# EUROPÄISCHE PATENTSCHRIFT

(11) **EP 2 928 884 B1**
(45) Veröffentlichungstag und Bekanntmachung des Hinweises auf die Patenterteilung: **24.05.2017**
(21) Anmeldenummer: 13798684.0
(22) Anmeldetag: 03.12.2013
(51) Int. Cl.: C07D 403/04, A61K 31/4184, A61P 5/00

(54) **NEUARTIGE BENZIMIDAZOLDERIVATE ALS EP4-ANTAGONISTEN**
NOVEL BENZIMIDAZOLE DERIVATIVES AS EP4 ANTAGONISTS
NOUVEAUX DÉRIVÉS DU BENZIMIDAZOLE COMME ANTAGONISTES EP4

(30) Priorität: 06.12.2012 EP 12195849
(43) Veröffentlichungstag der Anmeldung: 14.10.2015
(73) Patentinhaber: Bayer Pharma Aktiengesellschaft, 13353 Berlin (DE)
(72) Erfinder: PETERS, Olaf, 99891 Tabarz (DE); BRÄUER, Nico, 14612 Falkensee (DE); BLUME, Thorsten, 42113 Wuppertal (DE); TER LAAK, Antonius, 12159 Berlin (DE); ZORN, Ludwig, 13509 Berlin (DE); NAGEL, Jens, 55442 Daxweiler (DE); KAULFUSS, Stefan, 10437 Berlin (DE); LANGER, Gernot, 14612 Falkensee (DE); KUHNKE, Joachim, 14482 Potsdam (DE)
(74) Vertreter: BIP Patents
(86) Internationale Anmeldenummer: PCT/EP2013/075309
(87) Internationale Veröffentlichungsnummer: WO 2014/086739

(56) Entgegenhaltungen:
- WO-A1-2011/102149
- WO-A2-2004/011439
- SAPIJANSKAITE B ET AL: CHEMINE TECHNOLOGIJA,, Nr. 2, 1. Januar 2005 (2005-01-01), Seiten 57-62, XP009175546,

## Beschreibung

Die vorliegende Erfindung betrifft neuartige EP4-Antagonisten und ihre Verwendung zur Behandlung und/oder Prophylaxe von Erkrankungen sowie ihre Verwendung als Arzneimittel undpharmazeutische Präparate, die die neuen Benzimidazol-5-carbonsäurederivate enthalten.

Das Krankheitsbild der Endometriose ist umfassend untersucht und beschrieben, wenn auch die pathogenen Mechanismen noch nicht vollständig bekannt sind. Charakteristisch für Endometriose ist eine persistierende Ansiedlung von Endometriumsgewebe außerhalb der Uterushöhle, die zu typischen Herden führt. Es bilden sich sogenannte endometriotische Läsionen, die sich in unterschiedlicher Verteilung und Ausprägung im muskulären Bereich des Uterus (Endometriosis interna, Adenomyose), an verschiedenen Stellen des Bauchraums, z.B. den Ligamenten, am parietalen Peritoneum des Douglas Raumes (peritoneale Endometriose), der Darmwand, am Ovar (sogenannte Endometrioma) oder rektovaginal (rektovaginale, häufig auch tiefinfiltrierende, Endometriose) nachgewiesen werden können. Das neu angesiedelte Gewebe behält wesentliche Eigenschaften des Ursprungsgewebes (Uterus, Endometrium). Die Endometriose hat einen entzündlichen Charakter und äußert sich häufig durch verschiedene Formen von Unterleibschmerzen. Man geht davon aus, dass 10-20% der Frauen im reproduktiven Alter von Endometriose betroffen sind. Kernsymptome der Endometriose sind chronische Unterleibsschmerzen, Dysmenorrhoe, Dyspareunie, Dysurie, Blutungsstörungen und Unfruchtbarkeit. Die Symptome treten zumeist kombiniert auf.
Es wird vermutet, dass Endometriumsgewebe, das durch retrograde Menstruation über den Eileiter in den Peritonealraum gelangt, sich im Peritonealgewebe einnisten kann und die bei Endometriose beobachteten Läsionen hervorruft (Stratton & Berkley, Giudice 2010). Diese Läsionen rufen im Verlaufe der Erkrankung fortschreitende lokale Entzündungen hervor und sind durch Hochregulation von COX₂ Enzym und eine vermehrte Prostaglandinsynthese gekennzeichnet (Chishima et al 2002; Sales & Jabbour 2003).

Die Wirkungen der Prostaglandine werden durch ihre G-Protein-gekoppelten Rezeptoren, die in der Zellmembran lokalisiert sind, vermittelt. Von besonderem Interesse ist das Prostaglandin E2 (PGE2), das unterschiedlichste zelluläre Wirkungen erzielt, indem es an funktionell verschiedene Rezeptorsubtypen, nämlich EP1, EP2, EP3 und EP4, bindet.

Der Rezeptor EP4 (PTGR₄) ist einer der 4 humanen Rezeptoren, die durch endogen gebildetes Prostaglandin E2 (PGE2) aktiviert werden. EP4 gehört zur Familie der membranständigen G-Protein gekoppelten Rezeptoren (GPCR) und verfügt vorwiegend über eine Gs Kopplung, die nach Aktivierung zu einer Akkumulation des intrazellulären Signalmoleküls cAMP führt. Die Expression des Rezeptors wurde auf periphären Nervenendigungen von Nozizeptoren, auf Makrophagen und Neutrophilen nachgewiesen. Für diese Zelltypen wurde eine große Bedeutung im Zusammenhang mit Endometriose nachgewiesen. Es wird angenommen, dass die lokale Entzündung der Endometriose-Läsionen einen wesentlichen Beitrag zu der Genese der beobachteten Schmerzsymptome leistet (Stratton & Berkley 2010; Giudice 2010).

Derzeitige Therapieansätze für die Behandlung einer diagnostizierten Endometriose sind sehr eingeschränkt.

So kann die Endometriose durch ein operatives Entfernen der endometriotischen Läsionen in einem laparoskopischen Eingriff behandelt werden. Dabei werden Endometrioseherde operativ mit Hitze (Elektrokauterisation) oder durch Ausschneiden (Exstirpation) entfernt. Zusätzlich können hierbei eventuell vorhandene Verwachsungen gelöst, Endometriosezysten entfernt und bei Kinderwunsch die Durchgängigkeit der Eileiter mittels Chromopertubation geprüft werden. Die Rückfallquote nach einem solchen Eingriff ist allerdings sehr hoch (25-30%). Die Hysterektomie, also das komplette Entfernen des Uterus, besteht in solchen besonders hartnäckigen Fällen als finale Therapieoption.

Bei besonders schweren Erkrankungen bringt manchmal erst die Entfernung beider Eierstöcke und Eileiter (beidseitige Salpingo-Oophorektomie, Adnexektomie) eine definitive Therapie.

Die Regelschmerzen und verlängerte bzw. verstärkte Blutungen, die von einer Endometriose in der Gebärmuttermuskulatur (adenomyosis uteri) ausgehen, können auch mit einer Gebärmutterentfernung erfolgreich behandelt werden.

Diese Eingriffe führen jedoch zu Unfruchtbarkeit und einer verfrühten Menopause mit den damit verbundenen Problemen, weshalb der Nutzen gut gegen die Nachteile abgewogen werden muss.
Neben invasiven operativen Eingriffen kann auch eine medikamentöse Therapie in

Betracht gezogen werden. Diese kommt häufig bei großflächigem, eventuell nicht komplett operablem Befall, zur Anwendung, wird aber auch bei leichter bis mittlerer Erkrankung eingesetzt. Neben vorwiegend symptomatischer Schmerztherapie mit nichtsteroidalen Analgetika (NSAID), kommen hierfür im Prinzip vier Substanzgruppen in Betracht:
(a) kombinierte orale Kontrazeptiva (bestehend aus Estrogen und Gestagen) (OCs)
(b) Gestagene
(c) GnRH-Analoge (GnRH = Gonadotropin-Releasing-Hormone) und
(d) Danazol®

Die kombinierten oralen Kontrazeptiva (a) regulieren den Zyklusverlauf und reduzieren die Menstruationsstärke. Daraus folgt vermutlich deren Wirksamkeit in Endometriose-Patientinnen. Allerdings ist die Patientinnen-Zufriedenheit mit dieser Behandlungsform gering, was vermutlich auf Nebenwirkungen durch Beeinflussung des Hormonhaushaltes und eine unzulängliche Schmerzkontrolle zurück zu führen ist. Zudem weisen neue Studien darauf hin, dass eine langjährige Verwendung der hormonellen Wirkstoffe mit erhöhter Rate an tiefinfiltrierender Endometriose assoziiert zu sein scheint, einer besonders schmerzhaften Endometrioseform.

Der Einsatz von OCs bei der Behandlung der Endometriose wird auch in der Patentliteratur beschrieben. So offenbart die EP 1257280, dass mikronisiertes Drospirenon zur Behandlung der Endometriose geeignet ist. Es wird dort in Absatz [0045] beschrieben, dass Zusammensetzungen von Drospirenon mit einem niedrigen Gehalt an Estrogen oder auch ohne jegliches Estrogen u.a. zur Behandlung der Endometriose geeignet sind. Dieses erklärt sich aus der gestagenen Eigenschaft des Drospirenons. In der EP1257280 werden Mengen von 0,5 bis 10 mg Drospirenon als wirksam beschrieben. Über die Dauer der Behandlung der Endometriose mit Drospirenon ist in dieser Schrift nichts offenbart.

In der WO2008/107373 werden Mineralokortikoid-Rezeptor-Antagonisten zur Herstellung eines Arzneimittels für die Behandlung der Endometriose beschrieben. Neben der Verwendung von Verbindungen mit reiner antimineralokortikoiden Wirkung werden dort auch Verbindungen vorgeschlagen, die darüber hinaus auch eine Wirkung am Progesteron-Rezeptor, am Estrogen-Rezeptor, am Glucokortikoid-Rezeptor und/oder am Androgen-Rezeptor zeigen. Insbesondere die in der WO2008/107373 offenbarten Verbindungen Spironolacton und das zuvor genannte Drospirenon weisen auch eine gestagene Wirkung auf.

Die in der WO2008/107373 genannte Verbindung Eplerenon als reiner MR Antagonist zeigt eine relativ schwache *in vitro* Potenz. Bevorzugt sind MR Antagonisten, die in *in vitro* Transaktivierungsassays eine mindestens 10-fach geringere IC₅₀ verglichen mit Eplerenon aufweisen.

Gestagene (b) werden ebenfalls bei Endometriose eingesetzt. Ansatzpunkt ist hier zum einen die Unterdrückung der Funktion der Eierstöcke und zum anderen das Herbeiführen der terminalen Differenzierung des Endometriums, der Dezidualisierung, die letztlich zum Gewebsuntergang führt.

Die Gestagene täuschen dem Körper eine Schwangerschaft vor und schaffen so eine veränderte hormonelle Situation. Es findet kein Eisprung mehr statt und die Gebärmutterschleimhaut bildet sich zurück. In der Regel lassen die Endometriosebeschwerden dann innerhalb von 6 bis 8 Wochen nach.

Depot-MPA (Medroxyprogesteronacetat) und Visanne© (Dienogest) sind für die Endometriosebehandlung zugelassen. Eine deutliche analgetische Wirkung von Visanne© tritt erst nach mehreren Wochen Behandlung ein (Petraglia et al 2011). Es gibt keine Hinweise auf die allgemein erwünschte zügige Schmerzlinderung. Bei MPA kann es aufgrund der antiestrogenen Wirkung der Verbindung bereits nach einer Anwendungsdauer von 6 Monaten zu einer Verringerung der Knochenmasse kommen. Es soll deshalb keinesfalls über einen längeren Zeitraum als von 2 Jahren angewendet werden. Unter Behandlung mit Visanne kann es als Nebenwirkung der gestagenen Eigenschaften zu einer unerwünschten Beeinflussung des Blutungsprofils kommen. (Fachinfo-Nebenwirkungen).

Gestagene beeinflussen im Allgemeinen neben dem Hormonzyklus auch das Blutungsprofil, mit Blutungsstörungen als einer häufigen Nebenwirkung von Gestagenen. Das betrifft auch Substanzen, die an anderen Hormonrezeptoren aktiv sind und gleichzeitig eine gestagene Aktivität aufweisen, wie beispielsweise Spironolacton. Durch eine fehlerhafte Angiogenese (Gefäßneubildung, ein Vorgang der zyklisch im Endometrium stattfindet) während der Dezidualisierung des Endometriums werden die Gefäßwände brüchig und es kommt zu den sogenannten Durchbruchblutungen, die unabhängig von einer Menstruationsblutung stattfinden und charakteristisch für die chronische Behandlung mit Gestagenen sind.

Die Gonadotropin-Releasing-Hormon-Analoga (GnRH) (c) stellen derzeit den Goldstandard der zugelassenen Therapeutika gegen alle Stadien der Endometriose dar. GnRH-Analoga blockieren die Hirnanhangsdrüse vollständig. Der Menstruationszyklus findet nicht mehr statt. Diese Substanzen versetzen den Körper der Frau somit vorübergehend künstlich in die Wechseljahre und das Endometriosegewebe kann daher auch nicht mehr mitbluten. Das Gewebe wird hypotroph.

Aufgrund des Nebenwirkungsprofils ist dieser Therapieansatz jedoch ebenfalls nur für den kurzfristigen Einsatz (bis zu 6 Monaten) geeignet. So induzieren GnRH-Agonisten postmenopausale Symptome, wie Hitzewallungen (80-90%), Schlafstörungen (60-90%), Trockenheit der Scheide (30%), Kopfschmerzen (20-30%), Stimmungsveränderungen (10%) und Abnahme der Knochendichte mit einhergehendem erhöhtem Osteoporoserisiko.

Abgesehen von den genannten Nebenwirkungen stellt sich nach Beendigung der Behandlung innerhalb von zwei bis drei Monaten der normale Zyklus wieder ein. Bei über 60% der betroffenen Frauen, kehren dann auch die Beschwerden der Endometriose zurück, so dass ein erneuter Behandlungszyklus erwogen werden muss.

Aufgrund der genannten Nachteile haben GnRH-Analoga bislang keine breite Anwendung bei der Behandlung der Endometriose erlangt, wenngleich diese, die in den 70er Jahren etablierte Standardtherapie mit Danazol®, einem gestagenen Androgen, aufgrund des etwas besseren Nebenwirkungsprofils, verdrängt haben.

Danazol® (d) war das erste "klassische" Therapeutikum der Endometriose und bis in die 70er-Jahre der Goldstandard. Danazol® führt bei längerer Anwendung, ähnlich wie das männliche Geschlechtshormon Testosteron, zu einer Vermännlichung der Frau. Als weitere Nebenwirkungen treten die für Androgene bekannten Wirkungen, wie Akne, Hyperandrogenismus, Hirsutismus und (irreversible) Stimmlagenveränderung auf (Hinweis Fachinfo).

Danazol® wirkt, wie auch die GnRH-Agonisten, auf die Hirnanhangsdrüse, die für die Produktion von Hormonen verantwortlich ist, die die Eierstöcke stimulieren. Dadurch wird die Produktion von Estrogenen in den Eierstöcken eingestellt.

Es besteht daher ein dringender Bedarf an alternativen Präparaten, die eine nicht invasive Behandlung der Endometriose erlauben und die nicht die Nachteile des Standes der Technik aufweisen.

Bisher ist kein EP4-Antagonist als Arzneimittel zugelassen. Es sind aber EP4-Antagonisten verschiedener Strukturklassen beschrieben, die sich signifikant von den erfindungsgemäßen Verbindungen unterscheiden, indem sie nicht deren Carbazolylbenzimidazol-Struktur aufweisen. So werden in den WO2005/0121508, WO2005102389 und WO2005/105733 (Pfizer) beispielsweise *N*-Benzyl-aryl-amide, *N-*Benzyl-heteroarylamide und [(1 H-Benzimidazol-1-yl)phenylethyl]aryl- und [(1 H-Benzimidazol-1-yl)phenylethyl]heteroaryl-sulfonylcarbamate für die Anwendung bei Schmerz, Entzündungen, Osteoarthritis und Rheumatoider Arthritis beschrieben. Pfizer beschreibt in WO2002032422, WO2002032900 und WO2003086371 auch Strukturen, die generisch Benzimidazole einschließen, jedoch an Position 2 nicht durch einen kondensierten Tricyclus, wie Carbazol substituiert sein können. Thiophen-*N*-benzylamide in WO2008017164 und WO2009020588, Indol-*N*-benzylamide in WO2007121578 und N-{[(6,8-dihydro-7H-pyrrolo[3,4-g]quinolin-7-yl)-aryl]methyl}sulfonylamide in WO2008104055 werden für nahezu das gleiche Indikationsspektrum durch Merck-Frosst adressiert. In WO2010019796 (Chemietek) werden generisch sehr breit mehrfach substituierte Heterobicyclen beansprucht, wobei die typischen Einheiten der erfindungsgemäßen Verbindungen, Carbazol und Benzimidazol, nicht in den sehr wenigen Beispielen vorkommen und tricyclische Substituenten wie das Carbazol auch generisch nicht adressiert werden. In WO2004067524 (Pharmagene Laboratories) werden Furanderivate für die Behandlung von Kopfschmerz und Migräne beschrieben, bei denen der Furanring linear mit zwei weiteren Aryl- oder Heteroaryleinheiten, jeweils mit sechs Ringatomen, verbunden ist.

EP2172447 (Astellas Pharma) beansprucht generisch in sehr breiter Weise Verbindungen, die aus zwei direkt miteinander verbundenen Heterocyclen bestehen können, von denen aber einer durch eine Aminocarbonylgruppe substituiert sein muss und die Aminogruppe durch einen Substituenten weiter substituiert sein muss, der eine Carboxylgruppe oder ein Carboxylsurrogat trägt für die Indikationen renale Insuffizienz und diabetische Nephropathie.

Es werden auch Verbindungen beschrieben, die keine EP4-Antagonisten sind, aber strukturell in Beziehung zu den erfindungsgemäßen Verbindungen stehen. US2004/0122046 (Pfizer) adressiert Carbazole, die direkt über Position 3 mit einem Heterocyclus verbunden sind, der auch Benzimidazol sein kann, als NPY-Rezeptorantagonisten zur Behandlung von Adipositas. Im Unterschied zu den erfindungsgemäßen Verbindungen ist das NH der Benzimidazoleinheit aber zwingend unsubstituiert und die beiden Sechsringe der Carbazoleinheit können keinen weiteren Substituenten tragen. WO03/000254 bzw. EP1162196 (Japan Tobacco) beansprucht in breiter Weise generisch Heterobicyclen, die direkt mit einem Heterocyclus verbunden sein können als Therapeutikum für Hepatitis C. Falls es sich bei dem Heterocyclus um ein Benzimidazol handelt, muss dieser im Unterschied zu den erfindungsgemäßen Verbindungen an Position 1 zwingend direkt durch eine Bindung mit einer Cycloalkyl- oder Cycloalkenyleinheit verbunden sein. Paratek beschreibt in WO2010/124097 substituierte Benzimidazole als Antiinfektiva. Jedoch trägt das Benzimidazol anders als in den erfindungsgemäßen Verbindungen an Position 4 zwingend eine Alkylgruppe die terminal mit einer Carbonsäure-, Phosphonsäure oder Sulfonsäurefunktion bzw. deren Derivaten substituiert ist; des Weiteren ist an Position 2 als direkter cyclischer Substituent Heterocycloalkyl, nicht aber Heteroaryl zugelassen. Ausgehend vom beschriebenen Stand der Technik bestand daher keine Veranlassung, die Strukturen des Standes der Technik erfindungsgemäß abzuwandeln, um Strukturen zu erhalten, die antagonistisch am EP4-Rezeptor wirken.

Aufgabe der vorliegenden Erfindung ist es, *in vivo* verfügbare und somit wirksame und stabile Verbindungen, die als potente und selektive Antagonisten des Rezeptors EP4 wirken, bereitzustellen und die sich daher zur Behandlung und/oder Prophylaxe von Erkrankungen wie z.B. Endometriose eignen.

Diese Aufgabe wurde durch die Verbindungen der allgemeinen Formel I gelöst, 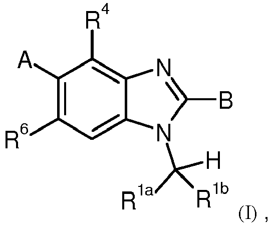 in der
- R^{1a}, R^{1b}: unabhängig voneinander für H, C₁-C₅-Alkyl, C₂-C₅-Alkenyl, C₂-C₅-Alkinyl, C₃-C₆-Cycloalkyl-(CH₂)ₘ, C₃-C₆-Heterocycloalkyl-(CH₂)ₙ, C₁-C₅-Alkoxy-C₁-C₃-Alkyl, C₃-C₆-Cycloalkoxy-C₁-C₃-Alkyl, Amino-C₁-C₃-Alkyl, C₁-C₅-Alkylamino-C₁-C₃-Alkyl, C₁-C₅-Dialkylamino-C₁-C₃-Alkyl oder Cyano stehen, wobei die gegebenenfalls vorhandene heterocyclische Einheit bevorzugt aus der Gruppe bestehend aus Oxetan, Tetrahydrofuran, Tetrahydropyran, 1,4-Dioxan, Morpholin, Azetidin, Pyrrolidin, Piperazin und Piperidin ausgewählt wird und wobei gegebenenfalls vorhandene Alkyl-, Cycloalkyl- oder Heterocycloalkylreste ein- oder mehrfach, gleich oder verschieden substituiert sein können mit Halogen, C₁-C₅-Alkyl, Hydroxy, Carboxy, Carboxy-C₁-C₅-Alkyl, C₁-C₅-Alkoxycarbonyl-C₁-C₅-Alkyl oder C₁-C₅-Alkylsulfonyl,
- R⁴: für H, F, Cl, C₁-C₂-Alkyl, C₃-C₅-Cycloalkyl, C₁-C₂-Alkoxy oder C₃-C₄-Cycloalkylmethyl steht, wobei die entsprechende Alkyl- oder Cycloalkyleinheit ein-oder mehrfach, gleich oder verschieden mit Halogen oder Hydroxy substituiert sein kann,
- A: für RO-CO(CH₂)ₚ steht, wobei R für H oder C₁-C₂-Alkyl steht,
- m: 0, 1, 2 oder 3 ist,
- n: 0, 1, 2 oder 3 ist,
- p: 0 ist, und
- B: aus den folgenden Strukturen ausgewählt wird,

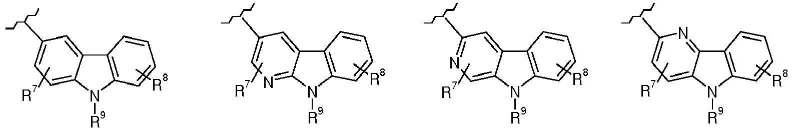

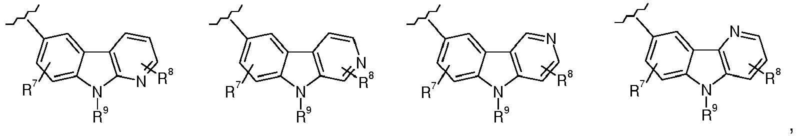

- R⁶: für H, F, Cl, CH₃, CF₃ CH₃O oder CF₃O steht,
- R⁷, R⁸: jeweils unabhängig voneinander für H, F, Cl, Cyano, SF₅, C₁-C₃-Alkyl, C₃-C₅-Cycloalkyl, C₁-C₂-Alkoxy oder C₃-C₄-Cycloalkylmethyl stehen, wobei die entsprechende Alkyl- oder Cycloalkyleinheit ein- oder mehrfach halogeniert sein kann, und
- R⁹: für C₁-C₅-Alkyl, C₂-C₅-Alkenyl, C₂-C₅-Alkinyl, C₃-C₆-Cycloalkyl-(CH₂)ₙ, C₃-C₆-Heterocycloalkyl-(CH₂)ₙ, oder C₁-C₇-Alkoxy-C₂-C₅-Alkyl steht, wobei die gegebenenfalls vorhandene heterocyclische Einheit aus der Gruppe bestehend aus Oxetan, Tetrahydrofuran, Tetrahydropyran, Morpholin, Pyrrolidin und Piperidin ausgewählt wird und wobei die gegebenenfalls vorhandenen Alkyl-, Cycloalkyl- oder Heterocycloalkyleinheiten ein- oder mehrfach, gleich oder verschieden mit Halogen, C₁-C₂-Alkyl, C₁-C₂-Alkoxy oder Carboxy substituiert sein kann,
und ihre Isomere, Diastereomere, Enantiomere, Solvate und Salze oder Cyclodextrin-Clathrate, für die Herstellung von Arzneimitteln.

Erfindungsgemäße Verbindungen sind die Verbindungen der Formel (I) und deren Stereoisomere, Tautomere, N-Oxide, Hydrate, Salze, Solvate und Solvate der Salze, sowie die von Formel (I) umfassten, nachfolgend als Ausführungsbeispiele genannten Verbindungen und deren Stereoisomere, Tautomere, N-Oxide, Hydrate, Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyl-diisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und lod, wie ²H (Deuterium), ³H (Tritium), ¹¹C, ¹³C, ¹⁴C, ¹⁵N ¹⁷O ¹⁸O ³²P ³³P ³³S, ³⁴S, ³⁵S, ³⁶S, ¹⁸F ³⁶Cl, ⁸²Br ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein, beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Die erfindungsgemäßen Verbindungen sind neu und haben eine antagonistische Wirkung am EP4-Rezeptor und dienen unter anderem der Behandlung der Endometriose.

Im Rahmen der vorliegenden Erfindung haben die Substituenten, soweit nicht anders spezifiziert, die folgende Bedeutung:
Alkyl steht für einen linearen oder verzweigten, gesättigten, monovalenten Kohlenwasserstoffrest mit mindestens 1 und höchstens 7 Kohlenstoffatomen (C₁-C₇-Alkyl). Eine gegebenenfalls vorgenommene Einschränkung des Bereiches für die Zahl der Kohlenstoffatome lässt sich direkt aus dem Präfix vor ,Alkyl' erkennen, beispielsweise bedeutet C₁-C₃-Alkyl, dass nur Alkylgruppen mit 1, 2 oder 3 Kohlenstoffatomen zugelassen sind. Beispielhaft seien genannt:
   Methyl, Ethyl, Propyl, Butyl, Pentyl, Hexyl, iso-Propyl, iso-Butyl, sec-Butyl, tert-Butyl, iso-Pentyl, 2-Methylbutyl, 1-Methylbutyl, 1-Ethylpropyl, 1,2-Dimethylpropyl, neo-Pentyl, 1,1-Dimethylpropyl, 4-Methylpentyl, 3-Methylpentyl, 2-Methylpentyl, 1-Methylpentyl, 2-Ethylbutyl, 1-Ethylbutyl, 3,3-Dimethylbutyl, 2,2-Dimethylbutyl, 1,1-Dimethylbutyl, 2,3-Dimethylbutyl, 1,3-Dimethylbutyl, 1,2-Dimethylbutyl. Die Alkylreste können gegebenenfalls ein- oder mehrfach durch Fluor substituiert sein.

Alkenyl und Alkinyl bezeichnen lineare oder verzweigte, ungesättigte, monovalente Kohlenwasserstoffreste, die sich von den zuvor genannten Alkylgruppen dadurch ableiten, dass der Rest mindestens zwei Kohlenstoffatome aufweist und dass eine Einfachbindung zwischen zwei mit der geeigneten Zahl von Wasserstoffatomen versehenen Kohlenstoffatomen durch eine Doppelbindung oder eine Dreifachbindung ersetzt ist. Beispielhaft seien genannt: Vinyl, Allyl, Buten-1-yl für Alkenyl und Ethinyl, Propargyl, Pentin-1-yl für Alkinyl. Die Zahl der Kohlenstoffatome ergibt sich aus dem Präfix, z.B. bedeutet C₂-C₅-Alkenyl eine Alkenylgruppe mit 2 bis 5 Kohlenstoffatomen.

Alkoxy steht für einen linearen oder verzweigten, gesättigten Alkyletherrest der Formel Alkyl-O mit mindestens 1 und höchstens 7 Kohlenstoffatomen (C₁-C₇-Alkoxy) wie beispielsweise Methoxy, Ethoxy, n-Propoxy, Isopropoxy, n-Butoxy, sec-Butoxy, Isobutoxy, tert-Butoxy, Pentyloxy, Hexyloxy und Heptyloxy. Die Alkoxyreste können gegebenenfalls ein- oder mehrfach durch Fluor substituiert sein.

Alkoxyalkyl steht für einen mit Alkoxy substituierten Alkylrest, wobei. Cₙ-Alkoxy-Cₘ-Alkyl bedeutet, dass dabei der Alkoxyteil n Kohlenstoffatome und der Alkylteil, über den der Rest gebunden ist, m Kohlenstoffatome aufweist.

Cycloalkoxy steht für einen Rest C₃-C₆-Cycloalkyl-O, wobei C₃-C₆-Cycloalkyl die nachstehend angegebene Bedeutung besitzt.

C₃-C₆-Cycloalkyl bezeichnet monocyclische Alkylreste mit 3 bis 6 Kohlenstoffatomen, wobei die Zahl der Ringatome modifiziert sein kann, wie dann in den Indizes ausgewiesen (z.B. bedeutet C₄-C₅-Cycloalkyl 4 oder 5 Ringatome). Beispielhaft seien genannt: Cyclopropyl, Cyclobutyl, Cyclopentyl und Cyclohexyl. Die Cycloalkylreste können gegebenenfalls ein- oder mehrfach durch Fluor substituiert sein.

Cycloalkoxyalkyl steht für einen mit Cycloalkoxy substituierten Alkylrest, wobei Cₙ-Cycloalkoxy-Cₘ-Alkyl bedeutet, dass dabei der Cycloalkoxyteil n Kohlenstoffatome und der Alkylteil, über den der Rest weiter bindet, m Kohlenstoffatome aufweist.

Aminoalkyl steht für eine mit einer Aminogruppe substituierten Alkylrest, wobei Amino-Cₙ-Alkyl bedeutet, dass die Alkylgruppe n Kohlenstoffatome aufweist.

Alkylamino steht für einen mit einer Alkylgruppe substituierten Aminorest, wobei beispielsweise C₁-C₅-Alkylamino einen mit einer 1 bis 5 Kohlenstoffatome besitzenden Alkylgruppe substituierten Aminorest bedeutet. Beispielhaft seien genannt: Methylamino, Ethylamino, Propylamino, Isopropylamino, *tert*-Butylamino, Pentylamino, Hexylamino, *N,N-*Dimethylamino, *N,N-*Diethylamino, *N*-Ethyl-*N*-methylamino, *N*-Methyl-N-propylamino, *N*-Isopropyl-*N*-propylamino, *N*-*tert*-Butyl-*N*-methylamino, *N*-Ethyl-*N-*pentylamino und *N*-Hexyl-*N*-methylamino

Dialkylamino steht entsprechend für einen mit zwei unabhängig gewählten Alkylgruppen substituierten Aminorest. Beispielhaft seien genannt: *N,N-*Dimethylamino, *N,N-*Diethylamino, *N*-Ethyl-*N*-methylamino, *N*-Methyl-N-propylamino, *N*-Isopropyl-*N-*propylamino, *N*-*tert*-Butyl-*N*-methylamino, *N*-Ethyl-*N*-pentylamino und *N*-Hexyl-*N-*methylamino.

Alkylaminoalkyl bzw. Dialkylaminoalkyl steht für eine Alkylgruppe, die mit einer Alkylaminogruppe bzw. einer Dialkylaminogruppe substituiert ist. So bedeutet beispielsweise C₁-C₅-Dialkylamino-C₁-C₃-Alkyl eine ein bis drei Kohlenstoffatome aufweisende Alkylgruppe, die mit einer Aminogruppe substituiert ist, die wiederum durch zwei unabhängig gewählte, ein bis fünf Kohlenstoffatome aufweisenden Alkylgruppen substituiert ist. Beispielhaft seien genannt: *N*-Methylamino-methyl, *N-*Methylamino-ethyl, *N*-Methylamino-propyl, *N,N-*Dimethylamino-methyl, *N,N-*Dimethyl-amino-ethyl, *N,N-*Dimethylamino-propyl, *N,N*-Diethylamino-methyl, *N,N-*Diethylaminoethyl, *N*-Ethyl-*N*-methylamino-methyl, *N*-Methyl-N-propylamino-methyl, *N*-Ethyl-*N-*methylamino-ethyl, *N*-Methyl-N-propylamino-ethyl.

Carboxyalkyl steht für einen mit einer Carboxylgruppe substituierten Alkylrest, wobei Carboxy-C₁-C₅-Alkyl bedeutet, dass der Alkylrest, an den die Carboxylgruppe gebunden ist, ein bis fünf Kohlenstoffe aufweisen kann. Beispielhaft seien genannt: Carboxymethyl, Carboxyethyl, Carboxypropyl, Carboxybutyl und Carboxypentyl.

Alkylcarbonyl bzw. Cycloalkylcarbonyl bzw. Aryl-(CH₂)ᵣ-carbonyl bzw. Pyridyl-(CH₂)ᵣ-carbonyl steht für eine über eine Carbonylgruppe CO bindende Alkylgruppe bzw. Cycloalkylgruppe bzw. Arylgruppe bzw. Pyridylgruppe.

Aryl-(CH₂)ᵣ-Sulfonyl bzw. Pyridyl-(CH₂)ᵣ-sulfonyl steht für einen Rest Aryl-(CH₂)ᵣ-SO₂ bzw. Pyridyl-(CH₂)ᵣ-SO₂.

Alkoxycarbonylalkyl steht für einen mit einer Alkoxycarbonylgruppe substituierten Alkylrest, wobei C₁-C₅-Alkoxycarbonyl-C₁-C₅-Alkyl bedeutet, dass der Alkoxyrest ein bis fünf Kohlenstoffatome aufweisen kann und über sein Sauerstoffatom an die Carbonylgruppe gebunden ist, die wiederum weiter an den Alkylrest bindet, der unabhängig vom Alkoxyrest ein bis fünf Kohlenstoffe aufweisen kann. Beispielhaft seien genannt: Methoxycarbonyl-methyl, Ethoxycarbonyl-methyl, Methoxycarbonyl-ethyl, Ethoxycarbonyl-ethyl, Methoxycarbonyl-propyl, Ethoxycarbonyl-propyl, Methoxycarbonyl-butyl, Ethoxycarbonyl-pentyl, Propoxycarbonyl-methyl, Butoxycarbonyl-methyl, tert.-Butoxycarbonyl-methyl, Neopentyloxycarbonyl-methyl.

Alkylsulfonyl steht für einen Rest Alkyl-SO₂, wobei der Alkylrest ein bis fünf Kohlenstoffatome besitzen kann.

Alkylsulfonimidoyl bzw. Cycloalkylsulfonimidoyl steht für eine Alkyl- bzw. Cycloalkylgruppe, die jeweils über einen Sulfoximinorest S(O)(NH) weiter bindet.

C₃-C₆-Heterocyclyl oder C₃-C₆-Heterocvcloalkyl oder 3- bis 6-gliedriges Heterocyclyl bezeichnet monocyclische Alkylreste, die 3 bis 6 Ringatome besitzen, wobei die Zahl der Ringatome modifiziert sein kann, wie dann in den Indizes ausgewiesen (z.B. bedeutet C₄-C₅-Heterocycloalkyl 4 oder 5 Ringatome) und die anstelle eines oder mehrerer Ring-Kohlenstoffatome ein oder mehrere Heteroatome, wie Sauerstoff, Schwefel und/oder Stickstoff oder eine Heterogruppe wie -S(O)-, -SO₂- enthalten. Die Bindungsvalenz kann an einem beliebigen Kohlenstoffatom oder an einem Stickstoffatom sein.

Beispielhaft genannt seien: Azetidinyl, Pyrrolidinyl, Piperidinyl, Piperazinyl, 1,4-Diazepanyl, Morpholinyl, Thiomorpholinyl, Oxetanyl, Tetrahydrofuranyl, Tetrahydropyranyl, 1,4-Dioxanyl, 2-Oxo-oxazolidinyl. Die Heterocyclylreste können gegebenenfalls ein- oder mehrfach durch Fluor, Hydroxy, Methoxy beziehungsweise mit Oxo substituiert sein.

Unter Halogen ist jeweils Fluor, Chlor oder Brom zu verstehen.

Ein C₆-C₁₀-gliedriger Arylrest bedeutet Phenyl oder Naphthyl. Dieser kann gegebenenfalls einfach durch Fluor, Chlor beziehungsweise eine Methylgruppe substituiert sein.

Unter einem C₅-C₁₀-Heteroarylrest sind mono- oder bicyclische Ringsysteme zu verstehen, die jeweils 5 - 10 Ringatome enthalten und die anstelle des Kohlenstoffs ein-oder mehrere, gleiche oder verschiedene Heteroatome, wie Sauerstoff, Schwefel oder Stickstoff enthalten können. Die Bindungsvalenz kann an einem beliebigen Kohlenstoffatom oder an einem Stickstoffatom sein.
Beispielsweise seien genannt: Thienyl, Thiazolyl, Furyl, Pyrrolyl, Oxazolyl, Imidazolyl, Pyrazolyl, Isoxazolyl, Isothiazolyl, Oxadiazolyl, Triazolyl, Thiadiazolyl, Tetrazolyl, Pyridyl, Pyridazinyl, Pyrimidinyl, Pyrazinyl, Triazinyl, Phthalidyl, Thiophthalidyl, Indolyl, Isoindolyl, Indazolyl, Benzothiazolyl, Benzofuryl, Benzothienyl, Benzimidazolyl, Benzoxazolyl, Azocinyl, Indolizinyl, Purinyl, Isochinolinyl, Chinolinyl, Chinolizinyl, Chinazolinyl, Chinoxalinyl, Cinnolinyl, Phthalazinyl, 1,7- or 1,8-Naphthyridinyl, Pteridinyl. Der C₅-C₁₀-gliedrige Heteroarylrest kann gegebenenfalls einfach durch Fluor, Chlor beziehungsweise eine Methylgruppe substituiert sein.

Ist eine basische Funktion enthalten, sind die physiologisch verträglichen Salze organischer und anorganischer Säuren geeignet, wie Salzsäure, Schwefelsäure, Phosphorsäure, Zitronensäure, Weinsäure u.a.

Bevorzugt sind Verbindungen der Formel I, wobei
- R^{1a}: für H oder C₁-C₅-Alkyl steht,
- R^{1b}: für H, C₁-C₅-Alkyl, C₂-C₅-Alkenyl, C₃-C₆-Cycloalkyl-(CH₂)ₘ, C₃-C₆-Heterocycloalkyl-(CH₂)ₙ, C₁-C₅-Alkoxy-C₁-C₃-Alkyl oder C₁-C₅-Dialkylamino-C₁-C₃-Alkyl steht, wobei die gegebenenfalls vorhandene heterocyclische Einheit bevorzugt aus der Gruppe bestehend aus Oxetan, Tetrahydrofuran, 1,4-Dioxan, Morpholin und Pyrrolidin ausgewählt wird und wobei gegebenenfalls vorhandene Alkyl- oder Cycloalkylreste ein- oder mehrfach, gleich oder verschieden substituiert sein können mit C₁-C₅-Alkyl, Hydroxy, oder C₁-C₅-Alkylsulfonyl,
- R⁴: für H, F, Cl, C₁-C₂-Alkyl oder C₁-C₂-Alkoxy steht,
- A: für RO-CO(CH₂)ₚ steht, wobei R für H oder C₁-C₂-Alkyl steht,
- m: 0 oder 1 ist,
- n: 0 oder 1 ist,
- p: 0 ist und
- B: aus den folgenden Strukturen ausgewählt wird,

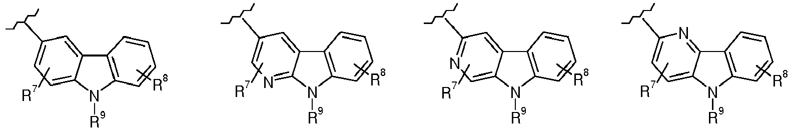

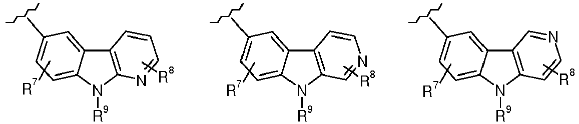

- R⁶: für H, F, CH₃ oder CH₃O steht,
- R⁷, R⁸: jeweils unabhängig voneinander für H, F, Cl, C₁-C₃-Alkyl oder C₁-C₂-Alkoxy stehen, und
- R⁹: für C₁-C₅-Alkyl, C₂-C₅-Alkenyl, C₂-C₅-Alkinyl, C₃-C₆-Cycloalkyl-(CH₂)ₙ oder C₁-C₇-Alkoxy-C₂-C₅-Alkyl steht,
und ihre Isomere, Diastereomere, Enantiomere, Solvate und Salze oder Cyclodextrin-Clathrate.

Bevorzugt sind Verbindungen der Formel I, wobei
- R^{1a}: für H oder Methyl steht,
- R^{1b}: für H, C₁-C₂-Alkyl, Vinyl, Cyclopropyl-(CH₂)ₘ, C₃-C₆-Heterocycloalkyl-(CH₂)ₙ, Methoxy-C₁-C₂-Alkyl oder (N,N-Dimethylamino)methyl steht, wobei die gegebenenfalls vorhandene heterocyclische Einheit bevorzugt aus der Gruppe bestehend aus Oxetan, Tetrahydrofuran, 1,4-Dioxan, Morpholin und Pyrrolidin ausgewählt wird und wobei gegebenenfalls vorhandene Alkyl- oder Cycloalkylreste ein- oder mehrfach, gleich oder verschieden substituiert sein können mit Methyl, Hydroxy, oder Methylsulfonyl,
- R⁴: für H, F, Cl, Methyl oder Methoxy steht,
- A: für RO-CO(CH₂)ₚ steht, wobei R für H oder C₁-C₂-Alkyl steht
- m: 0 oder 1 ist,
- n: 0 oder 1 ist,
- p: 0 ist und
- B: aus den folgenden Strukturen ausgewählt wird,

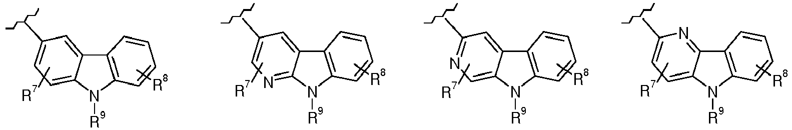

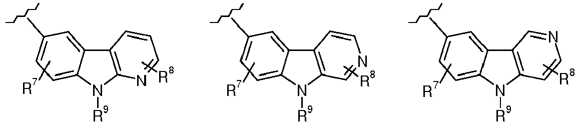

- R⁶: für H, F, CH₃ oder CH₃O steht,
- R⁷, R⁸: jeweils unabhängig voneinander für H, F, Cl, Methyl oder Methoxy stehen, und
- R⁹: für C₁-C₃-Alkyl, Allyl, Propargyl, C₃-C₄-Cycloalkyl-(CH₂)ₙ oder Methoxyethyl steht,
und ihre Isomere, Diastereomere, Enantiomere, Solvate und Salze oder Cyclodextrin-Clathrate.

Bevorzug sind Verbindungen der Formel (I), wobei
- R^{1a}: für H oder Methyl steht,
- R^{1b}: für H, C₁-C₂-Alkyl, Vinyl, Cyclopropyl-(CH₂)ₘ, C₃-C₆-Heterocycloalkyl-(CH₂)ₙ, Methoxy-C₁-C₂-Alkyl oder (N,N-Dimethylamino)methyl steht, wobei die gegebenenfalls vorhandene heterocyclische Einheit bevorzugt aus der Gruppe bestehend aus Oxetan, Tetrahydrofuran, 1,4-Dioxan, Morpholin und Pyrrolidin ausgewählt wird und wobei gegebenenfalls vorhandene Alkyl- oder Cycloalkylreste ein- oder mehrfach, gleich oder verschieden substituiert sein können mit Methyl, Hydroxy, oder Methylsulfonyl,
- R⁴: für H, F, Cl, Methyl oder Methoxy steht,
- A: für RO-CO(CH₂)ₚ steht, wobei R für H steht,
- m: 0 oder 1 ist,
- n: 0 oder 1 ist,
- p: 0 ist und
- B: aus den folgenden Strukturen ausgewählt wird,

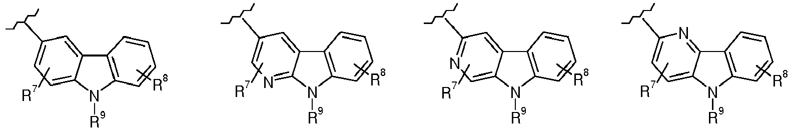

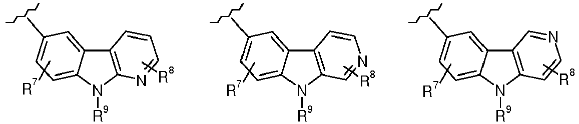

- R⁶: für H, F, CH₃ oder CH₃O steht,
- R⁷, R⁸: jeweils unabhängig voneinander für H, F, Cl, Methyl oder Methoxy stehen, und
- R⁹: für C₁-C₃-Alkyl, Allyl, Propargyl, C₃-C₄-Cycloalkyl-(CH₂)ₙ oder Methoxyethyl steht,
und ihre Isomere, Diastereomere, Enantiomere, Solvate und Salze oder Cyclodextrin-Clathrate.

Bevorzugt sind Verbindungen der Formel (I), wobei
- R^{1a}: für H steht,
- R^{1b}: für Methoxymethyl steht,
- R⁴: für H, F, Cl, Methyl oder Methoxy steht,
- A: für RO-CO(CH₂)ₚ steht, wobei R für H steht,
- n: 0 oder 1 ist,
- p: 0 ist, und
- B: aus den folgenden Strukturen ausgewählt wird,

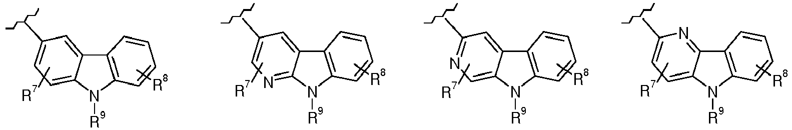

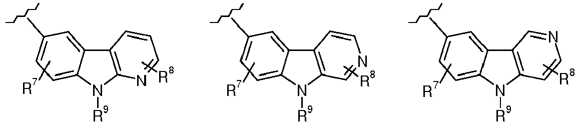

- R⁶: für H, F, CH₃ oder CH₃O steht,
- R⁷, R⁸: jeweils unabhängig voneinander für H, F, Cl, Methyl oder Methoxy stehen, und
- R⁹: für C₁-C₃-Alkyl, Allyl, Propargyl, C₃-C₄-Cycloalkyl-(CH₂)ₙ oder Methoxyethyl steht,
und ihre Isomere, Diastereomere, Enantiomere, Solvate und Salze oder Cyclodextrin-Clathrate.

Ebenfalls offenbart sind Verbindungen der Formel I, in denen
- R^{1a}: für H steht, und
- R^{1b}: für Methoxy-C₁-C₂-Alkyl steht.

Ebenfalls offenbart sind Verbindungen der Formel I, in denen
- R^{1a}: für H steht, und
- R^{1b}: für Methoxymethyl steht.

Ebenfalls offenbart sind Verbindungen der Formel I, in denen
- R⁴: für H oder C₁-C₂-Alkyl steht.

Ebenfalls offenbart sind Verbindungen der Formel I, in denen
- R⁴: für H steht.

Ebenfalls offenbart sind Verbindungen der Formel I, in denen
- R⁴: für C₁-C₂-Alkyl steht.

Ebenfalls offenbart sind Verbindungen der Formel I, in denen
- R⁴: für Methyl steht.

Ebenfalls offenbart sind Verbindungen der Formel I, in denen
- R⁶: für H steht.

Ebenfalls offenbart sind Verbindungen der Formel I, in denen
- A: für RO-CO(CH₂)ₚ, steht, wobei R für H oder C₁-C₂-Alkyl steht und p = 0 ist.

Ebenfalls offenbart sind Verbindungen der Formel I, in denen
- A: für RO-CO(CH₂)ₚ, steht, wobei R für H steht und p = 0 ist.

Ebenfalls offenbart sind Verbindungen der Formel I, in denen
- A: für RO-CO(CH₂)ₚ, steht, wobei R für C₁-C₂-Alkyl steht und p = 0 ist.

Ebenfalls offenbart sind Verbindungen der Formel I, in denen
- A: für RO-CO(CH₂)ₚ, steht, wobei R für Methyl steht und p = 0 ist.

Ebenfalls offenbart sind Verbindungen der Formel I, in denen
- B: für 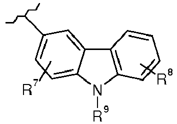 steht.

Ebenfalls offenbart sind Verbindungen der Formel I, in denen
- R⁷, R⁸: jeweils unabhängig voneinander für H, Cl oder Methyl stehen.

Ebenfalls offenbart sind Verbindungen der Formel I, in denen
- R⁷: für H steht.

Ebenfalls offenbart sind Verbindungen der Formel I, in denen
- R⁸: für H, Cl oder Methyl steht.

Ebenfalls offenbart sind Verbindungen der Formel I, in denen
- R⁸: für H steht.

Ebenfalls offenbart sind Verbindungen der Formel I, in denen
- R⁸: für Cl steht.

Ebenfalls offenbart sind Verbindungen der Formel I, in denen
- R⁸: für Methyl steht.

Ebenfalls offenbart sind Verbindungen der Formel I, in denen
- R⁹: für C₁-C₃-Alkyl steht.

Ebenfalls offenbart sind Verbindungen der Formel I, in denen
- R⁹: für Ethyl steht.

Die folgenden Verbindungen entsprechend der vorliegenden Erfindung sind ganz besonders bevorzugt:
1. Methyl-1-allyl-2-(9-ethyl-9H-carbazol-3-yl)-1 H-benzimidazol-5-carboxylat
2. 1-Allyl-2-(9-ethyl-9H-carbazol-3-yl)-1H-benzimidazol-5-carbonsäure
3. Methyl-2-(9-ethyl-9H-carbazol-3-yl)-1-(2-methoxyethyl)-1 H-benzimidazol-5-carboxylat
4. 2-(9-Ethyl-9H-carbazol-3-yl)-1-(2-methoxyethyl)-1 H-benzimidazol-5-carbonsäure
5. Methyl-1-(cyclopropylmethyl)-2-(9-ethyl-9H-carbazol-3-yl)-1 H-benzimidazol-5-carboxylat
6. 1-(Cyclopropylmethyl)-2-(9-ethyl-9H-carbazol-3-yl)-1 H-benzimidazol-5-carbonsäure
7. Methyl-4-chlor-1-(cyclopropylmethyl)-2-(9-ethyl-9H-carbazol-3-yl)-1 H-benzim idazol-5-carboxylat
8. 4-Chlor-1-(cyclopropylmethyl)-2-(9-ethyl-9H-carbazol-3-yl)-1 H-benzimidazol-5-carbonsäure
9. 1-(Cyclopropylmethyl)-2-(9-ethyl-9H-carbazol-3-yl)-4-methyl-1 H-benzimidazol-5-carbonsäure
10.2-(9-Ethyl-7-fluor-9H-carbazol-3-yl)-1-(2-methoxyethyl)-1H-benzimidazol-5-carbonsäure
11.2-(9-Ethyl-5-fluor-9H-carbazol-3-yl)-1-(2-methoxyethyl)-1H-benzimidazol-5-carbonsäure
12.2-(9-Ethyl-8-fluor-9H-carbazol-3-yl)-1-(2-methoxyethyl)-1H-benzimidazol-5-carbonsäure
13. 1-(Cyclopropylmethyl)-2-[9-(2-methoxyethyl)-9H-carbazol-3-yl]-1H-benzimidazol-5-carbonsäure
14.1-(Cyclopropylmethyl)-2-(9-ethyl-9H-pyrido[2,3-b]indol-3-yl)-1H-benzimidazol-5-carbonsäure
15. 2-(9-Ethyl-6-methoxy-9H-carbazol-3-yl)-1-(2-methoxyethyl)-1 H-benzimidazol-5-carbonsäure
16.2-(9-Allyl-9H-carbazol-3-yl)-1-(cyclopropylmethyl)-1H-benzimidazol-5-carbonsäure
17.1-(Cyclopropylmethyl)-2-(9-methyl-9H-carbazol-3-yl)-1H-benzimidazol-5-carbonsäure
18.1-(Cyclopropylmethyl)-2-[9-(cyclopropylmethyl)-9H-carbazol-3-yl]-1H-benzimidazol-5-carbonsäure
19.2-[9-(Cyclopropylmethyl)-9H-carbazol-3-yl]-1-(2-methoxyethyl)-1H-benzimidazol-5-carbonsäure
20.Ethyl-2-(9-ethyl-9H-carbazol-3-yl)-1-[2-(pyrrolidin-1-yl)ethyl]-1H-benzimidazol-5-carboxylat
21.2-(9-Ethyl-9H-carbazol-3-yl)-1-[2-(pyrrolidin-1-yl)ethyl]-1H-benzimidazol-5-carbonsäure
22.2-(5-Ethyl-5H-pyrido[3,2-b]indol-2-yl)-1-(2-methoxyethyl)-1H-benzimidazol-5-carbonsäure
23.1-(Cyclopropylmethyl)-2-(9-ethyl-6-methoxy-9H-carbazol-3-yl)-1H-benzimidazol-5-carbonsäure
24.2-(9-Ethyl-9H-carbazol-3-yl)-1-[2-(morpholin-4-yl)ethyl]-1H-benzimidazol-5-carbonsäure
25.Ethyl-1-[2-(dimethylamino)ethyl]-2-(9-ethyl-9H-carbazol-3-yl)-1H-benzimidazol-5-carboxylat
26.1-[2-(Dimethylamino)ethyl]-2-(9-ethyl-9H-carbazol-3-yl)-1H-benzimidazol-5-carbonsäure
27. 2-(9-Ethyl-9H-carbazol-3-yl)-1-(2-hydroxyethyl)-1 H-benzimidazol-5-carbonsäure
28. Ethyl-2-(9-ethyl-9H-carbazol-3-yl)-1-isopropyl-1 H-benzimidazol-5-carboxylat
29. 2-(9-Ethyl-9H-carbazol-3-yl)-1-isopropyl-1 H-benzimidazol-5-carbonsäure
30. 2-(9-Ethyl-9H-carbazol-3-yl)-1-methyl-1 H-benzimidazol-5-carbonsäure
31. Methyl-1-ethyl-2-(9-ethyl-9H-carbazol-3-yl)-1 H-benzimidazol-5-carboxylat
32. 2-(9-Allyl-9H-carbazol-3-yl)-1-(2-methoxyethyl)-1 H-benzimidazol-5-carbonsäure
33.Ethyl-1-(2-methoxyethyl)-2-[9-(2-methoxyethyl)-9H-carbazol-3-yl]-1H-benzim idazol-5-carboxylat
34.1-(2-Methoxyethyl)-2-[9-(2-methoxyethyl)-9H-carbazol-3-yl]-1H-benzimidazol-5-carbonsäure
35. Ethyl-2-(9-ethyl-9H-carbazol-3-yl)-1-(3-methoxypropyl)-1 H-benzimidazol-5-carboxylat
36. 2-(9-Ethyl-9H-carbazol-3-yl)-1-(3-methoxypropyl)-1 H-benzimidazol-5-carbonsäure
37.1-(Cyclopropylmethyl)-2-(9-ethyl-9H-carbazol-3-yl)-6-methoxy-1H-benzimidazol-5-carbonsäure
38. 2-(9-Ethyl-9H-carbazol-3-yl)-6-methoxy-1-(2-methoxyethyl)-1 H-benzimidazol-5-carbonsäure
39.1-(Cyclopropylmethyl)-2-(9-ethyl-9H-carbazol-3-yl)-4-methoxy-1H-benzimidazol-5-carbonsäure
40. 2-(9-Ethyl-9H-carbazol-3-yl)-1-(2-methoxyethyl)-4-methyl-1 H-benzimidazol-5-carbonsäure
41.1-(Cyclopropylmethyl)-2-(9-ethyl-9H-pyrido[2,3-b]indol-3-yl)-4-methyl-1H-benzimidazol-5-carbonsäure
42.1-(Cyclopropylmethyl)-2-(9-ethyl-9H-beta-carbolin-6-yl)-1H-benzimidazol-5-carbonsäure
43.1-(Cyclopropylmethyl)-2-(5-ethyl-5H-pyrido[4,3-b]indol-8-yl)-1H-benzimidazol-5-carbonsäure
44.1-(Cyclopropylmethyl)-2-(9-ethyl-9H-carbazol-3-yl)-6-methyl-1H-benzimidazol-5-carbonsäure
45. 2-(9-Ethyl-9H-carbazol-3-yl)-1-(2-methoxyethyl)-6-methyl-1 H-benzimidazol-5-carbonsäure
46. 2-(9-Ethyl-9H-carbazol-3-yl)-6-fluor-1-(2-methoxyethyl)-1 H-benzimidazol-5-carbonsäure
47.1-(Cyclopropylmethyl)-2-(9-ethyl-9H-carbazol-3-yl)-6-fluor-1H-benzimidazol-5-carbonsäure
48. 2-(9-Ethyl-9H-carbazol-3-yl)-4-fluor-1-(2-methoxyethyl)-1 H-benzimidazol-5-carbonsäure
49.1-(Cyclopropylmethyl)-2-(9-ethyl-9H-carbazol-3-yl)-4-fluor-1H-benzimidazol-5-carbonsäure
50.1-(Cyclopropylmethyl)-2-(9-ethyl-9H-pyrido[2,3-b]indol-6-yl)-1H-benzimidazol-5-carbonsäure
51.1-(2-Cyclopropylethyl)-2-(9-ethyl-9H-carbazol-3-yl)-1H-benzimidazol-5-carbonsäure
52.1-(2-Methoxyethyl)-2-(9-propyl-9H-carbazol-3-yl)-1H-benzimidazol-5-carbonsäure
53.1-(2-Methoxyethyl)-2-[9-(prop-2-in-1-yl)-9H-carbazol-3-yl]-1H-benzimidazol-5-carbonsäure
54.1-(Cyclopropylmethyl)-2-(9-propyl-9H-carbazol-3-yl)-1H-benzimidazol-5-carbonsäure
55.1-[(2,2-Dimethylcyclopropyl)methyl]-2-(9-ethyl-9H-carbazol-3-yl)-1H-benzimidazol-5-carbonsäure
56.Ethyl-2-(9-ethyl-9H-carbazol-3-yl)-1-(oxetan-3-ylmethyl)-1H-benzimidazol-5-carboxylat
57. 2-(9-Ethyl-9H-carbazol-3-yl)-1-(oxetan-3-ylmethyl)-1 H-benzimidazol-5-carbonsäure
58.2-[9-(Cyclobutylmethyl)-9H-carbazol-3-yl]-1-(2-methoxyethyl)-1H-benzimidazol-5-carbonsäure
59.2-[9-(Cyclobutylmethyl)-9H-carbazol-3-yl]-1-(cyclopropylmethyl)-1H-benzimidazol-5-carbonsäure
60.2-(9-Ethyl-9H-carbazol-3-yl)-1-[2-(trifluormethoxy)ethyl]-1H-benzimidazol-5-carbonsäure
61.1-(Cyclopropylmethyl)-2-(9-ethyl-1-methyl-9H-beta-carbolin-3-yl)-1H-benzimidazol-5-carbonsäure
62. 2-(9-Ethyl-9H-carbazol-3-yl)-1-(oxetan-2-ylmethyl)-1 H-benzimidazol-5-carbonsäure
63. 2-(9-Ethyl-9H-carbazol-3-yl)-1-(tetrahydrofuran-2-ylmethyl)-1 H-benzimidazol-5-carbonsäure
64.2-(9-Ethyl-9H-carbazol-3-yl)-1-[(2R)-2-hydroxy-3-methoxypropyl]-1H-benzimidazol-5-carbonsäure
65.2-(9-Ethyl-9H-carbazol-3-yl)-1-[(2S)-2-hydroxy-3-methoxypropyl]-1H-benzimidazol-5-carbonsäure
66.2-(9-Ethyl-9H-carbazol-3-yl)-1-[2-(methylsulfonyl)ethyl]-1H-benzimidazol-5-carbonsäure
67.1-(2-Cyclopropyl-2-hydroxyethyl)-2-(9-ethyl-9H-carbazol-3-yl)-1H-benzimidazol-5-carbonsäure
68.1-[(2S)-2,3-Dihydroxypropyl]-2-(9-ethyl-9H-carbazol-3-yl)-1H-benzimidazol-5-carbonsäure
69.1-(1,4-Dioxan-2-ylmethyl)-2-(9-ethyl-9H-carbazol-3-yl)-1H-benzimidazol-5-carbonsäure
70.1-(1,4-Dioxan-2-ylmethyl)-2-(9-ethyl-9H-carbazol-3-yl)-1H-benzimidazol-5-carbonsäure
71.2-(9-Ethyl-6-methyl-9H-carbazol-3-yl)-1-(2-methoxyethyl)-4-methyl-1H-benzimidazol-5-carbonsäure
72. 2-(6-Chlor-9-ethyl-9H-carbazol-3-yl)-1-(2-methoxyethyl)-4-methyl-1 H-benzimidazol-5-carbonsäure
73. 2-(8-Chlor-9-ethyl-9H-carbazol-3-yl)-1-(2-methoxyethyl)-4-methyl-1 H-benzimidazol-5-carbonsäure

Gegenstand der vorliegenden Erfindung sind die Verbindungen der Formel (I), zur Behandlung und/oder Prophylaxe von Krankheiten.

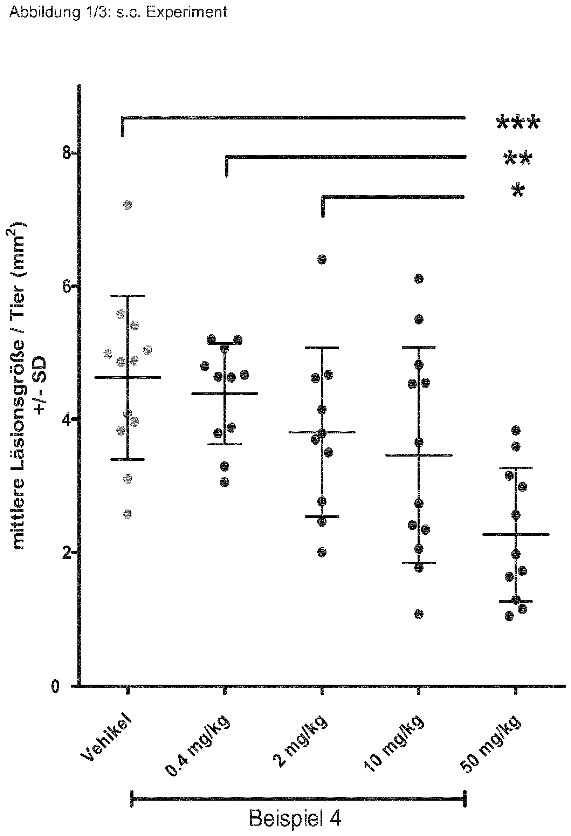

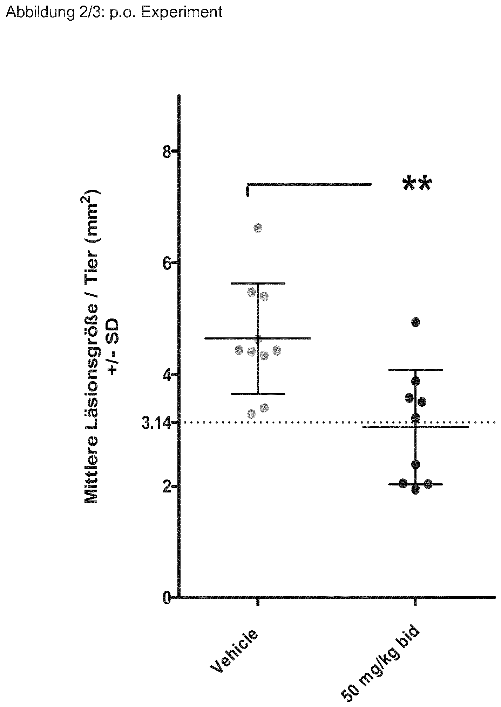

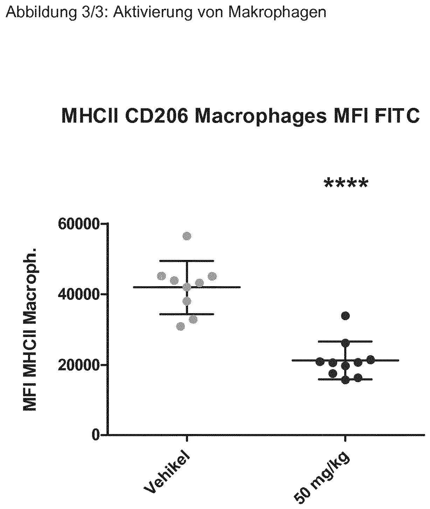

Im präventiven Endometriose Tiermodell wurde für EP4-Antagonisten erstmalig gezeigt, dass bei der Anwendung der erfindungsgemäßen Verbindung im genannten Dosisbereich nach subkutaner (Abbildung 1) sowie nach oraler Verabreichung (Abbildung 2) eine signifikante Verkleinerung der endometrialen Läsionen und eine immunmodulatorische Wirkung *in vivo* zu beobachten ist. Damit werden 2 wesentliche Aspekte der Endometriose durch die Anwendung der angemeldeten Verbindungen adressiert. Der immunmodulatorische Effekt wurde durch 2 Merkmale identifiziert:
1) die in der Kontrollgruppe beobachtete Infiltration von Neutrophilen in die Läsionen blieb bei den Dosisgruppen 10 mg/kg und 50 mg/kg aus (Abbildung 1, s.c. Experiment);
2) in einer FACS Analyse der Peritonealflüssigkeit, die im Rahmen der Sektion gesammelt wurde, konnte eine signifikante Reduktion der Makrophagenaktivierung in der hohen Dosisgruppe nachgewiesen werden (Abbildung 3). Der hormonelle Zyklus der Tiere blieb unbeeinflusst. Es wurden keine gastrointestinalen Schäden nach 28-tägiger Applikation im genannten Dosisbereich festgestellt. Insgesamt lieferte die histopathologische Untersuchung von Magen, Dünndarm, Niere, Leber und Herz der Versuchstiere nach 28 Tagen Behandlung keine Auffälligkeiten gegenüber den Organen der Kontrollgruppe.

Die erfindungsgemäßen Verbindungen der allgemeinen Formel I binden an den EP4Rezeptor und haben antagonistische Wirkung.
Gegenstand der vorliegenden Erfindung sind daher Verbindungen der Formel (I), die antagonistisch am EP4-Rezeptor wirken, zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

Ebenfalls Gegenstand der vorliegenden Erfindung ist die Verwendung einer Verbindung gemäß Formel (I) zur Herstellung eines Arzneimittels zur Behandlung und/oder Prophylaxe von Krankheiten. Dabei kann es sich um die Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen handeln.

Die antagonistische Wirkung kann durch einen Antagonismustest (siehe Beispiel 3.2.1 der biologischen Beispiele) bestimmt werden. So bindet beispielsweise die erfindungsgemäße Verbindung 4 mit einem IC₅₀-Wert von ca 6 nM an den EP4-Rezeptor.

Unter Antagonisten sind solche Moleküle zu verstehen, die an ihre entsprechenden Rezeptoren binden und welche die Initiierung des/der mit dem Rezeptor gekoppelten Signaltransduktionswege/s durch den oder die natürlich vorkommenden Liganden inhibieren. Üblicherweise kompetitieren die Antagonisten mit dem natürlich vorkommenden Liganden des Rezeptors um die Bindung an den Rezeptor. Aber auch andere Modifikationen des Rezeptors durch Moleküle, die verhindern, dass die mit dem Rezeptor gekoppelten Signaltransduktionswege durch den oder die natürlich vorkommenden Liganden aktiviert werden, sind möglich (z.B. nicht-kompetitive, sterische Modifikationen des Rezeptors).

Bevorzugterweise binden die Antagonisten reversibel an ihre korrespondierenden Rezeptoren.

Der EP4-Antagonist hat eine bevorzugte Affinität für den Rezeptor EP4 gegenüber jedem anderen EP-Subtyp. Der Antagonismus wird in Gegenwart des natürlichen Agonisten gemessen (PGE2).

Ebenfalls Gegenstand der vorliegenden Erfindung auf Grund der antagonistischen Wirkung am Rezeptor EP4 sind Arzneimittel zur Behandlung und/oder Prophylaxe von Erkrankungen, zu denen infektiöse Erkrankungen, Krebs, Herz-/Kreislauf-Erkrankungen, angiogenetische Erkrankungen, Störungen der Uteruskontraktion, akuter und chronischer Schmerz, inflammatorische Erkrankungen, neuroinflammatorische Erkrankungen, neurodegenerative Erkrankungen, Autoimmunerkrankungen, immunabhängige Erkrankungen/Therapien, nephrologische Erkrankungen, ophthamologische Erkrankungen zählen.

Unter infektiösen Erkrankungen sind durch unizelluläre Parasiten hervorgerufene Erkrankungen (z.B. Klebsiella, Streptococcus) zu verstehen. Bei den infektiösen Erkrankungen können die Arzneimittel immunmodulatorisch so wirken, dass die Erkrankungen prophylaktisch (Verringerung der Infektionsgefahr, wie beispielsweise bei Knochenmarkstransplantationen) oder therapeutisch behandelt werden können. Unter Krebs sind solide Tumoren und Leukämien; unter viralen Infektionen z. B. Cytomegalus-Infektionen, Hepatitis, Hepatitis B und C und HIV- Erkrankungen; unter Herz-Kreislauf-Erkrankungen ischämische Reperfusionserkrankung, Stenosen, Arteriosklerosen, Restenosen, Arthritis, Kawasaki-Syndrom und Aneurysmen; unter angiogenetischen Erkrankungen sind neben Endometriose die Fibrose und Fibroide im Uterus; unter Störungen der Uteruskontraktion z.B. Menstruationsbeschwerden; unter Schmerz beispielsweise inflammatorische Hyperalgesie, Arthritis, Arthrose, neuropathischer Schmerz, Gicht, Eingeweideschmerz, Rückenschmerzen, Kopfschmerzen, Migräne, Zahnschmerzen, Schmerzen durch Sonnenbrand und Schmerzen durch Brandverletzungen, unter inflammatorischen Erkrankungen beispielsweise entzündliche Darmerkrankungen; unter neuroinflammatorischen und neurodegenerativen Erkrankungen z.B. Multiple Sklerose, Alzheimer, Parkinson, ALS, Schlaganfall; unter immunabhängigen Erkrankungen/Therapien z.B. Transplantationen, bei denen eine Immunmodulation den Therapieerfolg erhöht; unter Autoimmunerkrankungen beispielsweise die ophthamologische Erkrankung Morbus Basedow, und unter nephrologischen Erkrankungen polyzystische Nierenerkrankungen, Glomerulonephritis, zu verstehen.

Die erfindungsgemäßen Verbindungen können dabei mit den üblichen pharmazeutischen Hilfsstoffen vermengt werden. Die EP4-Antagonisten werden in einer dem Fachmann an sich bekannten Weise formuliert.

Ebenfalls Gegenstand der vorliegenden Erfindung ist die Verwendung einer Verbindung gemäß Formel (I) zur Herstellung eines Arzneimittels.

Ebenfalls Gegenstand der vorliegenden Erfindung sind Arzneimittel, die die erfindungsgemäßen Verbindungen enthalten, mit geeigneten Formulierungs- und Trägerstoffen.

Die therapeutisch wirksame Dosis ist abhängig vom Körpergewicht, Applikationsweg, individuellem Verhalten, der Art der Zubereitung und Zeitpunkt bzw. Intervall, zu welchem die Applikation erfolgt. Ein typischer Dosisbereich für eine Frau mit 70 kg Körpergewicht liegt zwischen 1-500 mg/Tag, vorzugsweise zwischen 5 und 20 mg/ Tag.

Ein weiterer Gegenstand der vorliegenden Erfindung betrifft Arzneimittel, enthaltend mindestens eine erfindungsgemäße Verbindung und mindestens einen oder mehrere weitere Wirkstoffe, insbesondere zur Behandlung und/oder Prophylaxe der Endometriose. Als geeignete Kombinationswirkstoffe seien beispielhaft und vorzugsweise genannt: selektive Estrogen Rezeptor Modulatoren (SERMs), Estrogenrezeptor (ER) Antagonisten, Aromataseinhibitoren, 17β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen Kinasen (Mkk3/6, Mek1/2, Erk1/2), Inhibitoren der Proteinkinase B (PKBα/β/γ; Akt1/2/3), Inhibitoren der Phosphoinositid-3-Kinasen (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, Neurokinin1 Rezeptor Anatgonisten, Paracetamol, selektiven COX2-Inhibitoren und/oder nichtselektiven COX1/COX2 Inhibitoren.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die zuvor genannten Indikationen.

Die Verbindungen können, sowohl nach oraler als auch parenteraler Gabe, für die zuvor genannten Indikationen eingesetzt werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Die Dosierung der Verbindungen der allgemeinen Formel I sollte in diesen Zubereitungen 0.01 % - 20 % betragen, um eine ausreichende pharmakologische Wirkung zu erzielen.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Die oben beschrieben Formulierungen und Darreichungsformen sind ebenfalls Gegenstand der vorliegenden Erfindung.

Sofern neben der erfindungsgemäßen Verbindung gemäß Formel I weitere Wirkstoffe enthalten sind, können diese in einer gemeinsamen Applikationsform formuliert sein oder gegebenenfalls auch als Kombinationspräparat verabreicht werden.

Für die orale Applikation eignen sich nach dem Stand der Technik funktionierende, die erfindungsgemäß zu verwendenden Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäß zu verwendenden Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Tinkturen, Vaginalkapseln und -zäpfchen, Tampons, Intrauterinpessare, wässrige Suspensionen (Lotionen, Schüttelmixturen), lipophile Suspensionen, Kristallsuspensionen, wässrige und ölige Injektionslösungen, Depotpräparationen, Salben, Fettsalben, Gele, Cremes, transdermale therapeutische Systeme (z.B. Pflaster), Milch, Pasten, Schäume, Streupuder, Implantate, intrauterine Spiralen, Vaginalringe oder Stents.

Bevorzugt sind die orale oder parenterale Applikation, insbesondere die orale und die intravenöse Applikation.

Die erfindungsgemäß zu verwendenden Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Bei oraler Applikation beträgt die Menge pro Tag etwa 0,01 bis 100 mg/kg Körpergewicht. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg/kg Körpergewicht je Tag betragen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Ebenfalls Gegenstand der vorliegenden Erfindung ist die Verwendung der erfindungsgemäßen Substanzen als EP4-Rezeptor-Antagonisten zur Prophylaxe und direkten Behandlung von Erkrankungen, die ursächlich im Zusammenhang mit dem EP4-Rezeptor stehen oder von Erkrankungen, die durch eine Beeinflussung des EP4-Rezeptors therapiert werden können.

Prostaglandine spielen eine wichtige Rolle bei der Angiogenese (Sales, Jabbour, 2003, Reproduction 126, 559 - 567; Kuwano et al., 2004, FASEB J. 18, 300-310; Kamiyama et al., 2006, Oncogene 25, 7019-7028; Chang et al., 2005, Prostaglandins & other Lipid Mediators 76, 48-58).

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567). Prostaglandine und hier speziell der EP4- und der EP2-Rezeptor sind in Zusammenhang mit starken Menstruationsblutungen gebracht worden (Smith et al., 2007 (Human Reproduction, Vol.22, No.5 pp. 1450-1456).

Gegenstand der vorliegenden Erfindung ist die Verwendung der Substanzen der allgemeinen Formel I für die Prophylaxe und Behandlung von Menstruationsbeschwerden und starken Menstruationsblutungen sowie Schmerzen während der Menstruation. Fibroide (Myome) sind gutartige Tumore im Uterus mit einer hohen Verbreitungsrate. Über die Stimulierung der Aromatase durch einen PGE2/cAMP-vermittelten Signalweg, sowie durch mögliche andere Mechanismen, besteht ein Zusammenhang zum Prostaglandinstoffwechsel (Imir et al., 2007, J Clin Endocrinol Metab 92, 1979-1982).

Gegenstand der vorliegenden Erfindung ist die Verwendung der Substanzen der allgemeinen Formel I für die Prophylaxe und Behandlung von Fibroiden (Myome).

Zunehmende Forschungsergebnisse belegen auch die Bedeutung der EP-Rezeptoren, bei einer Vielzahl von Krebsarten (z. B. Brustkrebs, Kolonkarzinom, Lungenkrebs, Prostatakrebs, Leukämie, Hautkrebs), was auf zukünftige Möglichkeiten des Einsatzes von Modulatoren (Antagonisten oder Agonisten) des EP4-Rezeptors für die Therapie und Vorbeugung (prophylaktisch und/oder adjuvant) von Krebs schließen lässt (Fulton et al., 2006, Cancer Res; 66(20): 9794-7; Hull et al., 2004, Mol Cancer Ther;3(8):1031-9; Wang et al., 2004, Seminars in Oncology, Vol 31, No 1, Suppl 3: pp 64-73).

Gegenstand der vorliegenden Erfindung ist die Verwendung der Substanzen der allgemeinen Formel I für die Behandlung und Vorbeugung von Krebserkrankungen.

Die Aktivierung von endothelialen Zellen spielt im pathogenen Prozess der Arteriosklerose eine wichtige Rolle. Neuere Forschungen zeigen eine Beteiligung des EP4-Rezeptors (Minami et al., 2008, J Biol Chem., Apr 11;283(15):9692-703. Epub 2008 Feb 12).

Gegenstand der vorliegenden Erfindung ist die Verwendung der Substanzen der allgemeinen Formel I für die Behandlung und Vorbeugung von Arteriosklerose.

Neuere wissenschaftliche Publikationen zeigen, dass bei neurodegenerativen, neuroentzündlichen und ischämischen Erkrankungen (Alzheimer, Parkinson, ALS, Schlaganfall) Prostaglandine und der EP4-Rezeptor wichtige Komponenten des Krankheitsgeschehens darstellen (Hoshino et al., 2007, J Biol Chem.; 282(45): 32676-88; Cimino et al., 2008, Current Medicinal Chemistry, 1863-1869).

Multiple Sklerose ist eine chronische Entzündung des Nervensystems. Prostaglandine, speziell PGE2 und über den EP4-Rezeptor vermittelte Effekte werden ursächlich mit den pathologischen Vorgängen bei Multipler Sklerose in Zusammenhang gebracht (Palumbo et al., 2011, Prostaglandins, Leukotrienes and Essential Fatty Acids 85: 29-35; Kihara et al., 2009, Proc Natl Acad Sci U. S. A, 106, Nr. 51: 21807-21812).

Die erfindungsgemäßen Verbindungen können dabei mit den üblichen pharmazeutischen Hilfsstoffen vermengt werden. Die EP4-Antagonisten werden in einer dem Fachmann an sich bekannten Weise formuliert.

Ebenfalls Gegenstand der vorliegenden Erfindung ist die Verwendung einer Verbindung gemäß Formel (I) zur Herstellung eines Arzneimittels.

Ebenfalls Gegenstand der vorliegenden Erfindung sind Arzneimittel, die die erfindungsgemäßen Verbindungen enthalten, mit geeigneten Formulierungs- und Trägerstoffen.

Die therapeutisch wirksame Dosis ist abhängig vom Körpergewicht, Applikationsweg, individuellem Verhalten, der Art der Zubereitung und Zeitpunkt bzw. Intervall, zu welchem die Applikation erfolgt. Ein typischer Dosisbereich für eine Frau mit 70 kg Körpergewicht liegt zwischen 1-500 mg/Tag, vorzugsweise zwischen 5 und 20 mg/ Tag.

Ein weiterer Gegenstand der vorliegenden Erfindung betrifft Arzneimittel, enthaltend mindestens eine erfindungsgemäße Verbindung und mindestens einen oder mehrere weitere Wirkstoffe, insbesondere zur Behandlung und/oder Prophylaxe der Endometriose. Als geeignete Kombinationswirkstoffe seien beispielhaft und vorzugsweise genannt: selektive Estrogen Rezeptor Modulatoren (SERMs), Estrogenrezeptor (ER) Antagonisten, Aromataseinhibitoren, 17β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen Kinasen (Mkk3/6, Mek1/2, Erk1/2), Inhibitoren der Proteinkinase B (PKBα/β/γ; Akt1/2/3), Inhibitoren der Phosphoinositid-3-Kinasen (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, Neurokinin1 Rezeptor Anatgonisten, Paracetamol, selektiven COX2-Inhibitoren und/oder nichtselektiven COX1/COX2 Inhibitoren.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die zuvor genannten Indikationen.

Die Verbindungen können, sowohl nach oraler als auch parenteraler Gabe, für die zuvor genannten Indikationen eingesetzt werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Die Dosierung der Verbindungen der allgemeinen Formel I sollte in diesen Zubereitungen 0.01 % - 20 % betragen, um eine ausreichende pharmakologische Wirkung zu erzielen.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Die oben beschrieben Formulierungen und Darreichungsformen sind ebenfalls Gegenstand der vorliegenden Erfindung.

Sofern neben der erfindungsgemäßen Verbindung gemäß Formel I weitere Wirkstoffe enthalten sind, können diese in einer gemeinsamen Applikationsform formuliert sein oder gegebenenfalls auch als Kombinationspräparat verabreicht werden.

Für die orale Applikation eignen sich nach dem Stand der Technik funktionierende, die erfindungsgemäß zu verwendenden Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäß zu verwendenden Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Tinkturen, Vaginalkapseln und -zäpfchen, Tampons, Intrauterinpessare, wässrige Suspensionen (Lotionen, Schüttelmixturen), lipophile Suspensionen, Kristallsuspensionen, wässrige und ölige Injektionslösungen, Depotpräparationen, Salben, Fettsalben, Gele, Cremes, transdermale therapeutische Systeme (z.B. Pflaster), Milch, Pasten, Schäume, Streupuder, Implantate, intrauterine Spiralen, Vaginalringe oder Stents.

Bevorzugt sind die orale oder parenterale Applikation, insbesondere die orale und die intravenöse Applikation.

Die erfindungsgemäß zu verwendenden Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.
Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Bei oraler Applikation beträgt die Menge pro Tag etwa 0,01 bis 100 mg/kg Körpergewicht. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg/kg Körpergewicht je Tag betragen.
Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.
Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Ebenfalls Gegenstand der vorliegenden Erfindung ist die Verwendung der erfindungsgemäßen Substanzen zur Herstellung eines Medikaments als EP4-Rezeptor-Antagonisten zur Prophylaxe und direkten Behandlung von Erkrankungen, die ursächlich im Zusammenhang mit dem EP4-Rezeptor stehen oder von Erkrankungen, die durch eine Beeinflussung des EP4-Rezeptors therapiert werden können.

Prostaglandine spielen eine wichtige Rolle bei der Angiogenese (Sales, Jabbour, 2003, Reproduction 126, 559 - 567; Kuwano et al., 2004, FASEB J. 18, 300-310; Kamiyama et al., 2006, Oncogene 25, 7019-7028; Chang et al., 2005, Prostaglandins & other Lipid Mediators 76, 48-58).

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567). Prostaglandine und hier speziell der EP4- und der EP2-Rezeptor sind in Zusammenhang mit starken Menstruationsblutungen gebracht worden (Smith et al., 2007 (Human Reproduction, Vol.22, No.5 pp. 1450-1456).

Gegenstand der vorliegenden Erfindung ist die Verwendung der Substanzen der allgemeinen Formel I für die Prophylaxe und Behandlung von Menstruationsbeschwerden und starken Menstruationsblutungen sowie Schmerzen während der Menstruation.

Fibroide (Myome) sind gutartige Tumore im Uterus mit einer hohen Verbreitungsrate. Über die Stimulierung der Aromatase durch einen PGE2/cAMP-vermittelten Signalweg, sowie durch mögliche andere Mechanismen, besteht ein Zusammenhang zum Prostaglandinstoffwechsel (Imir et al., 2007, J Clin Endocrinol Metab 92, 1979-1982).

Gegenstand der vorliegenden Erfindung ist die Verwendung der Substanzen der allgemeinen Formel I für die Prophylaxe und Behandlung von Fibroiden (Myome).

Zunehmende Forschungsergebnisse belegen auch die Bedeutung der EP-Rezeptoren, bei einer Vielzahl von Krebsarten (z. B. Brustkrebs, Kolonkarzinom, Lungenkrebs, Prostatakrebs, Leukämie, Hautkrebs), was auf zukünftige Möglichkeiten des Einsatzes von Modulatoren (Antagonisten oder Agonisten) des EP4-Rezeptors für die Therapie und Vorbeugung (prophylaktisch und/oder adjuvant) von Krebs schließen lässt (Fulton et al., 2006, Cancer Res; 66(20): 9794-7; Hull et al., 2004, Mol Cancer Ther;3(8):1031-9; Wang et al., 2004, Seminars in Oncology, Vol 31, No 1, Suppl 3: pp 64-73).

Gegenstand der vorliegenden Erfindung ist die Verwendung der Substanzen der allgemeinen Formel I für die Behandlung und Vorbeugung von Krebserkrankungen.

Die Aktivierung von endothelialen Zellen spielt im pathogenen Prozess der Arteriosklerose eine wichtige Rolle. Neuere Forschungen zeigen eine Beteiligung des EP4-Rezeptors (Minami et al., 2008, J Biol Chem., Apr 11;283(15):9692-703. Epub 2008 Feb 12).

Gegenstand der vorliegenden Erfindung ist die Verwendung der Substanzen der allgemeinen Formel I für die Behandlung und Vorbeugung von Arteriosklerose.

Neuere wissenschaftliche Publikationen zeigen, dass bei neurodegenerativen, neuroentzündlichen und ischämischen Erkrankungen (Alzheimer, Parkinson, ALS, Schlaganfall) Prostaglandine und der EP4-Rezeptor wichtige Komponenten des Krankheitsgeschehens darstellen (Hoshino et al., 2007, J Biol Chem.; 282(45): 32676-88; Cimino et al., 2008, Current Medicinal Chemistry, 1863-1869).

Multiple Sklerose ist eine chronische Entzündung des Nervensystems. Prostaglandine, speziell PGE2 und über den EP4-Rezeptor vermittelte Effekte werden ursächlich mit den pathologischen Vorgängen bei Multipler Sklerose in Zusammenhang gebracht (Palumbo et al., 2011, Prostaglandins, Leukotrienes and Essential Fatty Acids 85: 29-35; Kihara et al., 2009, Proc Natl Acad Sci U. S. A, 106, Nr. 51: 21807-21812).

Gegenstand der vorliegenden Erfindung ist die Verwendung der Substanzen der allgemeinen Formel I für die Behandlung und Vorbeugung von neurodegenerativen, neuroentzündlichen und ischämischen Erkrankungen wie beispielsweise Alzheimer, Parkinson, ALS, Schlaganfall sowie für die Behandlung der Multiplen Sklerose.

Polyzystische Nierenerkrankungen stehen ebenfalls im Zusammenhang mit dem EP4-Rezeptor (Liu et al., 2012, Am J Physiol Renal Physiol. 2012 Aug 29. [Epub ahead of print.)

Gegenstand der vorliegenden Erfindung ist die Verwendung der Substanzen der allgemeinen Formel I für die Behandlung und Vorbeugung von polyzystischen Nierenerkrankungen.

Es gibt Hinweise, dass eine inflammatorische, gesteigerte Schmerzempfindlichkeit behandelt werden kann, indem gezielt EP4-Rezeptoren moduliert werden. Des Weiteren steht der EP4-Rezeptor in Zusammenhang mit weiteren Schmerzarten (Zeilhofer, 2007, Biochemical Pharmacology 73; 165- 174). Murase et al. (Eur J Pharmacol. 2008 Feb 2;580(1-2):116-21) berichten von einem Zusammenhang zwischen EP4-Rezeptor-Blockade und einer symptomatischen Erleichterung der Beschwerden, die bei Osteoarthritis und / oder rheumatoider Arthritis auftreten.

Gegenstand der vorliegenden Erfindung ist die Verwendung der erfindungsgemäßen Substanzen für die Behandlung und Vorbeugung von Schmerz verschiedenen Ursprungs wie beispielsweise der inflammatorischen Hyperalgesie oder der Arthritis.

Neuere wissenschaftliche Veröffentlichungen weisen auf eine Verwendung von EP4-Inhibitoren zur Vorbeugung und/oder Behandlung von Infektionen der Atmungswege hin. Serezani et al. (Am Respir Cell Mol Biol Vol 37. pp 562-570, 2007) beschreibt, dass über die Aktivierung des EP4-Rezeptors durch PGE2 Makrophagen des Atmungstraktes in ihrer Fähigkeit beeinträchtigt werden, Bakterien zu zerstören. Bakterieninfektionen führen zu einer vermehrten Produktion von Prostaglandinen, unter anderem PGE2, das über diesen Mechanismus die körpereigene Abwehr gegen Bakterien schwächt. Wie in dieser Publikation gezeigt, kann durch eine Inaktivierung des EP4-Rezeptors (und des EP2-Rezeptors) diese Fähigkeit der Bakterienbekämpfung wieder hergestellt werden.

Gegenstand der vorliegenden Erfindung ist die Verwendung der erfindungsgemäßen Substanzen für die Vorbeugung und Behandlung von Infektionskrankheiten der Lunge.

Darmentzündliche Erkrankungen (z.B. Morbus Crohn) stehen ebenfalls im Zusammenhang mit dem Prostaglandin EP4-Rezeptor (Sheibanie et al., 2007, The Journal of Immunology, 178: 8138-8147).

Gegenstand der vorliegenden Erfindung ist die Verwendung der erfindungsgemäßen Substanzen für die Vorbeugung und Behandlung von darmentzündlichen Erkrankungen.

Bei Knochenmarkstransplantationen kommt es oft zu Komplikationen durch Infektionen, wobei eine Überproduktion von PGE2 im Zusammenhang mit einer verminderten Immunabwehr steht (Ballinger et al., 2006, The Journal of Immunology, 177: 5499-5508).

Gegenstand der vorliegenden Erfindung ist die Verwendung der erfindungsgemäßen Substanzen für die Prophylaxe und Behandlung bei Knochenmarkstransplantationen.

Morbus Basedow (im Englischen "Graves Disease" genannt) ist eine Autoimmunerkrankung der Schilddrüse, bei der das klinische Bild auch pathologische Veränderungen im Auge umfassen kann (endokrine Orbitopathie; Hervortreten der Augäpfel (Exophtalmus)). Hierbei aktivieren einwandernde Lymphozyten vorhandene Fibroblasten, was unter anderem zu einer Anhäufung von Mucopolysachariden führt. Mögliche Folgen sind Beeinträchtigungen des Sehvermögens bis hin zur Blindheit. Untersuchungen zeigen, dass Interleukin-6 eine maßgebende Bedeutung für die pathologischen Mechansimen besitzt und über PGE2 wirkt (Wang et al., 1995, J Clin Endocrinol Metab 80: 3553-3560).

Gegenstand der vorliegenden Erfindung ist die Verwendung der erfindungsgemäßen Substanzen für die Prophylaxe und Behandlung bei Orbitopathie in Zusammenhang mit Morbus Basedow (Graves Disease) oder anderen pathologischen Erkrankungen des Auges.

Der natürliche Ligand (Agonist) des EP4-Rezeptors ist das PGE2, dessen Synthese über Cyclooxygenasen (COX)-Enzyme (COX-1, COX-2) vermittelt wird. Diese Enzyme sind in den erwähnten Krankheitsbildern, Indikationen und deren Entstehung meist über eine verstärkte Expression und Aktivität beteiligt. Deshalb ist bei allen erwähnten Anwendungsmöglichkeiten eine Kombination eines COX-Inhibitors (COX-2 und/oder COX-1) möglich, mit dem Ziel,
a) eine höhere und effektivere pharmakologische Wirksamkeit als mit einer Substanzklasse zu erreichen und
b) eine niedrigere Dosierung einer der beiden oder beider Substanzklassen zu ermöglichen, was zu einer Reduzierung möglicher Nebenwirkungen und einer besseren Verträglichkeit führt.

Gegenstand der vorliegenden Erfindung sind daher auch Arzneimittel enthaltend eine Verbindung der allgemeinen Formel (I) in Kombination mit einem COX-Inhibitor zur Behandlung von Erkrankungen (Kombinationspräparate). Als COX-Inhibitoren seien beispielsweise die nicht selektiven COX-Inhbitoren wie Aspirin, Naproxen, Indomethacin, Ibuprofen genannt oder die selektiven COX-Inhibitoren Meloxicam, Ketoprofen, Piroxicam, Tenoxicam, Nimesulide, Mefanemic Acid, Ketoralac, Celecoxib (4-[5-(4-methylphenyl)-3-(trifluormethyl)-1H-pyrazol-1-yl]benzenesulfonamide) Parecoxib (N-[4-(5-methyl-3-phenyl-4-isoxazolyl)phenyl]sulfonylpropionamide), Rofecoxib (4-(4-mesylphenyl)-3-phenylfuran-2(5H)-one), Valdecoxib (4-[5-methyl-3-phenyl-4-isoxazoyl]benzenesulfonamide), NS-398 (N-methyl-2-cyclohexanoxy-4-nitrobenzenesulfonamide), Lumiracoxib [2-(2'-chlor-6'-fluorphenyl)-amino-5-methylbenzeneacetic acid], Ceracoxib und Etoricoxib.

Diese Kombinationspräparate können für die Behandlung folgender Erkrankungen eingesetzt werden: infektiöse Erkrankungen, Krebs, Herz-/Kreislauf-Erkrankungen, angiogenetische Erkrankungen, Störungen der Uteruskontraktion, Schmerz, inflammatorische Erkrankungen, neuroinflammatorische Erkrankungen, neurodegenerative Erkrankungen, Autoimmunerkrankungen, immunabhängige Erkrankungen/Therapien, nephrologische Erkrankungen, ophthamologische Erkrankungen.

Im Folgenden sind die alternativen Reaktionsschemata dargestellt, nachdem die erfindungsgemäßen Verbindungen hergestellt werden können, jeweils in Abhängigkeit von der Verfügbarkeit der Ausgangsmaterialien. Für alle Schemata belegen Ausführungsbeispiele die Reaktionsführung detailliert.

Die in den Schemata 1-7 aufgeführten Reste R, R^{1a}, R^{1b}, R⁴, R⁵, R^{5'}, R⁶, R⁷, R⁸, R⁹, A und B besitzen die im Anspruch aufgeführten Bedeutungen und dienen zur Illustration der Synthese, ohne den Umfang der beanspruchten Verbindungen auf diese Beispiele zu beschränken.

So können beispielsweise durch Umsetzung von substituierten o-Phenylendiaminen der allgemeinen Formel II oder XI mit Aldehyden der Formel III, XXIV oder XXI Benzimidazole der allgemeinen Struktur IV oder XII hergestellt werden. Dieses kann zum Beispiel dadurch erreicht werden, dass die Komponenten II und III in Gegenwart von Säuren und einem Oxidationsmittel erhitzt werden. Die so generierten Verbindungen IV und XII können dann nach in der Literatur bekannten Verfahren an den Stickstoffatomen des Imidazols substituiert werden, vorzugsweise mit Alkylhalogeniden, Oxiranen oder anderen Nucleophilen (Schema 1, Schema 3). Bei dieser Synthesevariante entstehen in der Regel die Isomere Va und Vb oder auch XIIa und Xllb, welche nach gängigen Methoden voneinander getrennt werden können. Gängige Methoden sind Trennverfahren wie beispielsweise Kristallisation, Chromatographie an Kieselgel oder auch Trennungen mittels Hochdruck- oder Hochleistungsflüssigchromatographie.

Carbonsäuren der Formel VI können mit einem Amin nach dem Fachmann bekannten Verfahren zu den erfindungsgemäßen Verbindungen der allgemeinen Formel I umgesetzt werden (Schema 1-4, allgemeine Formel VII).

Die Umsetzung zu Amiden der Formel VII findet beispielsweise statt, indem man eine Carbonsäure der Formel VI in Anwesenheit eines tertiären Amins, beispielsweise Triethylamin, mit Isobutylchloroformiat in ein gemischtes Anhydrid überführt wird, welches mit einem Alkalisalz des entsprechenden Amins in einem inerten Lösungsmittel beziehungsweise Lösungsmittelgemisch, zum Beispiel Tetrahydrofuran, N,N-Dimethylformamid, Dimethoxyethan bei Temperaturen zwischen -30°C und +60°C zu den Zielverbindungen der Formel I reagiert.

Es ist ebenfalls möglich, eine Carbonsäure VI mit Reagenzien wie beispielsweise Dicyclohexylcarbodiimid (DCC), 1-(3-Dimethylaminopropyl)-3-ethylcarbodiimid (EDCI), N-Hydroxybenzotriazol (HOBT), N-[(Dimethylamino)-(3H-[1,2,3]triazolo[4,5-b]pyridin-3-yloxy)methyliden]-N-methylmethanaminiumhexafluorophosphat (HATU) zu aktivieren. Zum Beispiel findet die Umsetzung mit HATU in einem inerten Solvens, beispielsweise N,N-Dimethylformamid, Dimethylsulfoxid in Anwesenheit des entsprechenden Amins und eines tertiären Amins, beispielsweise Triethylamin, Diisopropylethylamin bei Temperaturen zwischen -30°C und +60°C statt.
Es ist ebenfalls möglich, eine Carbonsäure der Formel VI mit einem anorganischen Säurechlorid, beispielsweise Phosphorpentachlorid, Phosphortrichlorid, Thionylchlorid in das entsprechende Carbonsäurechlorid und anschließend in Pyridin oder einem inerten Lösungsmittel, wie zum Beispiel N,N-Dimethylformamid in Anwesenheit des entsprechenden Amins und eines tertiären Amins, beispielsweise Triethylamin bei Temperaturen zwischen -30°C und +60°C in die Zielverbindungen der allgemeinen Formel I zu überführen.

Die erfindungsgemäßen Verbindungen der allgemeinen Formel I lassen sich ebenso aus Aminen der allgemeinen Formel XXXII durch Umsetzung mit Carbonsäuren, Carbonsäurechloriden oder Carbonsäureanhydriden erzielen.

Die erfindungsgemäßen Verbindungen der allgemeinen Formel I lassen sich ebenso aus Bromimidazolen der allgemeinen Formel XIII (XIIIa und/oder XIIIb) unter Palladium(0)-Katalyse durch Umsetzung mit einem entsprechenden Alkohol oder Amin und Kohlenmonoxid (CO) bzw. einer Kohlenmonoxidquelle, wie z.B. Molybdaenhexacarbonyl in einem geeigneten Lösungsmittel beziehungsweise - gemisch, zum Beispiel 1,4-Dioxan/Wasser oder Tetrahydrofuran, Zugabe einer Base wie beispielsweise Natriumcarbonat oder 1,8-Diazabicyclo(5.4.0)undec-7-en (DBU) sowie eines Katalysator-Liganden-Gemischs, zum Beispiel Palladium(II)-acetat oder trans-Bis(acetato)bis[o-(di-o-tolylphosphino)benzyl]dipalladium(II) / Tri-tert-butylphosphinotetrafluoroborat bei Temperaturen zwischen 80°C und 160°C (ggf. unter Mikrowellenbestrahlung zwischen 80-200 Watt), und im Falle der Verwendung von Kohlenmonoxid bei einem CO-Druck von 5-15bar, erhalten (Schema 3, Schema 4). Diese Methode ist nicht auf Methylester, d.h. auf die Verwendung von Methanol beschränkt, sondern auch auf andere Ester erweiterbar. So lassen sich beispielsweise durch Verwendung von Ethanol anstelle Methanol auf diese Art und Weise die entsprechenden Ethylester synthetisieren.

Die Carbonsäuren der allgemeinen Formel VI lassen sich beispielsweise aus Estern der Formel Va durch Esterverseifung in einem geeigneten Lösungsmittel beziehungsweise Lösungsmittelgemisch, zum Beispiel Methanol, Ethanol, Tetrahydrofuran, Wasser unter Zusatz einer wässrigen Lösung eines Alkalihydroxids, zum Beispiel Natriumhydroxid, Lithiumhydroxid bei Temperaturen zwischen 20°C und 60°C erhalten (Schema 1 -4).

Die Verbindungen der allgemeinen Formel XXIV lassen sich beispielsweise aus den entsprechenden Anilinen XX herstellen, indem XX nach dem Fachmann bekannten Verfahren zu Formel XXII cyclisiert und anschließend zu XXIII oxidiert werden. Die so generierten Verbindungen XXIII können nach in der Literatur bekannten Verfahren am Stickstoff des Carbazols alkyliert werden (Schema 5) und dann in Reaktionen eingesetzt werden, in denen die erfindungsgemäßen Benzimidazole der Formel I dargestellt werden.

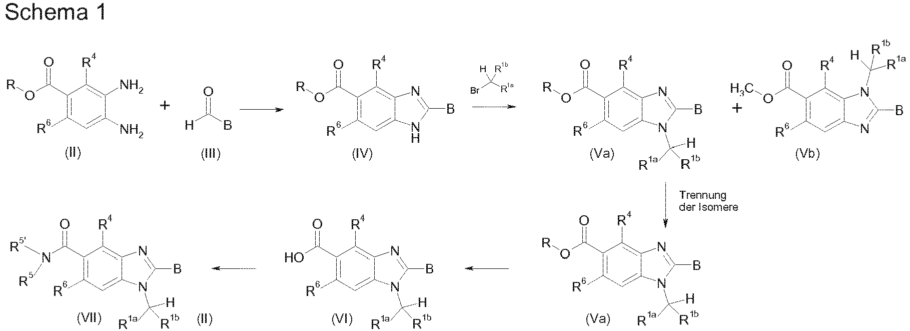

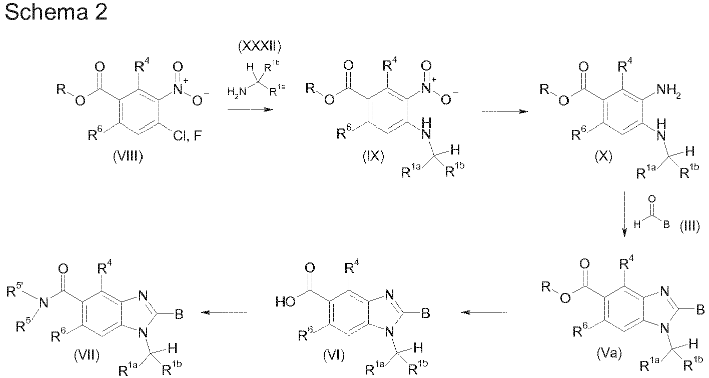

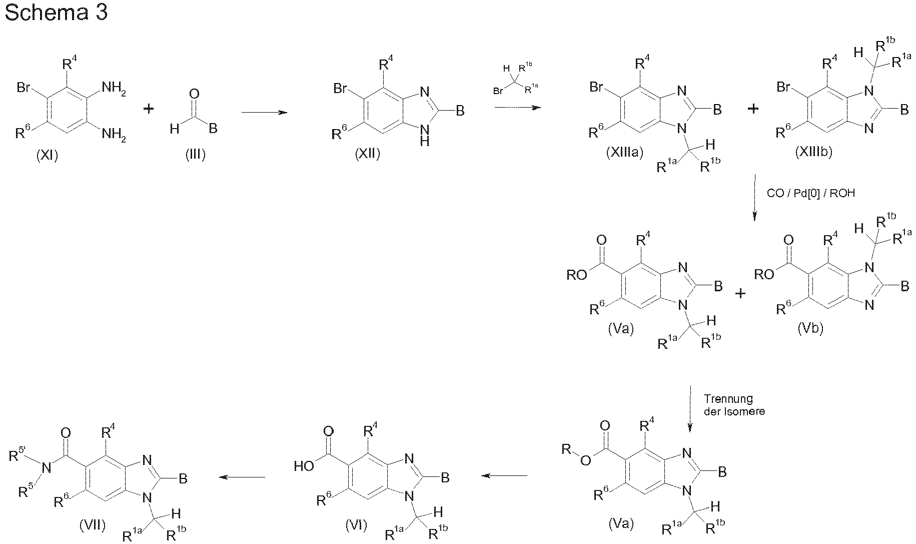

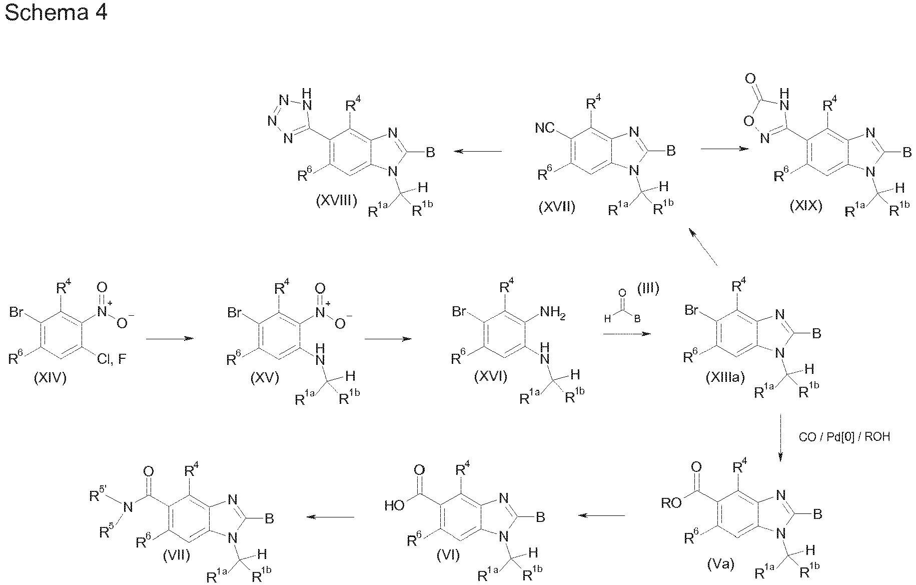

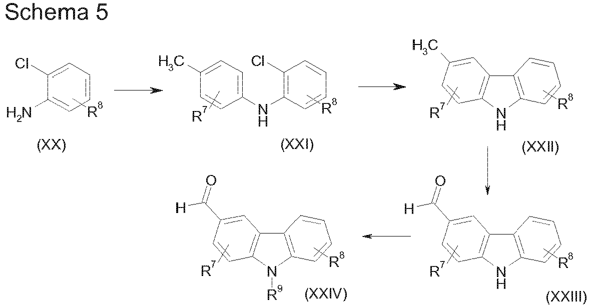

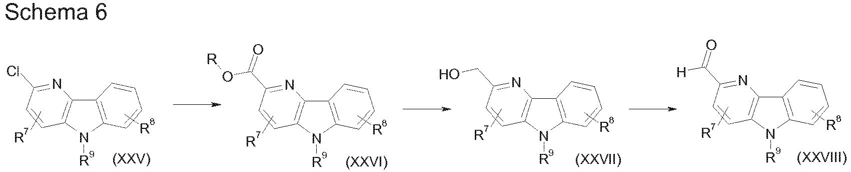

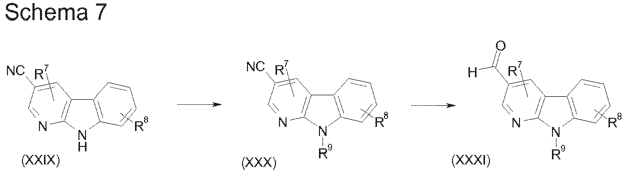

### Herstellung der erfindungsgemäßen Verbindungen

Die nachfolgenden Beispiele erläutern die Herstellung der erfindungsgemäßen Verbindungen der allgemeinen Formel (I), ohne den Umfang der beanspruchten Verbindungen auf diese Beispiele zu beschränken.
Die erfindungsgemäßen Verbindungen der allgemeinen Formel (I) lassen sich wie nachstehend beschrieben herstellen und charakterisieren.

### Abkürzungen

- CO: Kohlenmonoxid
- DBU: 1,8-Diazabicyclo[5.4.0]undec-7-en
- DCM: Dichlormethan
- DMF: N,N-Dimethylformamid
- DMSO: Dimethylsulfoxid
- h: Stunde(n)
- HPLC: Hochdruck-, Hochleistungsflüssigchromatographie
- konz.: konzentriert
- M: Molar
- min: Minute(n)
- N: Normal
- NMR: Kernresonanzspektroskopie
- RT: Raumtemperatur
- tert: tertiär
- THF: Tetrahydrofuran

NMR-Peak Formen sind so angegeben wie sie im Spektrum erscheinen, mögliche Effekte höherer Ordnung wurden nicht berücksichtigt.

### Experimenteller Teil

### Intermediat 1

### Methyl-2-(9-ethyl-9H-carbazol-3-yl)-1 H-benzimidazol-5-carboxylat

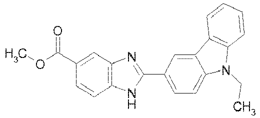

25.7 g (135 mmol) Natriumdisulfit wurden in 60 ml Wasser gelöst und mit einer Lösung von 14.8 g (66 mmol) 9-Ethyl-9H-carbazol-3-carbaldehyd in 135 ml THF versetzt. Danach wurden 20 g (120 mmol) Methyl-3,4-diaminobenzoat in 90 ml THF hinzugefügt, 3 h zum Rückfluss erhitzt und unter Abkühlung auf RT 15 h gerührt. Zum Reaktionsgemisch wurden 150 ml gesättigte Natriumhydrogencarbonatlösung gegeben, mehrmals mit Dichlormethan extrahiert, die gesammelten organischen Phasen mit Natriumsulfat getrocknet und bis zur Trockene eingedampft. Der Rückstand wurde in wenig Dichlormethan aufgenommen und zur Kristallisation gebracht. So wurden 20.56 g (84%) der Titelverbindung erhalten.
¹H-NMR (300 MHz, DMSO-d6), δ [ppm]= 1.33 (t, 3H), 3.85 (s, 3H), 4.48 (q, 2H), 7.26 (t, 1 H), 7.49 (t, 1 H), 7.59 - 7.70 (m, 2H), 7.74 - 7.87 (m, 2H), 8.11 - 8.34 (m, 3H), 8.97 (d, 1H), 12.60 - 13.70 (1H).

### Intermediat 2

### Ethyl-4-[(2-methoxyethyl)amino]-3-nitrobenzoat

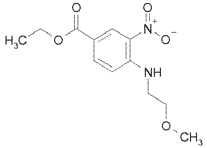

40.0 g (0.17 mol) Ethyl-4-chlor-3-nitrobenzoat wurden in 200 ml DMSO gegeben, 20.9 g (0.28 mol) 2-Methoxyethanamin hinzugefügt, 6 h auf 60 °C erhitzt und dann über Nacht auf RT abgekühlt. Das Reaktionsgemisch wurde auf 200 ml gesättigte Natriumhydrogencarbonatlösung gegossen, der entstandene Niederschlag abfiltriert und mit 100 ml Wasser gewaschen. Der Niederschlag wurde getrocknet. So wurden 45.5g (78%) der Titelverbindung erhalten.
¹H-NMR (300 MHz, DMSO-d6), δ [ppm]= 1.31 (t, 3H), 3.31 - 3.32 (m, 1H), 3.57 - 3.62 (m, 4H), 4.29 (q, 2H), 7.19 (d, 1 H), 7.97 (dd, 1 H), 8.50 - 8.56 (m, 1 H), 8.61 (d, 1 H).

### Intermediat 3

### 4-[(2-Methoxyethyl)amino]-3-nitrobenzoesäure

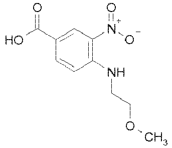

26.0 g (0.097 mol) Ethyl-4-[(2-methoxyethyl)amino]-3-nitrobenzoat wurden in 100 ml Ethanol gegeben, mit 55 mL 2M Natronlauge versetzt und 1 h zum Rückfluss erhitzt. Nach dem Abkühlen wurden 75 ml 2M Salzsäure hinzugefügt und fünfmal mit Dichlormethan extrahiert. Die gesammelten organischen Phasen wurden über Natriumsulfat getrocknet, filtriert und eingeengt. So wurden 21.8 g (93%) der Titelverbindung erhalten.
¹H-NMR (300 MHz, DMSO-d6), δ [ppm]= 3.31 (s, 3H), 3.57 -3.61 (4H), 7.17 (d, 1H), 7.96 (dd, 1 H), 8.47 - 8.53 (br. s., 1 H), 8.61 (d, 1 H), 12.40 - 13.30 (1 H).

### Intermediat 4

### 3-Amino-4-[(2-methoxyethyl)amino]benzoesäure

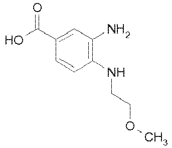

21,8 g (0.09 mol) 4-[(2-Methoxyethyl)amino]-3-nitrobenzoesäure wurden in 500 ml Ethanol gelöst, mit 2,5g Palladium/Kohle (10%) versetzt und bei RT unter Einleitung von Wasserstoff 4 h gerührt. Danach wurden erneut 2,5g Palladium/Kohle (10%) hinzugefügt und weitere 2 h Wasserstoff eingeleitet. Der Katalysator wurde abfiltriert und die Lösung eingeengt. So wurden 19.0 g (82%) der Titelverbindung erhalten.
¹H-NMR (400 MHz, DMSO-d6), δ [ppm]= 3.23 - 3.31 (m, 5H), 3.41 - 3.48 (m, 2H), 5.35 - 5.65 (1 H), 5.70 - 6.20 (2H), 6.15 (d, 1 H), 6.70 (s, 1H), 7.16 (d, 1H), 11.40 - 12.50 (1H).

### Intermediat 5

### 3-Amino-4-[(cyclopropylmethyl)amino]benzoesäure

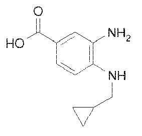

In Analogie zu Intermediat 4 wurde aus Ethyl-4-chlor-3-nitrobenzoat und 1-Cyclopropylmethanamin in drei Stufen 3-Amino-4-[(cyclopropylmethyl)amino]benzoesäure erhalten.
¹H-NMR (300 MHz, DMSO-d6), δ [ppm]= 0.21 - 0.27 (m, 2H), 0.46 - 0.53 (m, 2H), 1.05 - 1.15 (m, 1H), 2.98 (d, 2H), 5.00 - 5.80 (3H), 6.47 (d, 1H), 7.22 - 7.28 (m, 2H), 11.10 - 12.35 (1 H).

### Intermediat 6

### 4-Brom-3-chlor-N-(cyclopropylmethyl)-2-nitroanilin

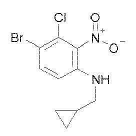

650 mg (2.59 mmol) 4-Brom-3-chlor-2-nitroanilin wurden in 5 ml Trifluoressigsäure gegeben, auf -15 °C gekühlt, portionsweise mit 822 mg (3.88 mmol) Natriumtriacetoxyborhydrid versetzt und 10 min bei -15 °C gerührt. Danach wurden 272 mg (3.88 mmol) Cyclopropancarbaldehyd in 5 ml Dichlormethan hinzugefügt und weitere 30 min bei -15 °C gerührt. Anschließend wurde das Reaktiongemisch auf kalte gesättigte Natriumhydrogencarbonatlösung gegossen und dreimal mit Dichlormethan extrahiert. Die organischen Phasen wurden filtriert und eingeengt. Auf diese Weise wurden 720 mg (87%) der Titelverbindung erhalten.
¹H-NMR (400 MHz, DMSO-d6), δ [ppm]= 0.19 - 0.23 (m, 2H), 0.41 - 0.46 (m, 2H), 1.00 - 1.09 (m, 1 H), 3.03 (t, 2H), 6.48 (t, 1 H), 6.92 (d, 1 H), 7.64 (d, 1 H).

### Intermediat 7

### 4-Brom-3-chlor-N1-(cyclopropylmethyl)benzol-1,2-diamin

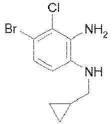

720 mg (2.36 mmol) 4-Brom-3-chlor-N-(cyclopropylmethyl)-2-nitroanilin wurden in 30 ml Ethanol gegeben, mit 2.13 g (9.43 mmol) Zinn-(II)-chloriddihydrat versetzt und anschließend 3 h bei 70 °C gerührt. Dann wurde das Reaktionsgemisch fast zur Trockene eingeengt, langsam mit gesättigter Natriumhydrogencarbonatlösung versetzt und dreimal mit Dichlormethan extrahiert. Die vereinten organischen Phasen wurden filtriert und anschließend eingeengt.
¹H-NMR (400 MHz, DMSO-d6), δ [ppm]= 0.19 - 0.24 (m, 2H), 0.46 - 0.52 (m, 2H), 1.04 - 1.10 (m, 1 H), 2.89 (dd, 2H), 4.98 - 5.05 (m, 1 H), 5.12 (s, 2H), 6.32 (d, 1 H), 6.80 (d, 1 H).

### Intermediat 8

### 3-[5-Brom-4-chlor-1-(cyclopropylmethyl)-1H-benzimidazol-2-yl]-9-ethyl-9H-carbazol

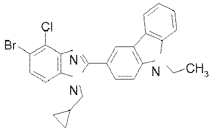

486 mg (2.56 mmol) Natriumdisulfit wurden in 3 ml Wasser gegeben, 5 min bei RT gerührt und dann mit einer Lösung von 253 mg (1.14 mmol) 9-Ethyl-9H-carbazol-3-carbaldehyd in 1 ml THF versetzt. Danach wurden 470 mg (1.71 mmol) 4-Brom-3-chlor-N1-(cyclopropylmethyl)benzol-1,2-diamin in 3 ml THF hinzugefügt, 10 min bei RT gerührt und anschließend 30 min zum Rückfluss erhitzt. Das Reaktionsgemisch wurde auf gesättigte Natriumhydrogencarbonatlösung gegeben und dreimal mit Ethylacetat extrahiert. Die organischen Phasen wurden eingeengt und der Rückstand chromatographisch an Kieselgel (Hexan/Ethylacetat 1:0 -> 4:6) gereinigt. So wurden 720 mg (79%) der Titelverbindung erhalten.
¹H-NMR (300 MHz, DMSO-d6), δ [ppm]= 0.10 - 0.18 (m, 2H), 0.30 - 0.39 (m, 2H), 0.95 - 1.10 (m, 1 H), 1.37 (t, 3H), 4.36 (d, 2H), 4.53 (q, 2H), 7.27 (t, 1 H), 7.49 - 7.56 (m, 1 H), 7.60 (d, 1 H), 7.67 - 7.76 (m, 2H), 7.79 - 7.84 (m, 1 H), 7.87 - 7.92 (m, 1 H), 8.32 (d, 1 H), 8.64 (d, 1 H).

### Intermediat 9

### Methyl-3-amino-4-[(cyclopropylmethyl)amino]-2-methylbenzoat

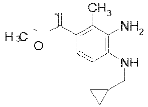

1.0 g (5.2 mmol) 4-Acetamido-2-methylbenzoesäure wurde in 5 ml konzentrierter Schwefelsäure gegeben und anschließend bei 0 °C ein Gemisch aus 0.22 ml konzentrierter Salpetersäure und 0.50 ml konzentrierter Schwefelsäure hinzugetropft. Das Reaktionsgemisch wurde auf RT erwärmt und über Nacht bei RT belassen. Dann wurde das Gemisch auf Eiswasser gegeben und der entstandene Niederschlag abfiltriert. So wurden 1.47 g 4-Amino-2-methyl-3-nitrobenzoesäure als Rohprodukt erhalten.

0.96 g (4.9 mmol) rohes 4-Amino-2-methyl-3-nitrobenzoesäure wurden in 40 ml Methanol gegeben, mit 2.4 ml konzentrierter Schwefelsäure versetzt und 18h zum Rückfluss erhitzt. Nach dem Abkühlen auf RT wurde der ausgefallene Niederschlag (Methyl-4-amino-2-methyl-3-nitrobenzoat, 228 mg, 22%) abfiltriert und ohne weitere Reinigung in die nächste Stufe eingesetzt.

220 mg (1.05 mmol) rohes Methyl-4-amino-2-methyl-3-nitrobenzoat in 1.1 ml Trifluoressigsäure wurden auf -15 °C gekühlt und portionsweise mit 333 mg (1.57 mmol) Natriumtriacetoxyborhydrid versetzt. Nach 10 min wurden 73 mg (1.05 mmol) Cyclopropancarbaldehyd in 2.2 ml Dichlormethan zugetropft und das Reaktionsgemisch nach weiteren 5 min auf gekühlte, gesättigte Natriumhydrogencarbonatlösung gegossen. Anschließend wurde dreimal mit Dichlormethan extrahiert, die organischen Phasen mit Natriumsulfat getrocknet, filtriert und das Filtrat eingeengt.

Der so erhaltene Rückstand (240 mg) wurde in Methanol aufgenommen und unter Normaldruck an Palladium (10%ig auf Kohlenstoff) hydriert. Der Katalysator wurde abfiltriert und das Filtrat eingeengt. So wurden 190 mg (79%) der Titelverbindung erhalten, welche ohne weitere Reinigung in der nächsten Stufe eingesetzt wurde.

### Intermediat 10

### 7-Fluor-3-methyl-9H-carbazol

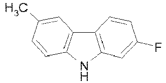

Eine Mischung aus 2.0 g (11.7 mmol) 1-Brom-4-methylbenzol, 1.7 g (11.7 mmol) 2-Chlor-5-fluoranilin, 5.6 g (58.5 mmol) Natrium-tert.-butylat, 131 mg (0.59 mmol) Palladium-(II)-acetat und 237 mg (0.82 mmol) Tri-tert.-butylphosphonium tetrafluoroborat wurden in 15 ml Toluol aufgenommen und 3 h in einer Mikrowelle auf 160 °C erhitzt. Nach dem Abkühlen wurde mit 1M Salzsäure bis pH 2 angesäuert, dreimal mit Dichlormethan extrahiert und die vereinten organisch Phasen eingeengt. Der Rückstand wurde chromatographisch an Kieselgel (Hexan/Ethylacetat 10:1) gereinigt. So wurden 465 mg (20%) der Titelverbindung erhalten.
¹H-NMR (400 MHz, Chloroform-d), δ [ppm]= 2.53 (s, 3H), 6.92 - 6.99 (m, 1 H), 7.08 (dd, 1 H), 7.20 - 7.25 (m, 1 H), 7.28 - 7.35 (m, 2H), 7.82 (s, 1 H), 7.92 - 7.98 (m, 1 H).

### Intermediat 11

### 7-Fluor-9H-carbazol-3-carbaldehyd

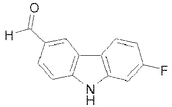

421 mg (2.11 mmol) 7-Fluor-3-methyl-9H-carbazol wurden in 5.5 ml Methanol/Wasser (10/1) gegeben, 2.0 g (8.90 mmol) 2,3-Dichlor-5,6-dicyano-1,4-benzochinon dazugegeben und 1.5 h bei RT gerührt. Das Reaktionsgemisch wurde über Celite filtriert, das Filtrat eingeengt und der Rückstand chromatographisch an Kieselgel (Dichlormethan) gereinigt. So wurden 263 mg (58%) der Titelverbindung erhalten. ¹H-NMR (300 MHz ,CHLOROFORM-d), δ [ppm]= 7.03 (td, 1H), 7.12 - 7.19 (m, 1H), 7.49 (d, 1 H), 7.93 (dd, 1 H), 8.02 (d, 1 H), 8.52 (s, 1 H), 9.60 (br. s., 1 H), 10.06 (s, 1 H).

### Intermediat 12

### 9-Ethyl-7-fluor-9H-carbazol-3-carbaldehyd

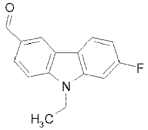

Unter Argon wurden 65 mg (1.36 mmol) Natriumhydrid (60%ige Mineralöldispersion) zweimal mit je 1 ml Toluol gewaschen und bei 0 °C mit einer Lösung von 263 mg (1.23 mmol) 7-Fluor-9H-carbazol-3-carbaldehyd in 4 ml DMF versetzt. Danach wurden 231 mg (1.48 mmol) lodethan hinzugefügt und das Gemisch für 17 h bei 50 ° gerührt. Nach dem Abkühlen wurde das Reaktionsgemisch mit 2 ml Wasser versetzt, 30 min gerührt und dreimal mit Ethylacetat extrahiert. Die organischen Phasen wurden mit Natriumsulfat getrocknet und eingeengt. So wurden 300 mg (100%) der Titelverbindung als Rohprodukt erhalten.

### Intermediat 13

### 9-Ethyl-5-fluor-9H-carbazol-3-carbaldehyd

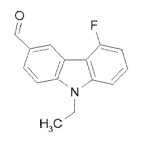

Ausgehend von 1-Brom-4-methylbenzol und 2-Chlor-3-fluoranilin wurde in Analogie zu den Intermediaten 10 - 12 über drei Stufen 9-Ethyl-5-fluor-9H-carbazol-3-carbaldehyd erhalten, welcher als Rohprodukt eingesetzt wurde

### Intermediat 14

### 9-Ethyl-8-fluor-9H-carbazol-3-carbaldehyd

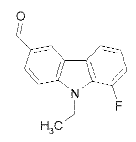

Ausgehend von 1-Brom-4-methylbenzol und 2-Chlor-6-fluoranilin wurde in Analogie zu den Intermediaten 10 - 12 über drei Stufen 9-Ethyl-8-fluor-9H-carbazol-3-carbaldehyd erhalten, welches als Rohprodukt eingesetzt wurde.

### Intermediat 15

### 9-(2-Methoxyethyl)-9H-carbazol-3-carbaldehyd

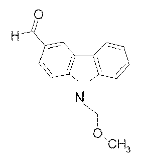

In Analogie zu Intermediat 12 wurde aus 9H-Carbazol-3-carbaldehyd und 1-Brom-2-methoxyethan 9-(2-Methoxyethyl)-9H-carbazol-3-carbaldehyd erhalten, welches ohne weitere Reinigung in der nächsten Stufe eingesetzt wurde.
¹H-NMR (400 MHz ,CHLOROFORM-d), δ [ppm]= 3.30 (s, 3H), 3.81 (t, 2H), 4.53 (t, 2H), 7.31 - 7.38 (m, 1 H), 7.45 - 7.60 (m, 3H), 8.02 (dd, 1 H), 8.16 (d, 1 H), 8.62 (d, 1 H), 10.11 (s, 1 H).

### Intermediat 16

### 9-Ethyl-6-methoxy-9H-carbazol-3-carbaldehyd

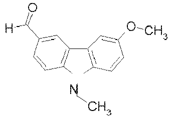

1.3 g (5.78 mmol) 6-Methoxy-9H-carbazol-3-carbaldehyd wurden in 78 ml DMF gelöst, dann 4.7 g (14.43 mmol) Caesiumcarbonat und 900 mg (5.78 mmol) lodethan hinzugefügt und 1 h bei 65°C gerührt. Nach dem Abkühlen wurde das Reaktionsgemisch filtriert und das Filtrat eingeengt. Auf diese Weise wurden 1,3 g (89%) der Titelverbindung erhalten.
¹H-NMR (300 MHz, DMSO-d6), δ [ppm]= 1.32 (t, 3H), 3.88 (s, 3H), 4.47 (q, 2H), 7.16 (dd, 1H), 7.62 (d, 1H), 7.73 (d, 1 H), 7.93 - 7.98 (m, 2H), 8.76 (d, 1 H), 10.03 (s, 1 H).

### Intermediat 17

### 9-Allyl-9H-carbazol-3-carbaldehyd

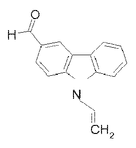

In Analogie zu Intermediat 16 wurde aus 9H-Carbazol-3-carbaldehyd und Allylbromid 9-Allyl-9H-carbazol-3-carbaldehyd erhalten.
¹H-NMR (300 MHz, DMSO-d6), δ [ppm]= 4.97 (dd, 1 H), 5.09 - 5.17 (m, 3H), 5.94 - 6.09 (m, 1 H), 7.28 - 7.35 (m, 1 H), 7.53 (td, 1 H), 7.64 - 7.69 (m, 1 H), 7.75 (d, 1 H), 7.99 (dd, 1 H), 8.31 (d, 1 H), 8.78 (d, 1 H), 10.07 (s, 1 H).

### Intermediat 18

### 2-Chlor-5-ethyl-5H-pyrido[3,2-b]indol

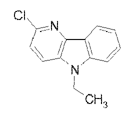

2-Chlor-5-ethyl-5H-pyrido[3,2-b]indol wurde ausgehend von 1-(1-Ethyl-1 H-indol-3-yl)ethanon analog der in WO 2004/046143 beschriebenen Synthese für 2-Chlor-5-methyl-5H-pyrido[3,2-b]indol hergestellt,
¹H-NMR (400 MHz, DMSO-d6), δ [ppm]= 1.30 - 1.34 (m, 3H), 4.50 (q, 2H), 7.29 - 7.34 (m, 1H), 7.52 (d, 1H), 7.61 (m, 1H), 7.75 (d, 1H), 8.16 - 8.21 (m, 2H).

### Intermediat 19

### Methyl-5-ethyl-5H-pyrido[3,2-b]indol-2-carboxylat

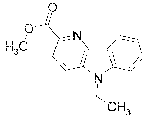

1.5 g (6.5 mmol) 2-Chlor-5-ethyl-5H-pyrido[3,2-b]indol wurden in 30 ml Methanol gelöst, mit 1.06 g (1.3 mmol) Bis(triphenylphosphin)-palladium-(11)-chlorid und 2.5 g (26.0 mmol) Kaliumacetat versetzt, und unter Kohlenmonoxid, bei 12 bar und 100 °C, 23 h in einem Autoklaven gerührt. Nach dem Abkühlen auf RT wurde das Gemisch filtriert, das Filtrat im Vakuum eingeengt und der so erhaltene Rückstand chromatographisch an Kieselgel (Hexan/Ethylacetat 7:3) gereinigt. So wurden 1.0 g (61%) der Titelverbindung erhalten.
¹H-NMR (300 MHz, DMSO-d6), δ [ppm]= 1.35 (t, 3H), 3.94 (s, 3H), 4.53 (q, 2H), 7.37 (t, 1H), 7.66 (m, 1 H), 7.76 - 7.81 (m, 1 H), 8.19 (d, 2H), 8.29 (d, 1 H).

### Intermediat 20

### 5-Ethyl-5H-pyrido[3,2-b]indol-2-carbaldehyd

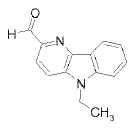

Unter Argon wurden 200 mg (0.79 mmol) Methyl-5-ethyl-5H-pyrido[3,2-b]indol-2-carboxylat in 4.5 ml Toluol gelöst, bei 0 °C mit 2.4 ml Düsobutylaluminiumhydridlösung (1.0 M in Toluol) versetzt, auf RT erwärmt und 2h bei RT gerührt. Dann wurden nacheinander 1.5 ml Ethanol, 1.5 ml Ethanol/Wasser (1:1), 1.5 ml Wasser hinzugefügt und mit 1 N Schwefelsäure auf pH=3 angesäuert. Danach wurde dreimal mit Ethylacetat extrahiert, die gesammelten organischen Phasen mit Natriumsulfat getrocknet, filtriert und eingeengt. Auf diese Weise wurden 162 mg (77%) (5-Ethyl-5H-pyrido[3,2-b]indol-2-yl)methanol als Rohprodukt erhalten, welches ohne weitere Reinigung in der nächsten Synthesestufe eingesetzt wurde.

100 mg (0.44 mmol) rohes (5-Ethyl-5H-pyrido[3,2-b]indol-2-yl)methanol wurden in 3.2 ml Dichlormethan gelöst, bei RT mit 50 mg (0.23 mmol) Pyridinium-chlorochromat versetzt und 1h bei dieser Temperatur gerührt. Danach wurde filtriert und das Filtrat im Vakuum eingeengt. Auf diese Weise wurden 54 mg (54%) der Titelverbindung erhalten. ¹H-NMR (400 MHz, DMSO-d6), δ [ppm]= 1.36 (t, 3H), 4.56 (d, 2H), 7.69 (t, 1H), 7.75 - 7.85 (m, 2H), 8.08 (d, 1H), 8.25 (d, 1H), 8.34 (d, 1H), 10.14 (s, 1H).

### Intermediat 21

### 9-Ethyl-9H-pyrido[2,3-b]indol-3-carbonitril

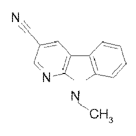

Zu 1.42 g (7.35 mmol) 9H-Pyrido[2,3-b]indol-3-carbonitril in 14 ml DMF wurden 5.99 g (18.37 mmol) Caesiumcarbonat und 1.15 g (7.35 mmol) lodethan gegeben und 1.5 h bei 65 °C gerührt. Nach dem Abkühlen wurde das Gemisch filtriert, das Filtrat eingeengt und der Rückstand chromatographisch an Kieselgel (Hexan/Ethylacetat 7:3) gereinigt. Auf diese Weise wurden 261 mg (16%) der Titelverbindung erhalten.
¹H-NMR (400 MHz ,CHLOROFORM-d), δ [ppm]= 1.50 (t, 3H), 4.58 (q, 2H), 7.39 (t, 1H), 7.53 - 7.57 (m, 1 H), 7.60 - 7.66 (m, 1 H), 8.12 (d, 1 H), 8.55 (d, 1 H), 8.76 (d, 1 H).

### Intermediat 22

### 9-Ethyl-9H-pyrido[2,3-b]indol-3-carbaldehyd

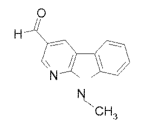

Zu einer Lösung von 250 mg (1.13 mmol) 9-Ethyl-9H-pyrido[2,3-b]indol-3-carbonitril in 15 ml Toluol wurden bei 0 °C unter Argon 1.4 ml (1.70 mmol) Diisobutylaluminiumhydrid in Toluol (25%ige Lösung) getropft und 1 h gerührt. Dann wurde das Gemisch auf Methanol gegeben, mit 1M Schwefelsäure angesäuert, danach mit gesättigter Natriumhydrogencarbonatlösung neutralisiert und dreimal mit Ethylacetat extrahiert. Die gesammelten organische Phasen wurden mit Natriumsulfat getrocknet, filtriert und eingeengt. Auf diese Weise wurden 160 mg (63%) der Titelverbindung erhalten.
¹H-NMR (400 MHz, DMSO-d6), δ [ppm]= 1.39 (t, 3H), 4.59 (q, 2H), 7.35 - 7.41 (m, 1 H), 7.59 - 7.65 (m, 1 H), 7.79 (d, 1 H), 8.37 (d, 1 H), 9.03 (s, 2H), 10.15 (s, 1 H).

### Intermediat 23

### Ethyl-3-amino-4-{[2-(pyrrolidin-1-yl)ethyl]amino}benzoat

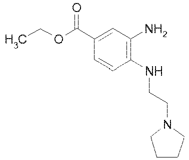

In Analogie zu Intermediat 2 wurden zunächst aus 1.0 g (4.4 mmol) Ethyl-4-chlor-3-nitrobenzoat und 0.8 g (7.0 mmol) 2-(Pyrrolidin-1-yl)ethanamin 1.2 g (88%) Ethyl-3-nitro-4-{[2-(pyrrolidin-1-yl)ethyl]amino}benzoat hergestellt, welches ohne weitere Reinigung in die nächste Stufe eingesetzt wurde.

Das Rohmaterial der letzten Stufe (1.2 g) wurde dann analog Intermediat 4 mit Wasserstoff an Palladium hydriert. Das so hergestellte Rohmaterial der Titelverbindung betrug 1.14 g (97%) und wurde ohne weitere Reinigung in der nächsten Stufe eingesetzt.

### Intermediat 24

### Ethyl-3-amino-4-[(cyclopropylmethyl)amino]benzoat

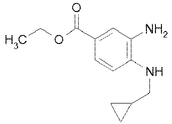

In Analogie zur Herstellung von Intermediat 2 wurde aus Ethyl-4-chlor-3-nitrobenzoat und 1-Cyclopropylmethanamin zunächst Ethyl-4-[(cyclopropylmethyl)amino]-3-nitrobenzoat hergestellt, welches dann analog der Herstellung von Intermediat 4 an Palladium mit Wasserstoff zur Titelverbindung umgesetzt wurde.

### Ethyl-4-[(cyclopropylmethyl)amino]-3-nitrobenzoat

¹H-NMR (400MHz, DMSO-d6): δ [ppm]= 0.30 - 0.36 (m, 2H), 0.50 - 0.56 (m, 2H), 1.12 - 1.24 (m, 1 H), 1.31 (t, 3H), 3.27 - 3.32 (m, 2H), 4.29 (q, 2H), 7.17 (d, 1 H), 7.97 (dd, 1 H), 8.56 (t, 1 H), 8.62 (d, 1 H).

### Ethyl-3-amino-4-[(cyclopropylmethyl)amino]benzoat

¹H-NMR (400MHz, DMSO-d6): δ [ppm]= 0.20 - 0.27 (m, 2H), 0.46 - 0.53 (m, 2H), 1.04 - 1.15 (m, 1 H), 1.26 (t, 3H), 2.95 - 3.01 (m, 2H), 4.18 (q, 2H), 4.77 (s, 2H), 5.26 (t, 1 H), 6.43 (d, 1 H), 7.15 - 7.22 (m, 2H).

### Intermediat 25

### 4-Brom-N1-(2-methoxyethyl)benzol-1,2-diamin

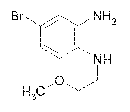

In Analogie zur Herstellung von Intermediat 2 wurde aus 4-Brom-1-fluor-2-nitrobenzol und 2-Methoxyethanamin zunächst 4-Brom-N-(2-methoxyethyl)-2-nitroanilin hergestellt, welches dann analog der Herstellung von Intermediat 4 an Raney-Nickel mit Wasserstoff zur Titelverbindung umgesetzt wurde.
¹H-NMR (400MHz, DMSO-d6): δ [ppm]= 3.26 - 3.33 (m, 5H), 3.48 - 3.54 (m, 2H), 3.55 - 3.61 (m, 2H), 7.10 (d, 1H), 7.66 (dd, 1H), 8.16 (d, 1H), 8.20 (br. s., 1H).

### Intermediat 26

### 3-[5-Brom-1-(2-methoxyethyl)-1 H-benzimidazol-2-yl]-9-ethyl-9H-carbazol

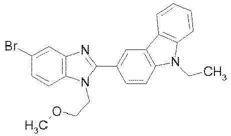

In Analogie zur Herstellung von Intermediat 8 wurde aus 0.87 g (3.5 mmol) 4-Brom-N1-(2-methoxyethyl)benzol-1,2-diamin und 0.53 g (2.4 mmol) 9-Ethyl-9H-carbazol-3-carbaldehyd 1.1g (68%) der Titelverbindung erhalten.
¹H-NMR (300 MHz, DMSO-d6), δ [ppm]= 1.37 (t, 3H), 3.14 (s, 3H), 3.71 (t, 2H), 4.47 - 4.59 (m, 4H), 7.27 (t, 1 H), 7.42 (dd, 1 H), 7.48 - 7.57 (m, 1 H), 7.66 - 7.72 (m, 2H), 7.79 (d, 1 H), 7.86 - 7.96 (m, 2H), 8.26 (d, 1 H), 8.66 (d, 1 H).

### Intermediat 27

### Ethyl-3-amino-4-{[2-(morpholin-4-yl)ethyl]amino}benzoat

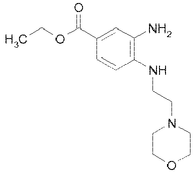

In Analogie zu Intermediat 2 wurden zunächst aus 1.0 g (4.4 mmol) Ethyl-4-chlor-3-nitrobenzoat und 0.91 g (7.0 mmol) 2-(Morpholin-4-yl)ethanamin 1.27 g (90%) Ethyl-3-amino-4-{[2-(morpholin-4-yl)ethyl]amino}benzoat hergestellt, welches ohne weitere Reinigung in die nächste Stufe eingesetzt wurde.

Das Rohmaterial der letzten Stufe (1.27 g) wurde dann analog Intermediat 4 mit Wasserstoff an Palladium hydriert. Das so hergestellte Rohmaterial der Titelverbindung betrug 1.19 g (90%) und wurde ohne weitere Reinigung in der nächsten Stufe eingesetzt.

### Intermediat 28

### Ethyl-3-amino-4-(isopropylamino)benzoat

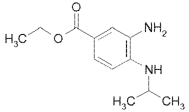

In Analogie zur Herstellung von Intermediat 2 wurde aus Ethyl-4-chlor-3-nitrobenzoat und Propan-2-amin zunächst Ethyl-4-(isopropylamino)-3-nitrobenzoat hergestellt, welches dann analog der Herstellung von Intermediat 4 an Palladium mit Wasserstoff zur Titelverbindung umgesetzt wurde.
¹H-NMR (400MHz, DMSO-d6): δ [ppm]= 1.18 (d, 6H), 1.26 (t, 3H), 3.65 (s, 1H), 4.18 (q, 2H), 4.80 - 5.30 (br., 3H), 6.46 (d, 1 H), 7.17 - 7.25 (m, 2H).

### Intermediat 29

### Ethyl-3-amino-4-[(2-methoxyethyl)amino]benzoat

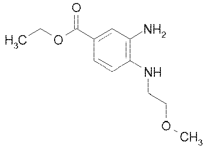

Ethyl-3-amino-4-[(2-methoxyethyl)amino]benzoat wurde analog Intermediat 4 aus Ethyl-4-[(2-methoxyethyl)amino]-3-nitrobenzoat durch Reduktion mit Wasserstoff an Palladium hergestellt.
¹H-NMR (400 MHz, DMSO-d6), δ [ppm]= 1.25 (t, 3H), 3.25 - 3.31 (m, 5H), 3.42 - 3.47 (m, 2H), 4.13 - 4.20 (m, 2H), 6.02 (br. s., 1H), 6.17 (d, 1H), 6.71 (d, 1H), 7.18 (dd, 1H), NH2 nicht angegeben.

### Intermediat 30

### Ethyl-3-amino-4-[(3-methoxypropyl)amino]benzoat

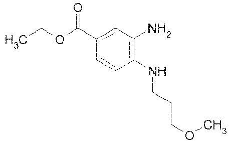

In Analogie zu Intermediat 2 wurden zunächst aus 1.0 g (4.4 mmol) Ethyl-4-chlor-3-nitrobenzoat und 0.62 g (7.0 mmol) 3-Methoxypropan-1-amin 1.20 g (88%) Ethyl-4-[(3-methoxypropyl)amino]-3-nitrobenzoat hergestellt, welches dann analog Intermediat 4 mit Wasserstoff an Palladium hydriert wurde. Das so hergestellte Rohmaterial der Titelverbindung betrug 1.15 g (93%).

### Ethyl-4-[(3-methoxypropyl)amino]-3-nitrobenzoat

¹H-NMR (300MHz, DMSO-d6): δ [ppm]= 1.26 - 1.38 (m, 3H), 1.88 (quin, 2H), 3.27 (s, 3H), 3.42 - 3.50 (m, 4H), 4.29 (q, 2H), 7.13 (d, 1H), 7.94 - 8.01 (m, 1H), 8.62 (d, 1H), 8.70 (t, 1 H).

### Ethyl-3-amino-4-[(3-methoxypropyl)amino]benzoat

¹H-NMR (400MHz, DMSO-d6): δ [ppm]= 1.26 (t, 3H), 1.82 (quin, 2H), 3.13 - 3.18 (m, 2H), 3.25 (s, 3H), 3.43 (t, 2H), 4.19 (q, 2H), 6.45 (d, 1 H), 7.20 (d, 1 H), 7.24 (dd, 1 H), NH und NH2 nicht angegeben.

### Intermediat 31

### 3-(5-Brom-6-methoxy-1H-benzimidazol-2-yl)-9-ethyl-9H-carbazol

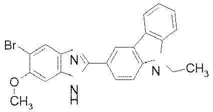

4.81 g (19.5 mmol) 4-Brom-5-methoxy-2-nitroanilin wurden in einer Mischung aus 58 ml Ethanol und 39 ml Wasser gelöst, anschließend mit 1.2 ml Essigsäure und 5.44 g (97.3 mmol) Eisenpulver versetzt und dann 2h zum Rückfluss erhitzt. Nach dem Abkühlen auf RT wurde über Celite filtriert, mit Ethylacetat gewaschen und das Filtrat im Vakuum bis zur Trockene eingeengt. Das so erhaltene Rohprodukt (4-Brom-5-methoxybenzol-1,2-diamin, 4.92 g) wurde ohne weitere Reinigung in der nächsten Stufe eingesetzt.

12.92 g (68.0 mmol) Natriumdisulfit wurden in 15 ml Wasser gegeben, 5 min bei RT gerührt und dann mit einer Lösung von 5.06 g (22.7 mmol) 9-Ethyl-9H-carbazol-3-carbaldehyd in 15 ml THF versetzt. Danach wurden 4.92 g (22.7 mmol) 4-Brom-5-methoxybenzol-1,2-diamin in 15 ml THF hinzugefügt, 10 min bei RT gerührt und anschließend 30 min zum Rückfluss erhitzt. Nach dem Abkühlen auf RT wurde das Reaktionsgemisch auf gesättigte Natriumhydrogencarbonatlösung gegeben und mehrmals mit Ethylacetat extrahiert. Die organischen Phasen wurden eingeengt und der Rückstand chromatographisch an Kieselgel gereinigt. So wurden 5.87 g (57%) der Titelverbindung erhalten.
¹H-NMR (400 MHz, DMSO-d6), δ [ppm]= 1.36 (t, 3H), 3.91 (s, 3H), 4.50 (d, 2H), 7.12 - 7.41 (m, 2H), 7.49-7.54 (m, 1H), 7.64 - 7.88 (m, 3H), 8.20-8.27 (m, 2H), 8.91 (d, 1H), 12.73 - 13.00 (br, 1 H).

### Intermediat 32

### 3-(5-Brom-4-fluor-1 H-benzimidazol-2-yl)-9-ethyl-9H-carbazol

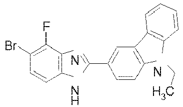

In Analogie zu Beispiel 31 wurde ausgehend von 4-Brom-3-fluor-2-nitroanilin zuerst 4-Brom-3-fluorbenzol-1,2-diamin hergestellt, welches anschließend mit 9-Ethyl-9H-carbazol-3-carbaldehyd zu 3-(5-Brom-4-fluor-1H-benzimidazol-2-yl)-9-ethyl-9H-carbazol umgesetzt wurde.
¹H-NMR (400MHz, DMSO-d6): δ [ppm]= 1.36 (t, 3H), 4.51 (q, 2H), 7.26 - 7.32 (m, 1 H), 7.33 - 7.43 (m, 2H), 7.53 (td, 1 H), 7.68 (d, 1 H), 7.81 (d, 1 H), 8.23 - 8.34 (m, 2H), 9.01 (s., 1H), 13.31 (br. s., 1H).

### Intermediat 33

### 3-(5-Brom-6-fluor-1 H-benzimidazol-2-yl)-9-ethyl-9H-carbazol

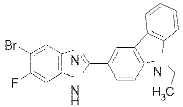

In Analogie zu Beispiel 31 wurde ausgehend von 4-Brom-5-fluor-2-nitroanilin zuerst 4-Brom-5-fluorbenzol-1,2-diamin hergestellt. Dieses wurde anschließend mit 9-Ethyl-9H-carbazol-3-carbaldehyd zu 3-(5-Brom-6-fluor-1H-benzimidazol-2-yl)-9-ethyl-9H-carbazol umgesetzt und direkt in weiteren Stufen eingesetzt.

### Intermediat 34

### Methyl-3-amino-4-[(2-methoxyethyl)amino]-2-methylbenzoat

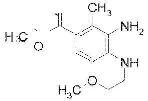

27.5 g (0.12 mol) eines Gemisches aus Methyl-4-chlor-2-methyl-5-nitrobenzoat und Methyl-4-chlor-2-methyl-3-nitrobenzoat, hergestellt nach M. Baumgarth et al., J. Med. Chem. 1997, 40, 2017-2034, wurden in 50 ml DMSO gegeben, mit 31 ml (0.36 mol) 2-Methoxyethanamin versetzt und 25 h bei 80° C gerührt. Dann wurde mit Wasser versetzt, mehrfach mit DCM extrahiert und die gesammelten organischen Phasen eingedampft. Der Rückstand wurde chromatographisch an Kieselgel (Hexan/DCM 1:0 → 0:1) aufgetrennt. Auf diese Weise wurden 9.3 g (29%) Methyl-4-[(2-methoxyethyl)amino]-2-methyl-3-nitrobenzoat und 15.5 g (49%) Methyl-4-[(2-methoxyethyl)amino]-2-methyl-5-nitrobenzoat erhalten.

### Methyl-4-[(2-methoxyethyl)amino]-2-methyl-3-nitrobenzoat

¹H-NMR (400 MHz, DMSO-d6), δ [ppm]= 2.37 (s, 3H), 3.26 (s, 3H), 3.36 (q, 2H), 3.47 (t, 2H), 3.77 (s, 3H), 6.42 (t, 1 H), 6.86 (d, 1 H), 7.83 (d, 1 H).

### Methyl-4-[(2-methoxyethyl)amino]-2-methyl-5-nitrobenzoat

¹H-NMR (400 MHz, DMSO-d6), δ [ppm]= 2.55 (s, 3H), 3.32 (s, 3H), 3.56 - 3.62 (m, 4H), 3.79 (s, 3H), 6.99 (s, 1 H), 8.39 - 8.44 (m, 1 H), 8.64 (s, 1 H).

3.33 g (12.4 mmol) Methyl-4-[(2-methoxyethyl)amino]-2-methyl-3-nitrobenzoat wurden in 80 ml THF/Methanol (1:1) gelöst und unter Normaldruck an Palladium (10%ig auf Kohlenstoff) hydriert. Der Katalysator wurde abfiltriert und das Filtrat eingeengt. So wurden 2.85 g (92%) rohes Methyl-3-amino-4-[(2-methoxyethyl)amino]-2-methylbenzoat erhalten, welches ohne weitere Reinigung in folgende Stufen eingesetzt wurde.
¹H-NMR (400 MHz, DMSO-d6), δ [ppm]= 2.31 (s, 3H), 3.25 - 3.31 (m, 5H), 3.53 (t, 2H), 3.70 (s, 3H), 4.44 (br, 2H), 5.20 (t, 1H), 6.37 (d, 1H), 7.18 (d, 1H).

### Intermediat 35 und Intermediat 36

### 6-Chlor-9-ethyl-9H-carbazol-3-carbaldehyd und 8-Chlor-9-ethyl-9H-carbazol-3-carbaldehyd

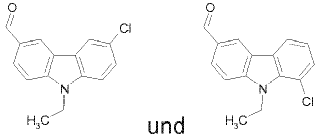

15.0 g (67.2 mmol) 9-Ethyl-9H-carbazol-3-carbaldehyd und 9.9 g (73.9 mmol) N-Chlorsuccinimid (NCS) wurden in 228 ml Acetonitril gegeben und 24 h bei RT gerührt. Dann wurde das Reaktionsgemisch eingedampft, der Rückstand in 340 ml DCM aufgenommen und anschließend mit 300 ml n-Hexan versetzt. Der Niederschlag wurde abfiltriert, das Filtrat zur Trockene eingedampft und der Eindampfrückstand chromatographisch an Kieselgel (Hexan/Ethylacetat 8:2 -> 7:3) gereinigt. So wurden 11.4 g (66%) 6-Chlor-9-ethyl-9H-carbazol-3-carbaldehyd und 4.8 g (25%) 8-Chlor-9-ethyl-9H-carbazol-3-carbaldehyd erhalten.

### 6-Chlor-9-ethyl-9H-carbazol-3-carbaldehyd

¹H-NMR (600 MHz, DMSO-d6), δ [ppm]= 1.33 (t, 3H), 4.51 (q, 2H), 7.55 (dd, 1H), 7.69 - 7.87 (m, 2H), 8.02 (dd, 1 H), 8.42 (d, 1 H), 8.81 (d, 1 H), 10.05 (s, 1 H).

### 8-Chlor-9-ethyl-9H-carbazol-3-carbaldehyd

¹H-NMR (400 MHz, DMSO-d6), δ [ppm]= 1.40 (t, 3H), 4.83 (q, 2H), 7.29 (t, 1H), 7.56 (dd, 1 H), 7.85 (d, 1 H), 8.05 (dd, 1 H), 8.31 (dd, 1 H), 8.80 (d, 1 H), 10.08 (s, 1 H).

### Beispiel 1

### Methyl-1-allyl-2-(9-ethyl-9H-carbazol-3-yl)-1 H-benzimidazol-5-carboxylat

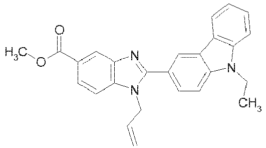

Zur einer Lösung von 1.5 g (4.1 mmol) Methyl-2-(9-ethyl-9H-carbazol-3-yl)-1 H-benzimidazol-5-carboxylat in 50 ml DMF wurden unter Argon zunächst 179 mg (4.45 mmol) Natriumhydrid (60%ige Mineralöldispersion) gegeben, anschließend 30 min bei RT gerührt und dann mit 0.42 ml (4.87 mmol) Allylbromid versetzt. Nach 30 h wurden 50 ml gesättigte Natriumhydrogencarbonatlösung hinzugefügt und das Gemisch mehrmals mit Dichlormethan extrahiert. Die gesammelten organischen Phasen wurden mit Wasser gewaschen, mit Natriumsulfat getrocknet und eingeengt. Der Rückstand wurde zunächst chromatographisch an Kieselgel (Hexan/Ethylacetat 7:3) gereinigt und das so erhaltene Rohprodukt (1.46 g) mittels präparativer HPLC aufgetrennt. Auf diese Weise wurden 613 mg (37%) Methyl-1-allyl-2-(9-ethyl-9H-carbazol-3-yl)-1 H-benzimidazol-5-carboxylat und 457 mg (28%) Methyl-1-allyl-2-(9-ethyl-9H-carbazol-3-yl)-1 H-benzimidazol-6-carboxylat erhalten.

### Methyl-1-allyl-2-(9-ethyl-9H-carbazol-3-yl)-1 H-benzimidazol-5-carboxylat

¹H-NMR (300 MHz ,CHLOROFORM-d), δ [ppm]= 1.48 (t, 3H), 3.97 (s, 3H), 4.43 (q, 2H), 4.89 - 4.98 (m, 2H), 5.19 (d, 1H), 5.42 (d, 1 H), 6.17 (ddt, 1 H), 7.27 - 7.32 (m, 1 H), 7.39 (d, 1H), 7.43 - 7.57 (m, 3H), 7.88 (dd, 1H), 8.04 (dd, 1H), 8.12 (d, 1H), 8.56 (dd, 2H).

### Methyl-1-allyl-2-(9-ethyl-9H-carbazol-3-yl)-1 H-benzimidazol-6-carboxylat

¹H-NMR (300 MHz ,CHLOROFORM-d), δ [ppm]= 1.49 (3H), 3.97 (3H), 4.44 (2H), 4.92-5.02 (2H), 5.21 (1 H), 5.44 (1 H), 6.12-6.29 (1 H), 7.28-7.32 (1 H), 7.44-7.59 (3H), 7.83-7.95 (2H), 8.05 (1 H), 8.13 (2H), 8.58 (1 H).

### Beispiel 2

### 1-Allyl-2-(9-ethyl-9H-carbazol-3-yl)-1 H-benzimidazol-5-carbonsäure

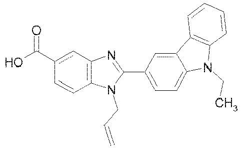

575 mg (1.40 mmol) Methyl-1-allyl-2-(9-ethyl-9H-carbazol-3-yl)-1 H-benzimidazol-5-carboxylat wurden in einer Mischung aus 7.5 ml Ethanol und 3 ml Dichlormethan gelöst, mit 11.2 ml 1.0 M Natronlauge versetzt und für 20 h auf 80 °C erhitzt. Nach dem Abkühlen auf RT wurde mit 1 M Salzsäure auf pH 2 angesäuert, mehrmals mit Ethylacetat extrahiert und die vereinigten organischen Phasen bis zur Trockene eingeengt. So wurden 389 mg (70%) der Titelverbindung erhalten.
¹H-NMR (400MHz, DMSO-d₆): d [ppm]= 1.38 (t, 3H), 4.57 (q, 2H), 5.12 - 5.42 (m, 3H), 5.35 (d, 1 H), 6.13 - 6.28 (m, 1H), 7.33 (t, 1H), 7.58 (td, 1H), 7.76 (d, 1H), 7.91 - 7.97 (m, 3H), 8.11 (dd, 1H), 8.26 (d, 1 H), 8.35 (d, 1 H), 8.74 (s, 1 H), 12.60 - 14.10 (1 H).

### Beispiel 3

### Methyl-2-(9-ethyl-9H-carbazol-3-yl)-1-(2-methoxyethyl)-1 H-benzimidazol-5-carboxylat

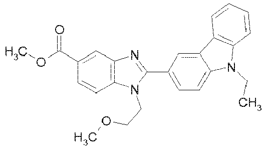

In Analogie zu Beispiel 1 wurden aus 3.0 g (8.12 mmol) Methyl-2-(9-ethyl-9H-carbazol-3-yl)-1 H-benzimidazol-5-carboxylat und 1.15 ml (12.18 mmol) 2-Bromethyl-methylether 721 mg (21%) der Titelverbindung und 761 mg (22%) des isomeren Methyl-2-(9-ethyl-9H-carbazol-3-yl)-1-(2-methoxyethyl)-1 H-benzimidazol-6-carboxylat erhalten.

### Methyl-2-(9-ethyl-9H-carbazol-3-yl)-1-(2-methoxyethyl)-1 H-benzimidazol-5-carboxylat

¹H-NMR (300 MHz ,CHLOROFORM-d), δ [ppm]= 1.50 (t, 3H), 3.33 (s, 3H), 3.84 (t, 2H), 3.98 (s, 3H), 4.45 (q, 2H), 4.53 (t, 2H), 7.29 - 7.34 (m, 1 H), 7.46 - 7.59 (m, 4H), 7.92 (dd, 1 H), 8.06 (dd, 1 H), 8.15 (d, 1 H), 8.56 (d, 1 H), 8.62 (d, 1 H).

### Methyl-2-(9-ethyl-9H-carbazol-3-yl)-1-(2-methoxyethyl)-1 H-benzimidazol-6-carboxylat

¹H-NMR (300 MHz ,CHLOROFORM-d), δ [ppm]= 1.50 (3H), 3.34 (3H), 3.88 (2H), 3.99 (3H), 4.44 (2H), 4.56 (2H), 7.28-7.33 (1 H), 7.45-7.58 (3H), 7.85 (1 H), 7.97 (1 H), 8.04 (1 H), 8.14 (1 H), 8.24 (1 H), 8.67 (1 H)

### Beispiel 4

### 2-(9-Ethyl-9H-carbazol-3-yl)-1-(2-methoxyethyl)-1H-benzimidazol-5-carbonsäure

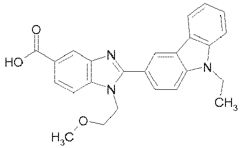

### Variante A

In Analogie zu Beispiel 2 wurden aus 685 mg (1.60 mmol) Methyl-2-(9-ethyl-9H-carbazol-3-yl)-1-(2-methoxyethyl)-1 H-benzimidazol-5-carboxylat 93 mg (14%) der Titelverbindung erhalten.
¹H-NMR (400 MHz, DMSO-d6), δ [ppm]= 1.38 (t, 3H), 3.14 (s, 3H), 3.73 (t, 2H), 4.53 (q, 2H), 4.59 (t, 2H), 7.27 (t, 1 H), 7.49 - 7.56 (m, 1 H), 7.69 (d, 1 H), 7.79 (t, 2H), 7.88 - 7.97 (m, 2H), 8.24 - 8.30 (m, 2H), 8.68 (d, 1 H), 12.40 - 13.00 (1 H).

### Beispiel 4

### Variante B

### 2-(9-Ethyl-9H-carbazol-3-yl)-1-(2-methoxyethyl)-1H-benzimidazol-5-carbonsäure

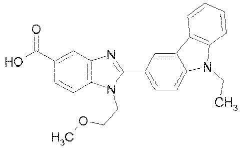

4.78 g (25.19 mmol) Natriumdisulfit wurden in 11 ml Wasser gegeben und dann mit einer Lösung von 2.5 g (11.20 mmol) 9-Ethyl-9H-carbazol-3-carbaldehyd in 25 ml THF versetzt. Danach wurden 3.53 g (16.80 mmol) 3-Amino-4-[(2-methoxyethyl)amino]benzoesäure in 10 ml THF hinzugefügt, 10 min bei RT gerührt und anschließend 2.5 h zum Rückfluss erhitzt. Nach dem Abkühlen wurde das Reaktionsgemisch mit 7.5 ml Wasser versetzt, mit 1 M Salzsäure auf pH 2 angesäuert und dreimal mit Dichlormethan extrahiert. Die gesammelten organischen Phasen wurden mit Wasser gewaschen, mit Natriumsulfat getrocknet und bis zur Trockene eingeengt. Nach Digerieren des Eindampfrückstandes mit Ethylacetat erhielt man 3.99 g (86%) der Titelverbindung.
¹H-NMR (400 MHz, DMSO-d6), δ [ppm]= 1.38 (t, 3H), 3.14 (s, 3H), 3.73 (t, 2H), 4.53 (q, 2H), 4.59 (t, 2H), 7.27 (t, 1 H), 7.49 - 7.56 (m, 1 H), 7.69 (d, 1 H), 7.79 (t, 2H), 7.88 - 7.97 (m, 2H), 8.24 - 8.30 (m, 2H), 8.68 (d, 1 H), 12.40 - 13.00 (1 H).

### Beispiel 5

### Methyl-1-(cyclopropylmethyl)-2-(9-ethyl-9H-carbazol-3-yl)-1 H-benzimidazol-5-carboxylat

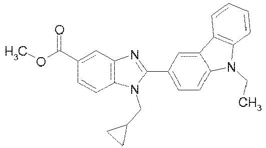

In Analogie zu Beispiel 1 wurden aus 20.4 g (8.12 mmol) Methyl-2-(9-ethyl-9H-carbazol-3-yl)-1 H-benzimidazol-5-carboxylat und 6.5 ml (66.3 mmol) (Brommethyl)cyclopropan 7.04 g (30%) der Titelverbindung und 5.96 g (25%) des isomeren Methyl-1-(cyclopropylmethyl)-2-(9-ethyl-9H-carbazol-3-yl)-1 H-benzimidazol-6-carboxylat erhalten. Methyl-1-(cyclopropylmethyl)-2-(9-ethyl-9H-carbazol-3-yl)-1 H-benzimidazol-5-carboxylat ¹H-NMR (300 MHz, DMSO-d6), δ [ppm]= 0.11 - 0.21 (m, 2H), 0.31 - 0.42 (m, 2H), 1.00 - 1.12 (m, 1H), 1.38 (t, 3H), 3.90 (s, 3H), 4.38 (d, 2H), 4.53 (q, 2H), 7.27 (t, 1H), 7.48 - 7.59 (m, 1 H), 7.69 (d, 1 H), 7.77 - 7.97 (m, 4H), 8.24 - 8.34 (m, 2H), 8.64 (d, 1 H). Methyl-1-(cyclopropylmethyl)-2-(9-ethyl-9H-carbazol-3-yl)-1 H-benzimidazol-6-carboxylat ¹H-NMR (300 MHz, DMSO-d6), δ [ppm]= 0.16 (q, 2H), 0.31 - 0.42 (m, 2H), 0.99 - 1.13 (m, 1H), 1.38 (t, 3H), 3.91 (s, 3H), 4.45 (d, 2H), 4.53 (q, 2H), 7.27 (t, 1H), 7.53 (t, 1H), 7.69 (d, 1 H), 7.75 - 7.85 (m, 2H), 7.87 - 7.95 (m, 2H), 8.28 - 8.37 (m, 2H), 8.66 (d, 1 H).

### Beispiel 6

### 1-(Cyclopropylmethyl)-2-(9-ethyl-9H-carbazol-3-yl)-1 H-benzimidazol-5-carbonsäure

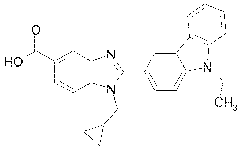

In Analogie zu Beispiel 4 / Variante A wurden aus 7.04 g (16.62 mmol) Methyl-1-(cyclopropylmethyl)-2-(9-ethyl-9H-carbazol-3-yl)-1H-benzimidazol-5-carboxylat 4.99 g (73%) der Titelverbindung erhalten.
¹H-NMR (300 MHz, DMSO-d6), δ [ppm]= 0.14 - 0.22 (m, 2H), 0.33 - 0.43 (m, 2H), 1.02 - 1.14 (m, 1H), 1.38 (t, 3H), 4.40 (d, 2H), 4.54 (q, 2H), 7.28 (t, 1H), 7.49 - 7.58 (m, 1H), 7.70 (d, 1 H), 7.80 - 7.99 (m, 4H), 8.26 - 8.35 (m, 2H), 8.66 (d, 1 H), 12.50 - 13.20 (1 H).

### Beispiel 7

### Methyl-4-chlor-1-(cyclopropylmethyl)-2-(9-ethyl-9H-carbazol-3-yl)-1 H-benzimidazol-5-carboxylat

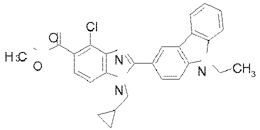

200 mg (0.38 mmol) (3-[5-Brom-4-chlor-1-(cyclopropylmethyl)-1 H-benzimidazol-2-yl]-9-ethyl-9H-carbazol wurden in 11 ml Methanol/DMSO (10:1) gelöst, mit 108 mg Bis(triphenylphosphin)-palladium-(II)-chlorid und 130 µl Triethylamin versetzt, und unter Kohlenmonoxid, bei 12.5bar und 100 °C, 48 h in einem Autoklaven gerührt. Anschließend wurde das Reaktionsgemisch abgekühlt, eingeengt, mit Wasser versetzt und der ausgefallene Feststoff abfiltriert. Der so erhaltene Rückstand wurde chromatographisch an Kieselgel (Hexan/Ethylacetat 7:3) gereinigt. So wurden 123 mg (68%) der Titelverbindung erhalten.
¹H-NMR (400 MHz, DMSO-d6), δ [ppm]= 0.16 (q, 2H), 0.33 - 0.39 (m, 2H), 0.99 - 1.10 (m, 1 H), 1.38 (t, 3H), 3.91 (s, 3H), 4.39 (d, 2H), 4.54 (q, 2H), 7.27 (t, 1 H), 7.51 - 7.56 (m, 1 H), 7.70 (d, 1 H), 7.78 - 7.85 (m, 3H), 7.89 - 7.93 (m, 1 H), 8.32 (d, 1 H), 8.65 (d, 1 H).

### Beispiel 8

### 4-Chlor-1-(cyclopropylmethyl)-2-(9-ethyl-9H-carbazol-3-yl)-1 H-benzimidazol-5-carbonsäure

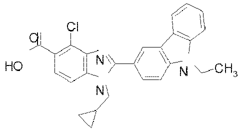

115 mg (0.25 mmol) Methyl-4-chlor-1-(cyclopropylmethyl)-2-(9-ethyl-9H-carbazol-3-yl)-1 H-benzimidazol-5-carboxylat wurden in 2.5 ml Methanol vorgelegt, dann mit 0.3 ml 2M Natronlauge versetzt und 1.5 h zum Rückfluss erhitzt. Nach dem Abkühlen wurde mit 2M Salzsäure auf pH 3 angesäuert und dreimal mit Dichlormethan extrahiert. Die organische Phase wurde filtriert, eingeengt und das so erhaltene Rohprodukt mittels HPLC aufgereinigt. Es wurden 88 mg (75%) der Titelverbindung erhalten.
¹H-NMR (300 MHz, DMSO-d6), δ [ppm]= 0.15 (q, 2H), 0.31 - 0.40 (m, 2H), 0.97 - 1.11 (m, 1 H), 1.38 (t, 3H), 4.38 (d, 2H), 4.54 (q, 2H), 7.27 (t, 1 H), 7.53 (t, 1 H), 7.70 (d, 1 H), 7.76 - 7.85 (m, 3H), 7.89 (d, 1H), 8.33 (d, 1H), 8.65 (d, 1 H), 12.95 - 13.20 (1H).

### Beispiel 9

### 1-(Cyclopropylmethyl)-2-(9-ethyl-9H-carbazol-3-yl)-4-methyl-1 H-benzimidazol-5-carbonsäure

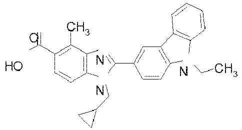

800 mg (3.41 mmol) Methyl-3-amino-4-[(cyclopropylmethyl)amino]-2-methylbenzoat und 381 mg (1.71 mmol) 9-Ethyl-9H-carbazol-3-carbaldehyd wurden in 32 ml Eisessig gegeben und unter Luftzufuhr 1 h zum Rückfluss erhitzt. Nach dem Abkühlen wurde die Mischung eingeengt und das so gewonnene rohe Methyl-1-(cyclopropylmethyl)-2-(9-ethyl-9H-carbazol-3-yl)-4-methyl-1 H-benzimidazol-5-carboxylat (1.4 g) ohne weitere Reinigung in die nächste Stufe eingesetzt.

1.4 g (3.2 mmol) rohes Methyl-1-(cyclopropylmethyl)-2-(9-ethyl-9H-carbazol-3-yl)-4-methyl-1 H-benzimidazol-5-carboxylat wurden in 9 ml Methanol vorgelegt, dann mit 4.3 ml 2M Natronlauge versetzt und 5 h zum Rückfluss erhitzt. Nach dem Abkühlen wurde mit Essigsäure auf pH 3 angesäuert, das ausgefallene Produkt abgetrennt und mittels präparativer HPLC aufgereinigt. Es wurden 227 mg (17%) der Titelverbindung erhalten. ¹H-NMR (400 MHz, DMSO-d6), δ [ppm]= 0.10 - 0.16 (m, 2H), 0.30 - 0.39 (m, 2H), 1.00 - 1.08 (m, 1 H), 1.38 (t, 3H), 2.89 (s, 3H), 4.32 (d, 2H), 4.53 (q, 2H), 7.26 (t, 1 H), 7.48 - 7.59 (m, 2H), 7.69 (d, 1H), 7.77 - 7.90 (m, 3H), 8.31 (d, 1H), 8.60 (d, 1H), 12.2 - 13.10 (1H).

### Beispiel 10

### 2-(9-Ethyl-7-fluor-9H-carbazol-3-yl)-1-(2-methoxyethyl)-1 H-benzimidazol-5-carbonsäure

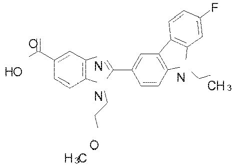

177 mg (2.25 mmol) Natriumdisulfit wurden in 0.5 ml Wasser gegeben und dann mit einer Lösung von 100 mg (0.41 mmol) 9-Ethyl-8-fluor-9H-carbazol-3-carbaldehyd in 1.0 ml THF versetzt. Danach wurden 131 mg (0.62 mmol) 3-Amino-4-[(2-methoxyethyl)-amino]benzoesäure in 0.5 ml THF hinzugefügt, 10 min bei RT gerührt und anschließend 1 h zum Rückfluss erhitzt. Das Reaktionsgemisch wurde mit 5 ml Wasser versetzt, mit 1M Salzsäure auf pH 2 angesäuert und der ausgefallene Niederschlag abfiltriert und getrocknet. Nach Aufreinigung mittels HPLC wurden 68 mg (38%) der Titelverbindung erhalten.
¹H-NMR (500 MHz, DMSO-d6), δ [ppm]= 1.36 (t, 3H), 3.13 (s, 3H), 3.71 (t, 2H), 4.47 - 4.61 (m, 4H), 7.07 - 7.12 (m, 1H), 7.60 (dd, 1H), 7.79 (t, 2H), 7.89 - 7.95 (m, 2H), 8.26 - 8.31 (m, 2H), 8.66 (d, 1H), 12.65 - 12.80 (1H).

### Beispiel 11

### 2-(9-Ethyl-5-fluor-9H-carbazol-3-yl)-1-(2-methoxyethyl)-1 H-benzimidazol-5-carbonsäure

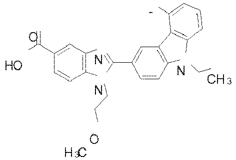

177 mg (2.25 mmol) Natriumdisulfit wurden in 0.5 ml Wasser gegeben und dann mit einer Lösung von 100 mg (0.41 mmol) 9-Ethyl-5-fluor-9H-carbazol-3-carbaldehyd in 1.0 ml THF versetzt. Danach wurden 131 mg (0.62 mmol) 3-Amino-4-[(2-methoxyethyl)amino]benzoesäure in 0.5 ml THF hinzugefügt, 10 min bei RT gerührt und anschließend 1 h zum Rückfluss erhitzt. Das Reaktionsgemisch wurde mit 5 ml Wasser versetzt, mit 1M Salzsäure auf pH 2 angesäuert und der ausgefallene Niederschlag abfiltriert und getrocknet. Nach Aufreinigung mittels HPLC wurden 32 mg (18%) der Titelverbindung erhalten.
¹H-NMR (500 MHz, DMSO-d6), δ [ppm]= 1.38 (t, 3H), 3.18 (s, 3H), 3.78 (t, 2H), 4.53 - 4.61 (m, 4H), 7.08 (dd, 1 H), 7.50 - 7.58 (m, 2H), 7.78 (d, 1 H), 7.85 - 7.94 (m, 2H), 8.02 (dd, 1 H), 8.28 (d, 1 H), 8.64 (d, 1 H), 12.72 (s, 1 H).

### Beispiel 12

### 2-(9-Ethyl-8-fluor-9H-carbazol-3-yl)-1-(2-methoxyethyl)-1 H-benzimidazol-5-carbonsäure

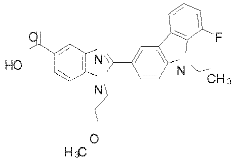

177 mg (2.25 mmol) Natriumdisulfit wurden in 0.5 ml Wasser gegeben und dann mit einer Lösung von 100 mg (0.41 mmol) 9-Ethyl-8-fluor-9H-carbazol-3-carbaldehyd in 1.0 ml THF versetzt. Danach wurden 131 mg (0.62 mmol) 3-Amino-4-[(2-methoxyethyl)amino]benzoesäure in 0.5 ml THF hinzugefügt, 10 min bei RT gerührt und anschließend 1 h zum Rückfluss erhitzt. Das Reaktionsgemisch wurde mit 5 ml Wasser versetzt, mit 1M Salzsäure auf pH 2 angesäuert und der ausgefallene Niederschlag abfiltriert und getrocknet. Nach Aufreinigung mittels HPLC wurden 51 mg (28%) der Titelverbindung erhalten.
¹H-NMR (500 MHz, DMSO-d6), δ [ppm]= 1.42 (t, 3H), 3.14 (s, 3H), 3.72 (t, 2H), 4.55 - 4.66 (m, 4H), 7.21 - 7.26 (m, 1 H), 7.36 (dd, 1 H), 7.79 (d, 1 H), 7.84 - 7.94 (m, 2H), 8.00 (dd, 1H), 8.12 (d, 1H), 8.27 (d, 1H), 8.71 (d, 1H), 12.60 - 12.90 (1H).

### Beispiel 13

### 1-(Cyclopropylmethyl)-2-[9-(2-methoxyethyl)-9H-carbazol-3-yl]-1 H-benzimidazol-5-carbonsäure

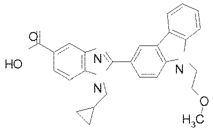

In Analogie zu Beispiel 9 wurden aus 67 mg (0.27 mmol) 9-(2-Methoxyethyl)-9H-carbazol-3-carbaldehyd und 109 mg (0.53 mmol) 3-Amino-4-[(cyclopropylmethyl)amino]benzoesäure 19 mg (19%) der Titelverbindung erhalten.
¹H-NMR (300 MHz ,CHLOROFORM-d), δ [ppm]= 0.17 - 0.26 (m, 2H), 0.50 - 0.59 (m, 2H), 1.15 - 1.30 (m, 1 H), 3.34 (s, 3H), 3.85 (t, 2H), 4.28 (d, 2H), 4.57 (t, 2H), 7.28 - 7.34 (m, 1 H), 7.51 - 7.64 (m, 4H), 7.85 (dd, 1 H), 8.11 - 8.18 (m, 2H), 8.51 (d, 1 H), 8.68 (d, 1H), 12.35- 12.85 (1H).

### Beispiel 14

### 1-(Cyclopropylmethyl)-2-(9-ethyl-9H-pyrido[2,3-b]indol-3-yl)-1 H-benzimidazol-5-carbonsäure

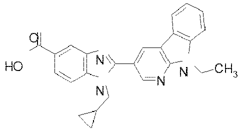

48 mg (0.21 mmol) 9-Ethyl-9H-pyrido[2,3-b]indol-3-carbaldehyd und 88 mg (0.43 mmol) 3-Amino-4-[(cyclopropylmethyl)amino]benzoesäure wurden in 4 ml Eisessig gegeben und unter Luftzufuhr 1 h zum Rückfluss erhitzt. Nach dem Abkühlen wurde die Mischung eingeengt und mittels präparativer HPLC gereinigt. Auf diese Weise wurden 24 mg (25%) der Titelverbindung erhalten.
¹H-NMR (300 MHz, DMSO-d6), δ [ppm]= 0.18 (q, 2H), 0.34 - 0.41 (m, 2H), 1.02 - 1.12 (1 H), 1.42 (t, 3H), 4.39 (d, 2H), 4.61 (q, 2H), 7.35 (t, 1 H), 7.61 (t, 1 H), 7.79 (d, 1 H), 7.83 - 7.88 (m, 1 H), 7.94 (dd, 1 H), 8.30 (d, 1 H), 8.37 (d, 1 H), 8.91 (d, 1 H), 9.04 (d, 1 H), 12.40 - 12.85 (1 H).

### Beispiel 15

### 2-(9-Ethyl-9H-carbazol-3-yl)-1-(2-methoxyethyl)-N-(methylsulfonyl)-1 H-benzimidazol-5-carboxamid

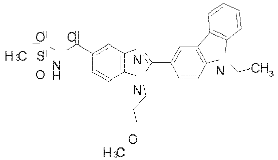

100 mg (0.24 mmol) 2-(9-Ethyl-9H-carbazol-3-yl)-1-(2-methoxyethyl)-1H-benzimidazol-5-carbonsäure wurden für 2 h in einer Mischung aus 0.11 ml (1.45 mmol) Thionylchlorid und 4 ml Toluol zum Rückfluss erhitzt und anschließend eingeengt. Der Rückstand wurde in 4 ml Dichlormethan aufgenommen und bei 0 °C zu einer Mischung aus 16 mg (0.36 mmol) Natriumhydrid (60%ige Mineralöldispersion) und 104 mg (1.08 mmol) Methansulfonamid in 2 ml DMF getropft. Nach Erwärmen auf RT wurde mit 1 M Salzsäure vorsichtig bis pH 3 angesäuert und dann mit Dichlormethan extrahiert. Die organische Phase wurde eingeengt und der Rückstand mittels präparativer HPLC aufgereinigt. Auf diese Weise wurden 25 mg (21 %) der Titelverbindung erhalten.
¹H-NMR (400 MHz, DMSO-d6), δ [ppm]= 1.38 (t, 3H), 3.13 (s, 3H), 3.39 (s, 3H), 3.72 (t, 2H), 4.50 - 4.63 (m, 4H), 7.27 (t, 1H), 7.53 (t, 1H), 7.69 (d, 1H), 7.81 (dd, 2H), 7.88 - 7.99 (m, 2H), 8.24 - 8.36 (m, 2H), 8.68 (s, 1 H), 12.09 (br. s., 1 H).

### Beispiel 16

### N-(Cyclopropylsulfonyl)-2-(9-ethyl-9H-carbazol-3-yl)-1-(2-methoxyethyl)-1H-benzimidazol-5-carboxamid

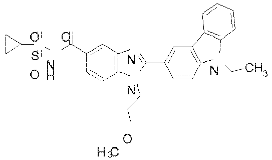

In Analogie zu Beispiel 15 wurden aus 300 mg (0.73 mmol) 2-(9-Ethyl-9H-carbazol-3-yl)-1-(2-methoxyethyl)-1 H-benzimidazol-5-carbonsäure 200 mg (51%) der Titelverbindung erhalten.
¹H-NMR (400 MHz, DMSO-d6), δ [ppm]= 1.11 - 1.22 (m, 4H), 1.38 (t, 3H), 3.13 (s, 3H), 3.16 - 3.23 (m, 1 H), 3.72 (t, 2H), 4.54 (q, 2H), 4.60 (t, 2H), 7.27 (t, 1 H), 7.50 - 7.56 (m, 1 H), 7.69 (d, 1 H), 7.79 - 7.85 (m, 2H), 7.89 - 7.97 (m, 2H), 8.28 (d, 1 H), 8.33 (d, 1 H), 8.69 (d, 1H), 12.03 (br. s., 1H).

### Beispiel 17

### N-[(3-Chlorphenyl)sulfonyl]-2-(9-ethyl-9H-carbazol-3-yl)-1-(2-methoxyethyl)-1 H-benzimidazol-5-carboxamid

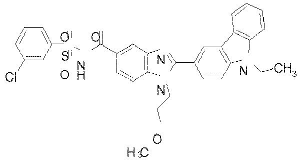

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure 10 mg (9%) der Titelverbindung erhalten.
¹H-NMR (400 MHz, DMSO-d6), δ [ppm]= 1.38 (t, 3H), 3.12 (s, 3H), 3.71 (t, 2H), 4.54 (q, 2H), 4.60 (t, 2H), 7.28 (t, 1 H), 7.51 - 7.56 (m, 1 H), 7.66 - 7.72 (m, 2H), 7.77 - 7.89 (m, 4H), 7.93 - 8.02 (m, 3H), 8.26 - 8.30 (m, 2H), 8.69 (s, 1H), 12.45 - 13.00 (1H).

### Beispiel 18

### 2-(9-Ethyl-9H-carbazol-3-yl)-N-(ethylsulfonyl)-1-(2-methoxyethyl)-1 H-benzimidazol-5-carboxamid

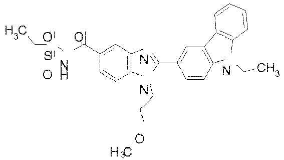

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure 45 mg (44%) der Titelverbindung erhalten.
¹H-NMR (400 MHz, DMSO-d6), δ [ppm]= 1.29 (t, 3H), 1.38 (t, 3H), 3.13 (s, 3H), 3.56 (q, 2H), 3.72 (t, 2H), 4.54 (q, 2H), 4.60 (t, 2H), 7.25 - 7.30 (m, 1 H), 7.50 - 7.56 (m, 1 H), 7.69 (d, 1 H), 7.79 - 7.85 (m, 2H), 7.89 - 7.97 (m, 2H), 8.28 (d, 1 H), 8.35 (d, 1 H), 8.68 (d, 1 H), 11.98 (br. s., 1 H).

### Beispiel 19

### 2-(9-Ethyl-9H-carbazol-3-yl)-1-(2-methoxyethyl)-N-[(4-methoxyphenyl)sulfonyl]-1 H-benzimidazol-5-carboxamid

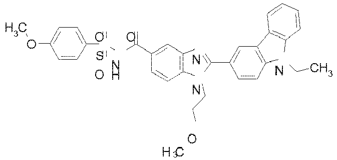

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure 45 mg (38%) der Titelverbindung erhalten.
¹H-NMR (400 MHz, DMSO-d6), δ [ppm]= 1.37 (t, 3H), 3.11 (s, 3H), 3.69 (t, 2H), 3.86 (s, 3H), 4.48 - 4.62 (m, 4H), 7.17 (d, 2H), 7.27 (t, 1 H), 7.50 - 7.55 (m, 1 H), 7.69 (d, 1 H), 7.76 - 7.83 (m, 3H), 7.91 - 8.01 (m, 3H), 8.24 - 8.29 (m, 2H), 8.67 (d, 1 H), 12.33 (br. s., 1 H).

### Beispiel 20

### 2-(9-Ethyl-9H-carbazol-3-yl)-1-(2-methoxyethyl)-N-({2-[4-(trifluormethyl)phenyl]ethyl}sulfonyl)-1H-benzimidazol-5-carboxamid

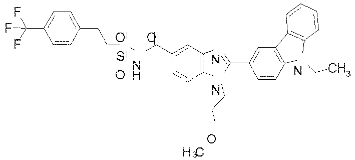

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure 70 mg (53%) der Titelverbindung erhalten.
¹H-NMR (400 MHz, DMSO-d6), δ [ppm]= 1.38 (t, 3H), 3.14 (s, 3H), 3.19 (t, 2H), 3.72 (t, 2H), 3.95 (t, 2H), 4.54 (q, 2H), 4.60 (t, 2H), 7.28 (t, 1 H), 7.50 - 7.56 (m, 3H), 7.60 - 7.64 (m, 2H), 7.70 (d, 1 H), 7.78 - 7.86 (m, 3H), 7.95 (dd, 1 H), 8.25 - 8.31 (m, 2H), 8.69 (d, 1 H), 11.90 - 12.20 (1 H).

### Beispiel 21

### 1-(Cyclopropylmethyl)-2-(9-ethyl-9H-carbazol-3-yl)-N-(2-methoxyethyl)-1 H-benzimidazol-5-carboxamid

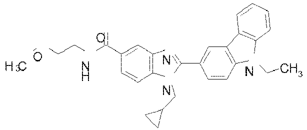

Nacheinander wurden 100 mg (0.24 mmol) 1-(Cyclopropylmethyl)-2-(9-ethyl-9H-carbazol-3-yl)-1 H-benzimidazol-5-carbonsäure, 70 mg (0.37 mmol) 1-(3-Dimethylaminopropyl)-3-ethylcarbodümidhydrochlorid (EDCI), 56 mg (0.37 mmol) 1-Hydroxy-1H-benzotriazol (HOBT-hydrat), 221 mg (1.71 mmol) N,N-Diisopropylethylamin und 92 mg (1.22 mmol) 2-Methoxyethanamin in 4.6 ml DMF gegeben und 3 h bei RT gerührt. Dann wurde das Reaktionsgemisch eingeengt, mit DMSO digeriert, filtriert und das Filtrat mittels präparativer HPLC getrennt. Auf diese Weise wurden 65 mg (56%) der Titelverbindung erhalten.
¹H-NMR (300 MHz, DMSO-d6), δ [ppm]= 0.16 (q, 2H), 0.32 - 0.40 (m, 2H), 1.05 (d, 1 H), 1.38 (t, 3H), 3.30 (s, 3H), 3.46 - 3.52 (m, 4H), 4.36 (d, 2H), 4.53 (q, 2H), 7.27 (t, 1H), 7.49 - 7.57 (m, 1 H), 7.69 (d, 1 H), 7.74 - 7.91 (m, 4H), 8.24 (d, 1 H), 8.30 (d, 1 H), 8.48 - 8.55 (m, 1 H), 8.62 (d, 1 H).

### Beispiel 22

### [1-(Cyclopropylmethyl)-2-(9-ethyl-9H-carbazol-3-yl)-1H-benzimidazol-5-yl](pyrrolidin-1-yl)methanon

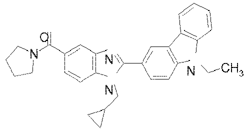

In Analogie zu Beispiel 21 wurden aus 100 mg (0.24 mmol) 1-(Cyclopropylmethyl)-2-(9-ethyl-9H-carbazol-3-yl)-1 H-benzimidazol-5-carbonsäure und 86 mg (1.22 mmol) Pyrrolidin 50 mg (44%) der Titelverbindung erhalten.
¹H-NMR (300 MHz, DMSO-d6), δ [ppm]= 0.16 (q, 2H), 0.33 - 0.42 (m, 2H), 1.01 - 1.14 (m, 1 H), 1.38 (t, 3H), 1.87 (d, 4H), 3.47 - 3.56 (m, 4H), 4.35 (d, 2H), 4.53 (q, 2H), 7.26 (t, 1 H), 7.44 - 7.56 (m, 2H), 7.66 - 7.91 (m, 5H), 8.30 (d, 1 H), 8.62 (d, 1 H).

### Beispiel 23

### [1-(Cyclopropylmethyl)-2-(9-ethyl-9H-carbazol-3-yl)-1 H-benzimidazol-5-yl](3-hydroxyazetidin-1-yl)methanon

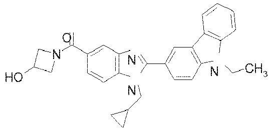

In Analogie zu Beispiel 21 wurden aus 100 mg (0.24 mmol) 1-(Cyclopropylmethyl)-2-(9-ethyl-9H-carbazol-3-yl)-1 H-benzimidazol-5-carbonsäure und 40 mg (1.22 mmol) 3-Hydroxyazetidinhydrochlorid 65 mg (54%) der Titelverbindung erhalten.
¹H-NMR (400 MHz, DMSO-d6), δ [ppm]= 0.16 - 0.22 (m, 2H), 0.36 - 0.43 (m, 2H), 1.05 - 1.14 (m, 1H), 1.38 (t, 3H), 3.85 (br. s., 1H), 4.11 (br. s., 1H), 4.30 (br. s., 1H), 4.40 (d, 2H), 4.50 - 4.60 (m, 4H), 5.55 - 5.95 (1 H), 7.28 (t, 1 H), 7.51 - 7.57 (m, 1 H), 7.63 - 7.73 (m, 2H), 7.82 - 7.96 (m, 4H), 8.30 (d, 1 H), 8.66 (d, 1 H).

### Beispiel 24

### 1-(Cyclopropylmethyl)-2-(9-ethyl-9H-carbazol-3-yl)-N-methyl-1 H-benzimidazol-5-carboxamid

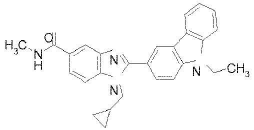

In Analogie zu Beispiel 21 wurden aus 100 mg (0.24 mmol) 1-(Cyclopropylmethyl)-2-(9-ethyl-9H-carbazol-3-yl)-1 H-benzimidazol-5-carbonsäure und Methylamin 60 mg (58%) der Titelverbindung erhalten.
¹H-NMR (400 MHz, DMSO-d6), δ [ppm]= 0.13 - 0.18 (m, 2H), 0.33 - 0.39 (m, 2H), 1.01 - 1.12 (m, 1H), 1.38 (t, 3H), 2.84 (d, 3H), 4.35 (d, 2H), 4.53 (q, 2H), 7.24 - 7.29 (m, 1H), 7.53 (td, 1 H), 7.69 (d, 1 H), 7.75 - 7.90 (m, 4H), 8.21 (d, 1 H), 8.30 (d, 1 H), 8.43 (q, 1 H), 8.62 (d, 1 H).

### Beispiel 25

### 1-(Cyclopropylmethyl)-2-(9-ethyl-9H-carbazol-3-yl)-N,N-dimethyl-1 H-benzimidazol-5-carboxamid

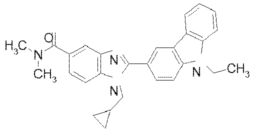

In Analogie zu Beispiel 21 wurden aus 100 mg (0.24 mmol) 1-(Cyclopropylmethyl)-2-(9-ethyl-9H-carbazol-3-yl)-1H-benzimidazol-5-carbonsäure und Dimethylamin 55 mg (51%) der Titelverbindung erhalten.
¹H-NMR (400 MHz, DMSO-d6), δ [ppm]= 0.17 (q, 2H), 0.34 - 0.42 (m, 2H), 1.02 - 1.14 (m, 1H), 1.38 (t, 3H), 3.02 (s, 6H), 4.35 (d, 2H), 4.53 (q, 2H), 7.26 (t, 1H), 7.34 (dd, 1H), 7.52 (t, 1H), 7.66 - 7.83 (m, 4H), 7.86 - 7.90 (m, 1H), 8.30 (d, 1H), 8.62 (d, 1H).

### Beispiel 26

### 1-(Cyclopropylmethyl)-2-(9-ethyl-9H-carbazol-3-yl)-1 H-benzimidazol-5-carboxamid

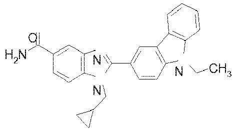

In Analogie zu Beispiel 21 wurden aus 100 mg (0.24 mmol) 1-(Cyclopropylmethyl)-2-(9-ethyl-9H-carbazol-3-yl)-1H-benzimidazol-5-carbonsäure und 65 mg (1.22 mmol) Ammoniumchlorid 70 mg (69%) der Titelverbindung erhalten.
¹H-NMR (400 MHz, DMSO-d6), δ [ppm]= 0.13 - 0.19 (m, 2H), 0.33 - 0.40 (m, 2H), 1.00 - 1.12 (m, 1H), 1.38 (t, 3H), 4.36 (d, 2H), 4.53 (q, 2H), 7.23 - 7.29 (m, 2H), 7.50 - 7.55 (m, 1H), 7.69 (d, 1H), 7.74 - 7.83 (m, 2H), 7.84 - 7.91 (m, 2H), 7.99 (br. s., 1H), 8.25 - 8.33 (m, 2H), 8.62 (d, 1H).

### Beispiel 27

### 1-(Cyclopropylmethyl)-2-(9-ethyl-9H-carbazol-3-yl)-N-(2,2,2-trifluorethyl)-1 H-benzimidazol-5-carboxamid

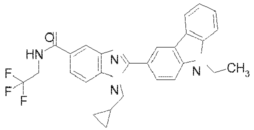

In Analogie zu Beispiel 21 wurden aus 100 mg (0.24 mmol) 1-(Cyclopropylmethyl)-2-(9-ethyl-9H-carbazol-3-yl)-1 H-benzimidazol-5-carbonsäure und 121 mg (1.22 mmol) 2,2,2-Trifluorethanamin 100 mg (83%) der Titelverbindung erhalten.
¹H-NMR (300 MHz, DMSO-d6), δ [ppm]= 0.12 - 0.20 (m, 2H), 0.31 - 0.40 (m, 2H), 0.99 - 1.12 (m, 1H), 1.38 (t, 3H), 4.06 - 4.21 (m, 2H), 4.37 (d, 2H), 4.54 (q, 2H), 7.27 (t, 1H), 7.48 - 7.57 (m, 1H), 7.69 (d, 1H), 7.78 - 7.92 (m, 4H), 8.27 - 8.33 (m, 2H), 8.63 (d, 1H), 9.08 (t, 1H).

### Beispiel 28

### 4-{[1-(Cyclopropylmethyl)-2-(9-ethyl-9H-carbazol-3-yl)-1H-benzimidazol-5-yl]carbonyl}piperazin-2-on

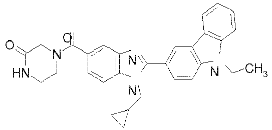

In Analogie zu Beispiel 21 wurden aus 100 mg (0.24 mmol) 1-(Cyclopropylmethyl)-2-(9-ethyl-9H-carbazol-3-yl)-1H-benzimidazol-5-carbonsäure und 122 mg (1.22 mmol) Piperazin-2-on 45 mg (37%) der Titelverbindung erhalten.
¹H-NMR (300 MHz, DMSO-d6), δ [ppm]= 0.17 (q, 2H), 0.33 - 0.42 (m, 2H), 1.03 - 1.13 (m, 1H), 1.38 (t, 3H), 3.20 - 3.30 (m, 1H), 3.60 - 3.80 (m, 2H), 4.10 (s, 2H), 4.37 (d, 2H), 4.53 (q, 2H), 7.27 (t, 1H), 7.38 (dd, 1H), 7.53 (t, 1 H), 7.69 (d, 1H), 7.76 - 7.84 (m, 3H), 7.86 - 7.92 (m, 1H), 8.13 (d, 1H), 8.30 (d, 1H), 8.63 (d, 1H).

### Beispiel 29

### [1-(Cyclopropylmethyl)-2-(9-ethyl-9H-carbazol-3-yl)-1 H-benzimidazol-5-yl](morpholin-4-yl)methanon

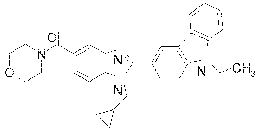

In Analogie zu Beispiel 21 wurden aus 100 mg (0.24 mmol) 1-(Cyclopropylmethyl)-2-(9-ethyl-9H-carbazol-3-yl)-1H-benzimidazol-5-carbonsäure und 106 mg (1.22 mmol) Morpholin 70 mg (60%) der Titelverbindung erhalten.
¹H-NMR (300 MHz, DMSO-d6), δ [ppm]= 0.13 - 0.20 (m, 2H), 0.33 - 0.41 (m, 2H), 1.01 - 1.15 (m, 1H), 1.38 (t, 3H), 3.49 - 3.71 (m, 8H), 4.36 (d, 2H), 4.53 (q, 2H), 7.27 (t, 1H), 7.35 (dd, 1H), 7.49 - 7.57 (m, 1H), 7.69 (d, 1H), 7.73 (d, 1H), 7.76 - 7.83 (m, 2H), 7.85 - 7.91 (m, 1H), 8.30 (d, 1H), 8.62 (d, 1H).

### Beispiel 30

### Azetidin-1-yl[2-(9-ethyl-9H-carbazol-3-yl)-1-(2-methoxyethyl)-1H-benzimidazol-5-yl]methanon

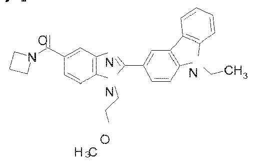

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 69 mg (1.22 mmol) Azetidin 33 mg (28%) der Titelverbindung erhalten.
¹H-NMR (300 MHz, DMSO-d6), δ [ppm]= 1.37 (t, 3H), 2.29 (2H), 3.15 (s, 3H), 3.73 (t, 2H), 4.09 (br., 2H), 4.40 (br., 2H), 4.48 - 4.61 (m, 4H), 7.27 (t, 1H), 7.49 - 7.61 (m, 2H), 7.66 - 7.83 (m, 3H), 7.90 - 7.97 (m, 2H), 8.27 (d, 1H), 8.68 (d, 1H).

### Beispiel 31

### 2-(9-Ethyl-9H-carbazol-3-yl)-N,1-bis(2-methoxyethyl)-1H-benzimidazol-5-carboxamid

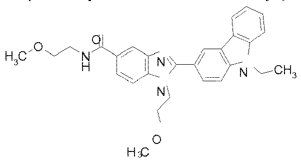

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 91 mg (1.21 mmol) 2-Methoxyethanamin 33 mg (29%) der Titelverbindung erhalten.
¹H-NMR (300 MHz, DMSO-d6), δ [ppm]= 1.38 (t, 3H), 3.14 (s, 3H), 3.30 (s, 3H), 3.44 - 3.54 (m, 4H), 3.72 (t, 2H), 4.48 - 4.61 (m, 4H), 7.27 (t, 1H), 7.49 - 7.56 (m, 1H), 7.66 - 7.86 (m, 4H), 7.94 (dd, 1H), 8.21 - 8.30 (m, 2H), 8.48 - 8.55 (m, 1H), 8.67 (d, 1H).

### Beispiel 32

### 2-(9-Ethyl-6-methoxy-9H-carbazol-3-yl)-1-(2-methoxyethyl)-1 H-benzimidazol-5-carbonsäure

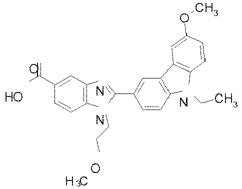

84 mg (0.44 mmol) Natriumdisulfit wurden in 1 ml Wasser gegeben, 5 min bei RT gerührt und dann mit einer Lösung von 50 mg (0.20 mmol) 9-Ethyl-6-methoxy-9H-carbazol-3-carbaldehyd in 1 ml THF versetzt. Danach wurden 62 mg (0.30 mmol) 3-Amino-4-[(2-methoxyethyl)amino]benzoesäure in 1 ml THF hinzugefügt, 10 min bei RT gerührt und anschließend 1 h zum Rückfluss erhitzt. Das Reaktionsgemisch wurde auf gesättigte Natriumhydrogencarbonatlösung gegeben und dreimal mit Ethylacetat extrahiert. Die organischen Phasen wurden eingeengt und der Rückstand mittels HPLC gereinigt. So wurden 20 mg (14%) der Titelverbindung erhalten.
¹H-NMR (300 MHz, DMSO-d6), δ [ppm]= 1.35 (t, 3H), 3.13 (s, 3H), 3.72 (t, 2H), 3.87 (s, 3H), 4.49 (d, 2H), 4.60 (br., 2H), 7.15 (dd, 1H), 7.59 (d, 1H), 7.76 (m, 2H), 7.84 - 7.97 (m, 3H), 8.26 (s, 1H), 8.67 (s, 1H), 12.00 - 13.30 (1H).

### Beispiel 33

### 2-(9-Allyl-9H-carbazol-3-yl)-1-(cyclopropylmethyl)-1 H-benzimidazol-5-carbonsäure

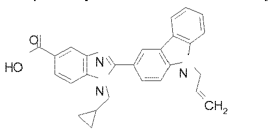

In Analogie zu Beispiel 9 wurden aus 57 mg (0.24 mmol) 9-Allyl-9H-carbazol-3-carbaldehyd und 100 mg (0.49 mmol) 3-Amino-4-[(cyclopropylmethyl)amino]benzoesäure 19 mg (18%) der Titelverbindung erhalten.
¹H-NMR (300 MHz ,CHLOROFORM-d), δ [ppm]= 0.18 - 0.27 (m, 2H), 0.50 - 0.60 (m, 2H), 1.23 (t, 1H), 4.29 (d, 2H), 5.00 (d, 2H), 5.12 (dd, 1H), 5.24 (dd, 1H), 6.06 (ddt, 1H), 7.28 - 7.35 (m, 1H), 7.43 - 7.48 (m, 1H), 7.50 - 7.58 (m, 3H), 7.84 (dd, 1H), 8.12 - 8.19 (m, 2H), 8.54 (d, 1H), 8.69 (d, 1H), COOH nicht angegeben.

### Beispiel 34

### 1-(Cyclopropylmethyl)-2-(9-methyl-9H-carbazol-3-yl)-1H-benzimidazol-5-carbonsäure

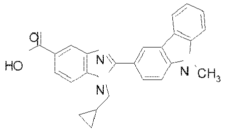

In Analogie zu Beispiel 32 wurden aus 101 mg (0.49 mmol) 9-Methyl-9H-carbazol-3-carbaldehyd und 150 mg (0.73 mmol) 3-Amino-4-[(cyclopropylmethyl)amino]-benzoesäure 55 mg (28%) der Titelverbindung erhalten.
¹H-NMR (300 MHz, DMSO-d6), δ [ppm]= 0.13 (q, 2H), 0.31 - 0.39 (m, 2H), 0.97 - 1.12 (m, 1H), 3.97 (s, 3H), 4.37 (d, 2H), 7.27 (t, 1H), 7.51 - 7.58 (m, 1H), 7.68 (d, 1H), 7.77 - 7.84 (m, 2H), 7.87 - 7.94 (m, 2H), 8.25 - 8.34 (m, 2H), 8.64 (d, 1H), 12.35 - 13.00 (1H).

### Beispiel 35

### 1-(Cyclopropylmethyl)-2-[9-(cyclopropylmethyl)-9H-carbazol-3-yl]-1 H-benzimidazol-5-carbonsäure

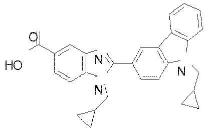

In Analogie zu Beispiel 9 wurden aus 60 mg (0.24 mmol) 9-(Cyclopropylmethyl)-9H-carbazol-3-carbaldehyd und 100 mg (0.49 mmol) 3-Amino-4-[(cyclopropylmethyl)amino]benzoesäure 14 mg (13%) der Titelverbindung erhalten.
¹H-NMR (300 MHz ,CHLOROFORM-d), δ [ppm]= 0.19 - 0.27 (m, 2H), 0.38 - 0.66 (m, 6H), 1.17 - 1.30 (m, 1 H), 1.31 - 1.47 (m, 1 H), 4.23 - 4.35 (m, 4H), 7.27 - 7.34 (m, 1H), 7.40 - 7.61 (m, 4H), 7.86 (dd, 1H), 8.08 - 8.19 (m, 2H), 8.53 (d, 1H), 8.70 (s, 1H), COOH nicht angegeben.

### Beispiel 36

### 2-[9-(Cyclopropylmethyl)-9H-carbazol-3-yl]-1-(2-methoxyethyl)-1H-benzimidazol-5-carbonsäure

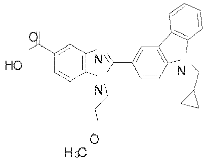

In Analogie zu Beispiel 9 wurden aus 59 mg (0.24 mmol) 9-(Cyclopropylmethyl)-9H-carbazol-3-carbaldehyd und 100 mg (0.48 mmol) 3-Amino-4-[(2-methoxyethyl)amino]benzoesäure6 mg (6%) der Titelverbindung erhalten.
¹H-NMR (300 MHz ,CHLOROFORM-d), δ [ppm]= 0.42 - 0.49 (m, 2H), 0.56 - 0.65 (m, 2H), 1.33 - 1.45 (m, 1H), 3.34 (s, 3H), 3.85 (t, 2H), 4.32 (d, 2H), 4.55 (t, 2H), 7.27 - 7.34 (m, 3H), 7.47 - 7.62 (m, 4H), 7.94 (dd, 1H), 8.10 - 8.19 (m, 1H), 8.61 - 8.71 (m, 1H), COOH nicht angegeben.

### Beispiel 37

### Ethyl-2-(9-ethyl-9H-carbazol-3-yl)-1-[2-(pyrrolidin-1-yl)ethyl]-1H-benzimidazol-5-carboxylat

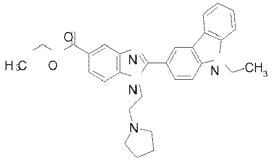

In Analogie zu Beispiel 4 / Variante B wurden aus 569 mg (2.55 mmol) 9-Ethyl-9H-carbazol-3-carbaldehyd und 1.14 g (3.8 mmol) rohem Ethyl-3-amino-4-{[2-(pyrrolidin-1-yl)ethyl]amino}benzoat 1.6 g (87%) der Titelverbindung erhalten.
¹H-NMR (400MHz, DMSO-d6): δ [ppm]= 1.33 (t, 6H), 1.46 (t, 4H), 2.20 (br. s., 4H), 2.75 (t, 2H), 4.32 (q, 2H), 4.47 - 4.54 (m, 4H), 7.23 (t, 1H), 7.49 (t, 1H), 7.66 (d, 1H), 7.73 - 7.80 (m, 2H), 7.89 (ddd, 2H), 8.21 - 8.26 (m, 2H), 8.62 (d, 1H).

### Beispiel 38

### 2-(9-Ethyl-9H-carbazol-3-yl)-1-[2-(pyrrolidin-1-yl)ethyl]-1H-benzimidazol-5-carbonsäure

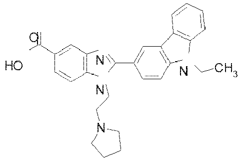

In Analogie zu Beispiel 2 wurden aus 1.5 g (3.1 mmol) Ethyl-2-(9-ethyl-9H-carbazol-3-yl)-1-[2-(pyrrolidin-1-yl)ethyl]-1 H-benzimidazol-5-carboxylat durch Verseifung mit Natronlauge 0.59 g (42%) der Titelverbindung erhalten.
¹H-NMR (400 MHz, DMSO-d6), δ [ppm]= 1.37 (t, 3H), 1.47 - 1.54 (m, 4H), 2.25 (br., 4H), 2.79 (t, 2H), 4.48 - 4.58 (m, 4H), 7.27 (t, 1H), 7.49 - 7.56 (m, 1H), 7.69 (d, 1H), 7.76 (d, 1H), 7.81 (d, 1H), 7.89 - 7.96 (m, 2H), 8.24 - 8.30 (m, 2H), 8.66 (d, 1H), 12.20-13.30 (br., 1H).

### Beispiel 39

### N-(tert-Butylsulfonyl)-2-(9-ethyl-9H-carbazol-3-yl)-1-(2-methoxyethyl)-1H-benzimidazol-5-carboxamid

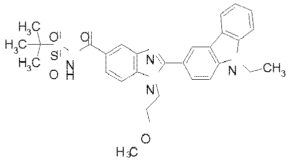

In Analogie zu Beispiel 21 wurden aus 100 mg (0.24 mmol) 2-(9-Ethyl-9H-carbazol-3-yl)-1-(2-methoxyethyl)-1H-benzimidazol-5-carbonsäure und 149 mg (1.09 mmol) 2-Methylpropan-2-sulfonamid 72 mg (55%) der Titelverbindung erhalten.
¹H-NMR (400 MHz, DMSO-d6), δ [ppm]= 1.38 (t, 3H), 1.44 (s, 9H), 3.13 (s, 3H), 3.72 (t, 2H), 4.53 (q, 2H), 4.60 (t, 2H), 7.25 - 7.30 (m, 1 H), 7.50 - 7.56 (m, 1 H), 7.69 (d, 1H), 7.78 - 7.88 (m, 3H), 7.95 (dd, 1H), 8.25 - 8.31 (m, 2H), 8.68 (d, 1H), 11.52 (br. s., 1H).

### Beispiel 40

### 2-(9-Ethyl-9H-carbazol-3-yl)-1-(2-methoxyethyl)-N-[(1-methylcyclopropyl)sulfonyl]-1H-benzimidazol-5-carboxamid

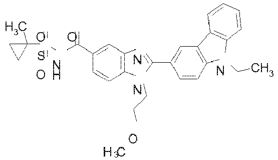

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 147 mg (1.09 mmol) 1-Methylcyclopropansulfonamid 46 mg (35%) der Titelverbindung erhalten.
¹H-NMR (400 MHz, DMSO-d6), δ [ppm]= 0.98 - 1.03 (m, 2H), 1.38 (t, 3H), 1.50 -1.55 (m, 5H), 3.13 (s, 3H), 3.72 (t, 2H), 4.54 (q, 2H), 4.60 (t, 2H), 7.27 (t, 1H), 7.53 (t, 1H), 7.69 (d, 1 H), 7.79 - 7.84 (dd, 2H), 7.88 - 7.92 (m, 1H), 7.95 (dd, 1H), 8.28 (d, 1H), 8.34 (d, 1H), 8.68 (d, 1H), 11.84 (br. s., 1H).

### Beispiel 41

### 2-(9-Ethyl-9H-carbazol-3-yl)-1-(2-methoxyethyl)-N-methyl-1H-benzimidazol-5-carboxamid

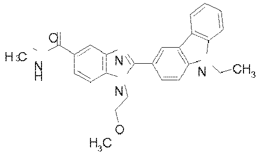

In Analogie zu Beispiel 21 wurden aus 100 mg (0.24 mmol) 2-(9-Ethyl-9H-carbazol-3-yl)-1-(2-methoxyethyl)-1H-benzimidazol-5-carbonsäure und 38 mg (1.3 mmol) Methylamin-Hydrochlorid 10 mg (10%) der Titelverbindung erhalten.
¹H-NMR (300 MHz, DMSO-d6), δ [ppm]= 1.38 (t, 3H), 2.84 (d, 3H), 3.14 (s, 3H), 3.73 (t, 2H), 4.49 - 4.60 (m, 4H), 7.27 (t, 1H), 7.49 - 7.56 (m, 1H), 7.66 - 7.84 (m, 4H), 7.94 (dd, 1H), 8.20 (d, 1H), 8.27 (d, 1H), 8.43 (d, 1H), 8.67 (d, 1H).

### Beispiel 42

### 2-(5-Ethyl-5H-pyrido[3,2-b]indol-2-yl)-1-(2-methoxyethyl)-1H-benzimidazol-5-carbonsäure

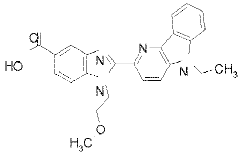

In Analogie zu Beispiel 14 wurden aus 50 mg (0.22 mmol) 5-Ethyl-5H-pyrido[3,2-b]indol-2-carbaldehyd und 141 mg (0.67 mmol) 3-Amino-4-[(2-methoxyethyl)amino]benzoesäure 9 mg (9%) der Titelverbindung erhalten.
¹H-NMR (300 MHz, DMSO-d6), δ [ppm]= 1.39 (t, 3H), 3.21 (s, 3H), 3.99 (t, 2H), 4.57 (q, 2H), 5.19 (t, 2H), 7.39 (t, 1H), 7.62 - 7.69 (m, 1H), 7.79 (s, 2H), 7.90 - 7.95 (m, 1H), 8.26 - 8.33 (m, 3H), 8.51 (d, 1H), 12.65 - 12.82 (br., 1H).

### Beispiel 43

### 1-(Cyclopropylmethyl)-2-(9-ethyl-6-methoxy-9H-carbazol-3-yl)-1 H-benzimidazol-5-carbonsäure

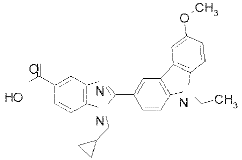

67 mg (0.30 mmol) Ethyl-3-amino-4-[(cyclopropylmethyl)amino]benzoat und 38 mg (0.15 mmol) 9-Ethyl-6-methoxy-9H-carbazol-3-carbaldehyd wurden in 1.4 ml Eisessig gegeben und unter Luftzufuhr 1 h zum Rückfluss erhitzt. Danach wurde das Reaktionsgemisch auf gesättigte Natriumchloridlösung gegeben, der entstandene Niederschlag zunächst abgetrennt (40 mg) und dann mittels präparativer HPLC gereinigt. Auf diese Weise wurden 13 mg (9%) der Titelverbindung erhalten.
¹H-NMR (300 MHz ,DMSO-d6), δ [ppm]= 0.15 (q, 2H), 0.30 - 0.39 (m, 2H), 0.99 - 1.10 (m, 1H), 1.35 (t, 3H), 3.86 (s, 3H), 4.39 (d, 2H), 4.44 - 4.55 (m, 2H), 7.15 (dd, 1H), 7.60 (d, 1H), 7.73 - 7.78 (m, 1H), 7.80 - 7.94 (m, 4H), 8.27 (s, 1H), 8.64 (s, 1H), 12.77 (br. s., 1H).

### Beispiel 44

### 2-(9-Ethyl-9H-carbazol-3-yl)-1-(2-methoxyethyl)-1H-benzimidazol-5-carbonitril

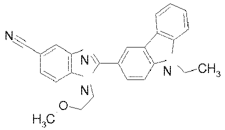

Eine Mischung aus 500 mg (1.1mmol) 3-[5-Brom-1-(2-methoxyethyl)-1H-benzimidazol-2-yl]-9-ethyl-9H-carbazol, 124 mg (1.1 mmol) Zinkcyanid und 64 mg (0.06 mmol) Tetrakis(triphenylphosphin)palladium(0) in 10 ml DMF wurden unter Argon 16h bei 80 °C gerührt. Nach dem Abkühlen wurde zum Reaktionsgemisch Wasser hinzugefügt und mit Ethylacetat extrahiert. Die organische Phase wurde mit gesättigter Natriumchloridlösung gewaschen, eingeengt und der erhaltene Rückstand an Kieselgel chromatographiert (Hexan/Ethylacetat 1:0 -> 2:3). So wurden 350 mg (80%) der Titelverbindung erhalten.
¹H-NMR (300MHz, DMSO-d6): δ [ppm]= 1.37 (t, 3H), 3.13 (s, 3H), 3.71 (t, 2H), 4.53 (d, 2H), 4.62 (t, 2H), 7.27 (t, 1H), 7.49 - 7.56 (m, 1H), 7.69 (dt, 2H), 7.81 (d, 1H), 7.89 - 7.98 (m, 2H), 8.21 - 8.30 (m, 2H), 8.69 (d, 1H).

### Beispiel 45

### 9-Ethyl-3-[1-(2-methoxyethyl)-5-(1H-tetrazol-5-yl)-1H-benzimidazol-2-yl]-9H-carbazol

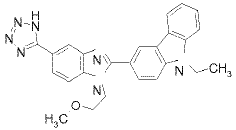

Eine Mischung aus 25 mg (0.11 mmol) 2-(9-Ethyl-9H-carbazol-3-yl)-1-(2-methoxyethyl)-1 H-benzimidazol-5-carbonitril, 89 mg (1.4 mmoll) Natriumazid und 73 mg (1.4 mmol) Ammoniumchlorid in 1.5 ml DMF wurde in der Mikrowelle für 3h auf 150 °C erhitzt (maximale Einstrahlung 30 Watt). Nach dem Abkühlen wurde die Reaktionsmischung filtriert und das Filtrat mittels präparativer HPLC aufgereinigt. So wurden 11 mg (20%) der Titelverbindung erhalten.
¹H-NMR (300MHz, DMSO-d6): δ [ppm]= 1.37 (t, 3H), 3.15 (s, 3H), 3.75 (t, 2H), 4.48 - 4.65 (m, 4H), 7.27 (t, 1H), 7.53 (t, 1 H), 7.69 (d, 1H), 7.81 (d, 1H), 7.89 - 8.03 (m, 3H), 8.28 (d, 1H), 8.36 (s, 1H), 8.71 (s, 1H), NH nicht angegeben.

### Beispiel 46

### 3-[2-(9-Ethyl-9H-carbazol-3-yl)-1-(2-methoxyethyl)-1H-benzimidazol-5-yl]-1,2,4-oxadiazol-5(4H)-on

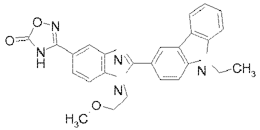

Eine Mischung aus 100 mg (0.25 mmol) 2-(9-Ethyl-9H-carbazol-3-yl)-1-(2-methoxyethyl)-1 H-benzimidazol-5-carbonitril, 106 mg (1.52 mmol) Hydroxylammoniumchlorid und 0.25 ml (1.77 mmol) Triethylamin wurde in 4 ml Ethanol für 2.5h zum Rückfluss erhitzt und danach im Vakuum eingeengt. Der Rückstand wurde anschließend in 10 ml DMF aufgenommen, 0.03 ml (0.33 mmol) Ethylchlorocarbonat und 0.09 ml Triethylamin hinzugefügt und für 11.5 h auf 95 °C erhitzt. Nach dem Abkühlen wurde mit Wasser und Ethylacetat versetzt und vom ausgefallenen Niederschlag abfiltriert. Die organische Phase wurde eingeengt und mittels präparativer HPLC aufgereinigt. Auf diese Weise wurden 30 mg (24%) der Titelverbindung erhalten. ¹H-NMR (300 MHz ,DMSO-d6), δ [ppm]= 1.37 (t, 3H), 3.14 (s, 3H), 3.73 (t, 2H), 4.48 - 4.63 (m, 4H), 7.27 (t, 1H), 7.53 (t, 1H), 7.69 (d, 1H), 7.73 - 7.83 (m, 2H), 7.86 - 7.90 (m, 1H), 7.96 (dd, 1H), 8.14 (d, 1H), 8.28 (d, 1H), 8.70 (d, 1H), 12.20 - 13.00 (1H)..

### Beispiel 47

### 2-(9-Ethyl-9H-carbazol-3-yl)-1-(2-methoxyethyl)-N-[(3-methylphenyl)sulfonyl]-1 H-benzimidazol-5-carboxamid

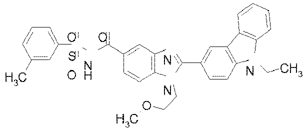

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure und dem entsprechenden Sulfonsäureamid 80 mg (70%) der Titelverbindung erhalten.
¹H-NMR (400 MHz, DMSO-d6), δ [ppm]= 1.37 (t, 3H), 2.43 (s, 3H), 3.11 (s, 3H), 3.69 (t, 2H), 4.49 - 4.61 (m, 4H), 7.27 (t, 1H), 7.50 - 7.56 (m, 3H), 7.69 (d, 1H), 7.77 - 7.86 (m, 5H), 7.94 (dd, 1H), 8.25 - 8.30 (m, 2H), 8.67 (d, 1H), 12.30 - 12.55 (br. s., 1H).

### Beispiel 48

### 2-(9-Ethyl-9H-carbazol-3-yl)-1-(2-methoxyethyl)-N-[(4-methylphenyl)sulfonyl]-1 H-benzimidazol-5-carboxamid

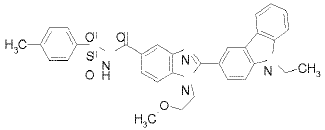

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 60 mg (54%) der Titelverbindung erhalten.
¹H-NMR (400 MHz, DMSO-d6), δ [ppm]= 1.37 (t, 3H), 2.41 (s, 3H), 3.11 (s, 3H), 3.69 (t, 2H), 4.49 - 4.61 (m, 4H), 7.27 (t, 1H), 7.46 (d, 2H), 7.49 - 7.56 (m, 1H), 7.69 (d, 1H), 7.76 - 7.83 (m, 3H), 7.90 - 7.96 (m, 3H), 8.25 - 8.29 (m, 2H), 8.67 (d, 1H), 12.31 - 12.52 (br. s., 1H).

### Beispiel 49

### N-[(4-Chlorphenyl)sulfonyl]-2-(9-ethyl-9H-carbazol-3-yl)-1-(2-methoxyethyl)-1H-benzimidazol-5-carboxamid

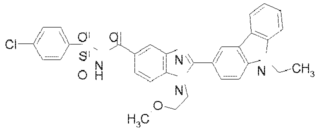

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure und dem entsprechenden Sulfonsäureamid 20 mg (17%) der Titelverbindung erhalten.
¹H-NMR (400 MHz, DMSO-d6), δ [ppm]= 1.37 (t, 3H), 3.11 (s, 3H), 3.70 (t, 2H), 4.49 - 4.63 (m, 4H), 7.27 (t, 1H), 7.50 - 7.56 (m, 1H), 7.67 - 7.76 (m, 3H), 7.79 - 7.86 (m, 3H), 7.94 (dd, 1H), 8.04 (d, 2H), 8.25 - 8.30 (m, 2H), 8.68 (d, 1H), 12.37 - 12.90 (br. s., 1H).

### Beispiel 50

### 2-(9-Ethyl-9H-carbazol-3-yl)-1-(2-methoxyethyl)-N-(phenylsulfonyl)-1H-benzimidazol-5-carboxamid

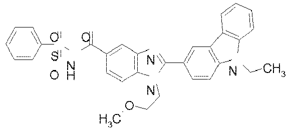

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure und dem entsprechenden Sulfonsäureamid 40 mg (37%) der Titelverbindung erhalten.
¹H-NMR (400 MHz, DMSO-d6), δ [ppm]= 1.37 (t, 3H), 3.11 (s, 3H), 3.69 (t, 2H), 4.49 - 4.61 (m, 4H), 7.27 (t, 1H), 7.50 - 7.56 (m, 1H), 7.63 - 7.76 (m, 4H), 7.77 - 7.84 (m, 3H), 7.94 (dd, 1H), 8.02 - 8.07 (m, 2H), 8.25 - 8.30 (m, 2H), 8.67 (d, 1H), 12.37 - 12.60 (br. s., 1 H).

### Beispiel 51

### 2-(9-Ethyl-9H-carbazol-3-yl)-1-(2-methoxyethyl)-N-(2-naphthylsulfonyl)-1H-benzimidazol-5-carboxamid

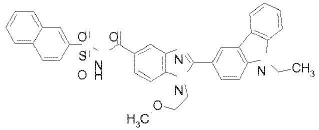

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 50 mg (41%) der Titelverbindung erhalten.
¹H-NMR (400 MHz, DMSO-d6), δ [ppm]= 1.37 (t, 3H), 3.10 (s, 3H), 3.69 (t, 2H), 4.49 - 4.61 (m, 4H), 7.27 (t, 1H), 7.52 (t, 1 H), 7.66 - 7.84 (m, 6H), 7.93 (dd, 1H), 8.02 (dd, 1H), 8.07 (d, 1H), 8.17 (d, 1H), 8.24 - 8.31 (m, 3H), 8.67 (d, 1H), 8.72 (s, 1H), 12.45 - 12.77 (br. s., 1H).

### Beispiel 52

### 2-(9-Ethyl-9H-carbazol-3-yl)-1-(2-methoxyethyl)-N-[(2-methoxyphenyl)sulfonyl]-1H-benzimidazol-5-carboxamid

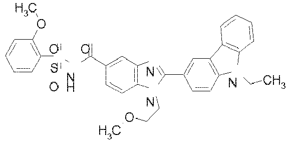

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 60 mg (51%) der Titelverbindung erhalten.
¹H-NMR (400 MHz, DMSO-d6), δ [ppm]= 1.38 (t, 3H), 3.12 (s, 3H), 3.70 (t, 2H), 3.88 (s, 3H), 4.49 - 4.61 (m, 4H), 7.18 (t, 1 H), 7.22 - 7.30 (m, 2H), 7.53 (t, 1H), 7.65 - 7.71 (m, 2H), 7.76 - 7.85 (m, 3H), 7.94 (td, 2H), 8.27 (d, 1H), 8.34 (s, 1H), 8.67 (s, 1H), 12.40 (br. s., 1H).

### Beispiel 53

### 2-(9-Ethyl-9H-carbazol-3-yl)-1-(2-methoxyethyl)-N-[(2-methylphenyl)sulfonyl]-1 H-benzimidazol-5-carboxamid

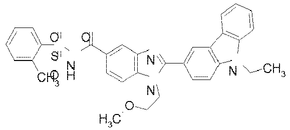

In Analogie zu Beispiel 15 wurden aus 70 mg (0.17 mmol) 2-(9-Ethyl-9H-carbazol-3-yl)-1-(2-methoxyethyl)-1 H-benzimidazol-5-carbonsäure und dem entsprechenden Sulfonsäureamid 32 mg (32%) der Titelverbindung erhalten.
¹H-NMR (300 MHz, DMSO-d6), δ [ppm]= 1.37 (t, 3H), 2.66 (s, 3H), 3.12 (s, 3H), 3.70 (t, 2H), 4.49 - 4.63 (m, 4H), 7.24 - 7.30 (m, 1 H), 7.39 - 7.63 (m, 4H), 7.69 (d, 1 H), 7.77 - 7.87 (m, 3H), 7.94 (dd, 1H), 8.09 (d, 1H), 8.27 (d, 1H), 8.33 (s, 1H), 8.68 (s, 1H), 12.47 - 12.81 (br. s., 1 H).

### Beispiel 54

### 2-(9-Ethyl-9H-carbazol-3-yl)-1-(2-methoxyethyl)-N-{[2-(trifluormethoxy)phenyl]sulfonyl}-1H-benzimidazol-5-carboxamid

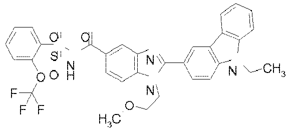

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 15 mg (12%) der Titelverbindung erhalten.
¹H-NMR (400 MHz, DMSO-d6), δ [ppm]= 1.38 (t, 3H), 3.13 (s, 3H), 3.72 (t, 2H), 4.50 - 4.64 (m, 4H), 7.28 (t, 1H), 7.50 - 7.67 (m, 3H), 7.70 (d, 1H), 7.79 - 7.91 (m, 4H), 7.96 (d, 1H), 8.17 (d, 1H), 8.27 (d, 1H), 8.34 (s, 1H), 8.70 (s, 1H), 12.73 - 13.17 (br. s., 1H).

### Beispiel 55

### N-(Benzylsulfonyl)-2-(9-ethyl-9H-carbazol-3-yl)-1-(2-methoxyethyl)-1 H-benzimidazol-5-carboxamid

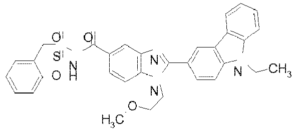

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 50 mg (43%) der Titelverbindung erhalten.
¹H-NMR (400 MHz, DMSO-d6), δ [ppm]= 1.38 (t, 3H), 3.14 (s, 3H), 3.72 (t, 2H), 4.50 - 4.63 (m, 4H), 4.90 (s, 2H), 7.27 (t, 1H), 7.33 - 7.41 (m, 5H), 7.50 - 7.56 (m, 1H), 7.69 (d, 1H), 7.82 (dd, 2H), 7.88 - 7.97 (m, 2H), 8.25 - 8.31 (m, 2H), 8.68 (d, 1H), 11.89 - 12.02 (br. s., 1H).

### Beispiel 56

### N-{[2-(3-Chlorphenyl)ethyl]sulfonyl}-2-(9-ethyl-9H-carbazol-3-yl)-1-(2-methoxyethyl)-1H-benzimidazol-5-carboxamid

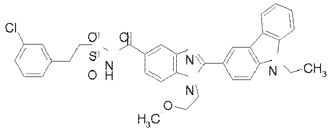

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 25 mg (21%) der Titelverbindung erhalten.
¹H-NMR (400 MHz, DMSO-d6), δ [ppm]= 1.38 (t, 3H), 3.08 - 3.15 (m, 5H), 3.72 (t, 2H), 3.87 - 3.94 (m, 2H), 4.50 - 4.64 (m, 4H), 7.22 - 7.34 (m, 4H), 7.41 (s, 1H), 7.53 (t, 1H), 7.70 (d, 1H), 7.79 - 7.85 (m, 2H), 7.85 - 7.90 (m, 1H), 7.95 (dd, 1H), 8.26 - 8.33 (m, 2H), 8.69 (d, 1H), 11.97 - 12.18 (br. s., 1H).

### Beispiel 57

### 2-(9-Ethyl-9H-carbazol-3-yl)-1-(2-methoxyethyl)-N-{[2-(2-methylphenyl)ethyl]sulfonyl}-1H-benzimidazol-5-carboxamid

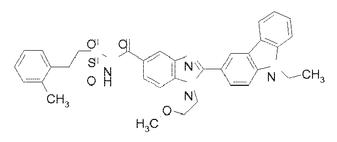

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 50 mg (42%) der Titelverbindung erhalten.
¹H-NMR (400 MHz, DMSO-d6), δ [ppm]= 1.38 (t, 3H), 2.26 (s, 3H), 3.03 - 3.09 (m, 2H), 3.14 (s, 3H), 3.72 (t, 2H), 3.76 - 3.82 (m, 2H), 4.54 (q, 2H), 4.61 (t, 2H), 7.10 - 7.18 (m, 3H), 7.19 - 7.24 (m, 1H), 7.28 (t, 1H), 7.53 (t, 1H), 7.70 (d, 1H), 7.83 (t, 2), 7.88 - 7.92 (m, 1H), 7.95 (dd, 1H), 8.28 (d, 1H), 8.34 (d, 1H), 8.69 (s, 1H), 12.04 - 12.23 (br. s., 1H).

### Beispiel 58

### 2-(9-Ethyl-9H-carbazol-3-yl)-N-[(4-fluorbenzyl)sulfonyl]-1-[(2-methoxyethyl)-1H-benzimidazol-5-carboxamid

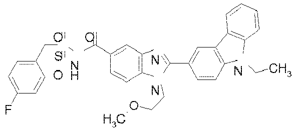

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 60 mg (51%) der Titelverbindung erhalten.
¹H-NMR (400 MHz, DMSO-d6), δ [ppm]= 1.38 (t, 3H), 3.14 (s, 3H), 3.72 (t, 2H), 4.53 (q, 2H), 4.60 (t, 2H), 4.91 (s, 2H), 7.19 - 7.31 (m, 3H), 7.38 - 7.44 (m, 2H), 7.50 - 7.56 (m, 1H), 7.69 (d, 1H), 7.82 (dd, 2H), 7.88 - 7.97 (m, 2H), 8.25 - 8.31 (m, 2H), 8.68 (d, 1H), 11.89 - 12.06 (br. s., 1H).

### Beispiel 59

### 2-(9-Ethyl-9H-carbazol-3-yl)-1-(2-methoxyethyl)-N-[(2-methoxyethyl)sulfonyl]-1H-benzimidazol-5-carboxamid

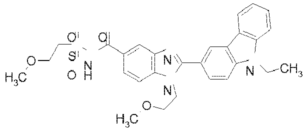

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 70 mg (66%) der Titelverbindung erhalten.
¹H-NMR (400 MHz, DMSO-d6), δ [ppm]= 1.38 (t, 3H), 3.14 (s, 3H), 3.22 (s, 3H), 3.72 (t, 2H), 3.75 - 3.85 (m, 4H), 4.54 (q, 2H), 4.60 (t, 2H), 7.27 (t, 1H), 7.53 (t, 1H), 7.69 (d, 1H), 7.79 - 7.84 (m, 2H), 7.87 - 7.91 (m, 1H), 7.95 (dd, 1H), 8.28 (d, 1H), 8.33 (d, 1H), 8.68 (d, 1H), 11.97 - 12.18 (br. s., 1H).

### Beispiel 60

### N-[(2,6-Dichlorbenzyl)sulfonyl]-2-(9-ethyl-9H-carbazol-3-yl)-1-(2-methoxyethyl)-1H-benzimidazol-5-carboxamid

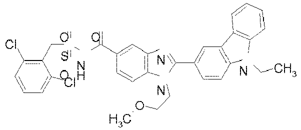

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 40 mg (66%) der Titelverbindung erhalten.
¹H-NMR (400 MHz, DMSO-d6), δ [ppm]= 1.38 (t, 3H), 3.15 (s, 3H), 3.73 (t, 2H), 4.50 - 4.63 (m, 4H), 5.22 (s, 2H), 7.28 (t, 1H), 7.41 - 7.47 (m, 1H), 7.50 - 7.59 (m, 3H), 7.70 (d, 1H), 7.79 - 7.85 (m, 2H), 7.89 - 7.97 (m, 2H), 8.28 (d, 1H), 8.32 (d, 1H), 8.69 (d, 1H), 12.22 - 12.43 (br. s., 1H).

### Beispiel 61

### N-{[2-(2-Chlorphenyl)ethyl]sulfonyl}-2-(9-ethyl-9H-carbazol-3-yl)-1-(2-methoxyethyl)-1H-benzimidazol-5-carboxamid

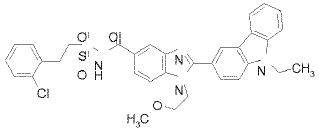

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure und dem entsprechenden Sulfonsäureamid 60 mg (48%) der Titelverbindung erhalten.
¹H-NMR (400 MHz, DMSO-d6), δ [ppm]= 1.38 (t, 3H), 3.14 (s, 3H), 3.17 - 3.23 (m, 2H), 3.72 (t, 2H), 3.81 - 3.88 (m, 2H), 4.54 (q, 2H), 4.61 (t, 2H), 7.23 - 7.32 (m, 3H), 7.40 - 7.48 (m, 2H), 7.50 - 7.56 (m, 1H), 7.69 (d, 1H), 7.79 - 7.85 (m, 2H), 7.86 - 7.91 (m, 1H), 7.95 (dd, 1H), 8.28 (d, 1H), 8.32 (d, 1H), 8.69 (d, 1H), 12.08 - 12.23 (br. s., 1H).

### Beispiel 62

### 2-(9-Ethyl-9H-carbazol-3-yl)-1-(2-methoxyethyl)-N-{[4-(trifluormethyl)benzyl]sulfonyl}-1H-benzimidazol-5-carboxamid

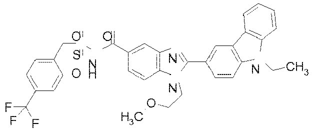

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 40 mg (32%) der Titelverbindung erhalten.
¹H-NMR (400 MHz, DMSO-d6), δ [ppm]= 1.38 (t, 3H), 3.14 (s, 3H), 3.72 (t, 2H), 4.54 (q, 2H), 4.61 (t, 2H), 5.05 (s, 2H), 7.28 (t, 1H), 7.53 (t, 1H), 7.60 (d, 2H), 7.70 (d, 1H), 7.76 - 7.86 (m, 4H), 7.89 - 7.97 (m, 2H), 8.26 - 8.31 (m, 2H), 8.68 (d, 1H), 11.93 - 12.14 (br. s., 1H).

### Beispiel 63

### 2-(9-Ethyl-9H-carbazol-3-yl)-1-(2-methoxyethyl)-N-[(5-methylpyridin-2-yl)sulfonyl]-1H-benzimidazol-5-carboxamid

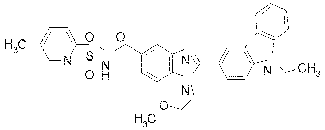

In Analogie zu Beispiel 15 wurden aus 70 mg (0.17 mmol) 2-(9-Ethyl-9H-carbazol-3-yl)-1-(2-methoxyethyl)-1 H-benzimidazol-5-carbonsäure und dem entsprechenden Sulfonsäureamid 32 mg (32%) der Titelverbindung erhalten.
¹H-NMR (300 MHz, DMSO-d6), δ [ppm]= 1.37 (t, 3H), 2.42 (s, 3H), 3.12 (s, 3H), 3.70 (t, 2H), 4.48 - 4.63 (m, 4H), 7.27 (t, 1H), 7.49 - 7.57 (m, 1H), 7.69 (d, 1H), 7.77 - 7.86 (m, 3H), 7.91 - 8.00 (m, 2H), 8.06 - 8.11 (m, 1H), 8.28 (d, 1H), 8.32 (s, 1H), 8.57 (s, 1H), 8.69 (d, 1H), 12.53 - 12.92 (br. s., 1H).

### Beispiel 64

### N-[(4-Chlorbenzyl)sulfonyl]-2-(9-ethyl-9H-carbazol-3-yl)-1-(2-methoxyethyl)-1H-benzimidazol-5-carboxamid

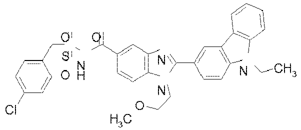

In Analogie zu Beispiel 15 wurden aus 70 mg (0.17 mmol) 2-(9-Ethyl-9H-carbazol-3-yl)-1-(2-methoxyethyl)-1H-benzimidazol-5-carbonsäure und dem entsprechenden Sulfonsäureamid 55 mg (52%) der Titelverbindung erhalten.
¹H-NMR (300 MHz, DMSO-d6), δ [ppm]= 1.37 (t, 3H), 3.14 (s, 3H), 3.72 (t, 2H), 4.49 - 4.65 (m, 4H), 4.93 (s, 2H), 7.28 (t, 1H), 7.36 - 7.41 (m, 2H), 7.44 - 7.49 (m, 2H), 7.53 (t, 1H), 7.70 (d, 1H), 7.79 - 7.98 (m, 4H), 8.25 - 8.31 (m, 2H), 8.69 (d, 1H), 11.87 - 12.14 (br. s., 1H).

### Beispiel 65

### 2-(9-Ethyl-9H-carbazol-3-yl)-1-(2-methoxyethyl)-N-{[2-(trifluormethoxy)benzyl]sulfonyl}-1H-benzimidazol-5-carboxamid

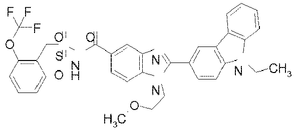

In Analogie zu Beispiel 15 wurden aus 70 mg (0.17 mmol) 2-(9-Ethyl-9H-carbazol-3-yl)-1-(2-methoxyethyl)-1H-benzimidazol-5-carbonsäure und dem entsprechenden Sulfonsäureamid 10 mg (9%) der Titelverbindung erhalten.
¹H-NMR (300 MHz, DMSO-d6), δ [ppm]= 1.38 (t, 3H), 3.14 (s, 3H), 3.72 (t, 2H), 4.49 - 4.63 (m, 4H), 5.02 (s, 2H), 7.27 (t, 1H), 7.37 - 7.47 (m, 2H), 7.49 - 7.57 (m, 2H), 7.58 - 7.63 (m, 1H), 7.70 (d, 1H), 7.79 - 7.86 (m, 2H), 7.88 - 7.98 (m, 2H), 8.25 - 8.31 (m, 2H), 8.68 (s, 1H), 12.04 - 12.20 (br. s., 1H).

### Beispiel 66

### N-[(2,2-Dimethylpropyl)sulfonyl]-2-(9-ethyl-9H-carbazol-3-yl)-1-(2-methoxyethyl)-1 H-benzimidazol-5-carboxamid

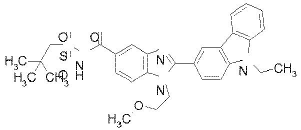

In Analogie zu Beispiel 15 wurden aus 100 mg (0.24 mmol) 2-(9-Ethyl-9H-carbazol-3-yl)-1-(2-methoxyethyl)-1H-benzimidazol-5-carbonsäure und dem entsprechenden Sulfonsäureamid 30 mg (22%) der Titelverbindung erhalten.
¹H-NMR (400 MHz, DMSO-d6), δ [ppm]= 1.15 (s, 9H), 1.37 (t, 3H), 3.12 - 3.14 (m, 3H), 3.57 (s, 2H), 3.72 (t, 2H), 4.54 (q, 2H), 4.60 (t, 2H), 7.25 - 7.30 (m, 1H), 7.53 (td, 1H), 7.70 (d, 1H), 7.80 - 7.86 (m, 2H), 7.89 - 7.92 (m, 1H), 7.95 (dd, 1H), 8.28 (d, 1H), 8.34 (d, 1H), 8.69 (d, 1H), 12.05 (br. s., 1H).

### Beispiel 67

### N-[(2-Chlor-6-methylbenzyl)sulfonyl]-2-(9-ethyl-9H-carbazol-3-yl)-1-(2-methoxyethyl)-1H-benzimidazol-5-carboxamid

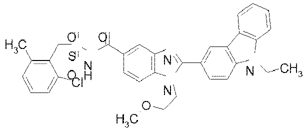

In Analogie zu Beispiel 15 wurden aus 70 mg (0.17 mmol) 2-(9-Ethyl-9H-carbazol-3-yl)-1-(2-methoxyethyl)-1H-benzimidazol-5-carbonsäure und dem entsprechenden Sulfonsäureamid 20 mg (18%) der Titelverbindung erhalten.
¹H-NMR (300 MHz, DMSO-d6), δ [ppm]= 1.38 (t, 3H), 2.53 (s, 3H), 3.15 (s, 3H), 3.73 (t, 2H), 4.49 - 4.65 (m, 4H), 5.16 (s, 2H), 7.24 - 7.39 (m, 4H), 7.53 (t, 1H), 7.70 (d, 1H), 7.79 - 7.88 (m, 2H), 7.91 - 7.99 (m, 2H), 8.28 (d, 1H), 8.35 (s, 1H), 8.70 (s, 1H), 12.22-12.43 (br. s., 1H).

### Beispiel 68

### 2-(9-Ethyl-9H-carbazol-3-yl)-1-(2-methoxyethyl)-N-{[(3-methylpyridin-2-yl)methyl]sulfonyl}-1H-benzimidazol-5-carboxamid

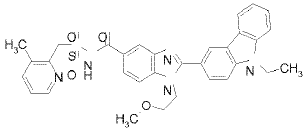

In Analogie zu Beispiel 15 wurden aus 70 mg (0.17 mmol) 2-(9-Ethyl-9H-carbazol-3-yl)-1-(2-methoxyethyl)-1 H-benzimidazol-5-carbonsäure und dem entsprechenden Sulfonsäureamid 20 mg (20%) der Titelverbindung erhalten.
¹H-NMR (400 MHz, DMSO-d6), δ [ppm]= 1.38 (t, 3H), 2.49 (s, 3H), 3.15 (s, 3H), 3.70 - 3.75 (m, 2H), 4.49 - 4.63 (m, 4H), 5.06 (s, 2H), 7.24 - 7.31 (m, 2H), 7.50 - 7.56 (m, 1H), 7.66 - 7.77 (m, 2H), 7.78 - 7.85 (m, 2H), 7.88 - 7.92 (m, 1H), 7.93 - 7.98 (m, 1H), 8.24 - 8.34 (m, 3H), 8.67 - 8.70 (m, 1H), 11.84 - 12.11 (br. s., 1H).

### Beispiel 69

### 2-(9-Ethyl-9H-carbazol-3-yl)-1-(2-methoxyethyl)-N-{[2-(3-methoxyphenyl)ethyl]sulfonyl}-1H-benzimidazol-5-carboxamid

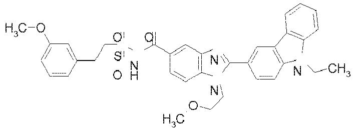

In Analogie zu Beispiel 15 wurden aus 80 mg (0.19 mmol) 2-(9-Ethyl-9H-carbazol-3-yl)-1-(2-methoxyethyl)-1H-benzimidazol-5-carbonsäure und dem entsprechenden Sulfonsäureamid 30 mg (25%) der Titelverbindung erhalten.
¹H-NMR (400 MHz, DMSO-d6), δ [ppm]= 1.38 (t, 3H), 3.03 - 3.08 (m, 2H), 3.13 (s, 3H), 3.70 - 3.74 (m, 5H), 3.84 - 3.91 (m, 2H), 4.54 (q, 2H), 4.61 (t, 2H), 6.75 (dd, 1H), 6.82 - 6.89 (m, 2H), 7.20 (t, 1H), 7.27 (t, 1H), 7.53 (t, 1H), 7.69 (d, 1H), 7.80 - 7.85 (m, 2H), 7.86 - 7.90 (m, 1H), 7.95 (dd, 1H), 8.28 (d, 1H), 8.32 (d, 1H), 8.69 (d, 1H), 12.07 (br. s., 1 H).

### Beispiel 70

### 2-(9-Ethyl-9H-carbazol-3-yl)-1-[2-(morpholin-4-yl)ethyl]-1H-benzimidazol-5-carbonsäure

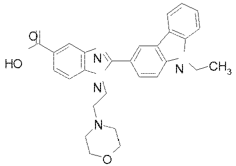

In Analogie zu Beispiel 37 wurden aus 1.19 g (4.06 mmol) rohem Ethyl-3-amino-4-{[2-(morpholin-4-yl)ethyl]amino}benzoat und 0.82 g (3.77 mmol) 9-Ethyl-9H-carbazol-3-carbaldehyd zunächst 1.85 g (76%) Ethyl-2-(9-ethyl-9H-carbazol-3-yl)-1-[2-(morpholin-4-yl)ethyl]-1H-benzimidazol-5-carboxylat als Rohprodukt gewonnen. Durch Verseifung von 1.75 g dieses Rohproduktes mit Natronlauge wurden 0.93 g (56%) der Titelverbindung erhalten.
¹H-NMR (300 MHz, DMSO-d6), δ [ppm]= 1.37 (t, 3H), 2.16 - 2.23 (m, 4H), 2.65 (t, 2H), 3.29 - 3.36 (m, 4H), 4.54 (d, 4H), 7.24 - 7.30 (m, 1H), 7.53 (t, 1H), 7.67 - 7.84 (m, 3H), 7.89 - 7.97 (m, 2H), 8.26 - 8.32 (m, 2H), 8.64 (s, 1H), 12.57 - 12.90 (br., 1H).

### Beispiel 71

### Ethyl-1-[2-(dimethylamino)ethyl]-2-(9-ethyl-9H-carbazol-3-yl)-1H-benzimidazol-5-carboxylat

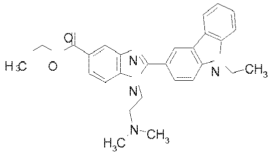

In Analogie zur Herstellung von Ethyl-3-amino-4-{[2-(pyrrolidin-1-yl)ethyl]amino}benzoat wurde zunächst Ethyl-3-amino-4-{[2-(dimethylamino)ethyl]amino}benzoat aus Ethyl-4-chlor-3-nitrobenzoat und N,N-Dimethylethan-1,2-diamin hergestellt, welches ohne weitere Reinigung mit 9-Ethyl-9H-carbazol-3-carbaldehyd analog Beispiel 4 / Variante B zur Titelverbindung umgesetzt wurde.
¹H-NMR (500MHz, DMSO-d6), δ [ppm]= 1.35 - 1.40 (m, 6H), 2.03 (s, 6H), 2.59 - 2.65 (m, 2H), 4.36 (q, 2H), 4.49 - 4.57 (m, 4H), 7.27 (t, 1H), 7.50 - 7.55 (m, 1H), 7.68 (d, 1H), 7.80 (dd, 2H), 7.92 (ddd, 2H), 8.26 - 8.31 (m, 2H), 8.65 (d, 1H).

### Beispiel 72

### 1-[2-(Dimethylamino)ethyl]-2-(9-ethyl-9H-carbazol-3-yl)-1H-benzimidazol-5-carbonsäure

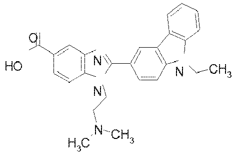

In Analogie zu Beispiel 2 wurden aus 1.7 g (3.74 mmol) Ethyl-1-[2-(dimethylamino)ethyl]-2-(9-ethyl-9H-carbazol-3-yl)-1H-benzimidazol-5-carboxylat durch Verseifung mit Natronlauge 1.42 g (85%) der Titelverbindung erhalten.
¹H-NMR (400 MHz, DMSO-d6), δ [ppm]= 1.38 (t, 3H), 2.03 (s, 6H), 2.62 (t, 2H), 4.47 - 4.57 (m, 4H), 7.27 (t, 1H), 7.50 - 7.56 (m, 1H), 7.69 (d, 1H), 7.76 (d, 1H), 7.81 (d, 1H), 7.89 - 7.94 (m, 2H), 8.25 - 8.31 (m, 2H), 8.66 (d, 1H), 12.54 - 12.87 (br., 1H).

### Beispiel 73

### 1-[2-(Dimethylamino)ethyl]-2-(9-ethyl-9H-carbazol-3-yl)-N-methyl-1 H-benzimidazol-5-carboxamid

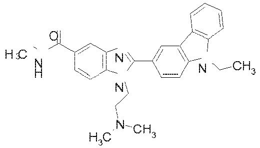

In Analogie zu Beispiel 21 wurden aus 100 mg (0.23 mmol) 1-[2-(Dimethylamino)ethyl]-2-(9-ethyl-9H-carbazol-3-yl)-1H-benzimidazol-5-carbonsäure 72 mg (66%) der Titelverbindung erhalten.
¹H-NMR (300 MHz, DMSO-d6), δ [ppm]= 1.37 (t, 3H), 2.04 (s, 6H), 2.62 (t, 2H), 2.83 (d, 3H), 4.45 - 4.58 (m, 4H), 7.27 (t, 1H), 7.53 (t, 1H), 7.67 - 7.75 (m, 2H), 7.79 - 7.84 (m, 2H), 7.89 - 7.94 (m, 1H), 8.20 (d, 1H), 8.29 (d, 1H), 8.45 (d, 1H), 8.65 (d, 1H).

### Beispiel 74

### N-(Cyclopropylsulfonyl)-1-[2-(dimethylamino)ethyl]-2-(9-ethyl-9H-carbazol-3-yl)-1H-benzimidazol-5-carboxamid

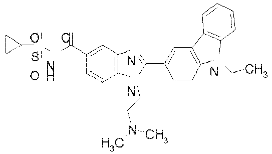

In Analogie zu Beispiel 15 wurden aus 100 mg (0.23 mmol) 1-[2-(Dimethylamino)ethyl]-2-(9-ethyl-9H-carbazol-3-yl)-1H-benzimidazol-5-carbonsäure und 127 mg (1.1 mmol) Cyclopropansulfonamid 2.6 mg (2%) der Titelverbindung erhalten.
¹H-NMR (400 MHz, DMSO-d6), δ [ppm]= 0.96 - 1.03 (m, 2H), 1.04 - 1.12 (m, 2H), 1.38 (t, 3H), 2.10 - 2.17 (m, 6H), 2.72 - 2.80 (m, 2H), 3.09 - 3.17 (m, 1H), 4.50 - 4.58 (m, 4H), 7.27 (t, 1H), 7.50 - 7.56 (m, 1H), 7.69 (d, 1H), 7.75 (d, 1H), 7.82 (d, 1H), 7.93 (td, 2H), 8.28 - 8.32 (m, 2H), 8.65 (d, 1H), 11.36 - 11.93 (br., 1H).

### Beispiel 75

### 1-[2-(Dimethylamino)ethyl]-2-(9-ethyl-9H-carbazol-3-yl)-N-[(4-methylphenyl)sulfonyl]-1H-benzimidazol-5-carboxamid

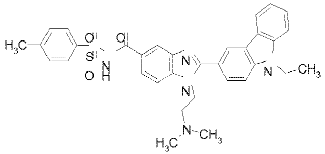

In Analogie zu Beispiel 15 wurden aus 50 mg (0.12 mmol) 1-[2-(Dimethylamino)ethyl]-2-(9-ethyl-9H-carbazol-3-yl)-1H-benzimidazol-5-carbonsäure und dem entsprechenden Sulfonsäureamid 5 mg (7%) der Titelverbindung erhalten.
¹H-NMR (300 MHz, DMSO-d6), δ [ppm]= 1.37 (t, 3H), 2.34 - 2.43 (m, 8H), 2.54 (s, 3H), 4.49 - 4.66 (m, 4H), 7.23 - 7.38 (m, 4H), 7.53 (t, 1H), 7.71 (t, 2H), 7.79 - 7.93 (m, 4H), 8.26 (d, 1H), 8.32 (d, 1H), 8.63 (d, 1H), 10.99 - 11.97 (br., 1H).

### Beispiel 76

### [2-(9-Ethyl-9H-carbazol-3-yl)-1-(2-methoxyethyl)-1H-benzimidazol-5-yl](pyrrolidin-1-yl)methanon

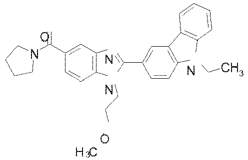

In Analogie zu Beispiel 21 wurden aus 100 mg (0.24 mmol) 2-(9-Ethyl-9H-carbazol-3-yl)-1-(2-methoxyethyl)-1H-benzimidazol-5-carbonsäure und 86 mg (1.21 mmol) Pyrrolidin 20 mg (17%) der Titelverbindung erhalten.
¹H-NMR (300 MHz, DMSO-d6), δ [ppm]= 1.38 (t, 3H), 1.79 - 1.95 (m, 4H), 3.16 (s, 3H), 3.51 (d, 4H), 3.74 (t, 2H), 4.49 - 4.60 (m, 4H), 7.24 - 7.29 (m, 1H), 7.46 (dd, 1H), 7.52 (td, 1H), 7.69 (d, 1H), 7.73 (d, 1H), 7.80 (d, 1H), 7.84 (d, 1H), 7.94 (dd, 1H), 8.27 (d, 1H), 8.67 (d, 1H).

### Beispiel 77

### 2-(9-Ethyl-9H-carbazol-3-yl)-1-(2-methoxyethyl)-N-(2,2,2-trifluorethyl)-1H-benzimidazol-5-carboxamid

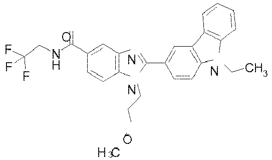

In Analogie zu Beispiel 21 wurden aus 100 mg (0.24 mmol) 2-(9-Ethyl-9H-carbazol-3-yl)-1-(2-methoxyethyl)-1H-benzimidazol-5-carbonsäure und 120 mg (1.21 mmol) 2,2,2-Trifluorethanamin 45 mg (37%) der Titelverbindung erhalten.
¹H-NMR (300 MHz, DMSO-d6), δ [ppm]= 1.37 (t, 3H), 3.14 (s, 3H), 3.72 (t, 2H), 4.07 - 4.21 (m, 2H), 4.48 - 4.63 (m, 4H), 7.27 (t, 1H), 7.49 - 7.56 (m, 1H), 7.69 (d, 1H), 7.77 - 7.83 (m, 2H), 7.85 - 7.90 (m, 1H), 7.95 (dd, 1H), 8.25 - 8.30 (m, 2H), 8.68 (d, 1H), 9.10 (t, 1H).

### Beispiel 78

### 2-(9-Ethyl-9H-carbazol-3-yl)-1-(2-methoxyethyl)-N-[2-(morpholin-4-yl)ethyl]-1H-benzimidazol-5-carboxamid

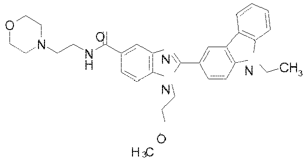

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 157 mg (1.21 mmol) 2-(Morpholin-4-yl)ethanamin 50 mg (39%) der Titelverbindung erhalten.
¹H-NMR (300 MHz, DMSO-d6), δ [ppm]= 1.38 (t, 3H), 2.42 - 2.54 (m, 6H), 3.14 (s, 3H), 3.44 (q, 2H), 3.57 - 3.62 (m, 4H), 3.72 (t, 2H), 4.48 - 4.61 (m, 4H), 7.27 (t, 1H), 7.49 - 7.56 (m, 1H), 7.67 - 7.84 (m, 4H), 7.94 (dd, 1H), 8.21 (d, 1H), 8.27 (d, 1H), 8.40 (t, 1H), 8.67 (d, 1H).

### Beispiel 79

### 1-(Cyclopropylmethyl)-2-(9-ethyl-9H-carbazol-3-yl)-N-[2-(morpholin-4-yl)ethyl]-1H-benzimidazol-5-carboxamid

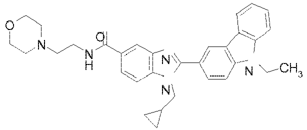

In Analogie zu Beispiel 21 wurden aus 100 mg (0.24 mmol) 1-(Cyclopropylmethyl)-2-(9-ethyl-9H-carbazol-3-yl)-1H-benzimidazol-5-carbonsäure und 159 mg (1.21 mmol) 2-(Morpholin-4-yl)ethanamin 85 mg (65%) der Titelverbindung erhalten.
¹H-NMR (300 MHz, DMSO-d6), δ [ppm]= 0.13 - 0.18 (m, 2H), 0.32 - 0.40 (m, 2H), 1.00 - 1.11 (m, 1 H), 1.38 (t, 3H), 2.42 - 2.54 (m, 6H), 3.44 (q, 2H), 3.56 - 3.62 (m, 4H), 4.36 (d, 2H), 4.53 (q, 2H), 7.26 (t, 1 H), 7.49 - 7.56 (m, 1 H), 7.69 (d, 1 H), 7.75 - 7.91 (m, 4H), 8.22 (s, 1 H), 8.30 (d, 1 H), 8.41 (t, 1 H), 8.62 (d, 1 H).

### Beispiel 80

### Azetidin-1-yl[1-(cyclopropylmethyl)-2-(9-ethyl-9H-carbazol-3-yl)-1H-benzimidazol-5-yl]methanon

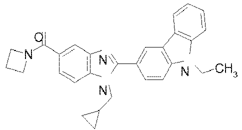

In Analogie zu Beispiel 21 wurden aus 100 mg (0.24 mmol) 1-(Cyclopropylmethyl)-2-(9-ethyl-9H-carbazol-3-yl)-1 H-benzimidazol-5-carbonsäure und 70 mg (1.22 mmol) Azetidin 55 mg (50%) der Titelverbindung erhalten.
¹H-NMR (300 MHz, DMSO-d6), δ [ppm]= 0.12 - 0.20 (m, 2H), 0.33 - 0.42 (m, 2H), 1.01 - 1.13 (m, 1H), 1.38 (t, 3H), 2.29 (quin, 2H), 4.09 (br. s., 2H), 4.32 - 4.45 (m, 4H), 4.53 (q, 2H), 7.26 (t, 1 H), 7.49 - 7.56 (m, 1 H), 7.59 (dd, 1 H), 7.69 (d, 1 H), 7.75 - 7.94 (m, 4H), 8.30 (d, 1 H), 8.62 (d, 1 H).

### Beispiel 81

### [2-(9-Ethyl-9H-carbazol-3-yl)-1-(2-methoxyethyl)-1H-benzimidazol-5-yl](morpholin-4-yl)methanon

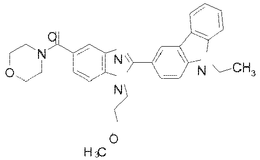

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 105 mg (1.21 mmol) Morpholin 35 mg (29%) der Titelverbindung erhalten.
¹H-NMR (400 MHz, DMSO-d6), δ [ppm]= 1.38 (t, 3H), 3.16 (s, 3H), 3.50 - 3.68 (m, 8H), 3.74 (t, 2H), 4.49 - 4.59 (m, 4H), 7.24 - 7.29 (m, 1 H), 7.34 (dd, 1 H), 7.53 (td, 1 H), 7.67 - 7.82 (m, 4H), 7.94 (dd, 1 H), 8.27 (d, 1 H), 8.67 (d, 1 H).

### Beispiel 82

### 2-(9-Ethyl-9H-carbazol-3-yl)-1-(2-methoxyethyl)-1H-benzimidazol-5-carboxamid

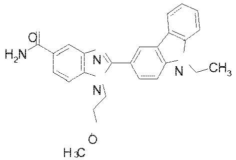

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 65 mg (1.21 mmol) Ammoniumchlorid 30 mg (29%) der Titelverbindung erhalten.
¹H-NMR (300 MHz, DMSO-d6), δ [ppm]= 1.38 (t, 3H), 3.14 (s, 3H), 3.73 (t, 2H), 4.48 - 4.62 (m, 4H), 7.27 (t, 2H), 7.48 - 7.56 (m, 1 H), 7.65 - 7.89 (m, 4H), 7.91 - 8.03 (m, 2H), 8.24 - 8.29 (m, 2H), 8.67 (d, 1 H).

### Beispiel 83

### 2-(9-Ethyl-9H-carbazol-3-yl)-1-(2-methoxyethyl)-N,N-dimethyl-1 H-benzimidazol-5-carboxamid

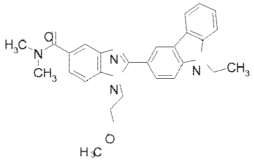

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 55 mg (1.21 mmol) Dimethylamin 45 mg (42%) der Titelverbindung erhalten.
¹H-NMR (300 MHz, DMSO-d6), δ [ppm]= 1.38 (t, 3H), 3.02 (s, 6H), 3.16 (s, 3H), 3.74 (t, 2H), 4.48 - 4.61 (m, 4H), 7.23 - 7.35 (m, 2H), 7.49 - 7.56 (m, 1 H), 7.66 - 7.83 (m, 4H), 7.94 (dd, 1 H), 8.27 (d, 1 H), 8.67 (d, 1 H).

### Beispiel 84

### 2-(9-Ethyl-9H-carbazol-3-yl)-1-(2-methoxyethyl)-N-methyl-N-(methylsulfonyl)-1 H-benzimidazol-5-carboxamid

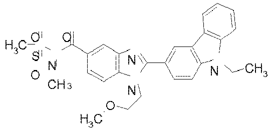

In Analogie zu Beispiel 15 wurden aus 80 mg (0.19 mmol) 2-(9-Ethyl-9H-carbazol-3-yl)-1-(2-methoxyethyl)-1 H-benzimidazol-5-carbonsäure und dem entsprechenden Sulfonsäureamid 30 mg (31%) der Titelverbindung erhalten.
¹H-NMR (300 MHz, DMSO-d6), δ [ppm]= 1.37 (t, 3H), 3.02 (s, 6H), 3.16 (s, 3H), 3.74 (t, 2H), 4.48 - 4.61 (m, 4H), 7.23 - 7.36 (m, 2H), 7.52 (t, 1 H), 7.65 - 7.83 (m, 4H), 7.95 (dd, 1 H), 8.27 (d, 1 H), 8.68 (d, 1 H).

### Beispiel 85

### 1-Allyl-2-(9-ethyl-9H-carbazol-3-yl)-N,N-dimethyl-1 H-benzimidazol-5-carboxamid

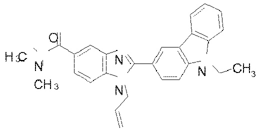

1-Allyl-2-(9-ethyl-9H-carbazol-3-yl)-N,N-dimethyl-1H-benzimidazol-5-carboxamid wurde in Analogie zu Beispiel 21 aus 1-Allyl-2-(9-ethyl-9H-carbazol-3-yl)-1 H-benzimidazol-5-carbonsäure mit N-[(1 H-Benzimidazol-1-yloxy)(dimethylamino)methylen]-N-methylmethanaminiumtetrafluoroborat (TBTU) und Dimethylamin hergestellt.

### Beispiel 86

### N-[2-(Dimethylamino)ethyl]-2-(9-ethyl-9H-carbazol-3-yl)-1-(2-methoxyethyl)-1H-benzimidazol-5-carboxamid

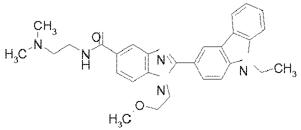

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 107 mg (1.21 mmol) N,N-Dimethylethan-1,2-diamin 7 mg (6%) der Titelverbindung erhalten.
¹H-NMR (300 MHz, DMSO-d6), δ [ppm]= 1.38 (t, 3H), 2.25 - 2.29 (m, 1 H), 3.17 (s, 3H), 3.27 - 3.40 (m, 10H), 3.75 (t, 2H), 4.48 - 4.60 (m, 4H), 7.21 - 7.30 (m, 2H), 7.49 - 7.56 (m, 1 H), 7.61 (d, 1 H), 7.66 - 7.82 (m, 3H), 7.94 (dd, 1 H), 8.27 (d, 1 H), 8.67 (d, 1 H).

### Beispiel 87

### 2-(9-Ethyl-9H-carbazol-3-yl)-1-(2-methoxyethyl)-N-[2-(pyrrolidin-1-yl)ethyl]-1H-benzimidazol-5-carboxamid

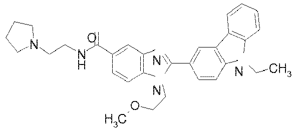

In Analogie zu Beispiel 21 wurden aus 100 mg (0.24 mmol) 2-(9-Ethyl-9H-carbazol-3-yl)-1-(2-methoxyethyl)-1 H-benzimidazol-5-carbonsäure und 138 mg (1.21 mmol) 2-(Pyrrolidin-1-yl)ethanamin 51 mg (39%) der Titelverbindung erhalten.
¹H-NMR (300 MHz, DMSO-d6), δ [ppm]= 1.37 (t, 3H), 1.69 (t, 4H), 2.46 - 2.56 (m, 4H), 2.61 (t, 2H), 3.14 (s, 3H), 3.42 (q, 2H), 3.72 (t, 2H), 4.49 - 4.60 (m, 4H), 7.27 (t, 1H), 7.48 - 7.56 (m, 1 H), 7.66 - 7.85 (m, 4H), 7.94 (dd, 1 H), 8.22 (d, 1 H), 8.27 (d, 1 H), 8.45 (t, 1 H), 8.67 (d, 1 H).

### Beispiel 88

### 1-Allyl-2-(9-ethyl-9H-carbazol-3-yl)-N-methyl-1 H-benzimidazol-5-carboxamid

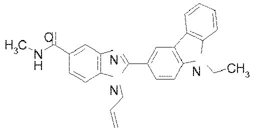

In Analogie zu Beispiel 21 wurden aus 101 mg (0.25 mmol) 2-(9-Ethyl-9H-carbazol-3-yl)-1-(2-methoxyethyl)-1 H-benzimidazol-5-carbonsäure und Methylammoniumhydrochlorid 29 mg (28%) der Titelverbindung erhalten.
¹H-NMR (400 MHz, DMSO-d6), δ [ppm]= 1.37 (t, 3H), 2.83 (d, 3H), 4.53 (q, 2H), 4.93 - 5.06 (m, 3H), 5.26 (dd, 1 H), 6.10 - 6.21 (m, 1 H), 7.25 - 7.29 (m, 1 H), 7.50 - 7.55 (m, 1 H), 7.60 (d, 1 H), 7.69 (d, 1 H), 7.79 - 7.83 (m, 2H), 7.85 - 7.90 (m, 1 H), 8.22 (d, 1 H), 8.26 (d, 1 H), 8.45 (q, 1 H), 8.61 (d, 1 H).

### Beispiel 89

### 1-Allyl-2-(9-ethyl-9H-carbazol-3-yl)-1 H-benzimidazol-5-carboxamid

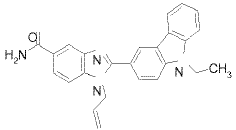

In Analogie zu Beispiel 21 wurde die Titelverbindung aus 2-(9-Ethyl-9H-carbazol-3-yl)-1-(2-methoxyethyl)-1 H-benzimidazol-5-carbonsäure und konzentrierter wässriger Ammoniumhydroxidlösung hergestellt (21 mg, 14%).
¹H-NMR (600 MHz, DMSO-d6), δ [ppm]= 1.37 (t, 3H), 4.53 (q, 2H), 4.94 - 5.06 (m, 3H), 5.26 (dd, 1H), 6.11 - 6.19 (m, 1H), 7.27 (t, 2H), 7.51 - 7.55 (m, 1H), 7.58 (d, 1H), 7.69 (d, 1 H), 7.81 (d, 1 H), 7.86 (ddd, 2H), 7.99 (br. s., 1 H), 8.26 (d, 1 H), 8.28 (d, 1 H), 8.61 (d, 1 H).

### Beispiel 90

### 2-(9-Ethyl-9H-carbazol-3-yl)-N,1-dimethyl-1 H-benzimidazol-5-carboxamid

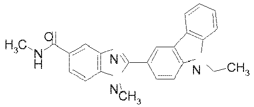

In Analogie zu Beispiel 21 wurden aus 500 mg (1.35 mmol) 2-(9-Ethyl-9H-carbazol-3-yl)-1-methyl-1 H-benzimidazol-5-carbonsäure und 110 mg (1.62 mmol) Methylaminhydrochlorid 106 mg (21%) der Titelverbindung erhalten.
¹H-NMR (600 MHz, DMSO-d6), δ [ppm]= 1.38 (t, 3H), 2.85 (d, 3H), 4.02 (s, 3H), 4.54 (q, 2H), 7.26 - 7.29 (m, 1 H), 7.51 - 7.55 (m, 1 H), 7.68 - 7.73 (m, 2H), 7.78 (dd, 1 H), 7.82 (d, 1 H), 7.97 - 8.00 (m, 1 H), 8.16 (d, 1 H), 8.33 (d, 1 H), 8.45 (q, 1 H), 8.71 (d, 1 H).

### Beispiel 91

### 2-(9-Ethyl-9H-carbazol-3-yl)-1-(2-hydroxyethyl)-1 H-benzimidazol-5-carbonsäure

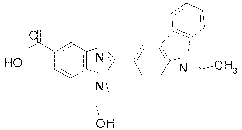

220 mg (0.53 mmol) 2-(9-Ethyl-9H-carbazol-3-yl)-1-(2-methoxyethyl)-1H-benzimidazol-5-carbonsäure wurden bei 0°C in 10 ml Dichlormethan (DCM) gegeben, durch ein Septum mit einer Lösung von 5.33 ml (5.33 mmol) Bortribromid in n-Heptan (1 M) versetzt und 8h bei 0°C gerührt. Überschüssiges Reagenz wurde mit Methanol zersetzt, das Gemisch im Vakuum eingeengt und der Rückstand chromatographisch an Kieselgel (DCM / Methanol 1:0 -> 4:1) und mittels präparativer HPLC gereinigt. So wurden 100 mg (47%) der Titelverbindung erhalten.
¹H-NMR (300 MHz ,DMSO-d6), δ [ppm]= 1.38 (t, 3H), 3.82 (t, 2H), 4.42 - 4.59 (m, 4H), 5.40 - 5.70 (1 H), 7.27 (t, 1 H), 7.48 - 7.57 (m, 1 H), 7.66 - 7.83 (m, 3H), 7.91 (dd, 1 H), 8.00 (dd, 1 H), 8.22 - 8.31 (m, 2H), 8.72 (d, 1 H), 11.80 - 12.60 (1 H).

### Beispiel 92

### Ethyl-2-(9-ethyl-9H-carbazol-3-yl)-1-isopropyl-1 H-benzimidazol-5-carboxylat

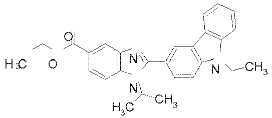

In Analogie zu Beispiel 37 wurden aus 90 mg (0.41 mmol) Ethyl-3-amino-4-(isopropylamino)benzoat und 60 mg (0.27 mmol) 9-Ethyl-9H-carbazol-3-carbaldehyd 127 mg (68 %) der Titelverbindung hergestellt.
¹H-NMR (300 MHz, DMSO-d6), δ [ppm]= 1.33 - 1.41 (m, 6H), 1.64 (d, 6H), 4.36 (q, 2H), 4.49 - 4.58 (m, 2H), 4.88 (quin, 1 H), 7.23 - 7.30 (m, 1 H), 7.50 - 7.56 (m, 1 H), 7.67 - 7.76 (m, 2H), 7.80 - 7.85 (m, 1 H), 7.87 - 7.91 (m, 1 H), 7.94 - 7.99 (m, 1 H), 8.27 - 8.32 (m, 2H), 8.51 (d, 1 H).

### Beispiel 93

### 2-(9-Ethyl-9H-carbazol-3-yl)-1-isopropyl-1 H-benzimidazol-5-carbonsäure

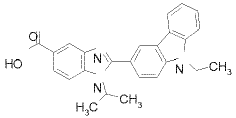

Durch Verseifung von Ethyl-2-(9-ethyl-9H-carbazol-3-yl)-1-isopropyl-1 H-benzimidazol-5-carboxylat mit Natronlauge wurde die Titelverbindung (34 mg, 33%) erhalten.
¹H-NMR (300 MHz, DMSO-d6), δ [ppm]= 1.39 (t, 3H), 1.74 (d, 6H), 4.58 (q, 2H), 5.01 (quin, 1 H), 7.33 (t, 1 H), 7.59 (t, 1 H), 7.76 (d, 1 H), 7.85 - 7.92 (m, 1 H), 7.95 - 8.01 (m, 1 H), 8.08 - 8.14 (m, 1 H), 8.26 - 8.33 (m, 2H), 8.36 (s, 1 H), 8.67 (s, 1 H), 12.86 - 13.64 (br., 1 H).

### Beispiel 94

### 2-(9-Ethyl-9H-carbazol-3-yl)-1-[2-(morpholin-4-yl)ethyl]-1 H-benzimidazol-5-carbonsäure

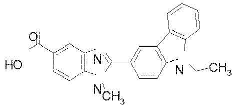

In Analogie zu Beispiel 38 wurde durch Verseifung von Ethyl-2-(9-ethyl-9H-carbazol-3-yl)-1-methyl-1 H-benzimidazol-5-carboxylat mit Natronlauge die Titelverbindung (9 mg, 10%) erhalten.
¹H-NMR (400 MHz, DMSO-d6), δ [ppm]= 1.38 (t, 3H), 4.01 (s, 3H), 4.54 (q, 2H), 7.27 (t, 1 H), 7.49 - 7.56 (m, 1 H), 7.66 - 7.72 (m, 2H), 7.81 (d, 1 H), 7.93 (dd, 1 H), 7.98 (dd, 1 H), 8.27 (s, 1 H), 8.32 (d, 1 H), 8.70 (d, 1 H), COOH nicht angegeben.

### Beispiel 95

### Methyl-1-ethyl-2-(9-ethyl-9H-carbazol-3-yl)-1 H-benzimidazol-5-carboxylat

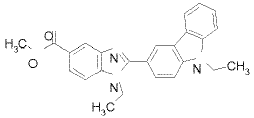

In Analogie zu Beispiel 1 wurden aus 9.22 g (25.0 mmol) Methyl-2-(9-ethyl-9H-carbazol-3-yl)-1 H-benzimidazol-5-carboxylat und 11.68 g (74.9 mmol) Ethyliodid 3.16 g (32%) Methyl-1-ethyl-2-(9-ethyl-9H-carbazol-3-yl)-1H-benzimidazol-5-carboxylat und 2.99 g (30%) des isomeren Methyl-1-ethyl-2-(9-ethyl-9H-carbazol-3-yl)-1 H-benzimidazol-6-carboxylat erhalten.

### Methyl-1-ethyl-2-(9-ethyl-9H-carbazol-3-yl)-1 H-benzimidazol-5-carboxylat

¹H-NMR (400 MHz , DMSO-d6), δ [ppm]= 1.37 (td, 6H), 3.90 (s, 3H), 4.45 (q, 2H), 4.53 (q, 2H), 7.24 - 7.29 (m, 1 H), 7.53 (td, 1 H), 7.69 (d, 1 H), 7.78 - 7.83 (m, 2H), 7.86 - 7.90 (m, 1 H), 7.94 (dd, 1 H), 8.28 - 8.33 (m, 2H), 8.61 (d, 1 H).

### Methyl-1-ethyl-2-(9-ethyl-9H-carbazol-3-yl)-1 H-benzimidazol-6-carboxylat

¹H-NMR (400 MHz , DMSO-d6), δ [ppm]= 1.38 (t, 6H), 3.91 (s, 3H), 4.48 - 4.57 (m, 4H), 7.24 - 7.30 (m, 1 H), 7.53 (td, 1 H), 7.69 (d, 1 H), 7.78 (d, 1 H), 7.81 - 7.84 (m, 1 H), 7.87 - 7.93 (m, 2H), 8.27 - 8.33 (m, 2H), 8.63 (d, 1 H).

### Beispiel 96

### 2-(9-Allyl-9H-carbazol-3-yl)-1-(2-methoxyethyl)-1H-benzimidazol-5-carbonsäure

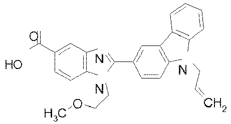

In Analogie zu Beispiel 14 wurden aus 56 mg (0.24 mmol) 9-Allyl-9H-carbazol-3-carbaldehyd und 100 mg (0.48 mmol) 3-Amino-4-[(2-methoxyethyl)amino]benzoesäure 3 mg (3%) der Titelverbindung erhalten.
¹H-NMR (300 MHz ,CHLOROFORM-d), δ [ppm]= 3.34 (s, 3H), 3.85 (t, 2H), 4.55 (t, 2H), 5.00 (d, 2H), 5.11 (d, 1H), 5.24 (d, 1H), 5.98 - 6.13 (m, 1H), 7.28 - 7.35 (m, 1H), 7.43 - 7.47 (m, 1H), 7.50 - 7.60 (m, 3H), 7.93 (dd, 1H), 8.10 - 8.19 (m, 2H), 8.63 - 8.68 (m, 2H), COOH nicht angegeben.

### Beispiel 97

### Ethyl-1-(2-methoxyethyl)-2-[9-(2-methoxyethyl)-9H-carbazol-3-yl]-1 H-benzimidazol-5-carboxylat

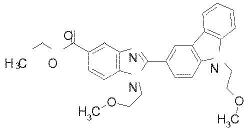

In Analogie zu Beispiel 14 wurden aus 32 mg (0.13 mmol) 9-Allyl-9H-carbazol-3-carbaldehyd und 60 mg (0.25 mmol) Ethyl-3-amino-4-[(2-methoxyethyl)amino]benzoat 30 mg (24%) der Titelverbindung erhalten.
¹H-NMR (300 MHz , DMSO-d6): δ [ppm]= 1.37 (t, 3H), 3.11 - 3.15 (m, 3H), 3.21 (s, 3H), 3.75 (dt, 4H), 4.36 (q, 2H), 4.62 (dt, 4H), 7.27 (t, 1 H), 7.48 - 7.55 (m, 1 H), 7.69 (d, 1 H), 7.78 - 7.84 (m, 2H), 7.89 - 7.96 (m, 2H), 8.23 - 8.30 (m, 2H), 8.67 (d, 1 H).

### Beispiel 98

### 1-(2-Methoxyethyl)-2-[9-(2-methoxyethyl)-9H-carbazol-3-yl]-1 H-benzimidazol-5-carbonsäure

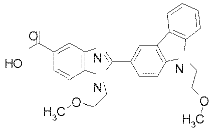

In Analogie zu Beispiel 38 wurde durch Verseifung von 30 mg Ethyl-1-(2-methoxyethyl)-2-[9-(2-methoxyethyl)-9H-carbazol-3-yl]-1 H-benzimidazol-5-carboxylat mit Natronlauge die Titelverbindung (18 mg, 61%) erhalten.
¹H-NMR (400MHz, DMSO-d6): δ [ppm]= 3.14 (s, 3H), 3.21 (s, 3H), 3.73 (t, 2H), 3.77 (t, 2H), 4.59 (t, 2H), 4.65 (t, 2H), 7.23 - 7.32 (m, 1 H), 7.51 (t, 1 H), 7.69 (d, 1 H), 7.80 (dd, 2H), 7.89 - 7.97 (m, 2H), 8.23 - 8.30 (m, 2H), 8.67 (d, 1 H), 12.74 (br. s., 1 H).

### Beispiel 99

### Ethyl-2-(9-ethyl-9H-carbazol-3-yl)-1-(3-methoxypropyl)-1 H-benzimidazol-5-carboxylat

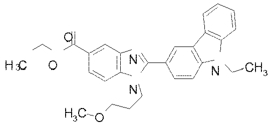

In Analogie zu Beispiel 14 wurde die Titelverbindung (100 mg, 51%) aus 100 mg (0.40 mmol) Ethyl-3-amino-4-[(2-methoxyethyl)amino]benzoat und 60 mg (0.26 mmol) 9-Ethyl-9H-carbazol-3-carbaldehyd gewonnen.
¹H-NMR (300 MHz , DMSO-d6), δ [ppm]= 1.37 (t, 6H), 1.93 - 2.04 (m, 2H), 3.05 (s, 3H), 3.22 (t, 2H), 4.36 (q, 2H), 4.48 - 4.60 (m, 4H), 7.28 (t, 1 H), 7.49 - 7.57 (m, 1 H), 7.70 (d, 1 H), 7.77 - 7.85 (m, 2H), 7.94 (td, 2H), 8.26 - 8.32 (m, 2H), 8.64 (d, 1 H).

### Beispiel 100

### 2-(9-Ethyl-9H-carbazol-3-yl)-1-(3-methoxypropyl)-1 H-benzimidazol-5-carbonsäure

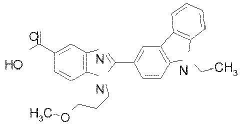

Durch Verseifung von 394 mg (0.89 mmol) Ethyl-2-(9-ethyl-9H-carbazol-3-yl)-1-(3-methoxypropyl)-1 H-benzimidazol-5-carboxylat mit Natronlauge wurde die Titelverbindung (7 mg, 2%) erhalten.
¹H-NMR (500 MHz, DMSO-d6), δ [ppm]= 1.38 (t, 3H), 1.96 - 2.03 (m, 2H), 3.08 (s, 3H), 3.23 - 3.27 (m, 2H), 4.50 - 4.58 (m, 4H), 7.27 (t, 1 H), 7.53 (td, 1 H), 7.69 (d, 1 H), 7.73 (d, 1 H), 7.81 (d, 1 H), 7.88 (dd, 1 H), 7.93 (dd, 1 H), 8.25 (d, 1 H), 8.29 (d, 1 H), 8.64 (d, 1 H), COOH nicht angegeben.

### Beispiel 101

### 2-[2-(9-Ethyl-9H-carbazol-3-yl)-1-(2-methoxyethyl)-1 H-benzimidazol-5-yl]propan-2-ol

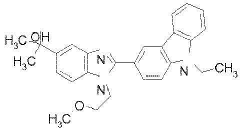

70 mg (0.16 mmol) Methyl-2-(9-ethyl-9H-carbazol-3-yl)-1-(2-methoxyethyl)-1 H-benzimidazol-5-carboxylat wurden in 5 ml Tetrahydrofuran (THF) gelöst und bei RT mit 1.0 ml (1.0 mmol) einer Lösung von Methylmagnesiumbromid in THF (1 M) versetzt. Das Reaktionsgemisch wurde auf 10 ml gesättigte Ammoniumchloridlösung gegossen und dreimal mit Ethylacetat extrahiert. Die gesammelten organischen Phasen wurden im Vakuum eingeengt und mittels präparativer HPLC aufgereinigt. Auf diese Weise wurden 37 mg (52%) der Titelverbindung erhalten.
¹H-NMR (500 MHz, DMSO-d6), δ [ppm]= 1.37 (t, 3H), 1.52 (s, 6H), 3.16 (s, 3H), 3.73 (t, 2H), 4.48 - 4.55 (m, 4H), 5.02 (s, 1 H), 7.24 - 7.28 (m, 1 H), 7.41 (dd, 1 H), 7.52 (ddd, 1 H), 7.57 (d, 1 H), 7.67 (d, 1 H), 7.76 - 7.79 (m, 2H), 7.92 (dd, 1 H), 8.26 (d, 1 H), 8.64 (d, 1 H).

### Beispiel 102

### 1-(Cyclopropylmethyl)-2-(9-ethyl-9H-carbazol-3-yl)-6-methoxy-1 H-benzimidazol-5-carbonsäure

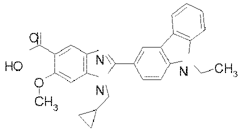

1.57 g (3.74 mmol) 3-(5-Brom-6-methoxy-1H-benzimidazol-2-yl)-9-ethyl-9H-carbazol wurden in 24 ml DMF gegeben, mit 1.01 g (7.47 mmol) (Brommethyl)cyclopropan und 4.87 g (14.94 mmol) Caesiumcarbonat versetzt und 2.5 h bei 60 °C gerührt. Nach dem Abkühlen auf RT wurde Wasser hinzugefügt, dreimal mit Ethylacetat extrahiert, die organischen Phasen mit gesättigter Natriumchloridlösung gewaschen, mit Natriumsulfat getrocknet und im Vakuum eingeengt.

Das so erhaltene Rohprodukt (2.0 g) wurde in 20 ml THF aufgenommen, 1.67 g Molybdänhexacarbonyl, 124 mg (0.42 mmol) Tri-tert.-butylphosphin-tetrafluoroborat, 325 mg (0.42 mmol) trans-Bis(acetato)bis[o-(di-o-tolylphosphino)benzyl]dipalladium(II) 1.54 ml (37.9 mmol) Methanol und 1.89 ml (12.65 mmol) 1,8-Diazabicyclo(5.4.0)undec-7-en hinzugefügt und in der Mikrowelle 25 min auf 125°C erhitzt (Einstrahlung 100W, 6 bar). Nach dem Abkühlen wurde das Reaktionsgemisch mit Wasser versetzt, mit Dichlormethan extrahiert und die organische Phase anschließend im Vakuum eingeengt.

Der Rückstand (1.18 g) wurde in 48 ml Methanol aufgenommen, mit 205 mg (5.15 mmol) Natriumhydroxid versetzt und bei 40 °C 24h gerührt. Das Gemisch wurde mit 2M Salzsäure neutralisiert, der ausgefallenen Niederschlag abgetrennt, getrocknet und mittels präparativer HPLC aufgereinigt. Auf diese Weise wurden 358 mg der Titelverbindung erhalten
¹H-NMR (400 MHz ,DMSO-d6), δ [ppm]= 0.10 - 0.20 (m, 2H), 0.29 - 0.40 (m, 2H), 1.04 (d, 1 H), 1.39 (t, 3H), 3.93 (s, 3H), 4.35 (d, 2H), 4.53 (q, 2H), 7.26 (t, 1 H), 7.39 (s, 1 H), 7.48 - 7.56 (m, 1 H), 7.67 (d, 1 H), 7.73 - 7.80 (m, 1 H), 7.81 - 7.89 (m, 1 H), 7.97 (s, 1 H), 8.25 - 8.33 (m, 1 H), 8.58 (d, 1 H), 12.25 - 12.39 (1 H).

### Beispiel 103

### 2-(9-Ethyl-9H-carbazol-3-yl)-6-methoxy-1-(2-methoxyethyl)-1H-benzimidazol-5-carbonsäure

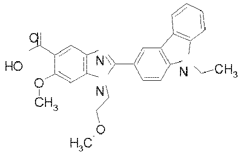

Ausgehend von 3-(5-Brom-6-methoxy-1H-benzimidazol-2-yl)-9-ethyl-9H-carbazol und (2-Bromethyl)methylether wurde in Analogie zu Beispiel 102 die Titelverbindung erhalten.
¹H-NMR (400MHz, DMSO-d6): δ [ppm]= 1.38 (t, 3H), 3.15 (s, 3H), 3.71 (t, 2H), 3.84 (s, 3H), 4.47 - 4.55 (m, 4H), 7.15 (s, 1 H), 7.22 - 7.29 (m, 1 H), 7.47 - 7.53 (m, 1H), 7.60 (s, 1 H), 7.66 (d, 1 H), 7.75 (d, 1 H), 7.89 (dd, 1 H), 8.25 (d, 1 H), 8.61 (d, 1 H), COOH nicht angegeben.

### Beispiel 104

### 1-(Cyclopropylmethyl)-2-(9-ethyl-9H-carbazol-3-yl)-4-methoxy-1 H-benzimidazol-5-carbonsäure

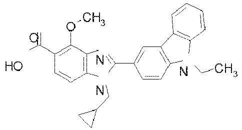

In Analogie zu Intermediat 31 wurde ausgehend von 4-Brom-3-methoxy-2-nitroanilin und 9-Ethyl-9H-carbazol-3-carbaldehyd zunächst 3-(5-Brom-4-methoxy-1H-benzimidazol-2-yl)-9-ethyl-9H-carbazol hergestellt, welches dann analog Beispiel 102 zur Titelverbindung umgesetzt wurde.
¹H-NMR (400 MHz ,DMSO-d6), δ [ppm]= 0.14 (q, 2H), 0.33 - 0.39 (m, 2H), 1.00 - 1.09 (m, 1 H), 1.38 (t, 3H), 4.29 - 4.33 (m, 2H), 4.34 (s, 3H), 4.53 (q, 2H), 7.26 (t, 1 H), 7.43 (d, 1 H), 7.49 - 7.56 (m, 1 H), 7.61 - 7.72 (m, 2H), 7.77 - 7.83 (m, 1 H), 7.85 - 7.90 (m, 1 H), 8.30 (d, 1 H), 8.61 (d, 1 H), 12.34 (br. s., 1 H).

### Beispiel 105

### 2-(9-Ethyl-9H-carbazol-3-yl)-1-(2-methoxyethyl)-4-methyl-1 H-benzimidazol-5-carbonsäure

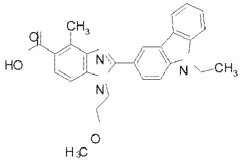

2-(9-Ethyl-9H-carbazol-3-yl)-1-(2-methoxyethyl)-4-methyl-1 H-benzimidazol-5-carbonsäure wurde in Analogie zu Beispiel 9, ausgehend von 4-Acetamido-2-methylbenzoesäure , hergestellt.
¹H-NMR (400 MHz, DMSO-d6), δ [ppm]= 1.39 (t, 3H), 2.90 (s, 3H), 3.14 (s, 3H), 3.70 (t, 2H), 4.46 - 4.58 (m, 4H), 7.26 (t, 1 H), 7.47 - 7.57 (m, 2H), 7.67 (d, 1 H), 7.78 (d, 1 H), 7.84 (d, 1H), 7.92 (dd, 1 H), 8.19 - 8.32 (m, 1H), 8.64 (d, 1 H), 12.30 - 12.70 (1 H).

### Beispiel 106

### 5-[2-(9-Ethyl-9H-carbazol-3-yl)-1-(2-methoxyethyl)-1H-benzimidazol-5-yl]-1,3,4-oxadiazol-2(3H)-on

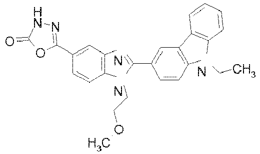

250 mg (0.61 mmol) 2-(9-Ethyl-9H-carbazol-3-yl)-1-(2-methoxyethyl)-1H-benzimidazol-5-carbonsäure wurden in 10 ml THF gegeben, mit 147 mg (0.91 mmol) 1,1'-Carbonyldiimidazol und 36 mg (0.30 mmol) 4-Dimethylaminopyridin versetzt und 1.5 h bei 60 °C gerührt. Nach dem Abkühlen auf RT wurden 0.10 ml (2.12 mmol) Hydrazinhydrat hinzugefügt, 2 h bei RT gerührt und das Gemisch dann im Vakuum eingeengt. Anschließend wurde in 10 ml DMF aufgenommen, mit 303 mg (1.87 mmol) 1,1'-Carbonyldiimidazol in 10 ml THF versetzt und 72h bei RT belassen. DasGemisch wurde erneut eingeengt und mittels präparativer HPLC augereinigt. Auf diese Weise wurden 200 mg der Titelverbindung gewonnen.
¹H-NMR (300 MHz ,DMSO-d6), δ [ppm]= 1.38 (t, 3H), 3.15 (s, 3H), 3.73 (t, 2H), 4.48 - 4.63 (m, 4H), 7.27 (t, 1 H), 7.53 (t, 1 H), 7.65 - 7.89 (m, 4H), 7.96 (dd, 1 H), 8.06 (d, 1 H), 8.27 (d, 1 H), 8.69 (d, 1 H), 12.47 (br. s., 1 H).

### Beispiel 107

### 1-(Cyclopropylmethyl)-2-(9-ethyl-9H-pyrido[2,3-b]indol-3-yl)-4-methyl-1 H-benzimidazol-5-carbonsäure

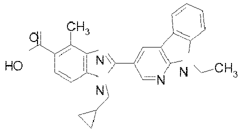

In Analogie zu Beispiel 9 wurde zuerst Methyl-1-(cyclopropylmethyl)-2-(9-ethyl-9H-pyrido[2,3-b]indol-3-yl)-4-methyl-1H-benzimidazol-5-carboxylat aus Methyl-3-amino-4-[(cyclopropylmethyl)amino]-2-methylbenzoat und 9-Ethyl-9H-pyrido[2,3-b]indol-3-carb-aldehyd hergestellt, welches anschließend mit Natronlauge verseift wurde.
¹H-NMR (300MHz, DMSO-d6): δ [ppm]= 0.16 (q, 2H), 0.32 - 0.41 (m, 2H), 0.95 - 1.05 (m, 1 H), 1.42 (t, 3H), 2.90 (s, 3H), 4.34 (d, 2H), 4.61 (q, 2H), 7.35 (t, 1 H), 7.58 - 7.66 (m, 2H), 7.78 (d, 1 H), 7.87 (d, 1 H), 8.38 (d, 1 H), 8.89 (d, 1 H), 9.01 (d, 1 H), COOH nicht angegeben.

### Beispiel 108

### 1-(Cyclopropylmethyl)-2-(9-ethyl-9H-beta-carbolin-6-yl)-1 H-benzimidazol-5-carbonsäure

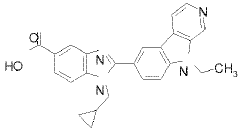

In Analogie zu Beispiel 38 wurde die Titelverbindung (20 mg, 79%) nach Verseifung des entsprechenden Ethylesters (27 mg, 0.1 mmol) erhalten.
¹H-NMR (300 MHz ,DMSO-d6), δ [ppm]= 0.16 (q, 2H), 0.32 - 0.40 (m, 2H), 0.95 - 1.08 (m, 1 H), 1.43 (t, 3H), 4.38 (d, 2H), 4.64 (q, 2H), 7.79 - 7.85 (m, 1 H), 7.89 - 7.97 (m, 2H), 8.03 - 8.08 (m, 1 H), 8.26 - 8.32 (m, 2H), 8.46 (d, 1 H), 8.78 (d, 1 H), 9.15 (s, 1 H), COOH nicht angegeben.

### Beispiel 109

### 1-(Cyclopropylmethyl)-2-(5-ethyl-5H-pyrido[4,3-b]indol-8-yl)-1 H-benzimidazol-5-carbonsäure

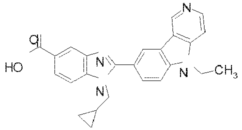

In Analogie zu Beispiel 38 wurde die Titelverbindung (25 mg) nach Verseifung des entsprechenden Ethylesters (27 mg, 0.1 mmol) erhalten.
¹H-NMR (300 MHz ,DMSO-d6), δ [ppm]= 0.15 (q, 2H), 0.32 - 0.40 (m, 2H), 0.95 - 1.08 (m, 1 H), 1.39 (t, 3H), 4.39 (d, 2H), 4.56 (q, 2H), 7.73 (d, 1 H), 7.81 - 7.87 (m, 1 H), 7.89 - 8.02 (m, 3H), 8.28 (d, 1H), 8.55 (d, 1H), 8.77 (d, 1H), 9.50 (s, 1H), COOH nicht angegeben.

### Beispiel 110

### 1-(Cyclopropylmethyl)-2-(9-ethyl-9H-carbazol-3-yl)-6-methyl-1 H-benzimidazol-5-carbonsäure

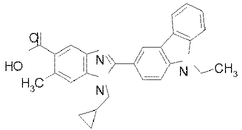

1-(Cyclopropylmethyl)-2-(9-ethyl-9H-carbazol-3-yl)-6-methyl-1 H-benzimidazol-5-carbonsäure wurde in Analogie zu Beispiel 9 hergestellt.
¹H-NMR (300 MHz ,DMSO-d6), δ [ppm]= 0.13 (q, 2H), 0.31 - 0.40 (m, 2H), 1.00 - 1.09 (m, 1 H), 1.38 (t, 3H), 2.70 (s, 3H), 4.33 (d, 2H), 4.53 (q, 2H), 7.26 (t, 1 H), 7.49 - 7.56 (m, 1 H), 7.63 (s, 1 H), 7.69 (d, 1 H), 7.77 - 7.82 (m, 1 H), 7.84 - 7.90 (m, 1 H), 8.20 (s, 1 H), 8.30 (d, 1 H), 8.62 (d, 1H)., 12.56 (br., s, 1 H).

### Beispiel 111

### 2-(9-Ethyl-9H-carbazol-3-yl)-1-(2-methoxyethyl)-6-methyl-1 H-benzimidazol-5-carbonsäure

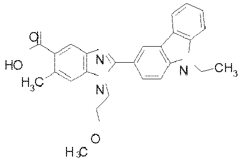

2-(9-Ethyl-9H-carbazol-3-yl)-1-(2-methoxyethyl)-6-methyl-1 H-benzimidazol-5-carbonsäure wurde in Analogie zu Beispiel 9 hergestellt.
¹H-NMR (300 MHz ,DMSO-d6), δ [ppm]= 1.37 (t, 3H), 2.70 (s, 3H), 3.14 (s, 3H), 3.72 (t, 2H), 4.50 - 4.58 (m, 4H), 7.27 (t, 1 H), 7.52 (t, 1 H), 7.61 (s, 1 H), 7.69 (d, 1 H), 7.80 (d, 1H), 7.94 (dd, 1H), 8.20 (s, 1H), 8.27 (d, 1H), 8.68 (d, 1H), 12.58 (br., s, 1H).

### Beispiel 112

### 1-(Cyclopropylmethyl)-2-(9-ethyl-9H-carbazol-3-yl)-6-fluor-1 H-benzimidazol-5-carbonsäure

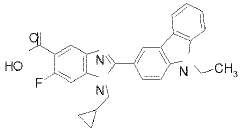

In Analogie zu Beispiel 102 wurde ausgehend von 3-(5-Brom-6-fluor-1 H-benzimidazol-2-yl)-9-ethyl-9H-carbazol und (Brommethyl)cyclopropan die Titelverbindung erhalten.
¹H-NMR (400 MHz ,DMSO-d6), δ [ppm]= 0.15 (q, 2H), 0.31 - 0.38 (m, 2H), 0.98 - 1.07 (m, 1 H), 1.38 (t, 3H), 4.34 (d, 2H), 4.52 (q, 2H), 7.26 (t, 1 H), 7.48 - 7.55 (m, 1 H), 7.64 - 7.75 (m, 2H), 7.77 - 7.82 (m, 1H), 7.85 - 7.90 (m, 1H), 8.15 (d, 1H), 8.29 (d, 1H), 8.62 (d, 1H),. 12.20 - 13.40 (1 H).

### Beispiel 113

### 2-(9-Ethyl-9H-carbazol-3-yl)-6-fluor-1-(2-methoxyethyl)-1 H-benzimidazol-5-carbonsäure

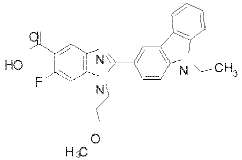

In Analogie zu Beispiel 102 wurde ausgehend von 3-(5-Brom-6-fluor-1 H-benzimidazol-2-yl)-9-ethyl-9H-carbazol und (2-Bromethyl)methylether die Titelverbindung erhalten.
¹H-NMR (400 MHz ,DMSO-d6), δ [ppm]= 1.38 (t, 3H), 3.14 (s, 3H), 3.69 (t, 2H), 4.49 - 4.58 (m, 4H), 7.27 (t, 1 H), 7.50 - 7.56 (m, 1 H), 7.66 - 7.71 (m, 2H), 7.79 (d, 1 H), 7.93 (dd, 1H), 8.14 (d, 1 H), 8.26 (d, 1H), 8.66 (d, 1H), 12.60 - 13.40 (1H).

### Beispiel 114

### 2-(9-Ethyl-9H-carbazol-3-yl)-4-fluor-1-(2-methoxyethyl)-1 H-benzimidazol-5-carbonsäure

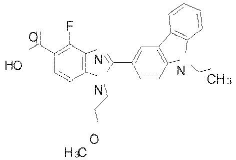

In Analogie zu Beispiel 102 wurde ausgehend von 20 g (49.0 mmol) 3-(5-Brom-4-fluor-1 H-benzimidazol-2-yl)-9-ethyl-9H-carbazol und 13.6 g (98 mmol) (2-Bromethyl)methyl-ether die Titelverbindung (432 mg) erhalten.
¹H-NMR (300 MHz ,DMSO-d6), δ [ppm]= 1 H-NMR (300MHz, DMSO-d6): δ [ppm]= 1.38 (t, 3H), 3.13 (s, 3H), 3.71 (t, 2H), 4.48 - 4.64 (m, 4H), 7.27 (t, 1 H), 7.49 - 7.57 (m, 1 H), 7.61 (d, 1 H), 7.69 (d, 1 H), 7.74 - 7.85 (m, 2H), 7.96 (dd, 1 H), 8.28 (d, 1 H), 8.70 (d, 1 H), 12.97 (br. s., 1 H).

### Beispiel 115

### 1-(Cyclopropylmethyl)-2-(9-ethyl-9H-carbazol-3-yl)-4-fluor-1 H-benzimidazol-5-carbonsäure

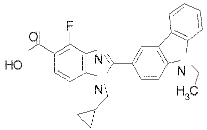

In Analogie zu Beispiel 102 wurde ausgehend von 3.26 g (8.0 mmol) 3-(5-Brom-4-fluor-1 H-benzimidazol-2-yl)-9-ethyl-9H-carbazol und 1.45 g (16.0 mmol) (Brommethyl)-cyclopropan die Titelverbindung (15 mg) erhalten.
¹H-NMR (400MHz, CHLOROFORM-d): δ [ppm]= 0.17 - 0.24 (m, 2H), 0.51 - 0.59 (m, 2H), 1.20 (d, 1H), 1.51 (t, 3H), 4.27 (d, 2H), 4.46 (q, 2H), 7.28 - 7.35 (m, 2H), 7.47 - 7.51 (m, 1H), 7.52 - 7.58 (m, 2H), 7.85 (dd, 1H), 7.97 - 8.02 (m, 1H), 8.16 (d, 1H), 8.52 (s, 1 H), COOH nicht angegeben.

### Beispiel 116

### 1-(Cyclopropylmethyl)-2-(9-ethyl-9H-pyrido[2,3-b]indol-6-yl)-1 H-benzimidazol-5-carbonsäure

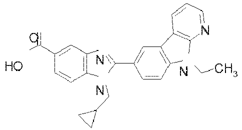

In Analogie zu Beispiel 38 wurde die Titelverbindung (20 mg, 59%) nach Verseifung des entsprechenden Ethylesters (35 mg, 0.1 mmol) erhalten.
¹H-NMR (400 MHz ,DMSO-d6), δ [ppm]= 0.13 - 0.19 (m, 2H), 0.33 - 0.39 (m, 2H), 1.02 - 1.11 (m, 1 H), 1.42 (t, 3H), 4.37 (d, 2H), 4.61 (q, 2H), 7.32 (dd, 1 H), 7.77 (d, 1 H), 7.88 - 7.99 (m, 3H), 8.26 (d, 1 H), 8.55 (dd, 1 H), 8.68 - 8.73 (m, 2H), COOH nicht angegeben.

### Beispiel 117

### 1-(2-Cyclopropylethyl)-2-(9-ethyl-9H-carbazol-3-yl)-1 H-benzimidazol-5-carbonsäure

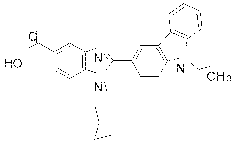

Zunächst wurde ausgehend von Ethyl-4-chlor-3-nitrobenzoat und 2-Cyclopropylethanamin, analog der Synthese von Intermediat 2, Ethyl-3-amino-4-{[2-cyclopropylethyl]amino}benzoat hergestellt, welches in Analogie zu Beispiel 9 mit 9-Ethyl-9H-carbazol-3-carbaldehyd umgesetzt und verseift wurde.
¹H-NMR (400MHz, DMSO-d6): δ [ppm]= -0.18 - -0.13 (m, 2H), 0.18 - 0.24 (m, 2H), 0.47 (d, 1 H), 1.38 (t, 3H), 1.63 (q, 2H), 4.50 - 4.58 (m, 4H), 7.28 (t, 1 H), 7.54 (t, 1 H), 7.70 (d, 1 H), 7.80 - 7.86 (m, 2H), 7.88 - 7.97 (m, 2H), 8.26 - 8.33 (m, 2H), 8.64 (s, 1 H).

### Beispiel 118

### 1-(2-Methoxyethyl)-2-(9-propyl-9H-carbazol-3-yl)-1 H-benzimidazol-5-carbonsäure

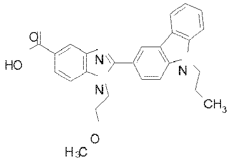

Zunächst wurde aus 9H-Carbazol-3-carbaldehyd und 3-Brompropan analog der Synthese von Intermediat 16, 9-Propyl-9H-carbazol-3-carbaldehyd hergestellt, welches dann in Analogie zu Beispiel 9 mit Ethyl-3-amino-4-[(2-methoxyethyl)amino]benzoat umgesetzt und anschließend verseift wurde.
1 H-NMR (400MHz, CHLOROFORM-d): δ [ppm]= 1.03 (t, 3H), 1.94 - 2.03 (m, 2H), 3.34 (s, 3H), 3.85 (t, 2H), 4.36 (t, 2H), 4.55 (t, 2H), 7.28 - 7.32 (m, 1 H), 7.46 - 7.58 (m, 4H), 7.94 (dd, 1 H), 8.09 - 8.17 (m, 2H), 8.62 - 8.68 (m, 2H), COOH nicht angegeben.

### Beispiel 119

### 1-(2-Methoxyethyl)-2-[9-(prop-2-in-1-yl)-9H-carbazol-3-yl]-1H-benzimidazol-5-carbonsäure

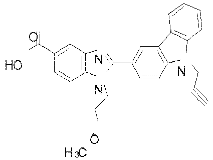

Zunächst wurde aus 9H-Carbazol-3-carbaldehyd und 3-Brompropin, analog der Synthese von Intermediat 16, 9-(Prop-2-in-1-yl)-9H-carbazol-3-carbaldehyd hergestellt, welches dann in Analogie zu Beispiel 9 mit Ethyl-3-amino-4-[(2-methoxyethyl)amino]benzoat umgesetzt und anschließend verseift wurde.
¹H-NMR (400MHz, CHLOROFORM-d): δ [ppm]= 2.31 - 2.35 (m, 1 H), 3.33 (s, 3H), 3.84 (t, 2H), 4.54 (t, 2H), 5.12 (d, 2H), 7.34 (ddd, 1 H), 7.53 - 7.58 (m, 3H), 7.65 (d, 1 H), 7.97 (dd, 1 H), 8.10 - 8.17 (m, 2H), 8.64 - 8.68 (m, 2H), COOH nicht angegeben..

### Beispiel 120

### 1-(Cyclopropylmethyl)-2-(9-propyl-9H-carbazol-3-yl)-1 H-benzimidazol-5-carbonsäure

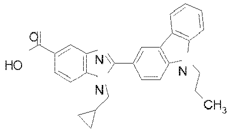

Zunächst wurde aus 9H-Carbazol-3-carbaldehyd und 3-Brompropan analog der Synthese von Intermediat 16, 9-Propyl-9H-carbazol-3-carbaldehyd hergestellt, welches dann in Analogie zu Beispiel 9 mit Ethyl-3-amino-4-[(cyclopropylmethyl)amino]benzoat umgesetzt und anschließend verseift wurde.
¹H-NMR (300MHz, CHLOROFORM-d): δ [ppm]= 0.18 - 0.25 (m, 2H), 0.49 - 0.58 (m, 2H), 1.03 (t, 3H), 1.16 - 1.28 (m, 1H), 1.99 (m, 2H), 4.29 (d, 2H), 4.36 (t, 2H), 7.28 - 7.33 (m, 1 H), 7.46 - 7.59 (m, 4H), 7.85 (dd, 1 H), 8.14 (t, 2H), 8.52 (s, 1 H), 8.67 (s, 1 H), COOH nicht angegeben.

### Beispiel 121

### 1-[(2,2-Dimethylcyclopropyl)methyl]-2-(9-ethyl-9H-carbazol-3-yl)-1 H-benzimidazol-5-carbonsäure

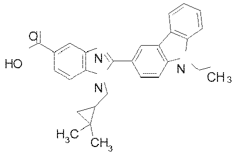

Zunächst wurde ausgehend von Ethyl-4-chlor-3-nitrobenzoat und 1-(2,2-Dimethyl-cycloprop-1-yl)methanamin, analog der Synthese von Intermediat 2, Ethyl-3-amino-4-{[(2,2-Dimethylcyclopropyl)methyl]amino}benzoat hergestellt, welches in Analogie zu Beispiel 9 mit 9-Ethyl-9H-carbazol-3-carbaldehyd umgesetzt und verseift wurde.
¹H-NMR (300MHz, DMSO-d6): δ [ppm]= 0.51 (dd, 2H), 0.70 (d, 1H), 0.85 (d, 6H), 1.37 (t, 3H), 4.26 - 4.66 (m, 4H), 7.27 (t, 1 H), 7.52 (t, 1 H), 7.69 (d, 1 H), 7.76 - 7.84 (m, 2H), 7.87 - 7.95 (m, 2H), 8.25 - 8.34 (m, 2H), 8.64 (d, 1 H).

### Beispiel 122

### Ethyl-2-(9-ethyl-9H-carbazol-3-yl)-1-(oxetan-3-ylmethyl)-1 H-benzimidazol-5-carboxylat

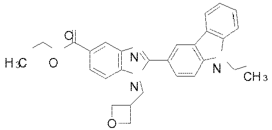

Zunächst wurde ausgehend von Ethyl-4-chlor-3-nitrobenzoat und Oxetan-3-yl-methanamin, analog der Synthese von Intermediat 2, Ethyl-3-amino-4-{[oxetan-3-ylmethyl]amino}benzoat hergestellt, welches in Analogie zu Beispiel 9 mit 9-Ethyl-9H-carbazol-3-carbaldehyd umgesetzt wurde.
¹H-NMR (300MHz, DMSO-d6): δ [ppm]= 1.38 (q, 6H), 4.01 - 4.10 (m, 2H), 4.31 - 4.44 (m, 4H), 4.54 (q, 2H), 4.86 (d, 2H), 7.28 (t, 1 H), 7.50 - 7.58 (m, 1 H), 7.70 (d, 1 H), 7.80 - 7.97 (m, 4H), 8.27 - 8.33 (m, 2H), 8.62 (d, 1 H).

### Beispiel 123

### 2-(9-Ethyl-9H-carbazol-3-yl)-1-(oxetan-3-ylmethyl)-1 H-benzimidazol-5-carbonsäure

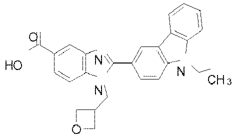

Analog Beispiel 2 wurde aus Ethyl-2-(9-ethyl-9H-carbazol-3-yl)-1-(oxetan-3-ylmethyl)-1 H-benzimidazol-5-carboxylat die Titelverbindung erhalten
¹H-NMR (300MHz, DMSO-d6): δ [ppm]= 1.39 (t, 3H), 3.22 - 3.39 (m, 1H), 4.08 (t, 2H), 4.42 (dd, 2H), 4.57 (q, 2H), 4.95 (d, 2H), 7.27 - 7.35 (m, 1 H), 7.56 (t, 1 H), 7.74 (d, 1 H), 7.88 - 7.98 (m, 2H), 8.04 (s, 2H), 8.26 - 8.35 (m, 2H), 8.71 (s, 1 H).

### Beispiel 124

### 2-[9-(Cyclobutylmethyl)-9H-carbazol-3-yl]-1-(2-methoxyethyl)-1 H-benzimidazol-5-carbonsäure

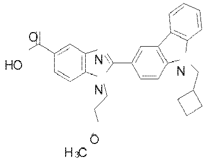

Zunächst wurde aus 9H-Carbazol-3-carbaldehyd und 1-Cyclobutylmethanamin, analog der Synthese von Intermediat 16, 9-(Cyclobutylmethyl)-9H-carbazol-3-carbaldehyd hergestellt, welches dann in Analogie zu Beispiel 9 mit Ethyl-3-amino-4-[(2-methoxyethyl)amino]benzoat umgesetzt und anschließend verseift wurde.
¹H-NMR (400MHz, CHLOROFORM-d): δ [ppm]= 1.89 - 1.95 (m, 4H), 2.03 - 2.12 (m, 2H), 2.97 - 3.06 (m, 1 H), 3.34 (s, 3H), 3.85 (t, 2H), 4.40 (d, 2H), 4.54 (t, 2H), 7.28 - 7.32 (m, 1 H), 7.48 - 7.61 (m, 4H), 7.93 (dd, 1 H), 8.10 - 8.16 (m, 2H), 8.61 - 8.67 (m, 2H), COOH nicht angegeben..

### Beispiel 125

### 2-[9-(Cyclobutylmethyl)-9H-carbazol-3-yl]-1-(cyclopropylmethyl)-1 H-benzimidazol-5-carbonsäure

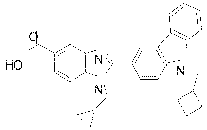

Zunächst wurde aus 9H-Carbazol-3-carbaldehyd und 1-Cyclobutylmethanamin, analog der Synthese von Intermediat 16, 9-(Cyclobutylmethyl)-9H-carbazol-3-carbaldehyd hergestellt, welches dann in Analogie zu Beispiel 9 mit Ethyl-3-amino-4-[(cyclopropylmethyl)amino]benzoat umgesetzt und anschließend verseift wurde.
¹H-NMR (400MHz, CHLOROFORM-d): δ [ppm]= 0.19 - 0.25 (m, 2H), 0.51 - 0.58 (m, 2H), 1.17 - 1.29 (m, 1H), 1.88 - 1.97 (m, 4H), 2.03 - 2.13 (m, 2H), 3.01 (quin, 1H), 4.29 (d, 2H), 4.40 (d, 2H), 7.28 - 7.32 (m, 1 H), 7.48 - 7.61 (m, 4H), 7.85 (dd, 1H), 8.11 - 8.17 (m, 2H), 8.51 (d, 1 H), 8.68 (s, 1 H), COOH nicht angegeben..

### Beispiel 126

### 5-{1-[2-(Dimethylamino)ethyl]-2-(9-ethyl-9H-carbazol-3-yl)-1H-benzimidazol-5-yl}-1,3,4-oxadiazol-2(3H)-on

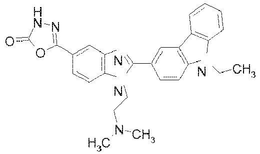

In Analogie zu Beispiel 106 wurde die Titelverbindung (53 mg) aus 100 mg (0.23 mmol) 1-[2-(Dimethylamino)ethyl]-2-(9-ethyl-9H-carbazol-3-yl)-1 H-benzimidazol-5-carbonsäure hergestellt.
¹H-NMR (300 MHz ,DMSO-d6), δ [ppm]= 1.37 (t, 3H), 2.02 (s, 6H), 2.57 - 2.65 (m, 2H), 4.47 - 4.60 (m, 4H), 7.27 (t, 1 H), 7.53 (t, 1 H), 7.67 - 7.78 (m, 2H), 7.79 - 7.89 (m, 2H), 7.93 (d, 1 H), 8.05 (s, 1 H), 8.29 (d, 1 H), 8.67 (s, 1 H), NH nicht angegeben.

### Beispiel 127

### 2-(9-Ethyl-9H-carbazol-3-yl)-1-(2-methoxyethyl)-1H-benzimidazol-5-sulfonamid

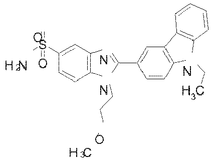

In Analogie zu Beispiel 4 / Variante B wurde die Titelverbindung (50 mg, 27%) aus 61 mg (0.27 mmol) 9H-Carbazol-3-carbaldehyd und 100 mg (0.41 mmol) 3-Amino-4-[(2-methoxyethyl)amino]benzolsulfonamid hergestellt.
¹H-NMR (400 MHz ,DMSO-d6), δ [ppm]= 1.38 (t, 3H), 3.14 (s, 3H), 3.72 (t, 2H), 4.53 (q, 2H), 4.61 (t, 2H), 7.24 - 7.32 (m, 3H), 7.53 (td, 1 H), 7.69 (d, 1 H), 7.76 (dd, 1 H), 7.81 (d, 1 H), 7.88 (d, 1 H), 7.95 (dd, 1 H), 8.14 (d, 1 H), 8.28 (d, 1 H), 8.69 (d, 1 H).

### Beispiel 128

### 9-Ethyl-3-[1-methyl-5-(1H-1,2,3-triazol-1-yl)-1H-benzimidazol-2-yl]-9H-carbazol

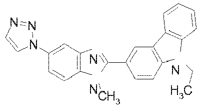

In Analogie zu Beispiel 4 / Variante B wurde die Titelverbindung (20 mg, 5%) aus 157 mg (0.71 mmol) 9H-Carbazol-3-carbaldehyd und 200 mg (1.06 mmol) N¹-Methyl-4-(1H-1,2,3-triazol-1-yl)benzol-1,2-diamin hergestellt.
¹H-NMR (400 MHz ,DMSO-d6), δ [ppm]= 1.37 (t, 3H), 4.05 (s, 3H), 4.54 (q, 2H), 7.24 - 7.31 (m, 1H), 7.53 (td, 1H), 7.70 (d, 1H), 7.80 - 7.88 (m, 3H), 7.98 - 8.03 (m, 2H), 8.19 (t, 1 H), 8.34 (d, 1 H), 8.73 (d, 1 H), 8.89 (d, 1 H).

### Beispiel 129

### 2-(9-Ethyl-9H-carbazol-3-yl)-N,1-dimethyl-1 H-benzimidazol-5-sulfonamid

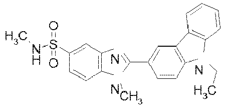

In Analogie zu Beispiel 4 / Variante B wurde die Titelverbindung (150 mg, 36%) aus 138 mg (0.62 mmol) 9H-Carbazol-3-carbaldehyd und 200 mg (0.93 mmol) 3-Amino-N-methyl-4-(methylamino)benzolsulfonamid hergestellt.
¹H-NMR (400 MHz ,DMSO-d6), δ [ppm]= 1.37 (t, 3H), 2.41 (d, 3H), 4.03 (s, 3H), 4.54 (q, 2H), 7.25 - 7.30 (m, 1 H), 7.39 (q, 1 H), 7.53 (ddd, 1 H), 7.67 - 7.74 (m, 2H), 7.85 (dd, 2H), 7.99 (dd, 1 H), 8.09 (d, 1 H), 8.33 (d, 1 H), 8.72 (d, 1 H).

### Beispiel 130

### 2-(9-Ethyl-9H-carbazol-3-yl)-1-[2-(trifluormethoxy)ethyl]-1H-benzimidazol-5-carbonsäure

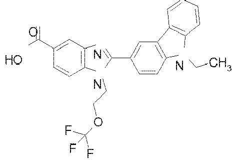

In Analogie zur Synthese von Intermediat 29 wurde zunächst Ethyl-3-amino-4-{[2-(trifluormethoxy)ethyl]amino}benzoat aus 2-(Trifluormethoxy)ethanamin und Ethyl-4-chlor-3-nitrobenzoat hergestellt, welches dann analog Beispiel 4 / Variante B mit 9H-Carbazol-3-carbaldehyd zu Ethyl-2-(9-ethyl-9H-carbazol-3-yl)-1-[2-(trifluor-methoxy)ethyl]-1 H-benzimidazol-5-carboxylat umgesetzt wurde. Nach der Verseifung dieses Esters mit Natronlauge analog Beispiel 2 wurde die Titelverbindung erhalten.
¹H-NMR (400 MHz ,DMSO-d6), δ [ppm]= 1.35 - 1.41 (m, 3H), 4.40 (t, 2H), 4.54 (q, 2H), 4.80 (t, 2H), 7.27 (t, 1 H), 7.53 (td, 1 H), 7.69 (d, 1 H), 7.79 - 7.89 (m, 3H), 7.94 (dd, 1 H), 8.25 - 8.30 (m, 2H), 8.60 (d, 1 H), COOH nicht angegeben.

### Beispiel 131

### 1-(Cyclopropylmethyl)-2-(9-ethyl-1-methyl-9H-beta-carbolin-3-yl)-1H-benzimidazol-5-carbonsäure

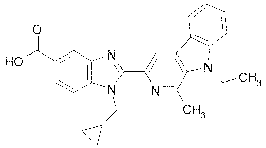

111 mg (0.44 mmol) 9-Ethyl-1-methyl-9H-beta-carbolin-3-carbonsäure wurden in 1 ml Toluol gegeben, mit 0.38 ml (5.24 mmol) Thionylchlorid versetzt, für 3 h zum Rückfluss erhitzt und dann im Vakuum eingeengt. Der Rückstand wurde in 3.8 ml Acetonitril aufgenommen, 180 mg (0.87 mmol) 3-Amino-4-[(cyclopropylmethyl)amino]benzoesäure hinzugefügt und in einer Mikrowelle für 30 min auf 180 °C erhitzt. Das Gemisch wurde im Vakuum eingeengt und mittels präparativer HPLC aufgereinigt. Auf diese Weise wurden 5 mg (3%) der Titelverbindung erhalten.
¹H-NMR (400 MHz ,DMSO-d6), δ [ppm]= 0.42 - 0.51 (m, 4H), 1.39 - 1.46 (m, 3H), 1.47 - 1.54 (m, 1H), 3.12 - 3.19 (m, 3H), 4.71 - 4.79 (m, 2H), 4.93 (d, 2H), 7.36 (t, 1H), 7.64 - 7.71 (m, 1 H), 7.78 - 7.86 (m, 2H), 7.90 - 7.97 (m, 1 H), 8.29 (d, 1 H), 8.46 (d, 1 H), 9.02 (s, 1 H), COOH nicht angegeben.

### Beispiel 132

### 1-(Cyclopropylmethyl)-2-(9-ethyl-9H-carbazol-3-yl)-6-methoxy-1 H-benzimidazol-5-carbonsäure

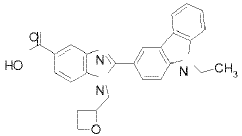

200 mg (0.54 mmol) Methyl-2-(9-ethyl-9H-carbazol-3-yl)-1 H-benzimidazol-5-carboxylat wurden in 3 ml DMF gegeben, mit 245 mg (1.62 mmol) 2-(Bromomethyl)oxetan und 530 mg (1.62 mmol) Caesiumcarbonat versetzt und 2.5 h bei 60 °C gerührt. Nach dem Abkühlen auf RT wurde Wasser hinzugefügt, nacheinander mit Ethylacetat und Toluol extrahiert, die organischen Phasen mit Natriumsulfat getrocknet und im Vakuum eingeengt.

Das so erhaltene Rohprodukt (0.37 g) wurde in 5 ml THF und 15 ml Methanol aufgenommen, mit 170 mg (4.3 mmol) Natriumhydroxid versetzt und bei 40 °C 24h gerührt. Das Gemisch wurde mit 2M Salzsäure neutralisiert, der ausgefallene Niederschlag abgetrennt, getrocknet und mittels präparativer HPLC aufgereinigt. Auf diese Weise wurden 21 mg (6%) der Titelverbindung erhalten.
¹H-NMR (400 MHz ,DMSO-d6), δ [ppm]= 1.39 (t, 3H), 2.31 - 2.43 (m, 1 H), 2.62 - 2.73 (m, 1H), 4.40 (dt, 1H), 4.47 - 4.62 (m, 4H), 4.79 (dd, 1H), 5.15 (qd, 1H), 7.27 (t, 1H), 7.49 - 7.55 (m, 1 H), 7.68 (d, 1 H), 7.77 - 7.82 (m, 2H), 7.90 (dd, 1 H), 7.96 (dd, 1 H), 8.22 - 8.27 (m, 2H), 8.71 (d, 1 H), COOH nicht angegeben.

### Beispiel 133

### 2-(9-Ethyl-9H-carbazol-3-yl)-1-(tetrahydrofuran-2-ylmethyl)-1 H-benzimidazol-5-carbonsäure

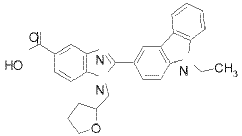

Analog der Vorschrift von Beispiel 132 wurden ausgehend von 200 mg (0.54 mmol) Methyl-2-(9-ethyl-9H-carbazol-3-yl)-1 H-benzimidazol-5-carboxylat und 196 mg (1.62 mmol) 2-(Chlormethyl)tetrahydrofuran 17 mg der Titelverbindung erhalten.
¹H-NMR (400 MHz ,DMSO-d6), δ [ppm]= 1.38 (t, 3H), 1.46 - 1.56 (m, 1H), 1.75 (quin, 2H), 1.88 - 1.99 (m, 1 H), 3.54 - 3.68 (m, 2H), 4.19 - 4.28 (m, 1 H), 4.39 - 4.57 (m, 4H), 7.27 (t, 1 H), 7.49 - 7.55 (m, 1 H), 7.68 (d, 1 H), 7.79 (dd, 2H), 7.87 - 7.96 (m, 2H), 8.24 - 8.28 (m, 2H), 8.67 (d, 1 H), COOH nicht angegeben.

### Beispiel 134

### 2-(9-Ethyl-9H-carbazol-3-yl)-1-[(2R)-2-hydroxy-3-methoxypropyl]-1 H-benzimidazol-5-carbonsäure

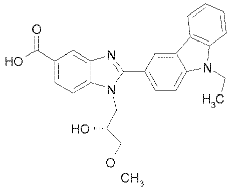

Analog der Vorschrift von Beispiel 132 wurden ausgehend von 200 mg (0.54 mmol) Methyl-2-(9-ethyl-9H-carbazol-3-yl)-1 H-benzimidazol-5-carboxylat und 143 mg (1.62 mmol) (2R)-2-(Methoxymethyl)oxiran 57 mg der Titelverbindung erhalten.
¹H-NMR (400 MHz ,DMSO-d6), δ [ppm]= 1.38 (t, 3H), 3.19 (s, 3H), 3.31 (m, 3H), 4.34 - 4.43 (m, 1 H), 4.46 - 4.57 (m, 3H), 7.26 (t, 1 H), 7.49 - 7.54 (m, 1 H), 7.65 - 7.74 (m, 2H), 7.78 (d, 1 H), 7.90 (dd, 1 H), 8.03 (dd, 1 H), 8.22 - 8.28 (m, 2H), 8.74 (d, 1 H), COOH und OH nicht angegeben.

### Beispiel 135

### 2-(9-Ethyl-9H-carbazol-3-yl)-1-[(2S)-2-hydroxy-3-methoxypropyl]-1 H-benzimidazol-5-carbonsäure

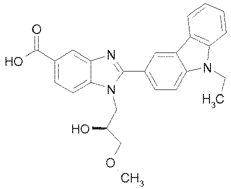

Analog der Vorschrift von Beispiel 132 wurden ausgehend von 200 mg (0.54 mmol) Methyl-2-(9-ethyl-9H-carbazol-3-yl)-1 H-benzimidazol-5-carboxylat und 143 mg (1.62 mmol) (2S)-2-(Methoxymethyl)oxiran 20 mg der Titelverbindung erhalten.
¹H-NMR (500 MHz ,DMSO-d6), δ [ppm]= 1.38 (t, 3H), 3.19 (s, 3H), 3.27 - 3.35 (m, 3H), 4.35 - 4.56 (m, 4H), 7.26 (t, 1 H), 7.49 - 7.54 (m, 1 H), 7.66 - 7.73 (m, 2H), 7.78 (d, 1 H), 7.90 (dd, 1H), 8.03 (dd, 1H), 8.23 - 8.27 (m, 2H), 8.74 (d, 1H), COOH und OH nicht angegeben.

### Beispiel 136

### 2-(9-Ethyl-9H-carbazol-3-yl)-1-[2-(methylsulfonyl)ethyl]-1H-benzimidazol-5-carbonsäure

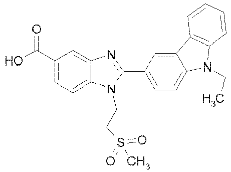

Analog der Vorschrift von Beispiel 132 wurden ausgehend von 200 mg (0.54 mmol) Methyl-2-(9-ethyl-9H-carbazol-3-yl)-1 H-benzimidazol-5-carboxylat und 304 mg (1.62 mmol) 1-Brom-2-(methylsulfonyl)ethan 4 mg der Titelverbindung erhalten.
¹H-NMR (500 MHz ,DMSO-d6), δ [ppm]= 1.39 (t, 3H), 3.00 (s, 3H), 3.81 - 3.87 (m, 2H), 4.54 (q, 2H), 4.81 - 4.86 (m, 2H), 7.27 (t, 1 H), 7.50 - 7.55 (m, 1 H), 7.69 (d, 1 H), 7.79 - 7.84 (m, 2H), 7.95 (dd, 2H), 8.27 (d, 1 H), 8.29 - 8.34 (m, 1 H), 8.64 (d, 1 H), COOH nicht angegeben.

### Beispiel 137

### 1-(2-Cyclopropyl-2-hydroxyethyl)-2-(9-ethyl-9H-carbazol-3-yl)-1 H-benzimidazol-5-carbonsäure

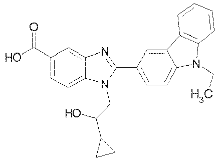

Analog der Vorschrift von Beispiel 132 wurden ausgehend von 200 mg (0.54 mmol) Methyl-2-(9-ethyl-9H-carbazol-3-yl)-1 H-benzimidazol-5-carboxylat und 137 mg (1.62 mmol) 2-Cyclopropyloxiran 10 mg der Titelverbindung erhalten.
¹H-NMR (300 MHz ,DMSO-d6), δ [ppm]= -0.04 (m, 1 H), 0.14 - 0.33 (m, 3H), 0.67 - 0.77 (m, 1H), 1.37 (t, 3H), 3.60 (t, 1 H), 4.45 - 4.59 (m, 4H), 5.12 (d, 1 H), 7.27 (t, 1 H), 7.52 (t, 1 H), 7.66 - 7.82 (m, 3H), 7.89 (dd, 1 H), 8.00 (dd, 1 H), 8.23 - 8.30 (m, 2H), 8.73 (d, 1 H), COOH nicht angegeben.

### Beispiel 138

### 1-[(2S)-2,3-Dihydroxypropyl]-2-(9-ethyl-9H-carbazol-3-yl)-1 H-benzimidazol-5-carbonsäure

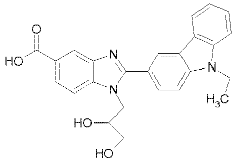

Analog der Vorschrift von Beispiel 132 wurden ausgehend von 200 mg (0.54 mmol) Methyl-2-(9-ethyl-9H-carbazol-3-yl)-1 H-benzimidazol-5-carboxylat und 118 mg (1.62 mmol) (2S)-Oxiran-2-ylmethanol 9 mg der Titelverbindung erhalten.
¹H-NMR (400MHz, DMSO-d6): δ [ppm]= 1.38 (t, 3H), 3.34 - 3.49 (m, 2H), 4.02 (d, 1 H), 4.34 (dd, 1 H), 4.48 - 4.57 (m, 3H), 7.26 (t, 1 H), 7.51 (t, 1 H), 7.67 (d, 1 H), 7.75 (dd, 2H), 7.90 (d, 1H), 8.07 (dd, 1H), 8.23 - 8.28 (m, 2H), 8.78 (s, 1H), COOH und OH nicht angegeben.

### Beispiel 139 und 140

### 1-[(2R)-1,4-Dioxan-2-ylmethyl]-2-(9-ethyl-9H-carbazol-3-yl)-1 H-benzimidazol-5-carbonsäure

### 1-[(2S)-1,4-Dioxan-2-ylmethyl]-2-(9-ethyl-9H-carbazol-3-yl)-1 H-benzimidazol-5-carbonsäure

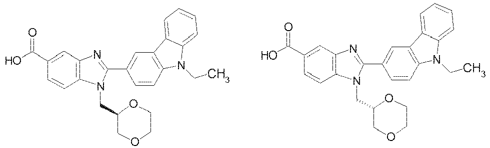

In Analogie zu Synthese von Intermediat 29 wurde zunächst *rac*-Ethyl-3-amino-4-[(1,4-dioxan-2-ylmethyl)amino]benzoat aus *rac*-1-(1,4-Dioxan-2-yl)methanamin und Ethyl-4-chlor-3-nitrobenzoat hergestellt, welches dann analog Beispiel 4 / Variante B mit 9H-Carbazol-3-carbaldehyd zu *rac*-Ethyl-1-(1,4-dioxan-2-ylmethyl)-2-(9-ethyl-9H-carbazol-3-yl)-1 H-benzimidazol-5-carboxylat umgesetzt wurde. Nach der Verseifung des Esters mit Natronlauge analog Beispiel 2 wurde die racemische Titelverbindung erhalten. Diese wurde mittels chiraler präparativer HPLC in die Enantiomere 1 und 2 der Titelverbindung aufgetrennt.

### Enantiomer 1

¹H-NMR (300 MHz ,DMSO-d6), δ [ppm]= 1.38 (t, 3H), 3.17 - 3.29 (m, 1H), 3.37 - 3.49 (m, 2H), 3.62 (dd, 2H), 3.73 (dd, 1H), 3.96 (d, 1H), 4.42 - 4.59 (m, 4H), 7.28 (t, 1H), 7.48 - 7.58 (m, 1 H), 7.70 (d, 1 H), 7.76 - 7.85 (m, 2H), 7.87 - 8.02 (m, 2H), 8.22 - 8.33 (m, 2H), 8.72 (d, 1 H), COOH nicht angegeben.

### Enantiomer 2

¹H-NMR (300 MHz ,DMSO-d6), δ [ppm]= 1.38 (t, 3H), 3.17 - 3.29 (m, 1H), 3.37 - 3.49 (m, 2H), 3.62 (dd, 2H), 3.73 (dd, 1H), 3.96 (d, 1H), 4.42 - 4.59 (m, 4H), 7.28 (t, 1H), 7.48 - 7.58 (m, 1 H), 7.70 (d, 1 H), 7.76 - 7.85 (m, 2H), 7.87 - 8.02 (m, 2H), 8.22 - 8.33 (m, 2H), 8.72 (d, 1 H), COOH nicht angegeben.

### Beispiel 141

### 2-(9-Ethyl-6-methyl-9H-carbazol-3-yl)-1-(2-methoxyethyl)-4-methyl-1 H-benzimidazol-5-carbonsäure

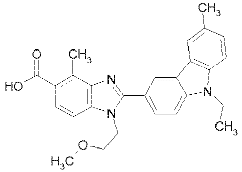

In Analogie zu Beispiel 4 / Variante B wurde zuerst Methyl-2-(9-ethyl-6-methyl-9H-carbazol-3-yl)-1-(2-methoxyethyl)-4-methyl-1 H-benzimidazol-5-carboxylat aus 1.0 g (4.2 mmol) Methyl-3-amino-4-[(2-methoxyethyl)amino]-2-methylbenzoat und 1.0 g (4.2 mmol) 9-Ethyl-6-methyl-9H-carbazol-3-carbaldehyd (CAS-Nr 122060-05-3) hergestellt.
¹H-NMR (400 MHz, DMSO-d6), δ [ppm]= 1.35 (t, 3H), 2.50 (s, 3H), 2.89 (s, 3H), 3.12 (s, 3H), 3.69 (t, 2H), 3.87 (s, 3H), 4.49 (q, 2H), 4.55 (t, 2H), 7.35 (dd, 1 H), 7.58 (t, 2H), 7.75 (d, 1H), 7.83 (d, 1H), 7.90 (dd, 1H), 8.06 (s, 1H), 8.59 (d, 1H).

Dieses wurde anschließend analog Beispiel 2 zu 2-(9-Ethyl-6-methyl-9H-carbazol-3-yl)-1-(2-methoxyethyl)-4-methyl-1 H-benzimidazol-5-carbonsäure umgesetzt. (920 mg 50%,).
¹H-NMR (300 MHz, DMSO-d6), δ [ppm]= 1.35 (t, 3H), 2.49 (s, 3H), 2.89 (s, 3H), 3.12 (s, 3H), 3.69 (t, 2H), 4.43 - 4.59 (m, 4H), 7.34 (dd, 1 H), 7.56 (m, 2H), 7.75 (d, 1 H), 7.81 - 7.93 (m, 2H), 8.07 (s, 1H), 8.59 (d, 1H), 12.25 - 12.95 (br., 1H).

### Beispiel 142

### 2-(6-Chlor-9-ethyl-9H-carbazol-3-yl)-1-(2-methoxyethyl)-4-methyl-1 H-benzimidazol-5-carbonsäure

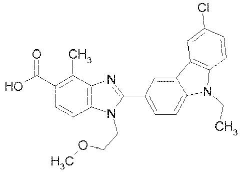

In Analogie zu Beispiel 4 / Variante B wurde aus 3.0 g (12.6 mmol) Methyl-3-amino-4-[(2-methoxyethyl)amino]-2-methylbenzoat und 3.2 g (12.6 mmol) 6-Chlor-9-ethyl-9H-carbazol-3-carbaldehyd zunächst Methyl-2-(6-chlor-9-ethyl-9H-carbazol-3-yl)-1-(2-methoxyethyl)-4-methyl-1 H-benzimidazol-5-carboxylat (5.3 g, 81%) hergestellt.
¹H-NMR (300 MHz, DMSO-d6), δ [ppm]= 1.36 (t, 3H), 2.88 (s, 3H), 3.09 (s, 3H), 3.66 (t, 2H), 3.86 (s, 3H), 4.48 - 4.60 (m, 4H), 7.53 (dd, 1 H), 7.60 (d, 1 H), 7.73 (d, 1 H), 7.79 - 7.87 (m, 2H), 7.96 (dd, 1H), 8.41 (d, 1H), 8.69 (d, 1H).

Dieses wurde anschließend analog Beispiel 2 zu 2-(6-Chlor-9-ethyl-9H-carbazol-3-yl)-1-(2-methoxyethyl)-4-methyl-1 H-benzimidazol-5-carbonsäure umgesetzt. (3.4 g, 64%). ¹H-NMR (400 MHz, DMSO-d6), δ [ppm]= 1.36 (t, 3H), 2.89 (s, 3H), 3.10 (s, 3H), 3.68 (t, 2H), 4.50 - 4.62 (m, 4H), 7.54 (dd, 1 H), 7.63 (d, 1 H), 7.74 (d, 1 H), 7.82 - 7.91 (m, 2H), 7.98 (dd, 1H), 8.41 (d, 1 H), 8.71 (d, 1H), 12.52 - 12.80 (br., 1H).

### Beispiel 143

### 2-(8-Chlor-9-ethyl-9H-carbazol-3-yl)-1-(2-methoxyethyl)-4-methyl-1 H-benzimidazol-5-carbonsäure

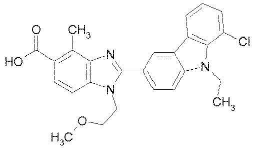

In Analogie zu Beispiel 4 / Variante B wurde aus 1.0 g (4.3 mmol) Methyl-3-amino-4-[(2-methoxyethyl)amino]-2-methylbenzoat und 740 mg (2.9 mmol) 8-Chlor-9-ethyl-9H-carbazol-3-carbaldehyd zunächst Methyl-2-(8-chlor-9-ethyl-9H-carbazol-3-yl)-1-(2-methoxyethyl)-4-methyl-1 H-benzimidazol-5-carboxylat (2.12 g, 99%) hergestellt.
¹H-NMR (400 MHz, DMSO-d6), δ [ppm]= 1.43 (t, 3H), 2.89 (s, 3H), 3.11 (s, 3H), 3.66 - 3.70 (m, 2H), 3.87 (s, 3H), 4.55 (t, 2H), 4.83 - 4.90 (m, 2H), 7.26 (t, 1H), 7.54 (dd, 1H), 7.61 (d, 1H), 7.84 (d, 1H), 7.88 (d, 1H), 7.96 - 8.01 (m, 1H), 8.30 (dd, 1H), 8.69 (d, 1H). Dieses wurde anschließend analog Beispiel 2 zu 2-(8-Chlor-9-ethyl-9H-carbazol-3-yl)-1-(2-methoxyethyl)-4-methyl-1 H-benzimidazol-5-carbonsäure umgesetzt. (700 mg, 33%). ¹H-NMR (300 MHz, DMSO-d6), δ [ppm]= 1.43 (t, 3H), 2.89 (s, 3H), 3.11 (s, 3H), 3.69 (t, 2H), 4.54 (t, 2H), 4.86 (q, 2H), 7.26 (t, 1H), 7.51 - 7.60 (m, 2H), 7.82 - 7.91 (m, 2H), 7.99 (dd, 1H), 8.27 - 8.33 (m, 1H), 8.69 (d, 1H), 12.56 (br. s., 1H).

### Biologische Beispiele:

### 1. Syngenes Maus-Endometriosemodell:

Die syngene Induktion der Endometriose in Mäusen stellt ein gängiges Tiermodell dar, um die Wirksamkeit von Substanzen für die Therapie der Endometriose zu testen. Die Endometriose wird experimentell durch Transplantation von murinen Uterusfragmenten einer Spendermaus desselben Stammes in die Bauchhöhle der Empfänger-Maus induziert. Es wurden weibliche Tiere des balb/c Stammes verwendet. Mittels Vaginalabstrich wird der Zyklus der Maus bestimmt. Es werden ausschließlich Spendertiere verwendet, die sich im Östrus befinden. Die Spendertiere werden getötet und die Uterushörner werden entnommen und anschließend longitudinal aufgeschnitten. Aus den Uteri werden mit einer Stanze 2 mm Bioptate ausgestanzt, die anschließend ins Empfängertier eingenäht werden. Die Empfängertiere werden narkotisiert und einer Laparotomie unterzogen. Während des Eingriffs werden 6 Uterusstanzen einer Donormaus an das parietale Peritoneum der Empfängermaus angenäht. Am Tag nach diesem Eingriff beginnt die 4-wöchige Behandlung mit den zu testenden Substanzen. Nach 28 Tagen werden die Tiere in einer finalen Laparotomie eröffnet und die Läsionsgrößen werden bestimmt. Die angewachsenen Läsionen werden fotografisch festgehalten und die Fläche wird mittels AxioVision Software vermessen. Pro Behandlungsgruppe werden 14 Tiere eingesetzt. Im s.c. Ansatz (Abbildung 1) wurde die erfindungsgemäße Substanz (Beispiel 4 in 4 verschiedenen Dosierungsschemata getestet und die Läsionsgröße im Vergleich zu den mit Vehikel behandelten Tieren ausgewertet. Folgende Dosierungen wurden getestet: 0,4 2 10 und 50 mg/kg/Tag. In der Abbildung 2 sind die mittleren Läsionsgrößen (in mm²) pro Tier dargestellt (y- Achse).

### 2. Durchflusszytometrie:

Zur Gewinnung der Zellen aus dem Peritoneum wurden 3 ml kaltes PBS (phosphate buffered saline) in das Peritoneum des toten Tieres injiziert und nach leichter Massage des Bauches wieder entnommen. Die Zellen dieser Peritoneallavage wurden bei 1400 UpM 2 min herunterzentrifugiert und in 500 µl PBS mit 2% FCS (fetal calf serum) wieder aufgenommen. Für jede Färbung mit verschiedenen Antikörper-Flourochromkonjugat-Mixen wurden 100 µl dieser Zellsuspension verwendet. Die Zellen wurden dafür in 96 Lochplatten wieder herunterzentrifugiert und in 50 µl einer Antikörperlösung in der 1:300 verdünnter anti CD11b- Pacific Blue (eBioscience), 1:200 verdünnter Anti F4/80-PE (eBioscience), 1:200 verdünnter Anti Gr1- APC-Cy7 (BD Pharmigen), 1:400 verdünnter Anti CD11c-PE-Cy7 (BD Pharmigen) und 1:400 verdünnter Anti MHCII-FITC (BD Pharmigen) resuspendiert und für 20 min im Dunkeln auf Eis gelagert. Anschließend wurden 150 µl PBS mit 2%FCS zugegeben und die Zellen erneut 2 min bei 1400 UpM herunter zentrifugiert. Die 200 µl Überstand wurden verworfen und die Zellen mit 200 µl PBS mit 2% FCS gewaschen und erneut abzentrifugiert. Anschließend wurden sie in 200 µl PBS mit 2%FCS und 1:5000 verdünntem 5mg/ml DAPI (Sigma) aufgenommen und die Fluoreszenz-Intensität der einzelne Fluorochrome pro Zelle mit einem FACS Canto II Durchflusszytometer gemessen. Dargestellt ist die durchschnittliche Intensität von MHCII-FITC auf allen DAPI negativen F4/80 und CD11 b doppelpositiven Makrophagen pro Maus.

### 3. Nachweis des Antagonismus des humanen Prostaglandin E2 (Subtyp EP₄) Rezeptorsignals

### 3. 1 Nachweisprinzip

### Nachweis der Antagonisierung des hEP4-Signals

Die Bindung des Agonisten Prostaglandin E2 (PGE2) an den EP4 Subtyp des humanen hPGE2-R (hEP4-R) induziert die Aktivierung membranständiger Adenylatzyklasen und damit die Bildung von cAMP. In Gegenwart des Phosphodiesteraseinhibitors IBMX akkumuliert dieses cAMP intrazellulär und wird nach Zell-Lyse in einem kompetitiven Nachweisverfahren eingesetzt. Bei diesem Verfahren konkurriert es mit einem fluoreszenzmarkierten cAMP (cAMP-d2) um die Bindung an einen Eu-Kryptat markierten Anti-cAMP Antikörpers. In Abwesenheit zellulären cAMPs bildet sich ein Komplex zwischen dem Eu-Kryptat markierten Anti-cAMP Antikörper und dem cAMP-d2 Molekül aus, welcher nach Anregung bei 337 nm einen auf FRET (fluorescence resonance energy transfer) basierenden Energietransfer zum cAMP-d2 Tracer ermöglicht und zu einem langanhaltenden Fluoreszenzsignal führt (Emission bei 665 und 620 nm). Dieses Signal wird zeitlich aufgelöst, d.h. nach Abklingen der Hintergrundsfluoreszenz in einem geeigneten Messgerät gemessen (time resolved; TR-FRET). Die Well-Ratio Ermittlung (Emision665nm/Emision620nm *10000) ermöglicht es zudem Einzelmessungsunterschiede in den Zugabemengen der Detektionsreagenzien zu normieren.

Durch Prostglandin-E2-Gabe und Anstieg des intrazellulären cAMPs kommt es zu einer Erniedrigung des FRET- Signals, welches im Falle einer antagonistischen Substanzwirkung wieder ansteigt.

### 3.2. Nachweisverfahren

### 3.2.1 Test auf Antagonismus (Angaben pro Vertiefung einer 384-Loch Platte):

Zu den in einer Testplatte vorgelegten Substanzlösungen (50 nl; 100% DMSO) werden 4 µl einer den hEP4 exprimierenden Zellsuspension (2500 Zellen/Well) zugegeben, die außerdem bereits den cAMP-D2 Tracer enthält. Nach einer 20 minütigen Vorinkubation bei Raumtemperatur werden 2 µl einer 3xPGE2 Lösung zugegeben und in Gegenwart einer EC80-Konzentration des Agonisten (0,4 nM) für weitere 60 min bei Raumtemperatur inkubiert (Volumen: ∼6 µl), bevor die Gesamtreaktion anschließend durch Zugabe von 2 µl Lysisbuffer gestoppt wird (Volumen: ∼8 µl). Weitere 20 min bei Raumtemperatur später wird das Zell-Lysat entsprechend den Herstellangaben in einem TR-FRET geeigneten Messgerät gemessen (vergleiche cAMP HTRF-Assay Kit: Cisbio International 62AM6PEJ high range)

### 3.2.2 Test auf Agonismus (Angaben pro Vertiefung einer 384-Loch Platte):

Zu den in einer Testplatte vorgelegten Substanzlösungen (50 nl; 100% DMSO) werden 4 µl einer den hEP4 exprimierenden Zellsuspension (2500 Zellen/Well) zugegeben, die außerdem bereits den cAMP-D2 Tracer enthält. Nach einer 20 minütigen Vorinkubation bei Raumtemperatur werden 2 µl Zellmedium zugegeben und für weitere 60 min bei Raumtemperatur inkubiert (Volumen: ∼6 µl), bevor die Gesamtreaktion anschließend durch Zugabe von 2 µl Lysisbuffer gestoppt wird (Volumen: ∼8 µl). Weitere 20 min bei Raumtemperatur später wird das Zell-Lysat entsprechend den Herstellangaben in einem TR-FRET geeigneten Messgerät gemessen (vgl. cAMP HTRF-Assay Kit: Cisbio International 62AM6PEJ high range)

### 4. Nachweis des Antagonismus des humanen Prostaglandin E2 (Subtyp EP2) Rezeptorsignals

### 4. 1 Nachweisprinzip

Die Bindung von PGE2 an den EP2-Subtyp des humanen PGE2-Rezeptors induziert die Aktivierung membranständiger Adenylatzyklasen und führt zur Bildung von cAMP. In Gegenwart des Phosphodiesteraseinhibitors IBMX wird das aufgrund dieser Stimulation akkumulierte und mittels Zell-Lyse freigesetzte cAMP in einem kompetitiven Nachweisverfahren eingesetzt. In diesem Test konkurriert das im Lysat vorhandene cAMP mit einem fluoreszenzmarkierten cAMP (cAMP-d2) um die Bindung an einen Eu-Kryptat markierten Anti-cAMP Antikörper.

In Abwesenheit von zellulärem cAMP entsteht ein maximales Signal, welches auf die Bindung dieses cAMP-d2 Moleküls an den Antikörpers zurückzuführen ist. Nach Anregung des cAMP-d2 Moleküls bei 337 nm kommt es zu einem Fluoreszenz Resonanz Energie Transfer (FRET) zu den Eu-Kryptat Molekülen des (damit markierten) Anti-cAMP Antikörpers, gefolgt von einem langanhaltenden Emissionssignal bei 665 nm (sowie bei 620 nM). Beide Signale werden in einem geeigneten Messgerät zeitlich versetzt, d.h. nach Abklingen der Hintergrundsfluoreszenz gemessen. Jegliche Erhöhung des durch Prostglandin-E₂-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 4.2. Nachweisverfahren

### 4.2.1. Test auf Antagonismus (Angaben pro Vertiefung einer 384-Loch-Platte):

Zu einer Testplatte mit den bereits vorgelegten Substanzlösungen (0.05 µl; 100 % DMSO, Konzentrationsbereich von 0.8 nM - 16.5 µM) wurden 4 µl einer cAMP-d2/Zellsuspension zugegeben (625000 Zellen/ml). Nach einer 20-minütigen Vorinkubation bei Raumtemperatur wurden 2 µl einer 3xPGE2 Lösung (1.5 nM, in PBS-IBMX) zugegeben und in Gegenwart des Agonisten für weitere 60 min bei Raumtemperatur inkubiert (Volumen: ∼ 6 µl). Anschließend wurde die Reaktion durch Zugabe von 2 µl Lysispuffer gestoppt und vor der eigentlichen Messung für weitere 20 min bei Raumtemperatur inkubiert (Volumen: ∼ 8 µl).

### 5. Nachweis des Antagonismus des humanen Prostaglandin D Rezeptorsignals

### 5. 1 Nachweisprinzip

Die Bindung von Prostaglandin D2 an den humanen PGD-Rezeptor induziert die Aktivierung membranständiger Adenylatzyklasen und führt zur Bildung von cAMP. In Gegenwart des Phosphodiesteraseinhibitors IBMX wird das aufgrund dieser Stimulation akkumulierte und mittels Zell-Lyse freigesetzte cAMP in einem kompetitiven Nachweisverfahren eingesetzt. In diesem Test konkurriert das im Lysat vorhandene cAMP mit einem fluoreszenzmarkierten cAMP (cAMP-d2) um die Bindung an einen Eu-Kryptat markierten Anti-cAMP Antikörper.

In Abwesenheit von zellulärem cAMP entsteht ein maximales Signal, welches auf die Bindung dieses cAMP-d2 Moleküls an den Antikörpers zurückzuführen ist. Nach Anregung des cAMP-d2 Moleküls bei 337 nm kommt es zu einem Fluoreszenz-Resonanz-EnergieTransfer (FRET) zu den Eu-Kryptat-Molekülen des (damit markierten) Anti-cAMP Antikörpers, gefolgt von einem langanhaltenden Emissionssignal bei 665 nm (sowie bei 620 nM). Beide Signale werden in einem geeigneten Messgerät zeitlich versetzt, d.h. nach Abklingen der Hintergrundsfluoreszenz gemessen. Jegliche Erhöhung des durch Prostglandin-E2-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 5.2. Nachweisverfahren

### 5.2.1. Test auf Antagonismus (Angaben pro Vertiefung einer 384-Loch-Platte):

Zu einer Testplatte mit den bereits vorgelegten Substanzlösungen (0.05 µl; 100 % DMSO, Konzentrationsbereich von 0.8 nM - 16.5 µM) wurden 4 µl einer cAMP-d2/Zellsuspension zugegeben (625000 Zellen/ml). Nach einer 20-minütigen Vorinkubation bei Raumtemperatur (RT) wurden 2 µl einer 3xPGD2 Lösung (6 nM, in PBS-IBMX) zugegeben und in Gegenwart des Agonisten für weitere 30 min bei RT inkubiert (Volumen: ∼ 6 µl). Anschließend wurde die Reaktion durch Zugabe von 2 µl Lysispuffer gestoppt und vor der eigentlichen Messung für weitere 20 min bei RT inkubiert (Volumen: ∼ 8 µl).

### Abbildungen

### Abbildung 1: Präventives Endometriosemodell: s.c. Experiment 1

Die Anwendung der erfindungsgemäßen Verbindung (Beispiel 4) führte im genannten Dosisbereich nach subkutaner Verabreichung zu einer signifikanten Verkleinerung der endometrialen Läsionen im Endometriosemodell in der Maus. Statistik: 1-Weg ANOVA gefolgt von Tukey Post-Hoc Test,*** p<0.005 vs Vehikel, * p<0.05 vs Vehikel; 3 Ausreißer (GRUBBS Test) ausgeschlossen (1 Tier von den Gruppen 0.4 / 2 / 10 jeweils).

### Abbildung 2: Präventives Endometriosemodel (p.o. Experiment)

Die Anwendung der erfindungsgemäßen Verbindung (Beispiel 4) führte im genannten Dosisbereich nach oraler Verabreichung zu einer signifikanten Verkleinerung der endometrialen Läsionen. Statistik:
** p<0.01, unpaired t-Test, ausgeschlossene Tiere: 1 Tier der Kontrollgruppe und 3 Tiere in der Behandlungsgruppe wegen Anwachsens der Läsionen am Darm.

### Abbildung 3: Makrophagenaktivierung

Die p.o. Applikation von Beispiel 4 führte im Endometriosemodell in der Maus zu einer hochsignifikant reduzierten Anzahl von aktivierten Makrophagen in der Peritonealflüssigkeit im Vergleich zur Vehikelkontrolle (Nachweis im FACS) **** p<0.001 im t-test.

**Tabelle 1: Antagonismus der humanen EP4 Rezeptors durch die erfindungsgemäßen Verbindungen**

| Beispiel | Antagonismus hEP4 IC₅₀ [M] |
|---|---|
| 1 | 3.1E-6 |
| 2 | 3.12E-7 |
| 3 | 1.75E-7 |
| 4 | 5.59E-9 |
| 5 | 4.04E-7 |
| 6 | 1.44E-8 |
| 7 | 1.43E-6 |
| 8 | 1.97E-8 |
| 9 | 7.12E-8 |
| 10 | 1.03E-8 |
| 11 | 6.36E-9 |
| 12 | 1.02E-8 |
| 13 | 1.8E-8 |
| 14 | 3.34E-8 |
| 15 | 2.38E-8 |
| 16 | 2.86E-8 |
| 17 | 1.47E-8 |
| 18 | 2.28E-8 |
| 19 | 3.22E-8 |
| 20 | 6.83E-7 |
| 21 | 1.03E-7 |
| 22 | 6.57E-8 |
| 23 | 1.43E-7 |
| 24 | 3.38E-7 |
| 25 | 8.63E-8 |
| 26 | 1.76E-7 |
| 27 | 1.17E-7 |
| 28 | 1.22E-7 |
| 29 | 2.19E-7 |
| 30 | 3.01E-8 |
| 31 | 4.28E-8 |
| 32 | 2.74E-8 |
| 33 | 2.87E-8 |
| 34 | 3.94E-8 |
| 35 | 3.38E-8 |
| 36 | 1.31E-8 |
| 37 | 3.78E-6 |
| 38 | 7.42E-7 |
| 39 | 7.68E-7 |
| 40 | 7.83E-8 |
| 41 | 9.67E-8 |
| 42 | 2.39E-7 |
| 43 | 5.46E-8 |
| 44 | 4.21E-7 |
| 45 | 1.34E-8 |
| 46 | 1.24E-8 |
| 47 | 2.56E-8 |
| 48 | 9.95E-8 |
| 49 | 6.15E-8 |
| 50 | 3.36E-8 |
| 51 | 9.55E-8 |
| 52 | 3.59E-8 |
| 53 | 3.53E-8 |
| 54 | 1.76E-8 |
| 55 | 1.17E-7 |
| 56 | 2.38E-7 |
| 57 | 2.39E-7 |
| 58 | 1.74E-7 |
| 59 | 1.69E-8 |
| 60 | 1.98E-8 |
| 61 | 1.67E-7 |
| 62 | 1.86E-7 |
| 63 | 1.17E-7 |
| 64 | 1.38E-7 |
| 65 | 1.33E-7 |
| 66 | 1.61E-8 |
| 67 | 3.69E-8 |
| 68 | 3.63E-8 |
| 69 | 1.55E-7 |
| 70 | 2.46E-6 |
| 71 | 8.72E-7 |
| 72 | 8.25E-8 |
| 73 | 3.82E-7 |
| 74 | 8.72E-8 |
| 75 | 2.14E-8 |
| 76 | 4.13E-8 |
| 77 | 4.92E-8 |
| 78 | 7.43E-8 |
| 79 | 1.06E-7 |
| 80 | 6.83E-8 |
| 81 | 1.23E-7 |
| 82 | 1.34E-7 |
| 83 | 1.22E-7 |
| 84 | 1.43E-7 |
| 85 | 1.86E-6 |
| 86 | 2.45E-7 |
| 87 | 2.09E-7 |
| 88 | 2.1E-6 |
| 89 | 3.64E-6 |
| 90 | 2.86E-6 |
| 91 | 1.13E-7 |
| 92 | 8.0E-6 |
| 93 | 7.33E-7 |
| 94 | 1.95E-7 |
| 95 | 1.03E-6 |
| 96 | 1.65E-8 |
| 97 | 4.12E-7 |
| 98 | 1.12E-7 |
| 99 | 1.71E-6 |
| 100 | 1.58E-7 |
| 101 | 9.89E-7 |
| 102 | 2.83E-6 |
| 103 | 3.11E-6 |
| 104 | 6.44E-8 |
| 105 | 6.43E-8 |
| 106 | 4.37E-8 |
| 107 | 4.33E-7 |
| 108 | 1.19E-7 |
| 109 | 1.78E-7 |
| 110 | 1.18E-6 |
| 111 | 6.12E-7 |
| 112 | 3.46E-8 |
| 113 | 5.95E-8 |
| 114 | 6.42E-9 |
| 115 | 1.16E-8 |
| 116 | 2.22E-8 |
| 117 | 2.21E-8 |
| 118 | 3.62E-8 |
| 119 | 4.37E-8 |
| 120 | 6.3E-8 |
| 121 | 1.0E-7 |
| 122 | 4.64E-7 |
| 123 | 5.15E-8 |
| 124 | 9.43E-8 |
| 125 | 2.57E-7 |
| 126 | 1.18E-7 |
| 127 | 3.05E-7 |
| 128 | 1.11E-7 |
| 129 | 9.44E-7 |
| 130 | 1.43E-8 |
| 131 | 4.8E-7 |
| 132 | 2.45E-8 |
| 133 | 7.0E-9 |
| 134 | 1.36E-7 |
| 135 | 3.4E-7 |
| 136 | 3.25E-7 |
| 137 | 1.86E-8 |
| 138 | 5.67E-7 |
| 139 | 2.52E-7 |
| 140 | 1.76E-7 |
| 141 | 1.53E-8 |
| 142 | 4.16E-9 |
| 143 | 5.19E-9 |

### Referenzen

Giudice L C; Endometriosis; N Engl J Med 2010;362:2389-98.
Chishima F, Hayakawa S, Sugita K, Kinukawa N, Aleemuzzaman S, Nemoto N, Yamamoto T, Honda M: Increased expression of cyclooxygenase-2 in local lesions of endometriosis patients. Am J Reprod. Immunol 2002; 48:50-56.
Sales K J and Jabbour H N; Cyclooxygenase enzymes and prostaglandins in pathology of the endometrium. Reproduction (2003) 126, 559-567.
Stratton P and Berkley K J; Chronic pelvic pain and endometriosis: translational evidence of the relationship and implications; Human Reproduction Update, Vol.0, No.0 pp. 1-21, 2010.
Petraglia F, Hornung D, Seitz C, Faustmann T, Gerlinger C, Luisi S, Lazzeri L, Strowitzki T; Reduced pelvic pain in women with endometriosis: efficacy of long-term dienogest treatment; Arch Gynecol Obstet, 2012 Jan;285(1):167-73.

## Patentansprüche

1. Verbindungen der allgemeinen Formel (I) 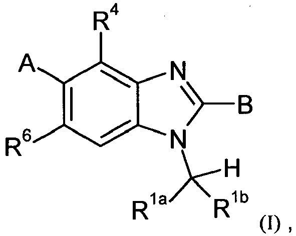 in der
R^{1a}, R^{1b} unabhängig voneinander für H, C₁-C₅-Alkyl, C₂-C₅-Alkenyl, C₂-C₅-Alkinyl, C₃-C₆-Cycloalkyl-(CH₂)ₘ, C₃-C₆-Heterocycloalkyl-(CH₂)ₙ, C₁-C₅-Alkoxy-C₁-C₃-Alkyl, C₃-C₆-Cycloalkoxy-C₁-C₃-Alkyl, Amino-C₁-C₃-Alkyl, C₁-C₅-Alkylamino-C₁-C₃-Alkyl, C₁-C₅-Dialkylamino-C₁-C₃-Alkyl oder Cyano stehen, wobei die gegebenenfalls vorhandene heterocyclische Einheit bevorzugt aus der Gruppe bestehend aus Oxetan, Tetrahydrofuran, Tetrahydropyran, 1,4-Dioxan, Morpholin, Azetidin, Pyrrolidin, Piperazin und Piperidin ausgewählt wird und wobei gegebenenfalls vorhandene Alkyl-, Cycloalkyl- oder Heterocycloalkylreste ein- oder mehrfach, gleich oder verschieden substituiert sein können mit Halogen, C₁-C₅-Alkyl, Hydroxy, Carboxy, Carboxy-C₁-C₅-Alkyl, C₁-C₅-Alkoxycarbonyl-C₁-C₅-Alkyl oder C₁-C₅-Alkylsulfonyl,
R⁴ für H, F, Cl, C₁-C₂-Alkyl, C₃-C₅-Cycloalkyl, C₁-C₂-Alkoxy oder C₃-C₄-Cycloalkylmethyl steht, wobei die entsprechende Alkyl- oder Cycloalkyleinheit ein-oder mehrfach, gleich oder verschieden mit Halogen oder Hydroxy substituiert sein kann,
A für RO-CO(CH₂)ₚ steht, wobei R für H oder C₁-C₂-Alkyl steht,
m 0, 1, 2 oder 3 ist,
n 0, 1, 2 oder 3 ist,
p 0 ist, und
B aus den folgenden Strukturen ausgewählt wird, 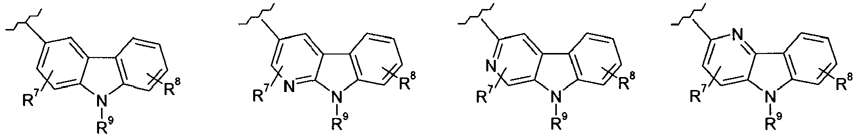 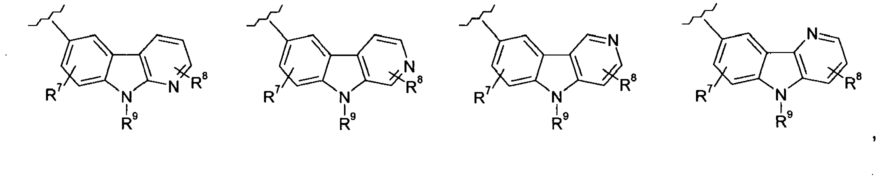
R⁶ für H, F, Cl, CH₃, CF₃ CH₃O oder CF₃O steht,
R⁷, R⁸ jeweils unabhängig voneinander für H, F, Cl, Cyano, SF₅, C₁-C₃-Alkyl, C₃-C₅-Cycloalkyl, C₁-C₂-Alkoxy oder C₃-C₄-Cycloalkylmethyl stehen, wobei die entsprechende Alkyl- oder Cycloalkyleinheit ein- oder mehrfach halogeniert sein kann, und
R⁹ für C₁-C₅-Alkyl, C₂-C₅-Alkenyl, C₂-C₅-Alkinyl, C₃-C₆-Cycloalkyl-(CH₂)ₙ, C₃-C₆-Heterocycloalkyl-(CH₂)ₙ, oder C₁-C₇-Alkoxy-C₂-C₅-Alkyl steht, wobei die gegebenenfalls vorhandene heterocyclische Einheit aus der Gruppe bestehend aus Oxetan, Tetrahydrofuran, Tetrahydropyran, Morpholin, Pyrrolidin und Piperidin ausgewählt wird und wobei die gegebenenfalls vorhandenen Alkyl-, Cycloalkyl- oder Heterocycloalkyleinheiten ein- oder mehrfach, gleich oder verschieden mit Halogen, C₁-C₂-Alkyl, C₁-C₂₋Alkoxy oder Carboxy substituiert sein kann,
und ihre Isomere, Diastereomere, Enantiomere, Solvate und Salze oder Cyclodextrin-Clathrate.

2. Verbindungen gemäß Anspruch 1, wobei
R^{1a} für H oder C₁-C₅-Alkyl steht,
R^{1b} für H, C₁-C₅-Alkyl, C₂-C₅-Alkenyl, C₃-C₆-Cycloalkyl-(CH₂)ₘ, C₃-C₆-Hetero-cycloalkyl-(CH₂)ₙ, C₁-C₅-Alkoxy-C₁-C₃-Alkyl oder C₁-C₅-Dialkylamino-C₁-C₃-Alkyl steht, wobei die gegebenenfalls vorhandene heterocyclische Einheit bevorzugt aus der Gruppe bestehend aus Oxetan, Tetrahydrofuran, 1,4-Dioxan, Morpholin und Pyrrolidin ausgewählt wird und wobei gegebenenfalls vorhandene Alkyl- oder Cycloalkylreste ein- oder mehrfach, gleich oder verschieden substituiert sein können mit C₁-C₅-Alkyl, Hydroxy, oder C₁-C₅-Alkylsulfonyl,
R⁴ für H, F, Cl, C₁-C₂-Alkyl oder C₁-C₂-Alkoxy steht,
A für RO-CO(CH₂)ₚ steht, wobei R für H oder C₁-C₂-Alkyl steht,
m 0 oder 1 ist,
n 0 oder 1 ist,
p 0 ist, und
B aus den folgenden Strukturen ausgewählt wird, 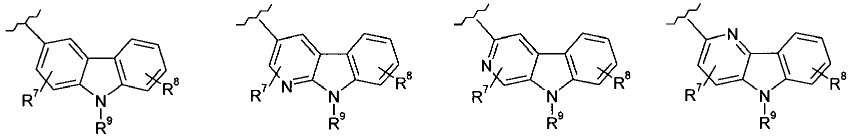 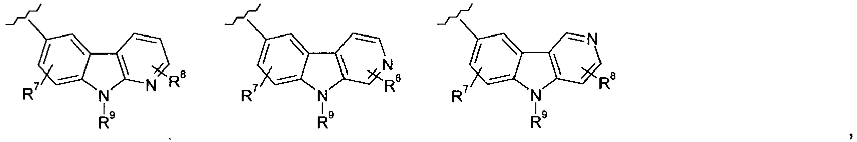
R⁶ für H, F, CH₃ oder CH₃O steht,
R⁷, R⁸ jeweils unabhängig voneinander für H, F, Cl, C₁-C₃-Alkyl oder C₁-C₂-Alkoxy stehen, und
R⁹ für C₁-C₅-Alkyl, C₂-C₅-Alkenyl, C₂-C₅-Alkinyl, C₃-C₆-Cycloalkyl-(CH₂)ₙ oder C₁-C₇-Alkoxy-C₂-C₅-Alkyl steht,
und ihre Isomere, Diastereomere, Enantiomere, Solvate und Salze oder Cyclodextrin-Clathrate.

3. Verbindungen gemäß Anspruch 1, wobei
R^{1a} für H oder Methyl steht,
R^{1b} für H, C₁-C₂-Alkyl, Vinyl, Cyclopropyl-(CH₂)ₘ, C₃-C₆-Heterocycloalkyl-(CH₂)ₙ, Methoxy-C₁-C₂-Alkyl oder (N,N-Dimethylamino)methyl steht, wobei die gegebenenfalls vorhandene heterocyclische Einheit bevorzugt aus der Gruppe bestehend aus Oxetan, Tetrahydrofuran, 1,4-Dioxan, Morpholin und Pyrrolidin ausgewählt wird und wobei gegebenenfalls vorhandene Alkyl- oder Cycloalkylreste ein- oder mehrfach, gleich oder verschieden substituiert sein können mit Methyl, Hydroxy, oder Methylsulfonyl,
R⁴ für H, F, Cl, Methyl oder Methoxy steht,
A für RO-CO(CH₂)ₚ steht, wobei R für H oder C₁-C₂-Alkyl steht,
m 0 oder 1 ist,
n 0 oder 1 ist,
p 0 ist, und
B aus den folgenden Strukturen ausgewählt wird, 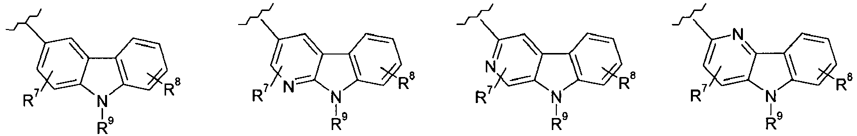 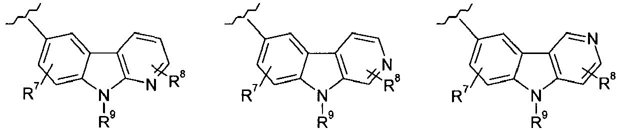
R⁶ für H, F, CH₃ oder CH₃O steht,
R⁷, R⁸ jeweils unabhängig voneinander für H, F, Cl, Methyl oder Methoxy stehen, und
R⁹ für C₁-C₃-Alkyl, Allyl, Propargyl, C₃-C₄-Cycloalkyl-(CH₂)ₙ oder Methoxyethyl steht,
und ihre Isomere, Diastereomere, Enantiomere, Solvate und Salze oder Cyclodextrin-Clathrate.

4. Verbindungen gemäß Anspruch 1, wobei
R^{1a} für H oder Methyl steht,
R^{1b} für H, C₁-C₂-Alkyl, Vinyl, Cyclopropyl-(CH₂)ₘ, C₃-C₆-Heterocycloalkyl-(CH₂)ₙ, Methoxy-C₁-C₂-Alkyl oder (N,N-Dimethylamino)methyl steht, wobei die gegebenenfalls vorhandene heterocyclische Einheit bevorzugt aus der Gruppe bestehend aus Oxetan, Tetrahydrofuran, 1,4-Dioxan, Morpholin und Pyrrolidin ausgewählt wird und wobei gegebenenfalls vorhandene Alkyl- oder Cycloalkylreste ein- oder mehrfach, gleich oder verschieden substituiert sein können mit Methyl, Hydroxy, oder Methylsulfonyl,
R⁴ für H, F, Cl, Methyl oder Methoxy steht,
A für RO-CO(CH₂)ₚ steht, wobei R für H steht,
m 0 oder 1 ist,
n 0 oder 1 ist,
p 0 ist, und
B aus den folgenden Strukturen ausgewählt wird, 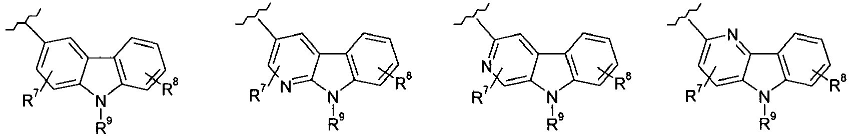 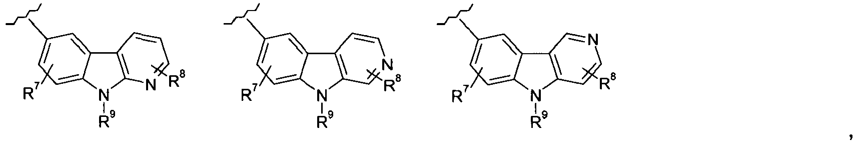
R⁶ für H, F, CH₃ oder CH₃O steht,
R⁷, R⁸ jeweils unabhängig voneinander für H, F, Cl, Methyl oder Methoxy stehen, und
R⁹ für C₁-C₃-Alkyl, Allyl, Propargyl, C₃-C₄-Cycloalkyl-(CH₂)ₙ oder Methoxyethyl steht,
und ihre Isomere, Diastereomere, Enantiomere, Solvate und Salze oder Cyclodextrin-Clathrate.

5. Verbindungen gemäß Anspruch 1, wobei
R^{1a} für H steht,
R^{1b} für Methoxymethyl steht,
R⁴ für H, F, Cl, Methyl oder Methoxy steht,
A für RO-CO(CH₂)ₚ steht, wobei R für H steht,
n 0 oder 1 ist,
p 0 ist, und
B aus den folgenden Strukturen ausgewählt wird, 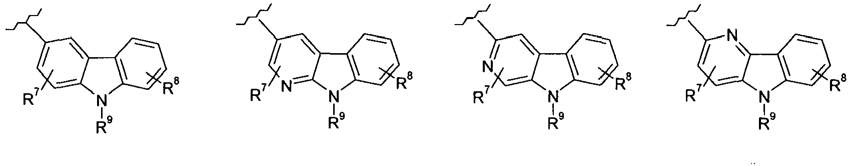 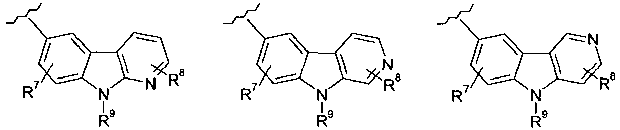
R⁶ für H, F, CH₃ oder CH₃O steht,
R⁷, R⁸ jeweils unabhängig voneinander für H, F, Cl, Methyl oder Methoxy stehen, und
R⁹ für C₁-C₃-Alkyl, Allyl, Propargyl, C₃-C₄-Cycloalkyl-(CH₂)ₙ oder Methoxyethyl steht,
und ihre Isomere, Diastereomere, Enantiomere, Solvate und Salze oder Cyclodextrin-Clathrate.

6. Verbindung gemäß Anspruch 1 ausgewählt aus einer Gruppe, die die folgenden Verbindungen enthält:
1. Methyl-1-allyl-2-(9-ethyl-9H-carbazol-3-yl)-1 H-benzimidazol-5-carboxylat
2. 1-Allyl-2-(9-ethyl-9H-carbazol-3-yl)-1H-benzimidazol-5-carbonsäure
3. Methyl-2-(9-ethyl-9H-carbazol-3-yl)-1-(2-methoxyethyl)-1 H-benzimidazol-5-carboxylat
4. 2-(9-Ethyl-9H-carbazol-3-yl)-1-(2-methoxyethyl)-1 H-benzimidazol-5-carbonsäure
5. Methyl-1-(cyclopropylmethyl)-2-(9-ethyl-9H-carbazol-3-yl)-1H-benzimidazol-5-carboxylat
6. 1-(Cyclopropylmethyl)-2-(9-ethyl-9H-carbazol-3-yl)-1H-benzimidazol-5-carbonsäure
7. Methyl-4-chlor-1-(cyclopropylmethyl)-2-(9-ethyl-9H-carbazol-3-yl)-1H-benzimidazol-5-carboxylat
8. 4-Chlor-1-(cyclopropylmethyl)-2-(9-ethyl-9H-carbazol-3-yl)-1H-benzimidazol-5-carbonsäure
9. 1-(Cyclopropylmethyl)-2-(9-ethyl-9H-carbazol-3-yl)-4-methyl-1 H-benzimidazol-5-carbonsäure
10. 2-(9-Ethyl-7-fluor-9H-carbazol-3-yl)-1-(2-methoxyethyl)-1H-benzimidazol-5-carbonsäure
11. 2-(9-Ethyl-5-fluor-9H-carbazol-3-yl)-1-(2-methoxyethyl)-1H-benzimidazol-5-carbonsäure
12. 2-(9-Ethyl-8-fluor-9H-carbazol-3-yl)-1-(2-methoxyethyl)-1 H-benzimidazol-5-carbonsäure
13. 1-(Cyclopropylmethyl)-2-[9-(2-methoxyethyl)-9H-carbazol-3-yl]-1H-benzimidazol-5-carbonsäure
14. 1-(Cyclopropylmethyl)-2-(9-ethyl-9H-pyrido[2,3-b]indol-3-yl)-1H-benzimidazol-5-carbonsäure
15. 2-(9-Ethyl-6-methoxy-9H-carbazol-3-yl)-1-(2-methoxyethyl)-1 H-benzimidazol-5-carbonsäure
16. 2-(9-Allyl-9H-carbazol-3-yl)-1-(cyclopropylmethyl)-1H-benzimidazol-5-carbonsäure
17. 1-(Cyclopropylmethyl)-2-(9-methyl-9H-carbazol-3-yl)-1H-benzimidazol-5-carbonsäure
18. 1-(Cyclopropylmethyl)-2-[9-(cyclopropylmethyl)-9H-carbazol-3-yl]-1H-benzimidazol-5-carbonsäure
19. 2-[9-(Cyclopropylmethyl)-9H-carbazol-3-yl]-1-(2-methoxyethyl)-1H-benzimidazol-5-carbonsäure
20. Ethyl-2-(9-ethyl-9H-carbazol-3-yl)-1-[2-(pyrrolidin-1-yl)ethyl]-1H-benzimidazol-5-carboxylat
21. 2-(9-Ethyl-9H-carbazol-3-yl)-1-[2-(pyrrolidin-1-yl)ethyl]-1H-benzimidazol-5-carbonsäure
22. 2-(5-Ethyl-5H-pyrido[3,2-b]indol-2-yl)-1-(2-methoxyethyl)-1H-benzimidazol-5-carbonsäure
23. 1-(Cyclopropylmethyl)-2-(9-ethyl-6-methoxy-9H-carbazol-3-yl)-1H-benzimidazol-5-carbonsäure
24. 2-(9-Ethyl-9H-carbazol-3-yl)-1-[2-(morpholin-4-yl)ethyl]-1H-benzimidazol-5-carbonsäure
25. Ethyl-1-[2-(dimethylamino)ethyl]-2-(9-ethyl-9H-carbazol-3-yl)-1H-benzimidazol-5-carboxylat
26. 1-[2-(Dimethylamino)ethyl]-2-(9-ethyl-9H-carbazol-3-yl)-1H-benzimidazol-5-carbonsäure
27. 2-(9-Ethyl-9H-carbazol-3-yl)-1-(2-hydroxyethyl)-1H-benzimidazol-5-carbonsäure
28. Ethyl-2-(9-ethyl-9H-carbazol-3-yl)-1-isopropyl-1 H-benzimidazol-5-carboxylat
29. 2-(9-Ethyl-9H-carbazol-3-yl)-1-isopropyl-1 H-benzimidazol-5-carbonsäure
30. 2-(9-Ethyl-9H-carbazol-3-yl)-1-methyl-1H-benzimidazol-5-carbonsäure
31. Methyl-1-ethyl-2-(9-ethyl-9H-carbazol-3-yl)-1 H-benzimidazol-5-carboxylat
32. 2-(9-Allyl-9H-carbazol-3-yl)-1-(2-methoxyethyl)-1H-benzimidazol-5-carbonsäure
33. Ethyl-1-(2-methoxyethyl)-2-[9-(2-methoxyethyl)-9H-carbazol-3-yl]-1H-benzimidazol-5-carboxylat
34. 1-(2-Methoxyethyl)-2-[9-(2-methoxyethyl)-9H-carbazol-3-yl]-1 H-benzimidazol-5-carbonsäure
35. Ethyl-2-(9-ethyl-9H-carbazol-3-yl)-1-(3-methoxypropyl)-1H-benzimidazol-5-carboxylat
36. 2-(9-Ethyl-9H-carbazol-3-yl)-1-(3-methoxypropyl)-1 H-benzimidazol-5-carbonsäure
37. 1-(Cyclopropylmethyl)-2-(9-ethyl-9H-carbazol-3-yl)-6-methoxy-1H-benzimidazol-5-carbonsäure
38. 2-(9-Ethyl-9H-carbazol-3-yl)-6-methoxy-1-(2-methoxyethyl)-1 H-benzimidazol-5-carbonsäure
39. 1-(Cyclopropylmethyl)-2-(9-ethyl-9H-carbazol-3-yl)-4-methoxy-1H-benzimidazol-5-carbonsäure
40. 2-(9-Ethyl-9H-carbazol-3-yl)-1-(2-methoxyethyl)-4-methyl-1 H-benzimidazol-5-carbonsäure
41. 1-(Cyclopropylmethyl)-2-(9-ethyl-9H-pyrido[2,3-b]indol-3-yl)-4-methyl-1 H-benzimidazol-5-carbonsäure
42. 1-(Cyclopropylmethyl)-2-(9-ethyl-9H-beta-carbolin-6-yl)-1H-benzimidazol-5-carbonsäure
43. 1-(Cyclopropylmethyl)-2-(5-ethyl-5H-pyrido[4,3-b]indol-8-yl)-1H-benzimidazol-5-carbonsäure
44. 1-(Cyclopropylmethyl)-2-(9-ethyl-9H-carbazol-3-yl)-6-methyl-1H-benzimidazol-5-carbonsäure
45. 2-(9-Ethyl-9H-carbazol-3-yl)-1-(2-methoxyethyl)-6-methyl-1H-benzimidazol-5-carbonsäure
46. 2-(9-Ethyl-9H-carbazol-3-yl)-6-fluor-1-(2-methoxyethyl)-1H-benzimidazol-5-carbonsäure
47. 1-(Cyclopropylmethyl)-2-(9-ethyl-9H-carbazol-3-yl)-6-fluor-1H-benzimidazol-5-carbonsäure
48. 2-(9-Ethyl-9H-carbazol-3-yl)-4-fluor-1-(2-methoxyethyl)-1H-benzimidazol-5-carbonsäure
49. 1-(Cyclopropylmethyl)-2-(9-ethyl-9H-carbazol-3-yl)-4-fluor-1H-benzimidazol-5-carbonsäure
50. 1-(Cyclopropylmethyl)-2-(9-ethyl-9H-pyrido[2,3-b]indol-6-yl)-1H-benzimidazol-5-carbonsäure
51. 1-(2-Cyclopropylethyl)-2-(9-ethyl-9H-carbazol-3-yl)-1H-benzimidazol-5-carbonsäure
52. 1-(2-Methoxyethyl)-2-(9-propyl-9H-carbazol-3-yl)-1 H-benzimidazol-5-carbonsäure
53. 1-(2-Methoxyethyl)-2-[9-(prop-2-in-1-yl)-9H-carbazol-3-yl]-1H-benzimidazol-5-carbonsäure
54. 1-(Cyclopropylmethyl)-2-(9-propyl-9H-carbazol-3-yl)-1H-benzimidazol-5-carbonsäure
55. 1-[(2,2-Dimethylcyclopropyl)methyl]-2-(9-ethyl-9H-carbazol-3-yl)-1H-benzimidazol-5-carbonsäure
56. Ethyl-2-(9-ethyl-9H-carbazol-3-yl)-1-(oxetan-3-ylmethyl)-1H-benzimidazol-5-carboxylat
57. 2-(9-Ethyl-9H-carbazol-3-yl)-1-(oxetan-3-ylmethyl)-1H-benzimidazol-5-carbonsäure
58. 2-[9-(Cyclobutylmethyl)-9H-carbazol-3-yl]-1-(2-methoxyethyl)-1H-benzimidazol-5-carbonsäure
59. 2-[9-(Cyclobutylmethyl)-9H-carbazol-3-yl]-1-(cyclopropylmethyl)-1H-benzimidazol-5-carbonsäure
60. 2-(9-Ethyl-9H-carbazol-3-yl)-1-[2-(trifluormethoxy)ethyl]-1H-benzimidazol-5-carbonsäure
61. 1-(Cyclopropylmethyl)-2-(9-ethyl-1-methyl-9H-beta-carbolin-3-yl)-1H-benzimidazol-5-carbonsäure
62. 2-(9-Ethyl-9H-carbazol-3-yl)-1-(oxetan-2-ylmethyl)-1H-benzimidazol-5-carbonsäure
63. 2-(9-Ethyl-9H-carbazol-3-yl)-1-(tetrahydrofuran-2-ylmethyl)-1H-benzimidazol-5-carbonsäure
64. 2-(9-Ethyl-9H-carbazol-3-yl)-1-[(2R)-2-hydroxy-3-methoxypropyl]-1 H-benzimidazol-5-carbonsäure
65. 2-(9-Ethyl-9H-carbazol-3-yl)-1-[(2S)-2-hydroxy-3-methoxypropyl]-1H-benzimidazol-5-carbonsäure
66. 2-(9-Ethyl-9H-carbazol-3-yl)-1-[2-(methylsulfonyl)ethyl]-1H-benzimidazol-5-carbonsäure
67. 1-(2-Cyclopropyl-2-hydroxyethyl)-2-(9-ethyl-9H-carbazol-3-yl)-1H-benzimidazol-5-carbonsäure
68. 1-[(2S)-2,3-Dihydroxypropyl]-2-(9-ethyl-9H-carbazol-3-yl)-1H-benzimidazol-5-carbonsäure
69. 1-(1,4-Dioxan-2-ylmethyl)-2-(9-ethyl-9H-carbazol-3-yl)-1H-benzimidazol-5-carbonsäure
70. 1-(1,4-Dioxan-2-ylmethyl)-2-(9-ethyl-9H-carbazol-3-yl)-1H-benzimidazol-5-carbonsäure
71. 2-(9-Ethyl-6-methyl-9H-carbazol-3-yl)-1-(2-methoxyethyl)-4-methyl-1H-benzimidazol-5-carbonsäure
72. 2-(6-Chlor-9-ethyl-9H-carbazol-3-yl)-1-(2-methoxyethyl)-4-methyl-1H-benzimidazol-5-carbonsäure
73. 2-(8-Chlor-9-ethyl-9H-carbazol-3-yl)-1-(2-methoxyethyl)-4-methyl-1H-benzimidazol-5-carbonsäure

7. Verbindung der Formel (I), wie in einem der Ansprüche 1 bis 6 definiert, zur Behandlung und/oder Prophylaxe von Krankheiten.

8. Verbindung der Formel (I), wie in einem der Ansprüche 1 bis 6 definiert, zur Verwendung in einem Verfahren zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

9. Verwendung einer Verbindung nach Anspruch 1, 2, 3, 4, 5 oder 6 zur Herstellung eines Arzneimittels zur Behandlung und/oder Prophylaxe von Krankheiten.

10. Verwendung einer Verbindung, wie in einem der Ansprüche 1 bis 6 definiert, zur Herstellung eines Arzneimittels zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

11. Arzneimittel enthaltend eine Verbindung, wie in einem der Ansprüche 1 bis 6 definiert, in Kombination mit einem oder mehreren weiteren Wirkstoffen insbesondere mit selektiven Estrogen Rezeptor Modulatoren (SERMs), Estrogenrezeptor (ER) Antagonisten, Aromataseinhibitoren, 17 β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen Kinasen (Mkk3/6, Mek1/2, Erk1/2), Inhibitoren der Proteinkinasen B (PKBα/β/γ; Akt1/2/3), Inhibitoren der Phosphoinositid-3-Kinasen (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, Neurokinin1 Rezeptor Antagonisten, Paracetamol, selektiven COX2-Inhibitoren und/oder nicht-selektiven COX1/COX2 Inhibitoren.

12. Arzneimittel enthaltend eine Verbindung der Formel (I) wie in einem der Ansprüche 1 bis 6 definiert, in Kombination mit einem inerten, nichttoxischen, pharmazeutisch geeigneten Hilfsstoff.

13. Arzneimittel nach Anspruch 11 oder 12 zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

## Claims

1. Compounds of the general formula (I) 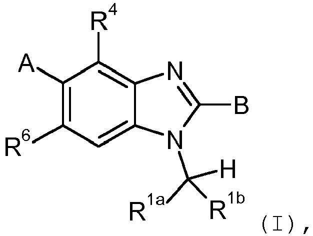 in which
R^{1a}, R^{1b} independently of one another represent H, C₁-C₅-alkyl, C₂-C₅-alkenyl, C₂-C₅-alkynyl, C₃-C₆-cycloalkyl-(CH₂)ₘ, C₃-C₆-heterocycloalkyl-(CH₂)ₙ, C₁-C₅-alkoxy-C₁-C₃-alkyl, C₃-C₆-cycloalkoxy-C₁-C₃-alkyl, amino-C₁-C₃-alkyl, C₁-C₅-alkylamino-C₁-C₃-alkyl, C₁-C₅-dialkylamino-C₁-C₃-alkyl or cyano, where the optionally present heterocyclic unit is preferably selected from the group consisting of oxetane, tetrahydrofuran, tetrahydropyran, 1,4-dioxane, morpholine, azetidine, pyrrolidine, piperazine and piperidine and where optionally present alkyl, cycloalkyl or heterocycloalkyl radicals can be mono- or polysubstituted, identically or differently by halogen, C₁-C₅-alkyl, hydroxyl, carboxyl, carboxy-C₁-C₅-alkyl, C₁-C₅-alkoxycarbonyl-C₁-C₅-alkyl or C₁-C₅-alkylsulphonyl,
R⁴ represents H, F, Cl, C₁-C₂-alkyl, C₃-C₅-cycloalkyl, C₁-C₂-alkoxy or C₃-C₄-cycloalkylmethyl, where the corresponding alkyl or cycloalkyl unit can be mono- or polysubstituted, identically or differently by halogen or hydroxyl,
A represents RO-CO(CH₂)ₚ, where R represents H or C₁-C₂-alkyl,
m is 0, 1, 2 or 3,
n is 0, 1, 2 or 3,
p is 0, and
B is selected from the following structures, 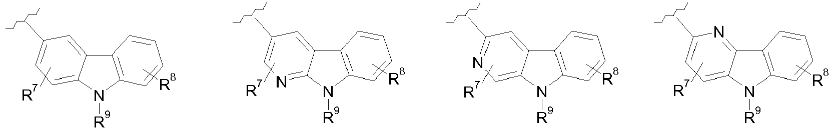 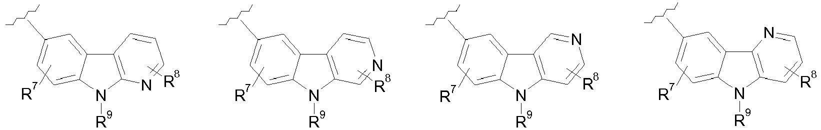
R⁶ , represents H, F, Cl, CH₃, CF₃ CH₃O or CF₃O,
R⁷, R⁸ in each case independently of one another represent H, F, Cl, cyano, SF₅, C₁-C₃-alkyl, C₃-C₅-cycloalkyl, C₁-C₂-alkoxy or C₃-C₄-cycloalkylmethyl, where the corresponding alkyl or cycloalkyl unit can be mono- or polyhalogenated, and
R⁹ represents C₁-C₅-alkyl, C₂-C₅-alkenyl, C₂-C₅-alkynyl, C₃-C₆-cycloalkyl-(CH₂)ₙ, C₃-C₆-heterocycloalkyl-(CH₂)ₙ, or C₁-C₇-alkoxy-C₂-C₅-alkyl, where the optionally present heterocyclic unit is selected from the group consisting of oxetane, tetrahydrofuran, tetrahydropyran, morpholine, pyrrolidine and piperidine and where the optionally present alkyl, cycloalkyl or heterocycloalkyl units can be mono- or polysubstituted, identically or differently by halogen, C₁-C₂-alkyl, C₁-C₂-alkoxy or carboxyl,
and their isomers, diastereomers, enantiomers, solvates and salts or cyclodextrin clathrates.

2. Compounds according to Claim 1, where
R^{1a} represents H or C₁-C₅-alkyl,
R^{1b} represents H, C₁-C₅-alkyl, C₂-C₅-alkenyl, C₃-C₆-cycloalkyl-(CH₂)ₘ, C₃-C₆-heterocycloalkyl-(CH₂)ₙ, C₁-C₅-alkoxy-C₁-C₃-alkyl or C₁-C₅-dialkylamino-C₁-C₃-alkyl, where the optionally present heterocyclic unit is preferably selected from the group consisting of oxetane, tetrahydrofuran, 1,4-dioxane, morpholine and pyrrolidine and where optionally present alkyl- or cycloalkyl radicals can be mono- or polysubstituted, identically or differently, by C₁-C₅-alkyl, hydroxyl, or C₁-C₅-alkylsulphonyl,
R⁴ represents H, F, Cl, C₁-C₂-alkyl or C₁-C₂-alkoxy,
A represents RO-CO(CH₂)ₚ, where R represents H or C₁-C₂-alkyl,
m is 0 or 1,
n is 0 or 1,
p is 0, and
B is selected from the following structures, 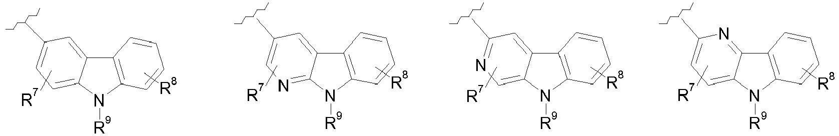 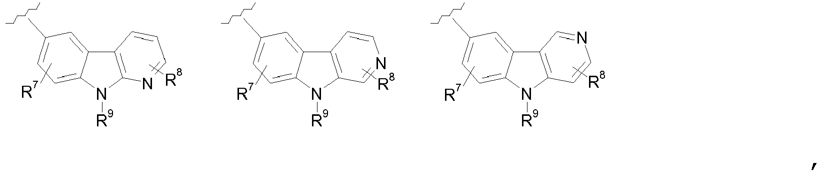
R⁶ represents H, F, CH₃ or CH₃O,
R⁷, R⁸ in each case independently of one another represent H, F, Cl, C₁-C₃-alkyl or C₁-C₂-alkoxy, and
R⁹ represents C₁-C₅-alkyl, C₂-C₅-alkenyl, C₂-C₅-alkynyl, C₃-C₆-cycloalkyl-(CH₂)ₙ or C₁-C₇-alkoxy-C₂-C₅-alkyl,
and their isomers, diastereomers, enantiomers, solvates and salts or cyclodextrin clathrates.

3. Compounds according to Claim 1, where
R^{1a} represents H or methyl,
R^{1b} represents H, C₁-C₂-alkyl, vinyl, cyclopropyl-(CH₂)ₘ, C₃-C₆-heterocycloalkyl-(CH₂)ₙ, methoxy-C₁-C₂-alkyl or (N,N-dimethylamino)-methyl, where the optionally present heterocyclic unit is preferably selected from the group consisting of oxetane, tetrahydrofuran, 1,4-dioxane, morpholine and pyrrolidine and where optionally present alkyl or cycloalkyl radicals can be mono- or polysubstituted, identically or differently, by methyl, hydroxyl, or methylsulphonyl,
R⁴ represents H, F, Cl, methyl or methoxy,
A represents RO-CO(CH₂)ₚ, where R represents H or C₁-C₂-alkyl,
m is 0 or 1,
n is 0 or 1,
p is 0, and
B is selected from the following structures, 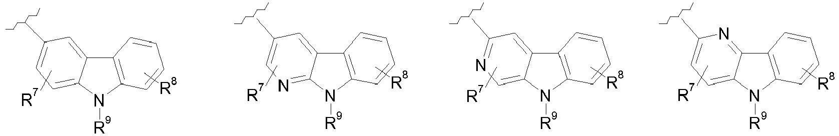 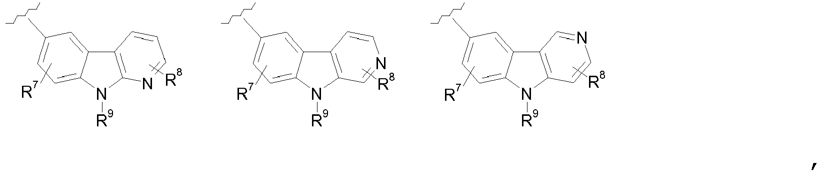
R⁶ represents H, F, CH₃ or CH₃O,
R⁷, R⁸ in each case independently of one another represent H, F, Cl, methyl or methoxy, and
R⁹ represents C₁-C₃-alkyl, allyl, propargyl, C₃-C₄-cycloalkyl-(CH₂)ₙ or methoxyethyl,
and their isomers, diastereomers, enantiomers, solvates and salts or cyclodextrin clathrates.

4. Compounds according to Claim 1, where
R^{1a} represents H or methyl,
R^{1b} represents H, C₁-C₂-alkyl, vinyl, cyclopropyl-(CH₂)ₘ, C₃-C₆-heterocycloalkyl-(CH₂)ₙ, methoxy-C₁-C₂-alkyl or (N,N-dimethylamino)-methyl, where the optionally present heterocyclic unit is preferably selected from the group consisting of oxetane, tetrahydrofuran, 1,4-dioxane, morpholine and pyrrolidine and where optionally present alkyl or cycloalkyl radicals can be mono- or polysubstituted, identically or differently, by methyl, hydroxyl, or methylsulphonyl,
R⁴ represents H, F, Cl, methyl or methoxy,
A represents RO-CO(CH₂)ₚ, where R represents H,
m is 0 or 1,
n is 0 or 1,
p is 0, and
B is selected from the following structures, 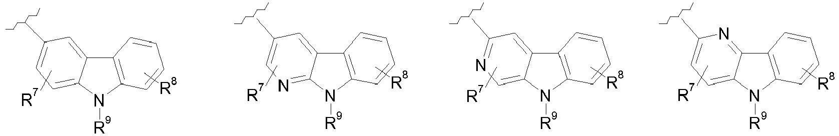 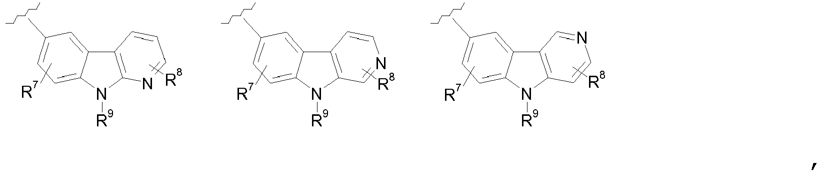
R⁶ represents H, F, CH₃ or CH₃O,
R⁷, R⁸ in each case independently of one another represent H, F, Cl, methyl or methoxy, and
R⁹ represents C₁-C₃-alkyl, allyl, propargyl, C₃-C₄-cycloalkyl-(CH₂)ₙ or methoxyethyl,
and their isomers, diastereomers, enantiomers, solvates and salts or cyclodextrin clathrates.

5. Compounds according to Claim 1, where
R^{1a} represents H,
R^{1b} represents methoxymethyl,
R⁴ represents H, F, Cl, methyl or methoxy,
A represents RO-CO(CH₂)ₚ, where R represents H,
n is 0 or 1,
p is 0, and
B is selected from the following structures, 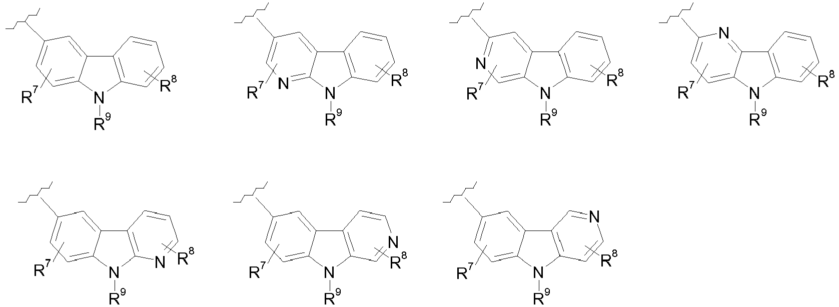
R⁶ , represents H, F, CH₃ or CH₃O,
R⁷, R⁸ in each case independently of one another represent H, F, Cl, methyl or methoxy, and
R⁹ represents C₁-C₃-alkyl, allyl, propargyl, C₃-C₄-cycloalkyl-(CH₂)ₙ or methoxyethyl,
and their isomers, diastereomers, enantiomers, solvates and salts or cyclodextrin clathrates.

6. Compound according to Claim 1 selected from a group that contains the following compounds:
1. Methyl 1-allyl-2-(9-ethyl-9H-carbazol-3-yl)-1H-benzimidazole-5-carboxylate
2. 1-Allyl-2-(9-ethyl-9H-carbazol-3-yl)-1H-benzimidazole-5-carboxylic acid
3.Methyl 2-(9-ethyl-9H-carbazol-3-yl)-1-(2-methoxyethyl)-1H-benzimidazole-5-carboxylate
4.2-(9-Ethyl-9H-carbazol-3-yl)-1-(2-methoxyethyl)-1H-benzimidazole-5-carboxylic acid
5.Methyl 1-(cyclopropylmethyl)-2-(9-ethyl-9H-carbazol-3-yl)-1H-benzimidazole-5-carboxylate
6. 1-(Cyclopropylmethyl)-2-(9-ethyl-9H-carbazol-3-yl)-1H-benzimidazole-5-carboxylic acid
7. Methyl 4-chloro-1-(cyclopropylmethyl)-2-(9-ethyl-9H-carbazol-3-yl)-1H-benzimid-azole-5-carboxylate
8. 4-Chloro-1-(cyclopropylmethyl)-2-(9-ethyl-9H-carbazol-3-yl)-1H-benzimidazole-5-carboxylic acid
9. 1-(Cyclopropylmethyl)-2-(9-ethyl-9H-carbazol-3-yl)-4-methyl-1H-benzimidazole-5-carboxylic acid
10. 2-(9-Ethyl-7-fluoro-9H-carbazol-3-yl)-1-(2-methoxyethyl)-1H-benzimidazole-5-carboxylic acid
11. 2-(9-Ethyl-5-fluoro-9H-carbazol-3-yl)-1-(2-methoxyethyl)-1H-benzimidazole-5-carboxylic acid
12. 2-(9-Ethyl-8-fluoro-9H-carbazol-3-yl)-1-(2-methoxyethyl)-1H-benzimidazole-5-carboxylic acid
13. 1-(Cyclopropylmethyl)-2-[9-(2-methoxyethyl)-9H-carbazol-3-yl]-1H-benzimidazole-5-carboxylic acid
14. 1-(Cyclopropylmethyl)-2-(9-ethyl-9H-pyrido[2,3-b]indol-3-yl)-1H-benzimidazole-5-carboxylic acid
15. 2-(9-Ethyl-6-methoxy-9H-carbazol-3-yl)-1-(2-methoxyethyl)-1H-benzimidazole-5-carboxylic acid
16. 2-(9-Allyl-9H-carbazol-3-yl)-1-(cyclopropylmethyl)-1H-benzimidazole-5-carboxylic acid
17. 1-(Cyclopropylmethyl)-2-(9-methyl-9H-carbazol-3-yl)-1H-benzimidazole-5-carboxylic acid
18. 1-(Cyclopropylmethyl)-2-[9-(cyclopropylmethyl)-9H-carbazol-3-yl]-1H-benzimidazole-5-carboxylic acid
19. 2-[9-(Cyclopropylmethyl)-9H-carbazol-3-yl]-1-(2-methoxyethyl)-1H-benzimidazole-5-carboxylic acid
20. Ethyl-2-(9-ethyl-9H-carbazol-3-yl)-1-[2-(pyrrolidin-1-yl)ethyl]-1H-benzimidazole-5-carboxylate
21. 2-(9-Ethyl-9H-carbazol-3-yl)-1-[2-(pyrrolidin-1-yl)ethyl]-1H-benzimidazole-5-carboxylic acid
22. 2-(5-Ethyl-5H-pyrido[3,2-b]indol-2-yl)-1-(2-methoxyethyl)-1H-benzimidazole-5-carboxylic acid
23. 1-(Cyclopropylmethyl)-2-(9-ethyl-6-methoxy-9H-carbazol-3-yl)-1H-benzimidazole-5-carboxylic acid
24. 2-(9-Ethyl-9H-carbazol-3-yl)-1-[2-(morpholin-4-yl)ethyl]-1H-benzimidazole-5-carboxylic acid
25. Ethyl-1-[2-(dimethylamino)ethyl]-2-(9-ethyl-9H-carbazol-3-yl)-1H-benzimidazole-5-carboxylate
26. 1-[2-(Dimethylamino)ethyl]-2-(9-ethyl-9H-carbazol-3-yl)-1H-benzimidazole-5-carboxylic acid
27. 2-(9-Ethyl-9H-carbazol-3-yl)-1-(2-hydroxyethyl)-1H-benzimidazole-5-carboxylic acid
28. Ethyl-2-(9-ethyl-9H-carbazol-3-yl)-1-isopropyl-1H-benzimidazole-5-carboxylate
29. 2-(9-Ethyl-9H-carbazol-3-yl)-1-isopropyl-1H-benzimidazole-5-carboxylic acid
30. 2-(9-Ethyl-9H-carbazol-3-yl)-1-methyl-1H-benzimidazole-5-carboxylic acid
31. Methyl 1-ethyl-2-(9-ethyl-9H-carbazol-3-yl)-1H-benzimidazole-5-carboxylate
32. 2-(9-Allyl-9H-carbazol-3-yl)-1-(2-methoxyethyl)-1H-benzimidazole-5-carboxylic acid
33. Ethyl 1-(2-methoxyethyl)-2-[9-(2-methoxyethyl)-9H-carbazol-3-yl]-1H-benzimidazole-5-carboxylate
34. 1-(2-Methoxyethyl)-2-[9-(2-methoxyethyl)-9H-carbazol-3-yl]-1H-benzimidazole-5-carboxylic acid
35. Ethyl 2-(9-ethyl-9H-carbazol-3-yl)-1-(3-methoxypropyl)-1H-benzimidazole-5-carboxylate
36. 2-(9-Ethyl-9H-carbazol-3-yl)-1-(3-methoxypropyl)-1H-benzimidazole-5-carboxylic acid
37. 1-(Cyclopropylmethyl)-2-(9-ethyl-9H-carbazol-3-yl)-6-methoxy-1H-benzimidazole-5-carboxylic acid
38. 2-(9-Ethyl-9H-carbazol-3-yl)-6-methoxy-1-(2-methoxyethyl)-1H-benzimidazole-5-carboxylic acid
39. 1-(Cyclopropylmethyl)-2-(9-ethyl-9H-carbazol-3-yl)-4-methoxy-1H-benzimidazole-5-carboxylic acid
40. 2-(9-Ethyl-9H-carbazol-3-yl)-1-(2-methoxyethyl)-4-methyl-1H-benzimidazole-5-carboxylic acid
41. 1-(Cyclopropylmethyl)-2-(9-ethyl-9H-pyrido[2,3-b]indol-3-yl)-4-methyl-1H-benzimidazole-5-carboxylic acid
42. 1-(Cyclopropylmethyl)-2-(9-ethyl-9H-beta-carbolin-6-yl)-1H-benzimidazole-5-carboxylic acid
43. 1-(Cyclopropylmethyl)-2-(5-ethyl-5H-pyrido[4,3-b]indol-8-yl)-1H-benzimidazole-5-carboxylic acid
44. 1-(Cyclopropylmethyl)-2-(9-ethyl-9H-carbazol-3-yl)-6-methyl-1H-benzimidazole-5-carboxylic acid
45. 2-(9-Ethyl-9H-carbazol-3-yl)-1-(2-methoxyethyl)-6-methyl-1H-benzimidazole-5-carboxylic acid
46. 2-(9-Ethyl-9H-carbazol-3-yl)-6-fluoro-1-(2-methoxyethyl)-1H-benzimidazole-5-carboxylic acid
47. 1-(Cyclopropylmethyl)-2-(9-ethyl-9H-carbazol-3-yl)-6-fluoro-1H-benzimidazole-5-carboxylic acid
48. 2-(9-Ethyl-9H-carbazol-3-yl)-4-fluoro-1-(2-methoxyethyl)-1H-benzimidazole-5-carboxylic acid
49. 1-(Cyclopropylmethyl)-2-(9-ethyl-9H-carbazol-3-yl)-4-fluoro-1H-benzimidazole-5-carboxylic acid
50. 1-(Cyclopropylmethyl)-2-(9-ethyl-9H-pyrido[2,3-b]indol-6-yl)-1H-benzimidazole-5-carboxylic acid
51. 1-(2-Cyclopropylethyl)-2-(9-ethyl-9H-carbazol-3-yl)-1H-benzimidazole-5-carboxylic acid
52. 1-(2-Methoxyethyl)-2-(9-propyl-9H-carbazol-3-yl)-1H-benzimidazole-5-carboxylic acid
53. 1-(2-Methoxyethyl)-2-[9-(prop-2-yn-1-yl)-9H-carbazol-3-yl]-1H-benzimidazole-5-carboxylic acid
54. 1-(Cyclopropylmethyl)-2-(9-propyl-9H-carbazol-3-yl)-1H-benzimidazole-5-carboxylic acid
55. 1-[(2,2-Dimethylcyclopropyl)methyl]-2-(9-ethyl-9H-carbazol-3-yl)-1H-benzimidazole-5-carboxylic acid
56. Ethyl 2-(9-ethyl-9H-carbazol-3-yl)-1-(oxetan-3-ylmethyl)-1H-benzimidazole-5-carboxylate
57. 2-(9-Ethyl-9H-carbazol-3-yl)-1-(oxetan-3-ylmethyl)-1H-benzimidazole-5-carboxylic acid
58. 2-[9-(Cyclobutylmethyl)-9H-carbazol-3-yl]-1-(2-methoxyethyl)-1H-benzimidazole-5-carboxylic acid
59. 2-[9-(Cyclobutylmethyl)-9H-carbazol-3-yl]-1-(cyclopropylmethyl)-1H-benzimidazole-5-carboxylic acid
60. 2-(9-Ethyl-9H-carbazol-3-yl)-1-[2-(trifluoromethoxy)ethyl]-1H-benzimidazole-5-carboxylic acid
61. 1-(Cyclopropylmethyl)-2-(9-ethyl-1-methyl-9H-beta-carbolin-3-yl)-1H-benzimidazole-5-carboxylic acid
62. 2-(9-Ethyl-9H-carbazol-3-yl)-1-(oxetan-2-ylmethyl)-1H-benzimidazole-5-carboxylic acid
63. 2-(9-Ethyl-9H-carbazol-3-yl)-1-(tetrahydrofuran-2-ylmethyl)-1H-benzimidazole-5-carboxylic acid
64. 2-(9-Ethyl-9H-carbazol-3-yl)-1-[(2R)-2-hydroxy-3-methoxypropyl]-1H-benzimidazole-5-carboxylic acid
65. 2-(9-Ethyl-9H-carbazol-3-yl)-1-[(2S)-2-hydroxy-3-methoxypropyl]-1H-benzimidazole-5-carboxylic acid
66. 2-(9-Ethyl-9H-carbazol-3-yl)-1-[2-(methylsulphonyl)ethyl]-1H-benzimidazole-5-carboxylic acid
67. 1-(2-Cyclopropyl-2-hydroxyethyl)-2-(9-ethyl-9H-carbazol-3-yl)-1H-benzimidazole-5-carboxylic acid
68. 1-[(2S)-2,3-Dihydroxypropyl]-2-(9-ethyl-9H-carbazol-3-yl)-1H-benzimidazole-5-carboxylic acid
69. 1-(1,4-Dioxan-2-ylmethyl)-2-(9-ethyl-9H-carbazol-3-yl)-1H-benzimidazole-5-carboxylic acid
70. 1-(1,4-Dioxan-2-ylmethyl)-2-(9-ethyl-9H-carbazol-3-yl)-1H-benzimidazole-5-carboxylic acid
71. 2-(9-Ethyl-6-methyl-9H-carbazol-3-yl)-1-(2-methoxyethyl)-4-methyl-1H-benzimidazole-5-carboxylic acid
72. 2-(6-Chloro-9-ethyl-9H-carbazol-3-yl)-1-(2-methoxyethyl)-4-methyl-1H-benzimidazole-5-carboxylic acid
73. 2-(8-Chloro-9-ethyl-9H-carbazol-3-yl)-1-(2-methoxyethyl)-4-methyl-1H-benzimidazole-5-carboxylic acid

7. Compound of the formula (I), as defined in one of Claims 1 to 6, for the treatment and/or prophylaxis of diseases.

8. Compound of the formula (I), as defined in one of Claims 1 to 6, for use in a method for the treatment and/or prophylaxis of endometriosis, of uterine leiomyomas, of uterine bleeding disorders, where the bleeding disorders can be severe and long-lasting bleeding, temporally irregular bleeding and pain, of dysmenorrhoea, of cancer, where the cancers can be lung, intestine, breast, skin, prostate, oesophageal cancer and leukaemia, of arteriosclerosis and of polycystic kidney diseases.

9. Use of a compound according to Claim 1, 2, 3, 4, 5 or 6 for the production of a medicament for the treatment and/or prophylaxis of diseases.

10. Use of a compound, such as defined in one of Claims 1 to 6, for the production of a medicament for the treatment and/or prophylaxis of endometriosis, of uterine leiomyomas, of uterine bleeding disorders, where the bleeding disorders can be severe and long-lasting bleeding, temporally irregular bleeding and pain, of dysmenorrhoea, of cancer, where the cancers can be lung, intestine, breast, skin, prostate, oesophageal cancer and leukaemia, of arteriosclerosis and of polycystic kidney diseases.

11. Medicament containing a compound, such as defined in one of Claims 1 to 6, in combination with one or more further active substances, in particular with selective oestrogen receptor modulators (SERMs), oestrogen receptor (ER) antagonists, aromatase inhibitors, 17β-HSD1 inhibitors, steroid sulphatase (STS) inhibitors, GnRH agonists and antagonists, kisspeptin receptor (KISSR) antagonists, selective androgen receptor modulators (SARMs), androgens, 5α-reductase inhibitors, selective progesterone receptor modulators (SPRMs), gestagens, antigestagens, oral contraceptives, inhibitors of mitogen activated protein (MAP) kinases and inhibitors of MAP kinases kinases (Mkk3/6, Mek1/2, Erk1/2), inhibitors of protein kinases B (PKBα/β/γ; Akt1/2/3), inhibitors of phosphoinositide-3 kinases (PI3K), inhibitors of cyclin-dependent kinase (CDK1/2), Inhibitors of the hypoxia-induced signal pathway (HIF1alpha inhibitors, activators of the prolylhydroxylases), histone deacetylase (HDAC) inhibitors, prostaglandin F receptor (FP) (PTGFR) antagonists, neurokinin 1 receptor antagonists, paracetamol, selective COX2 inhibitors and/or non-selective COX1/COX2 inhibitors.

12. Medicament containing a compound of the formula (I) such as defined in one of Claims 1 to 6, in combination with an inert, non-toxic, pharmaceutically suitable excipient.

13. Medicament according to Claim 11 or 12 for the treatment and/or prophylaxis of endometriosis, of uterine leiomyomas, of uterine bleeding disorders, where the bleeding disorders can be severe and long-lasting bleeding, temporally irregular bleeding and pain, of dysmenorrhoea, of cancer, where the cancers can be lung, intestine, breast, skin, prostate, oesophageal cancer and leukaemia, of arteriosclerosis and of polycystic kidney diseases.

## Revendications

1. Composés de formule générale (I) 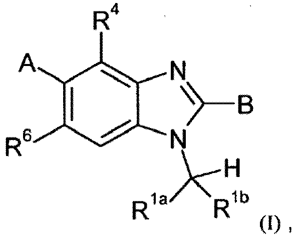 dans laquelle
R^{1a}, R^{1b} représentent, indépendamment l'un de l'autre, H, C₁-C₅-alkyle, C₂-C₅-alcényle, C₂-C₅-alcynyle, C₃-C₆-cycloalkyl-(CH₂)ₘ, C₃-C₆-hétérocycloalkyl-(CH₂)ₙ, C₁-C₅-alcoxy-C₁-C₃-alkyle, C₃-C₆-cycloalcoxy-C₁-C₃-alkyle, amino-C₁-C₃-alkyle, C₁-C₅-alkylamino-C₁-C₃-alkyle, C₁-C₅-dialkylamino-C₁-C₃-alkyle ou cyano, l'unité hétérocyclique le cas échéant présente étant de préférence choisie dans le groupe constitué par oxétane, tétrahydrofuranne, tétrahydropyranne, 1,4-dioxane, morpholine, azétidine, pyrrolidine, pipérazine et pipéridine et les radicaux alkyle, cycloalkyle ou hétérocycloalkyle le cas échéant présents pouvant être monosubstitués ou polysubstitués, de manière identique ou différente, par halogène, C₁-C₅-alkyle, hydroxy, carboxy, carboxy-C₁-C₅-alkyle, C₁-C₅-alcoxycarbonyl-C₁-C₅-alkyle ou C₁-C₅-alkylsulfonyle,
R⁴ représente H, F, Cl, C₁-C₂-alkyle, C₃-C₅-cycloalkyle, C₁-C₂-alcoxy ou C₃-C₄-cycloalkylméthyle, l'unité alkyle ou cycloalkyle correspondante pouvant être monosubstituée ou polysubstituée, de manière identique ou différente, par halogène ou hydroxy,
A représente RO-CO(CH₂)ₚ,
R représentant H ou C₁-C₂-alkyle,
m représente 0, 1, 2 ou 3,
n représente 0, 1, 2 ou 3,
p représente 0 et
B est choisi parmi les structures suivantes, 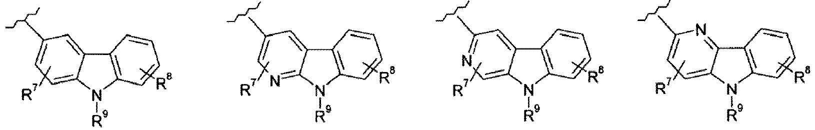 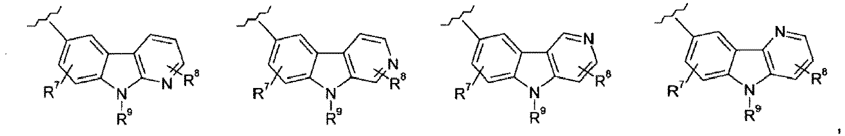
R⁶ représente H, F, Cl, CH₃, CF₃, CH₃O ou CF₃O,
R⁷, R⁸ représentent, à chaque fois indépendamment l'un de l'autre, H, F, Cl, cyano, SF₅, C₁-C₃-alkyle, C₃-C₅-cycloalkyle, C₁-C₂-alcoxy ou C₃-C₄-cycloalkylméthyle, l'unité alkyle ou cycloalkyle correspondante pouvant être monohalogénée ou polyhalogénée et
R⁹ représente C₁-C₅-alkyle, C₂-C₅-alcényle, C₂-C₅-alcynyle, C₃-C₆-cycloalkyl-(CH₂)ₙ, C₃-C₆-hétérocycloalkyl-(CH₂)ₙ ou C₁-C₇-alcoxy-C₂-C₅-alkyle, l'unité hétérocyclique le cas échéant présente étant choisie dans le groupe constitué par oxétane, tétrahydrofuranne, tétrahydropyranne, morpholine, pyrrolidine et pipéridine et les unités alkyle, cycloalkyle ou hétérocycloalkyle le cas échéant présentes pouvant être monosubstituées ou polysubstituées, de manière identique ou différente, par halogène, C₁-C₂-alkyle, C₁-C₂-alcoxy ou carboxy,
et leurs isomères, diastéréo-isomères, énantiomères, solvates et sels ou clathrates de cyclodextrine.

2. Composés selon la revendication 1, où
R^{1a} représente H ou C₁-C₅-alkyle,
R^{1b} représente H, C₁-C₅-alkyle, C₂-C₅-alcényle, C₃-C₆-cycloalkyl-(CH₂)ₘ, C₃-C₆-hétérocycloalkyl-(CH₂)ₙ, C₁-C₅-alcoxy-C₁-C₃-alkyle ou C₁-C₅-dialkylamino-C₁-C₅-alkyle, l'unité hétérocyclique le cas échéant présente étant de préférence choisie dans le groupe constitué par oxétane, tétrahydrofuranne, 1,4-dioxane, morpholine et pyrrolidine et les radicaux alkyle ou cycloalkyle le cas échéant présents pouvant être monosubstitués ou polysubstitués, de manière identique ou différente, par C₁-C₅-alkyle, hydroxy ou C₁-C₅-alkylsulfonyle,
R⁴ représente H, F, Cl, C₁-C₂-alkyle ou C₁-C₂-alcoxy,
A représente RO-CO(CH₂)ₚ,
R représentant H ou C₁-C₂-alkyle,
m représente 0 ou 1,
n représente 0 ou 1,
p représente 0 et
B est choisi parmi les structures suivantes, 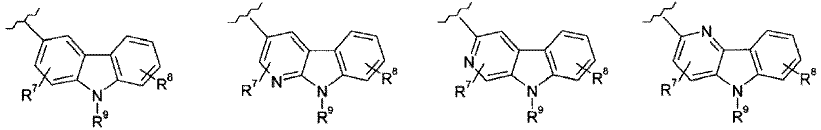 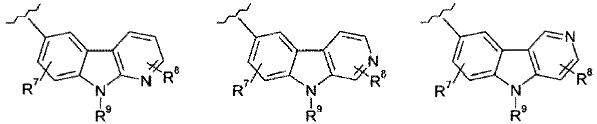
R⁶ représente H, F, CH₃ ou CH₃O,
R⁷, R⁶ représentent, à chaque fois indépendamment l'un de l'autre, H, F, Cl, C₁-C₃-alkyle ou C₁-C₂-alcoxy et
R⁹ représente C₁-C₅-alkyle, C₂-C₅-alcényle, C₂-C₅-alcynyle, C₃-C₆-cycloalkyl-(CH₂)ₙ ou C₁-C₇-alcoxy-C₂-C₅-alkyle,
et leurs isomères, diastéréo-isomères, énantiomères, solvates et sels ou clathrates de cyclodextrine.

3. Composés selon la revendication 1, où
R^{1a} représente H ou méthyle.
R^{1b} représente H, C₁-C₂-alkyle, vinyle, cyclopropyl-(CH₂)ₘ, C₃-C₆-hétérocycloalkyl-(CH₂)ₙ, méthoxy-C₁-C₂-alkyle ou (N,N-diméthylamino)méthyle, l'unité hétérocyclique le cas échéant présente étant de préférence choisie dans le groupe constitué par oxétane, tétrahydrofuranne, 1,4-dioxane, morpholine et pyrrolidine et les radicaux alkyle ou cycloalkyle le cas échéant présents pouvant être monosubstitués ou polysubstitués, de manière identique ou différente, par méthyle, hydroxy ou méthylsulfonyle,
R⁴ représente H, F, Cl, méthyle ou méthoxy,
A représente RO-CO(CH₂)ₚ,
R représentant H ou C₁-C₂-alkyle,
m représente 0 ou 1,
n représente 0 ou 1,
p représente 0 et
B est choisi parmi les structures suivantes, 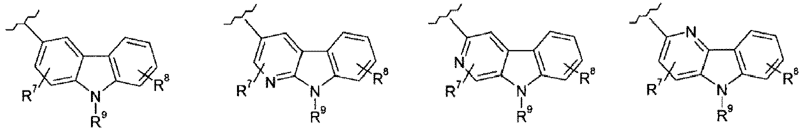 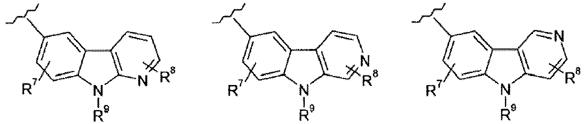
R⁶ représente H, F, CH₃ ou CH₃O,
R⁷, R⁶ représentent, à chaque fois indépendamment l'un de l'autre, H, F, Cl, méthyle ou méthoxy et
R⁹ représente C₁-C₃-alkyle, allyle, propargyle, C₃-C₄-cycloalkyl-(CH₂)ₙ ou méthoxyéthyle,
et leurs isomères, diastéréo-isomères, énantiomères, solvates et sels ou clathrates de cyclodextrine.

4. Composés selon la revendication 1, où
R^{1a} représente H ou méthyle.
R^{1b} représente H, C₁-C₂-alkyle, vinyle, cyclopropyl-(CH₂)ₘ, C₃-C₆-hétérocycloalkyl-(CH₂)ₙ, méthoxy-C₁-C₂-alkyle ou (N,N-diméthylamino)méthyle, l'unité hétérocyclique le cas échéant présente étant de préférence choisie dans le groupe constitué par oxétane, tétrahydrofuranne, 1,4-dioxane, morpholine et pyrrolidine et les radicaux alkyle ou cycloalkyle le cas échéant présents pouvant être monosubstitués ou polysubstitués, de manière identique ou différente, par méthyle, hydroxy ou méthylsulfonyle,
R⁴ représente H, F, Cl, méthyle ou méthoxy,
A représente RO-CO(CH₂)ₚ, R représentant H,
m représente 0 ou 1,
n représente 0 ou 1,
p représente 0 et
B est choisi parmi les structures suivantes, 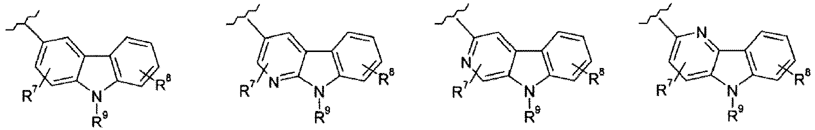 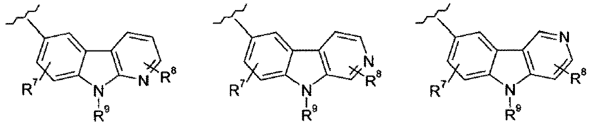
R⁶ représente H, F, CH₃ ou CH₃O,
R⁷, R⁸ représentent, à chaque fois indépendamment l'un de l'autre, H, F, Cl, méthyle ou méthoxy et
R⁹ représente C₁-C₃-alkyle, allyle, propargyle, C₃-C₄-cycloalkyl-(CH₂)ₙ ou méthoxyéthyle,
et leurs isomères, diastéréo-isomères, énantiomères, solvates et sels ou clathrates de cyclodextrine.

5. Composés selon la revendication 1, où
R^{1a} représente H,
R^{1b} représente méthoxyméthyle,
R⁴ représente H, F, Cl, méthyle ou méthoxy,
A représente RO-CO(CH₂)ₚ, R représentant H,
n représente 0 ou 1,
p représente 0 et
B est choisi parmi les structures suivantes, 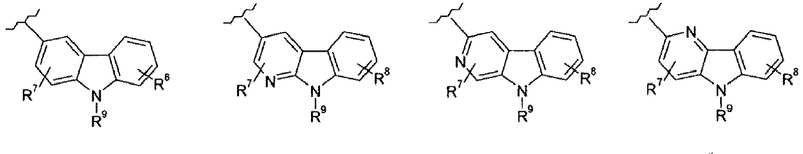 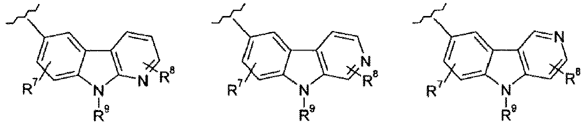
R⁶ représente H, F, CH₃ ou CH₃O,
R⁷, R⁸ représentent, à chaque fois indépendamment l'un de l'autre, H, F, Cl, méthyle ou méthoxy et
R⁹ représente C₁-C₃-alkyle, allyle, propargyle, C₃-C₄-cycloalkyl-(CH₂)ₙ ou méthoxyéthyle,
et leurs isomères, diastéréo-isomères, énantiomères, solvates et sels ou clathrates de cyclodextrine.

6. Composé selon la revendication 1, choisi dans un groupe qui contient les composés suivants :
1. 1-allyl-2-(9-éthyl-9H-carbazol-3-yl)-1H-benzimidazole-5-carboxylate de méthyle
2. acide 1-allyl-2-(9-éthyl-9H-carbazol-3-yl)-1H-benzimidazole-5-carboxylique
3. 2-(9-éthyl-9H-carbazol-3-yl)-1-(2-méthoxyéthyl)-1H-benzimidazole-5-carboxylate de méthyle
4. acide 2-(9-éthyl-9H-carbazol-3-yl)-1-(2-méthoxyéthyl)-1H-benzimidazole-5-carboxylique
5. 1-(cyclopropylméthyl)-2-(9-éthyl-9H-carbazol-3-yl)-1H-benzimidazole-5-carboxylate de méthyle
6. acide 1-(cyclopropylméthyl)-2-(9-éthyl-9H-carbazol-3-yl)-1H-benzimidazole-5-carboxylique
7. 4-chloro-1-(cyclopropylméthyl)-2-(9-éthyl-9H-carbazol-3-yl)-1H-benzimidazole-5-carboxylate de méthyle
8. acide 4-chloro-1-(cyclopropylméthyl)-2-(9-éthyl-9H-carbazol-3-yl)-1H-benzimidazole-5-carboxylique
9. acide 1-(cyclopropylméthyl)-2-(9-éthyl-9H-carbazol-3-yl)-4-méthyl-1H-benzimidazole-5-carboxylique
10. acide 2-(9-éthyl-7-fluoro-9H-carbazol-3-yl)-1-(2-méthoxyéthyl)-1H-benzimidazole-5-carboxylique
11. acide 2-(9-éthyl-5-fluoro-9H-carbazol-3-yl)-1-(2-méthoxyéthyl)-1H-benzimidazole-5-carboxylique
12. acide 2-(9-éthyl-8-fluoro-9H-carbazol-3-yl)-1-(2-méthoxyéthyl)-1H-benzimidazole-5-carboxylique
13. acide 1-(cyclopropylméthyl)-2-[9-(2-méthoxyéthyl)-9H-carbazol-3-yl]-1H-benzimidazole-5-carboxylique
14. acide 1-(cyclopropylméthyl)-2-(9-éthyl-9H-pyrido[2,3-b]indol-3-yl)-1H-benzimidazole-5-carboxylique
15. acide 2-(9-éthyl-6-méthoxy-9H-carbazol-3-yl)-1-(2-méthoxyéthyl)-1H-benzimidazole-5-carboxylique
16. acide 2-(9-allyl-9H-carbazol-3-yl)-1-(cyclopropylméthyl)-1H-benzimidazole-5-carboxylique
17. acide 1-(cyclopropylméthyl)-2-(9-méthyl-9H-carbazol-3-yl)-1H-benzimidazole-5-carboxylique
18. acide 1-(cyclopropylméthyl)-2-[9-(cyclopropylméthyl)-9H-carbazol-3-yl]-1H-benzimidazole-5-carboxylique
19. acide 2-[9-(cyclopropylméthyl)-9H-carbazol-3-yl]-1-(2-méthoxyéthyl)-1H-benzimidazole-5-carboxylique
20. 2-(9-éthyl-9H-carbazol-3-yl)-1-[2-(pyrrolidin-1-yl)éthyl]-1H-benzimidazole-5-carboxylate d'éthyle
21. acide 2-(9-éthyl-9H-carbazol-3-yl)-1-[2-(pyrrolidin-1-yl)éthyl]-1H-benzimidazole-5-carboxylique
22. acide 2-(5-éthyl-5H-pyrido[3,2-b]indol-2-yl)-1-(2-méthoxyéthyl)-1H-benzimidazole-5-carboxylique
23. acide 1-(cyclopropylméthyl)-2-(9-éthyl-6-méthoxy-9H-carbazol-3-yl)-1H-benzimidazole-5-carboxylique
24. acide 2-(9-éthyl-9H-carbazol-3-yl)-1-[2-(morpholin-4-yl)éthyl]-1H-benzimidazole-5-carboxylique
25. 1-[2-(diméthylamino)éthyl]-2-(9-éthyl-9H-carbazol-3-yl)-1H-benzimidazole-5-carboxylate d'éthyle
26. acide 1-[2-(diméthylamino)éthyl]-2-(9-éthyl-9H-carbazol-3-yl)-1H-benzimidazole-5-carboxylique
27. acide 2-(9-éthyl-9H-carbazol-3-yl)-1-(2-hydroxyéthyl)-1H-benzimidazole-5-carboxylique
28. 2-(9-éthyl-9H-carbazol-3-yl)-1-isopropyl-1H-benzimidazole-5-carboxylate d'éthyle
29. acide 2-(9-éthyl-9H-carbazol-3-yl)-1-isopropyl-1H-benzimidazole-5-carboxylique
30. acide 2-(9-éthyl-9H-carbazol-3-yl)-1-méthyl-1H-benzimidazole-5-carboxylique
31. 1-éthyl-2-(9-éthyl-9H-carbazol-3-yl)-1H-benzimidazole-5-carboxylate de méthyle
32. acide 2-(9-allyl-9H-carbazol-3-yl)-1-(2-méthoxyéthyl)-1H-benzimidazole-5-carboxylique
33. 1-(2-méthoxyéthyl)-2-[9-(2-méthoxyéthyl)-9H-carbazol-3-yl]-1H-benzimidazole-5-carboxylate d'éthyle
34. acide 1-(2-méthoxyéthyl)-2-[9-(2-méthoxyéthyl)-9H-carbazol-3-yl]-1H-benzimidazole-5-carboxylique
35. 2-(9-éthyl-9H-carbazol-3-yl)-1-(3-méthoxypropyl)-1H-benzimidazole-5-carboxylate d'éthyle
36. acide 2-(9-éthyl-9H-carbazol-3-yl)-1-(3-méthoxypropyl)-1H-benzimidazole-5-carboxylique
37. acide 1-(cyclopropylméthyl)-2-(9-éthyl-9H-carbazol-3-yl)-6-méthoxy-1H-benzimidazole-5-carboxylique
38. acide 2-(9-éthyl-9H-carbazol-3-yl)-6-méthoxy-1-(2-méthoxyéthyl)-1H-benzimidazole-5-carboxylique
39. acide 1-(cyclopropylméthyl)-2-(9-éthyl-9H-carbazol-3-yl)-4-méthoxy-1H-benzimidazole-5-carboxylique
40. acide 2-(9-éthyl-9H-carbazol-3-yl)-1-(2-méthoxyéthyl)-4-méthyl-1H-benzimidazole-5-carboxylique
41. acide 1-(cyclopropylméthyl)-2-(9-éthyl-9H-pyrido[2,3-b]indol-3-yl)-4-méthyl-1H-benzimidazole-5-carboxylique
42. acide 1-(cyclopropylméthyl)-2-(9-éthyl-9H-bêta-carbolin-6-yl)-1H-benzimidazole-5-carboxylique
43. acide 1-(cyclopropylméthyl)-2-(5-éthyl-5H-pyrido[4,3-b]indol-8-yl)-1H-benzimidazole-5-carboxylique
44. acide 1-(cyclopropylméthyl)-2-(9-éthyl-9H-carbazol-3-yl)-6-méthyl-1H-benzimidazole-5-carboxylique
45. acide 2-(9-éthyl-9H-carbazol-3-yl)-1-(2-méthoxyéthyl)-6-méthyl-1H-benzimidazole-5-carboxylique
46. acide 2-(9-éthyl-9H-carbazol-3-yl)-6-fluoro-1-(2-méthoxyéthyl)-1H-benzimidazole-5-carboxylique
47. acide 1-(cyclopropylméthyl)-2-(9-éthyl-9H-carbazol-3-yl)-6-fluoro-1H-benzimidazole-5-carboxylique
48. acide 2-(9-éthyl-9H-carbazol-3-yl)-4-fluoro-1-(2-méthoxyéthyl)-1H-benzimidazole-5-carboxylique
49. acide 1-(cyclopropylméthyl)-2-(9-éthyl-9H-carbazol-3-yl)-4-fluoro-1H-benzimidazole-5-carboxylique
50. acide 1-(cyclopropylméthyl)-2-(9-éthyl-9H-pyrido[2,3-b]indol-6-yl)-1H-benzimidazole-5-carboxylique
51. acide 1-(2-cyclopropyléthyl)-2-(9-éthyl-9H-carbazol-3-yl)-1H-benzimidazole-5-carboxylique
52. acide 1-(2-méthoxyéthyl)-2-(9-propyl-9H-carbazol-3-yl)-1H-benzimidazole-5-carboxylique
53. acide 1-(2-méthoxyéthyl)-2-[9-(prop-2-yn-1-yl)-9H-carbazol-3-yl]-1H-benzimidazole-5-carboxylique
54. acide 1-(cyclopropylméthyl)-2-(9-propyl-9H-carbazol-3-yl)-1H-benzimidazole-5-carboxylique
55. acide 1-[(2,2-diméthylcyclopropyl)méthyl]-2-(9-éthyl-9H-carbazol-3-yl)-1H-benzimidazole-5-carboxylique
56. 2-(9-éthyl-9H-carbazol-3-yl)-1-(oxétan-3-ylméthyl)-1H-benzimidazole-5-carboxylate d'éthyle
57. acide 2-(9-éthyl-9H-carbazol-3-yl)-1-(oxétan-3-ylméthyl)-1H-benzimidazole-5-carboxylique
58. acide 2-[9-(cyclobutylméthyl)-9H-carbazol-3-yl]-1-(2-méthoxyéthyl)-1H-benzimidazole-5-carboxylique
59. acide 2-[9-(cyclobutylméthyl)-9H-carbazol-3-yl]-1-(cyclopropylméthyl)-1H-benzimidazole-5-carboxylique
60. acide 2-(9-éthyl-9H-carbazol-3-yl)-1-[2-(trifluorométhoxy)éthyl]-1H-benzimidazole-5-carboxylique
61. acide 1-(cyclopropylméthyl)-2-(9-éthyl-1-méthyl-9H-bêta-carbolin-3-yl)-1H-benzimidazole-5-carboxylique
62. acide 2-(9-éthyl-9H-carbazol-3-yl)-1-(oxétan-2-ylméthyl)-1H-benzimidazole-5-carboxylique
63. acide 2-(9-éthyl-9H-carbazol-3-yl)-1-(tétrahydrofurann-2-ylméthyl)-1H-benzimidazole-5-carboxylique
64. acide 2-(9-éthyl-9H-carbazol-3-yl)-1-[(2R)-2-hydroxy-3-méthoxypropyl]-1H-benzimidazole-5-carboxylique
65. acide 2-(9-éthyl-9H-carbazol-3-yl)-1-[(2S)-2-hydroxy-3-méthoxypropyl]-1H-benzimidazole-5-carboxylique
66. acide 2-(9-éthyl-9H-carbazol-3-yl)-1-[2-(méthylsulfonyl)éthyl]-1H-benzimidazole-5-carboxylique
67. acide 1-(2-cyclopropyl-2-hydroxyéthyl)-2-(9-éthyl-9H-carbazol-3-yl)-1H-benzimidazole-5-carboxylique
68. acide 1-[(2S)-2,3-dihydroxypropyl]-2-(9-éthyl-9H-carbazol-3-yl)-1H-benzimidazole-5-carboxylique
69. acide 1-(1,4-dioxan-2-ylméthyl)-2-(9-éthyl-9H-carbazol-3-yl)-1H-benzimidazole-5-carboxylique
70. acide 1-(1,4-dioxan-2-ylméthyl)-2-(9-éthyl-9H-carbazol-3-yl)-1H-benzimidazole-5-carboxylique
71. acide 2-(9-éthyl-6-méthyl-9H-carbazol-3-yl)-1-(2-méthoxyéthyl)-4-méthyl-1H-benzimidazole-5-carboxylique
72. acide 2-(6-chloro-9-éthyl-9H-carbazol-3-yl)-1-(2-méthoxyéthyl)-4-méthyl-1H-benzimidazole-5-carboxylique
73. acide 2-(8-chloro-9-éthyl-9H-carbazol-3-yl)-1-(2-méthoxyéthyl)-4-méthyl-1H-benzimidazole-5-carboxylique.

7. Composé de formule (I), tel que défini dans l'une quelconque des revendications 1 à 6, pour le traitement et/ou la prophylaxie de maladies.

8. Composé de formule (I), tel que défini dans l'une quelconque des revendications 1 à 6, pour une utilisation dans un procédé pour le traitement et/ou la prophylaxie de l'endométriose, de léiomyomes utérins, de troubles hémorragiques utérins, les troubles hémorragiques pouvant être des saignements importants et longs, des saignements temporairement irréguliers ainsi que des douleurs, de la dysménorrhée, du cancer, les maladies cancéreuses pouvant être le cancer des poumons, de l'intestin, du sein, de la peau, de la prostate, de l'oesophage et la leucémie, de l'artériosclérose et de maladies rénales polykystiques.

9. Utilisation d'un composé selon la revendication 1, 2, 3, 4, 5 ou 6 pour la préparation d'un médicament destiné au traitement et/ou à la prophylaxie de maladies.

10. Utilisation d'un composé, tel que défini dans l'une quelconque des revendications 1 à 6, pour la préparation d'un médicament pour le traitement et/ou la prophylaxie de l'endométriose, de léiomyomes utérins, de troubles hémorragiques utérins, les troubles hémorragiques pouvant être des saignements importants et longs, des saignements temporairement irréguliers ainsi que des douleurs, de la dysménorrhée, du cancer, les maladies cancéreuses pouvant être le cancer des poumons, de l'intestin, du sein, de la peau, de la prostate, de l'oesophage et la leucémie, de l'artériosclérose et de maladies rénales polykystiques.

11. Médicament contenant un composé, tel que défini dans l'une quelconque des revendications 1 à 6, en combinaison avec une ou plusieurs autres substances actives, en particulier avec des modulateurs sélectifs du récepteur des estrogènes (SERM), des antagonistes du récepteur des estrogènes (ER), des inhibiteurs d'aromatases, des inhibiteurs de 17ß-HSD1, des inhibiteurs de la sulfatase stéroïde (STS), des agonistes et des antagonistes de GnRH, des antagonistes du récepteur de la kisspeptine (KISSR), des modulateurs sélectifs du récepteur des androgènes (SARM), des androgènes, des inhibiteurs de la 5α-réductase, des modulateurs sélectifs du récepteur des progestérones (SPRM), des gestagènes, des antigestagènes, des contraceptifs oraux, des inhibiteurs des protéine kinases activées par des agents mitogènes (MAP) ainsi que des inhibiteurs des MAP kinases kinases (Mkk3/6, Mek1/2, Erk1/2), des inhibiteurs des protéine kinases B (PKBα/ß/γ ; Akt1/2/3), des inhibiteurs des phosphoinositide-3-kinases (PI3K), des inhibiteurs des kinases cycline-dépendantes (CDK1/2), des inhibiteurs de la voie de signalisation induite par l'hypoxie (inhibiteurs HIF1alpha, activateurs des prolylhydroxylases), des inhibiteurs de l'histone désacétylase (HDAC), des antagonistes du récepteur de la prostaglandine F (FP) (PTGFR), des antagonistes du récepteur de la neurokinine1, le paracétamol, des inhibiteurs sélectifs de COX2 et/ou des inhibiteurs non sélectifs de COX1/COX2.

12. Médicament contenant un composé de formule (I), tel que défini dans l'une quelconque des revendications 1 à 6, en combinaison avec un adjuvant inerte, non toxique, pharmaceutiquement approprié.

13. Médicament selon la revendication 11 ou 12 pour le traitement et/ou la prophylaxie de l'endométriose, de léiomyomes utérins, de troubles hémorragiques utérins, les troubles hémorragiques pouvant être des saignements importants et longs, des saignements temporairement irréguliers ainsi que des douleurs, de la dysménorrhée, du cancer, les maladies cancéreuses pouvant être le cancer des poumons, de l'intestin, du sein, de la peau, de la prostate, de l'oesophage et la leucémie, de l'artériosclérose et de maladies rénales polykystiques.
